(19) Europäisches Patentamt
European Patent Office
Office européen des brevets

(11) EP 4 273 143 B1

(12) EUROPEAN PATENT SPECIFICATION

(45) Date of publication and mention of the grant of the patent:
**21.05.2025 Bulletin 2025/21**

(21) Application number: **21914730.3**

(22) Date of filing: **31.12.2021**

(51) International Patent Classification (IPC):
***C07D 403/14*** (2006.01) ***C07D 403/06*** (2006.01)
***C07D 401/06*** (2006.01) ***C07D 409/14*** (2006.01)
***C07D 417/14*** (2006.01) ***C07D 405/14*** (2006.01)
***A61K 31/496*** (2006.01) ***A61K 31/5377*** (2006.01)
***A61P 35/00*** (2006.01)

(52) Cooperative Patent Classification (CPC):
**C07F 5/025; A61P 35/00; C07C 309/04; C07D 401/14; C07D 403/06; C07D 413/14**

(86) International application number:
**PCT/CN2021/143913**

(87) International publication number:
**WO 2022/144002 (07.07.2022 Gazette 2022/27)**

# (54) DERIVATIVE OF 2,5-DIKETOPIPERAZINE COMPOUND, AND PREPARATION METHOD THEREFOR, PHARMACEUTICAL COMPOSITION THEREOF AND USE THEREOF

DERIVAT EINER 2,5-DIKETOPIPERAZINVERBINDUNG UND HERSTELLUNGSVERFAHREN DAFÜR, PHARMAZEUTISCHE ZUSAMMENSETZUNG DARAUS UND VERWENDUNG DAVON

DÉRIVÉ D’UN COMPOSÉ DE 2,5-DICÉTOPIPÉRAZINE, SON PROCÉDÉ DE PRÉPARATION, COMPOSITION PHARMACEUTIQUE DE CELUI-CI ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.12.2020 CN 202011624889**

(43) Date of publication of application:
**08.11.2023 Bulletin 2023/45**

(73) Proprietor: **Dalian WZ Probiotics AD Health Co., Ltd**
**Dalian, Liaoning 116602 (CN)**

(72) Inventors:
- **LI, Wenbao William**
**Shenzhen, Guangdong 518000 (CN)**
- **DING, Zhongpeng**
**Shenzhen, Guangdong 518000 (CN)**
- **LI, Feifei**
**Shenzhen, Guangdong 518000 (CN)**
- **XIE, Lianghui**
**Shenzhen, Guangdong 518000 (CN)**
- **XU, Yun**
**Dalian, Liaoning 116602 (CN)**
- **WANG, Xinwen**
**Dalian, Liaoning 116602 (CN)**

(74) Representative: **Greaves Brewster LLP**
**Copa House**
**Station Road**
**Cheddar, Somerset BS27 3AH (GB)**

(56) References cited:
**WO-A1-2012/035436 CN-A- 106 279 039**
**CN-A- 107 286 137 CN-A- 110 240 592**
**CN-A- 110 835 335**

- **DING, ZHONGPENG ET AL. .: "Structure-based design and synthesis of novel furan-diketopiperazine-type derivatives as potent microtubule inhibitors for treating cancer.", BIOORGANIC & MEDICINAL CHEMISTRY., vol. 28, no. 10, 13 March 2020 (2020-03-13), XP086135480, DOI: 10.1016/j.bmc.2020.115435**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present application claims the priority of Chinese patent application 2020116248892 filed on December 31, 2020.

TECHNICAL FIELD

The present disclosure belongs to the technical field of medicinal chemistry, and specifically relates to a derivative of a 2,5-diketopiperazine compound, a preparation method therefor, a pharmaceutical composition thereof, and a use thereof.

BACKGROUND

Plinabulin (NPI-2358) belongs to derivatives of 2,5-diketopiperazine compound, which is a derivative obtained by structural modification of a metabolite (low molecular cyclic dipeptide Phenylahistin) produced by marine fungus Aspergillus sp., and is a tubulin binding agent. Plinabulin can selectively act near the colchicine-binding site in endothelial tubulin, inhibit tubulin polymerization, and block microtubule assembly, thereby destroying the endothelial cytoskeleton and inhibiting tumor blood flow. At the same time, Plinabulin also inhibits the migration of endothelial cells and makes the tumor vasculature dysfunctional. Experiments have shown that Plinabulin acts on cells to arrest cells in early mitosis and induce cell death. Prior art (WO2012035436) has disclosed plinabulin prodrug analogs and their use for treating various disease conditions including cancer. Recent studies have found that Plinabulin is a differentiation immune and stem cell regulator, as well as a guanine nucleotide exchange factor (GEF-H1) activator, which can target and change the tumor microenvironment and destroy tumor vasculature through multiple mechanisms of action; Plinabulin acts as a potent antigen-presenting cell (APC) inducer (by activating dendritic cell maturation), and its long-lasting anticancer effect is associated with T cell activation (Cell Reports 2019, 28:13, 3367-3386).

The candidate drug is currently being developed by BeyondSpring Pharmaceuticals and has completed clinical phase III trials in China, the United States and other countries. On the one hand, it is used in combination with docetaxel for the treatment of non-small cell lung cancer, and on the other hand, it is used for the prevention of chemotherapy-induced neutropenia (CIN) in non-myeloid malignancies.

Plinabulin has a chemical structural formula as follows:

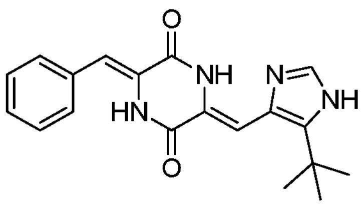

Plinabulin has a molecular formula of $C_{19}H_{20}N_4O_2$, a molecular weight of 336.39, and a CAS number of 714272-27-2. It has good stability, but poor water solubility, making it difficult to formulate into a drug.

CONTENT OF THE PRESENT INVENTION

The technical problem to be solved by the present disclosure is the defect of poor water solubility of Plinabulin, which is unfavorable for drug formation, and the present disclosure provides a derivative of a 2,5-diketopiperazine compound, a preparation method therefor, a pharmaceutical composition thereof, and a use thereof. The compounds of the present disclosure have novel structures, good activity, and water solubility.

The present disclosure solves the above technical problem through the following solutions.

The present disclosure provides a compound of formula (I), a stereoisomer thereof, a tautomer thereof, a pharmaceutically acceptable salt thereof, or a solvate of any one of the foregoing (referring to the foregoing compound of formula (I), the stereoisomer thereof, the tautomer thereof, or the pharmaceutically acceptable salt thereof):

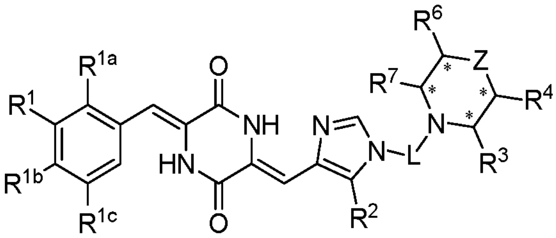

(I)

a carbon atom with "*" is a carbon atom or a chiral carbon atom, and when the carbon atom with "*" is the chiral carbon atom, it is in an S configuration and/or an R configuration;

$R^1$ is hydrogen, deuterium, halogen, $C_1$-$C_8$ alkyl, $C_1$-$C_8$ alkyl substituted by one or more than one halogen, $C_1$-$C_8$ alkoxy, $C_1$-$C_8$ alkoxy substituted by one or more than one halogen, benzoyl, benzoyl substituted by one or more than one halogen, phenoxy, or phenoxy substituted by one or more than one halogen;

$R^{1a}$, $R^{1b}$, and $R^{1c}$ are independently hydrogen, deuterium, halogen, or $C_1$-$C_8$ alkyl;

or, $R^1$, $R^{1a}$ together with the carbon atoms to which they are attached form a $C_6$-$C_{10}$ aryl or a 3- to 10-membered heteroaryl with 1-5 heteroatoms selected from one or more than one of N, O, and S;

or, $R^1$, $R^{1b}$ together with the carbon atoms to which they are attached form a $C_6$-$C_{10}$ aryl or a 3- to 10-membered heteroaryl with 1-5 heteroatoms selected from one or more than one of N, O, and S;

or, $R^{1b}$, $R^{1c}$ together with the carbon atoms to which they are attached form a $C_6$-$C_{10}$ aryl or a 3- to 10-membered heteroaryl with 1-5 heteroatoms selected from one or more than one of N, O, and S;

$R^2$ is $C_1$-$C_8$ alkyl or $C_3$-$C_{10}$ cycloalkyl;

L is $C_1$-$C_8$ heteroalkylene with 1-4 heteroatoms selected from one or more than one of N, O, S, and Se or $C_1$-$C_8$ alkylene;

$R^3$, $R^4$, $R^6$, and $R^7$ are independently hydrogen, deuterium, $C_1$-$C_8$ alkyl, $C_1$-$C_8$ alkyl substituted by one or more than one $R^{3-1}$, $C_1$-$C_8$ alkoxy, $C_3$-$C_{10}$ cycloalkyl, $C_6$-$C_{10}$ aryl, or 3- to 10-membered heterocycloalkyl with 1-5 heteroatoms selected from one or more than one of N, O, and S;

$R^{3-1}$ is independently O-$R^{3-1-1}$, $R^{3-1-1}$ is $C_1$-$C_8$ alkyl or $C_1$-$C_8$ alkyl substituted by one or more than one $R^{3-1-2}$;

$R^{3-1-2}$ is independently $C_6$-$C_{10}$ aryl;

Z is O or N($R^5$);

$R^5$ is hydrogen, deuterium, $C_1$-$C_8$ alkyl, $C_6$-$C_{10}$ aryl substituted by one or more than one $R^{5-1}$, benzyl, benzyl substituted by one or more than one $R^{5-2}$, -C(=O)-$R^{5-3}$, or -$S(=O)_2$-$R^{5-4}$;

$R^{5-1}$ is independently halogen;

$R^{5-2}$ is independently

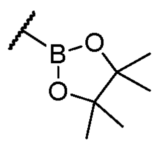

;

$R^{5-3}$ is hydrogen, $C_1$-$C_8$ alkoxy, benzyloxy, benzyloxy substituted by one or more than one $R^{5-2}$, $C_1$-$C_8$ alkyl, $C_3$-$C_{10}$ cycloalkyl, $C_2$-$C_8$ alkenyl, $C_6$-$C_{10}$ aryl, or $NR^{5-3-1}R^{5-3-2}$; $R^{5-3-1}$ and $R^{5-3-2}$ are independently hydrogen, $C_1$-$C_8$ alkyl, or $C_3$-$C_{10}$ cycloalkyl;

$R^{5-4}$ is $C_1$-$C_8$ alkyl or $C_6$-$C_{10}$ aryl.

In a preferred embodiment of the present disclosure, some groups in the compound of formula (I), the stereoisomer thereof, the tautomer thereof, the pharmaceutically acceptable salt thereof, or the solvate of any one of the foregoing are defined as follows, and the groups not mentioned are as described in any one embodiment of the present disclosure (referred to as "in an embodiment of the present disclosure" for short),

when $R^1$ is halogen, the halogen is fluorine, chlorine, bromine, or iodine, such as fluorine.

In an embodiment of the present disclosure, when $R^1$ is $C_1$-$C_8$ alkyl, the $C_1$-$C_8$ alkyl is $C_1$-$C_4$ alkyl, such as methyl.

In an embodiment of the present disclosure, when $R^1$ is $C_1$-$C_8$ alkyl substituted by one or more than one halogen, the $C_1$-$C_8$ alkyl substituted by one or more than one halogen is $C_1$-$C_4$ alkyl substituted by one or more than one halogen, such as trifluoromethyl.

In an embodiment of the present disclosure, when $R^1$ is $C_1$-$C_8$ alkoxy, the $C_1$-$C_8$ alkoxy is $C_1$-$C_4$ alkoxy, such as methoxy.

In an embodiment of the present disclosure, when $R^1$ is $C_1$-$C_8$ alkoxy substituted by one or more than one halogen, the $C_1$-$C_8$ alkoxy substituted by one or more than one halogen is $C_1$-$C_4$ alkoxy substituted by one or more than one halogen, such as trifluoromethoxy.

In an embodiment of the present disclosure, when $R^1$ is benzoyl substituted by one or more than one halogen, the halogen is fluorine, chlorine, bromine, or iodine, such as fluorine. Preferably, the benzoyl substituted by one or more than one halogen is

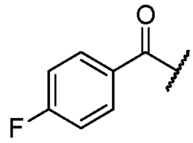

.

In an embodiment of the present disclosure, when $R^1$ is phenoxy substituted by one or more than one halogen, the halogen is fluorine, chlorine, bromine, or iodine, such as fluorine. Preferably, the phenoxy substituted by one or more than one halogen is

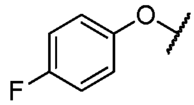

.

In an embodiment of the present disclosure, when $R^{1a}$, $R^{1b}$, and $R^{1c}$ are independently halogen, the halogen is fluorine, chlorine, bromine, or iodine, such as fluorine.

In an embodiment of the present disclosure, when $R^{1a}$, $R^{1b}$, and $R^{1c}$ are independently $C_1$-$C_8$ alkyl, the $C_1$-$C_8$ alkyl is $C_1$-$C_4$ alkyl, such as methyl.

In an embodiment of the present disclosure, when $R^1$, $R^{1a}$ together with the carbon atoms to which they are attached form the $C_6$-$C_{10}$ aryl, the $C_6$-$C_{10}$ aryl is phenyl.

In an embodiment of the present disclosure, when $R^1$, $R^{1a}$ together with the carbon atoms to which they are attached form the 3- to 10-membered heteroaryl with 1-5 heteroatoms selected from one or more than one of N, O, and S, the 3- to 10-membered heteroaryl with 1-5 heteroatoms selected from one or more than one of N, O, and S is 4- to 8-membered heteroaryl with 1-2 heteroatoms selected from one or more than one of N, O, and S; such as pyridyl; and further such as

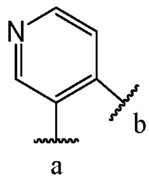

,

with an a-terminal attached to the carbon atom to which $R^1$ is attached and a b-terminal attached to the carbon atom to which $R^{1a}$ is attached.

In an embodiment of the present disclosure, when $R^2$ is $C_1$-$C_8$ alkyl, the $C_1$-$C_8$ alkyl is $C_1$-$C_4$ alkyl, such as methyl, ethyl, isopropyl, or tert-butyl.

In an embodiment of the present disclosure, when $R^2$ is $C_3$-$C_{10}$ cycloalkyl, the $C_3$-$C_{10}$ cycloalkyl is $C_3$-$C_6$ cycloalkyl, such as cyclopropyl.

In an embodiment of the present disclosure, when L is $C_1$-$C_8$ alkylene, the $C_1$-$C_8$ alkylene is $C_3$-$C_8$ alkylene, such as

, 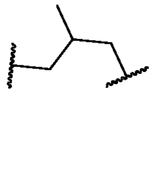, 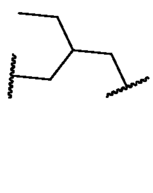, 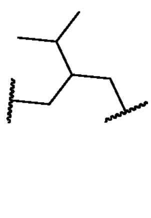,

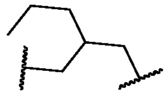, or 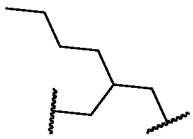,

and further such as

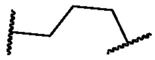.

In an embodiment of the present disclosure, when $R^3$, $R^4$, $R^6$, and $R^7$ are independently $C_1$-$C_8$ alkyl, the $C_1$-$C_8$ alkyl is $C_1$-$C_4$ alkyl, such as methyl or ethyl, and further such as methyl.

In an embodiment of the present disclosure, when $R^3$, $R^4$, $R^6$, and $R^7$ are independently $C_1$-$C_8$ alkyl substituted by one or more than one $R^{3-1}$, the $C_1$-$C_8$ alkyl is $C_1$-$C_4$ alkyl, such as methyl.

In an embodiment of the present disclosure, when $R^{3-1-1}$ is $C_1$-$C_8$ alkyl, the $C_1$-$C_8$ alkyl is $C_1$-$C_4$ alkyl, such as methyl, ethyl, n-propyl, or n-butyl.

In an embodiment of the present disclosure, when $R^{3-1-1}$ is $C_1$-$C_8$ alkyl substituted by one or more than one $R^{3-1-2}$, the $C_1$-$C_8$ alkyl is $C_1$-$C_4$ alkyl, such as methyl.

In an embodiment of the present disclosure, when $R^{3-1-2}$ is independently $C_6$-$C_{10}$ aryl, the $C_6$-$C_{10}$ aryl is phenyl.

In an embodiment of the present disclosure, when $R^5$ is $C_1$-$C_8$ alkyl, the $C_1$-$C_8$ alkyl is $C_1$-$C_4$ alkyl, such as methyl, ethyl, n-propyl, isopropyl, n-butyl, or 2-methylpropyl, and further such as methyl.

In an embodiment of the present disclosure, when $R^5$ is $C_6$-$C_{10}$ aryl substituted by one or more than one $R^{5-1}$, the $C_6$-$C_{10}$ aryl is phenyl or naphthyl, such as phenyl.

In an embodiment of the present disclosure, when $R^{5-1}$ is halogen, the halogen is fluorine, chlorine, bromine, or iodine, such as fluorine.

In an embodiment of the present disclosure, when $R^{5-3}$ is $C_1$-$C_8$ alkoxy, the $C_1$-$C_8$ alkoxy is $C_1$-$C_4$ alkoxy, such as tert-butoxy.

In an embodiment of the present disclosure, when $R^{5-3}$ is $C_1$-$C_8$ alkyl, the $C_1$-$C_8$ alkyl is methyl, ethyl, n-propyl, isopropyl, tert-butyl, or $-CH(C_2H_5)CH_2CH_3$. Preferably, the $C_1$-$C_8$ alkyl is $C_1$-$C_4$ alkyl.

In an embodiment of the present disclosure, when $R^{5-3}$ is $C_3$-$C_{10}$ cycloalkyl, the $C_3$-$C_{10}$ cycloalkyl is cyclopropyl, cyclobutyl, cyclopentyl, cyclohexyl, or adamantyl. Preferably, the $C_3$-$C_{10}$ cycloalkyl is $C_3$-$C_6$ cycloalkyl.

In an embodiment of the present disclosure, when $R^{5-3}$ is $C_2$-$C_8$ alkenyl, the $C_2$-$C_8$ alkenyl is $C_2$-$C_4$ alkenyl, such as

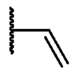, 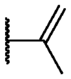, 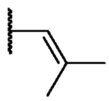, or 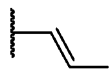,

and further such as

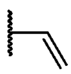 and 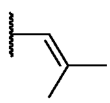.

In an embodiment of the present disclosure, when $R^{5-3}$ is $C_6$-$C_{10}$ aryl, the $C_6$-$C_{10}$ aryl is phenyl or naphthyl, such as phenyl.

In an embodiment of the present disclosure, when $R^{5-3-1}$ and $R^{5-3-2}$ are independently $C_1$-$C_8$ alkyl, the $C_1$-$C_8$ alkyl is $C_1$-$C_4$ alkyl, such as isopropyl or tert-butyl.

In an embodiment of the present disclosure, when $R^{5-3-1}$ and $R^{5-3-2}$ are independently $C_3$-$C_{10}$ cycloalkyl, the $C_3$-$C_{10}$ cycloalkyl is $C_3$-$C_6$ cycloalkyl, such as cyclopentyl or cyclohexyl.

In an embodiment of the present disclosure, when $R^{5-4}$ is $C_1$-$C_8$ alkyl, the $C_1$-$C_8$ alkyl is $C_1$-$C_4$ alkyl, such as methyl.

In an embodiment of the present disclosure, when $R^{5-4}$ is $C_6$-$C_{10}$ aryl, the $C_6$-$C_{10}$ aryl is phenyl or naphthyl, such as phenyl.

In an embodiment of the present disclosure, $R^1$ is hydrogen, halogen, $C_1$-$C_8$ alkyl, $C_1$-$C_8$ alkoxy, $C_1$-$C_8$ alkoxy substituted by one or more than one halogen, benzoyl, benzoyl substituted by one or more than one halogen, phenoxy, or

phenoxy substituted by one or more than one halogen.

In an embodiment of the present disclosure, $R^{1b}$ is hydrogen or halogen; such as hydrogen or fluorine.

In an embodiment of the present disclosure, $R^{1c}$ is hydrogen or halogen, such as hydrogen or fluorine.

In an embodiment of the present disclosure, $R^2$ is $C_1$-$C_8$ alkyl.

In an embodiment of the present disclosure, L is $C_1$-$C_8$ alkylene.

In an embodiment of the present disclosure, $R^3$, $R^4$, $R^6$, and $R^7$ are independently hydrogen or $C_1$-$C_8$ alkyl.

In an embodiment of the present disclosure, $R^5$ is benzyl, -C(=O)-$R^{5-3}$, or -S(=O)$_2$-$R^{5-4}$.

In an embodiment of the present disclosure, $R^{5-3}$ is hydrogen, $C_1$-$C_4$ alkyl, $C_3$-$C_6$ cycloalkyl, $C_2$-$C_8$ alkenyl, benzyloxy substituted by one or more than one $R^{5-2}$, $C_6$-$C_{10}$ aryl, or $NR^{5-3-1}R^{5-3-2}$.

In an embodiment of the present disclosure,

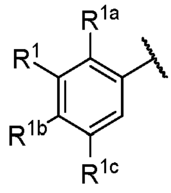 is 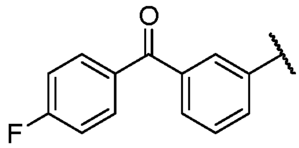,

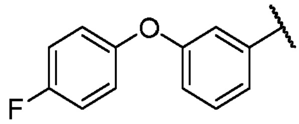, 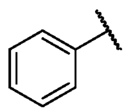, 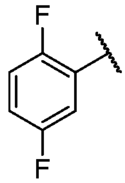, 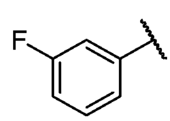, 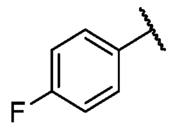, 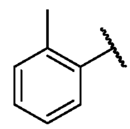,

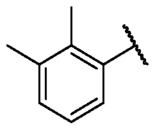, 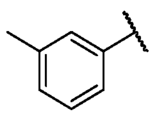, 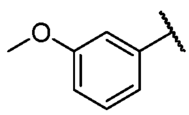, 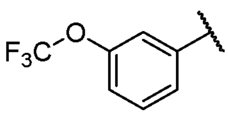, 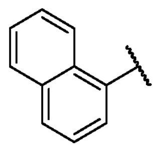, 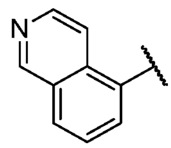,

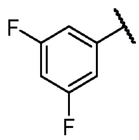, or 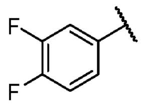.

In an embodiment of the present disclosure, $R^2$ is isopropyl or cyclopropyl.

In an embodiment of the present disclosure, L is

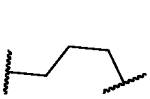 or 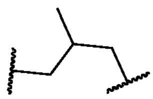.

In an embodiment of the present disclosure,

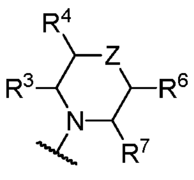 is 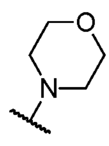, 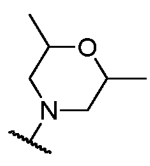,

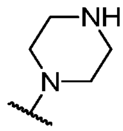, 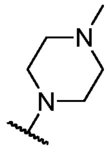, 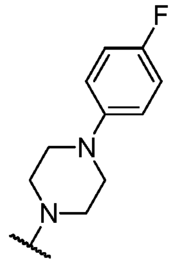, 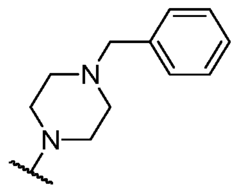, 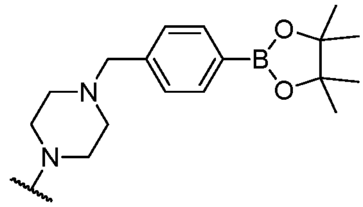,

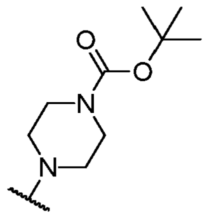, 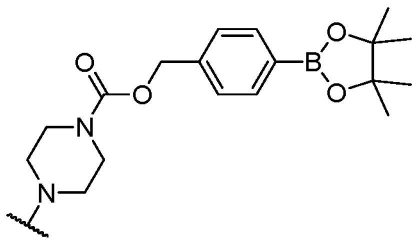, 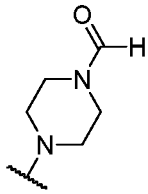, 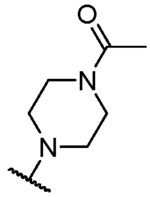, 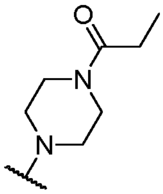,

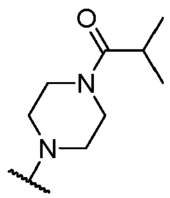, 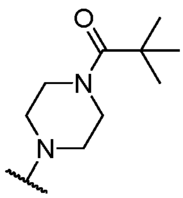, 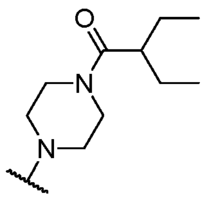, 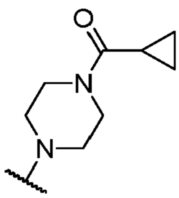, 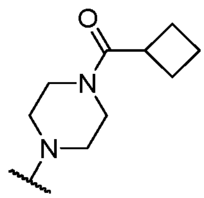,

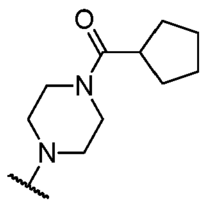, 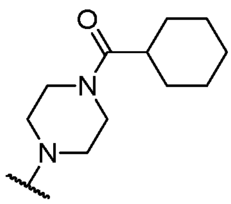, 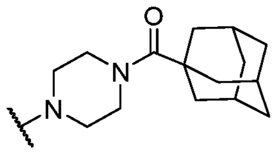, 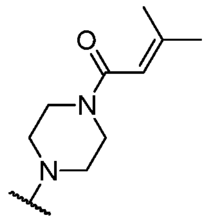,

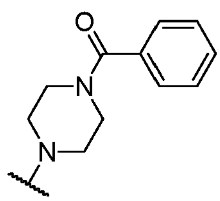, 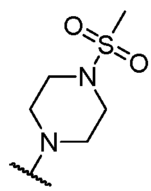, 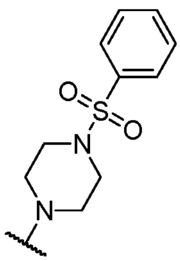, 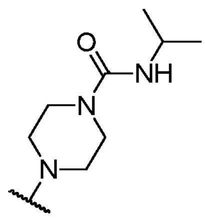, 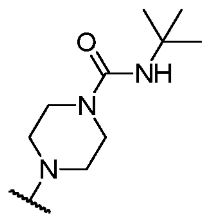,

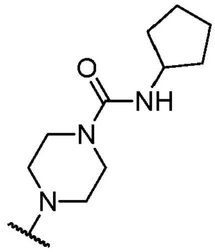, 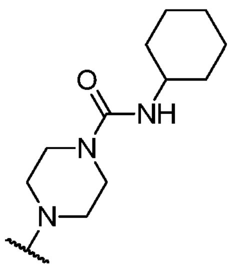, 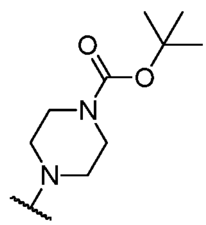, 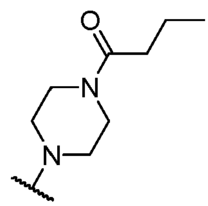, 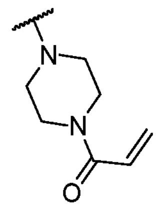,

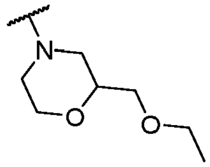,
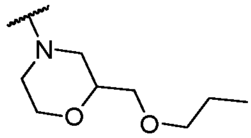,
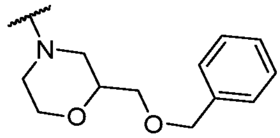,
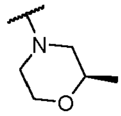, or
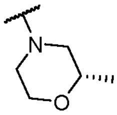.

Preferably, the compound of formula (I) is any one of the following compounds:

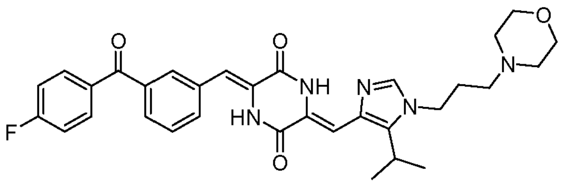,
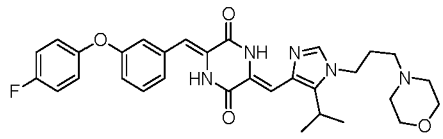,
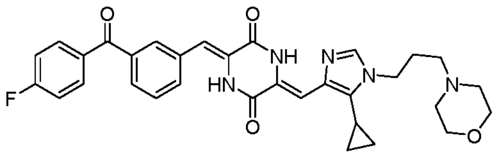,
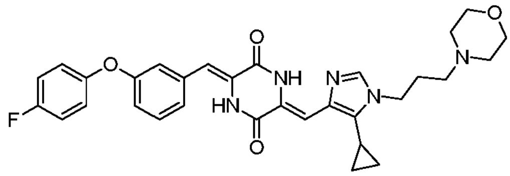,
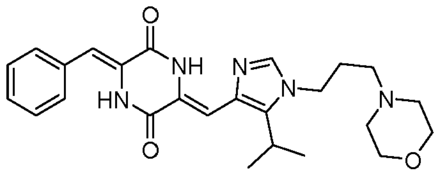,
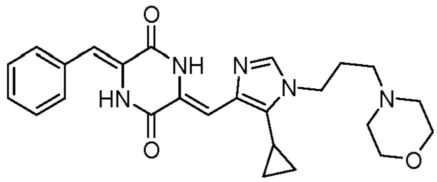,
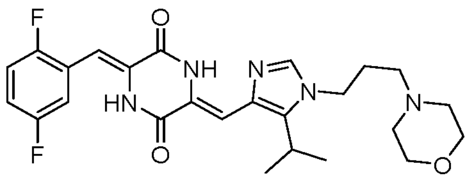,
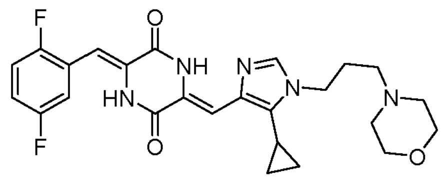,
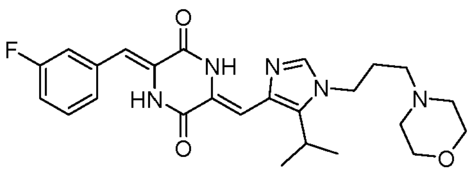,
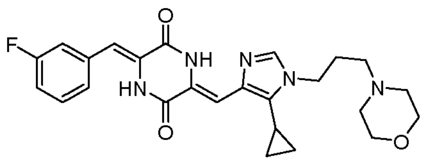,
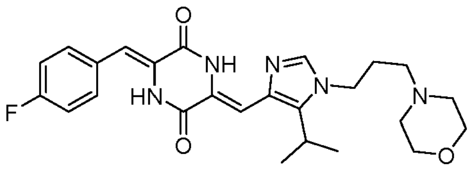,
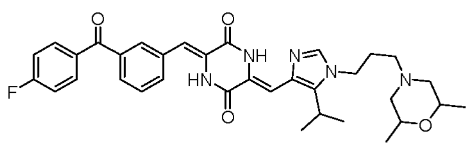,

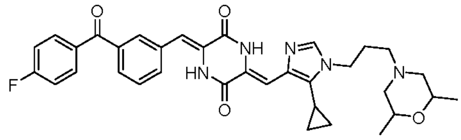,

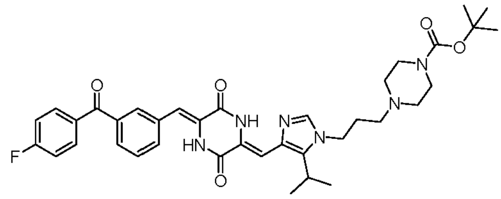,

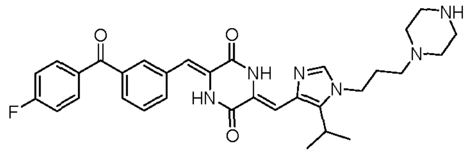,

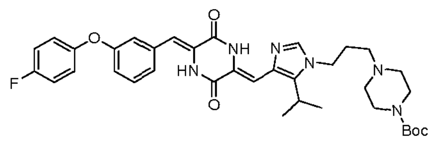,

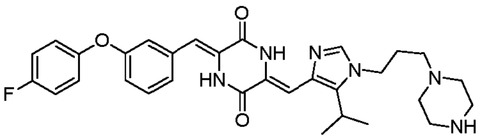,

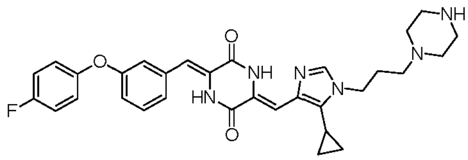,

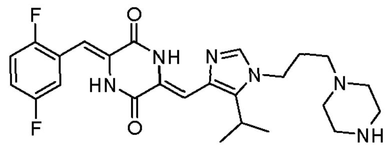,

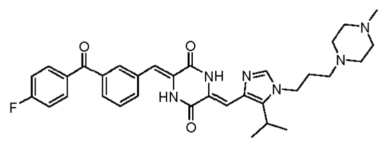,

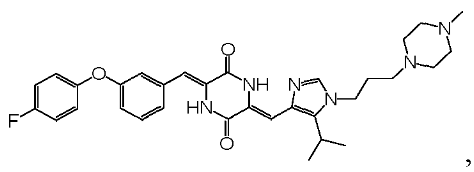,

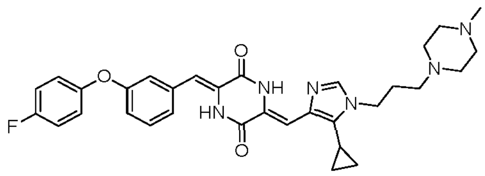,

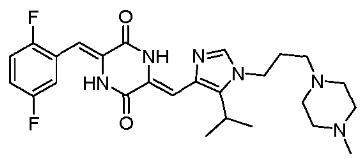,

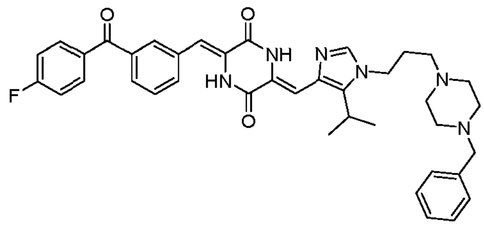,

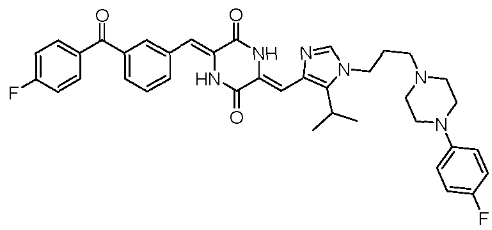,

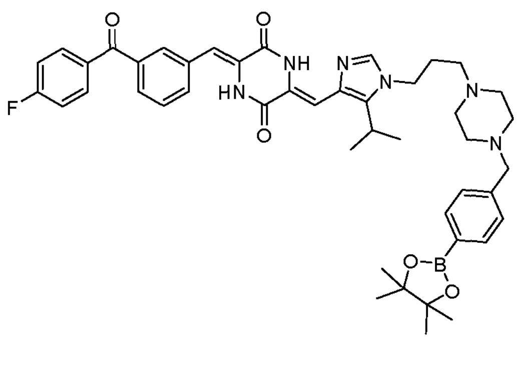,

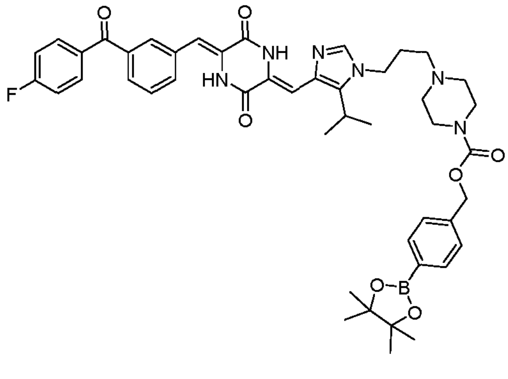,

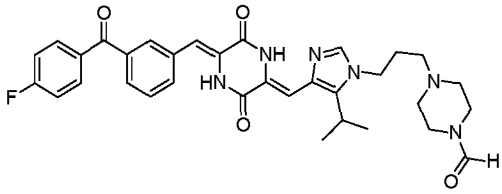,

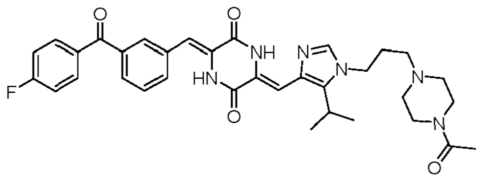,

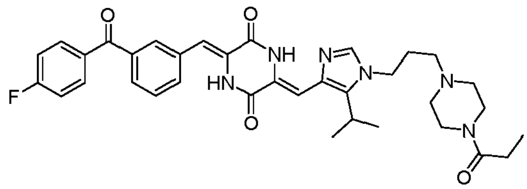,

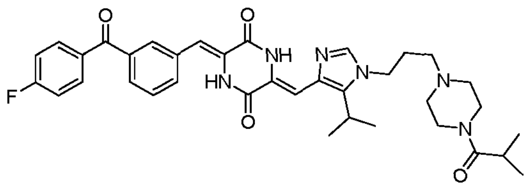,

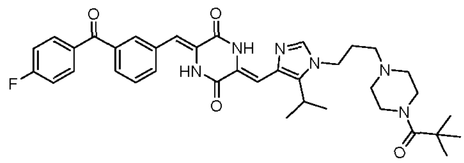,

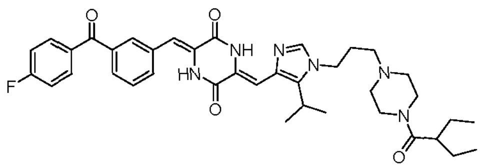,

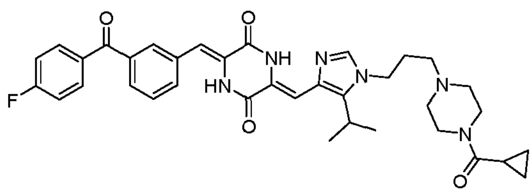,

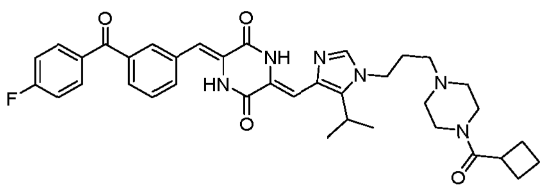,

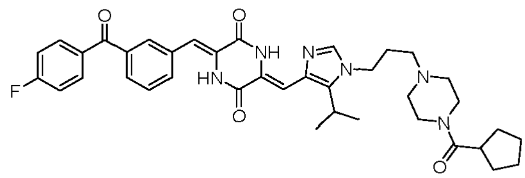,

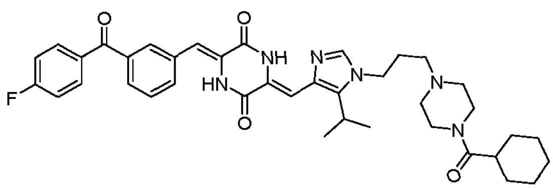,

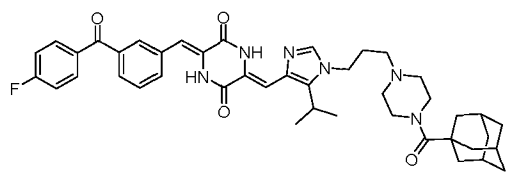,

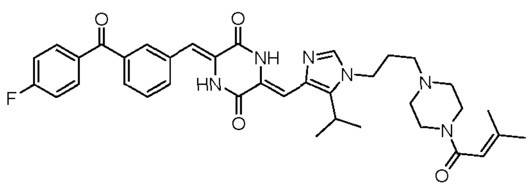,

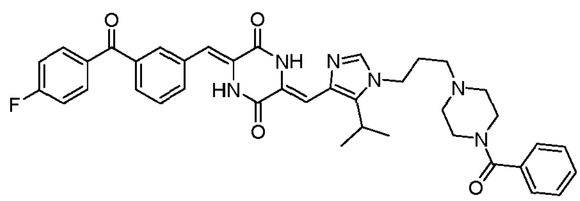,

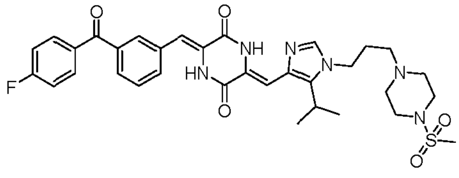,

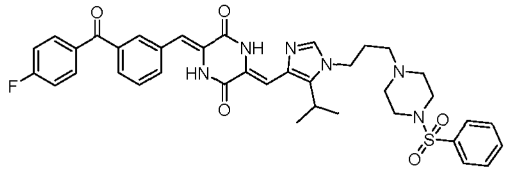,

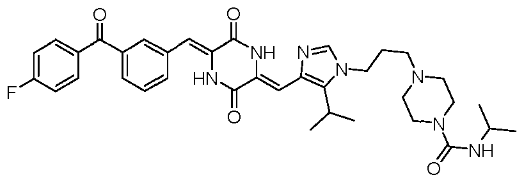,

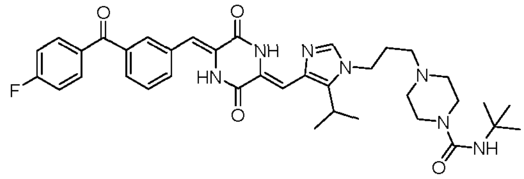,

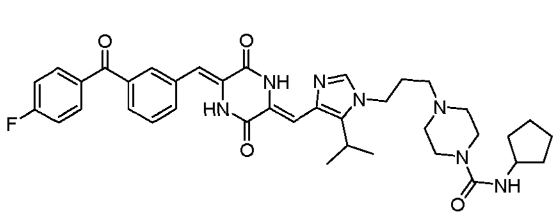,

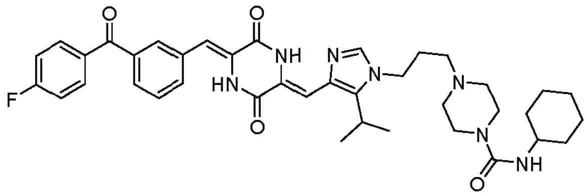,

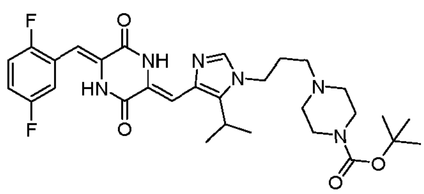,

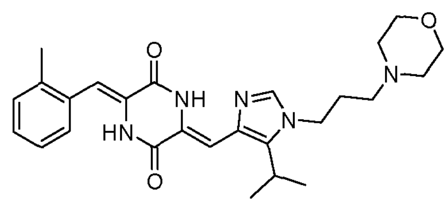,

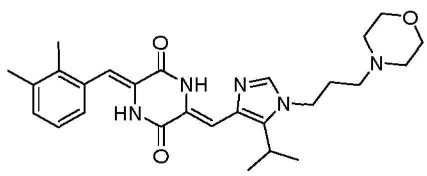,

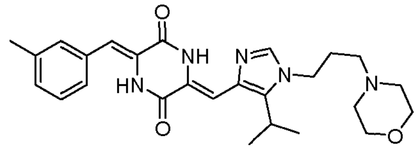,

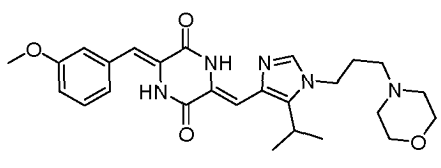,

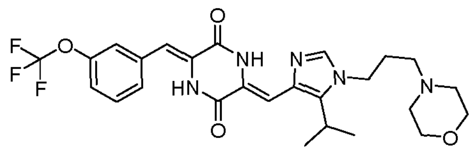,

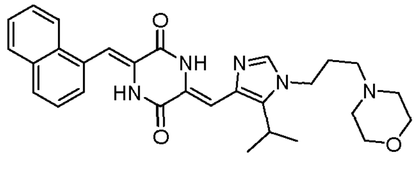,

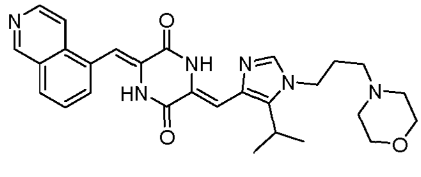,

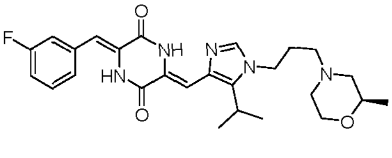,

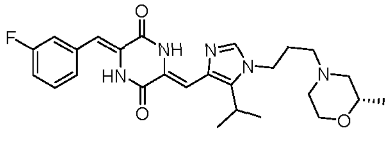,

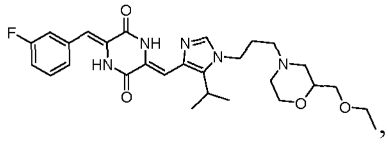,

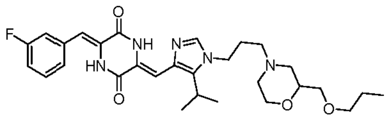,

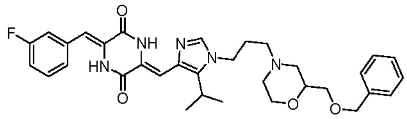,

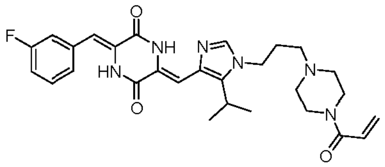,

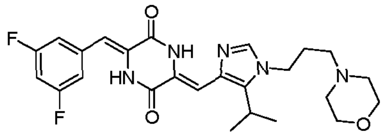,

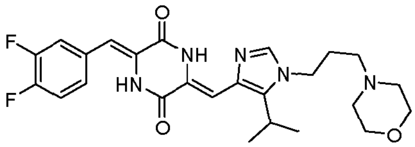,

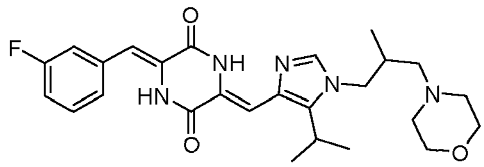,

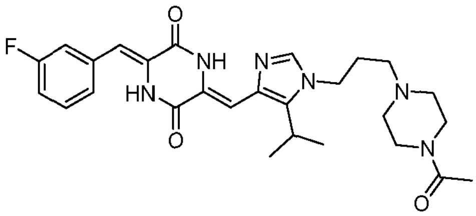,

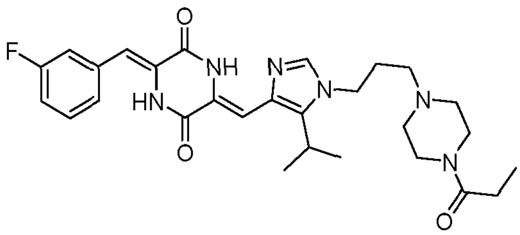,

or

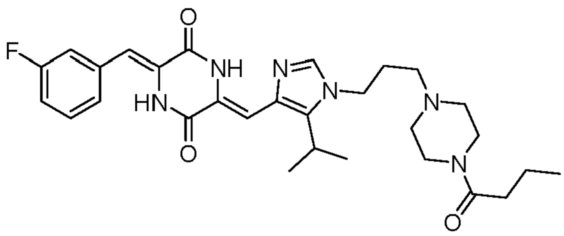.

Preferably, the compound of formula (I) is any one of the following compounds:
a compound with a retention time of 6.29 min under the following conditions, which is one stereoisomer of

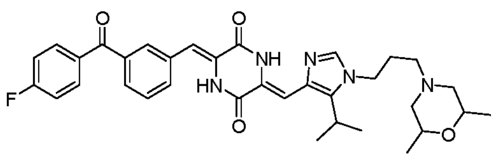:

chromatographic column: Acclaim 120, C18, 5 μm, 4.6 * 150 mm, mobile phase: mobile phase A: MeOH, mobile phase B: 0.1% formic acid aqueous solution; flow rate: 1 mL/min, the mobile phase is washed according to the following gradient elution procedure:

| Time (min) | Mobile phase A (%, V/V) | Mobile phase B (%, V/V) |
| --- | --- | --- |
| 0 | 40 | 60 |
| 0→6 | 40→100 | 60→0 |

(continued)

| Time (min) | Mobile phase A (%, V/V) | Mobile phase B (%, V/V) |
|---|---|---|
| 6-10 | 100-40 | 0→60 |

a compound with a retention time of 6.48 min under the following conditions, which is one stereoisomer of

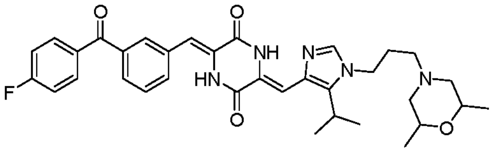
;

chromatographic column: Acclaim 120, C18, 5 μm, 4.6 * 150 mm, mobile phase: mobile phase A: MeOH, mobile phase B: 0.1% formic acid aqueous solution; flow rate: 1 mL/min, the mobile phase is washed according to the following gradient elution procedure:

| Time (min) | Mobile phase A (%, V/V) | Mobile phase B (%, V/V) |
|---|---|---|
| 0 | 40 | 60 |
| 0→6 | 40→100 | 60→0 |
| 6-10 | 100-40 | 0-60 |

The test conditions for the above retention time are not limited to the compound, and as long as the retention time obtained by using the above test conditions is the same as that described above or within the error range, and the compound is one stereoisomer of the compounds defined by the above retention times, the compound falls within the scope of protection of the present disclosure.

The pharmaceutically acceptable salt of the compound of formula (I) may be a salt prepared from the compound of formula (I) and a pharmaceutically acceptable acid, and the pharmaceutically acceptable acid may be a conventional acid in the art, such as an inorganic acid or an organic acid. The inorganic acid is preferably hydrochloric acid, and the organic acid is preferably methanesulfonic acid. Preferably, the pharmaceutically acceptable acid is hydrochloric acid or methanesulfonic acid. Preferably, the pharmaceutically acceptable salt of the compound of formula (I) is a salt formed by the compound of formula (I) and the pharmaceutically acceptable acid in a molar ratio of 1:2.

Preferably, the pharmaceutically acceptable salt of the compound of formula (I) is any one of the following compounds:

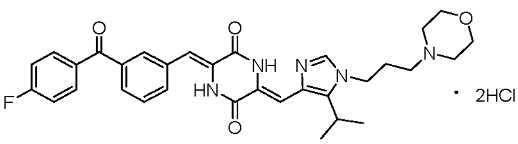
· 2HCl ,

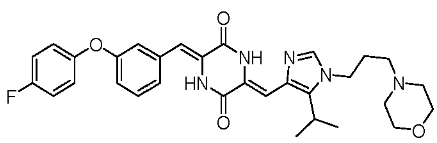
· 2HCl ,

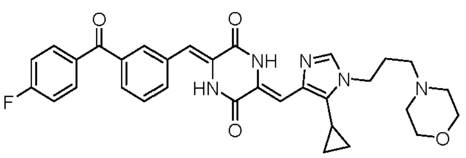
· 2HCl ,

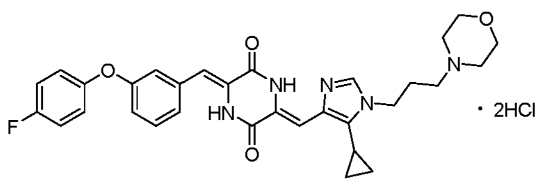
· 2HCl ,

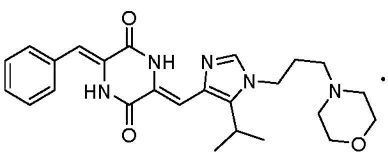
· 2HCl ,

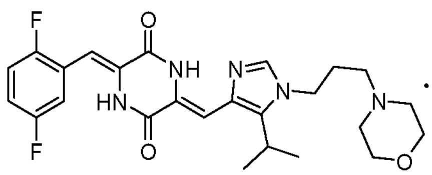
· 2HCl ,

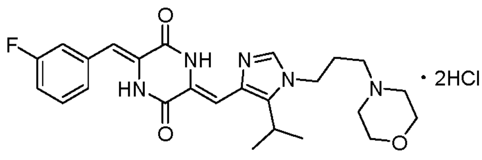
,

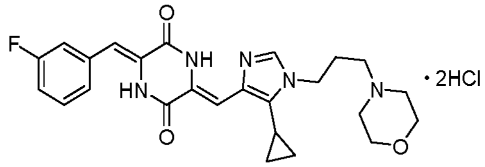
,

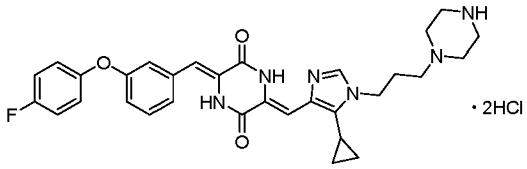
,

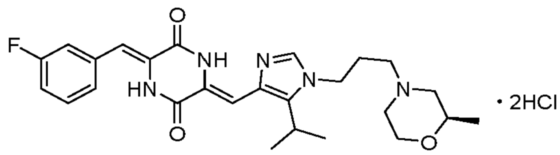
,

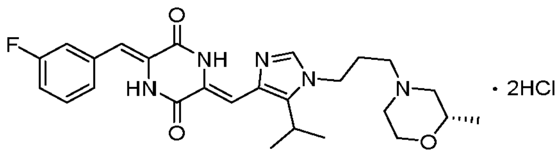
,

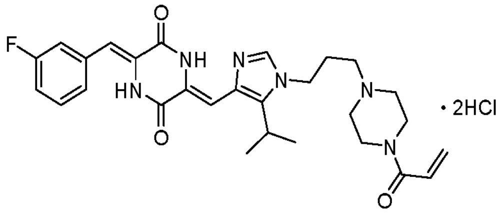
,

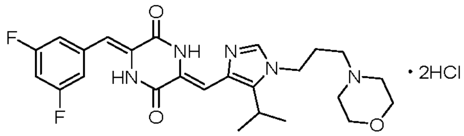
,

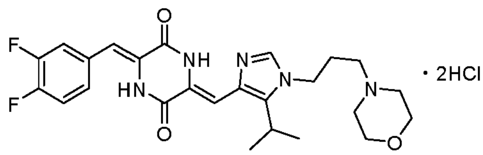
,

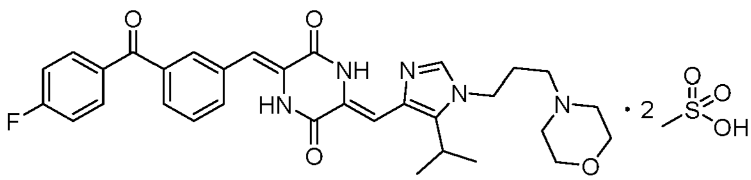
,

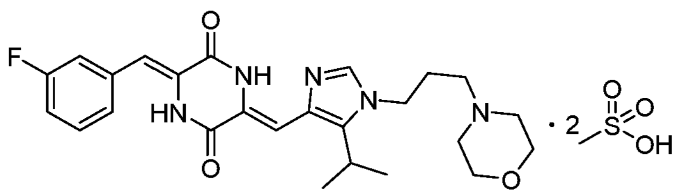
,

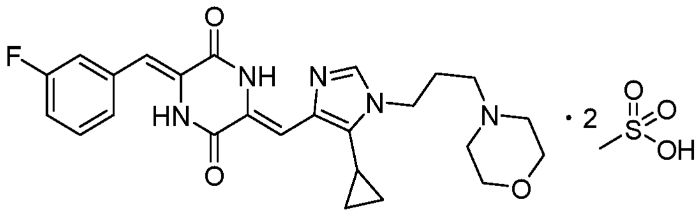
,

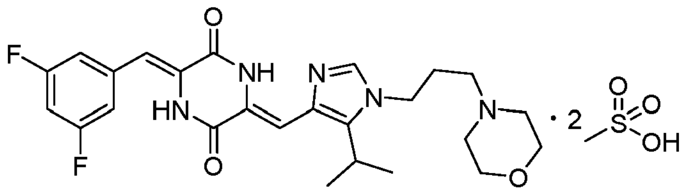

,

or

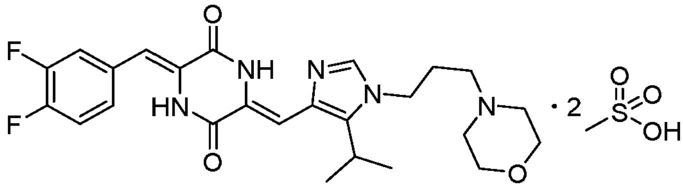

.

The present disclosure also provides a preparation method for the compound of formula (I), which comprises the following steps: carrying out a condensation reaction as shown below between a compound of formula (II) and a compound of formula (III) to obtain the compound of formula (I);

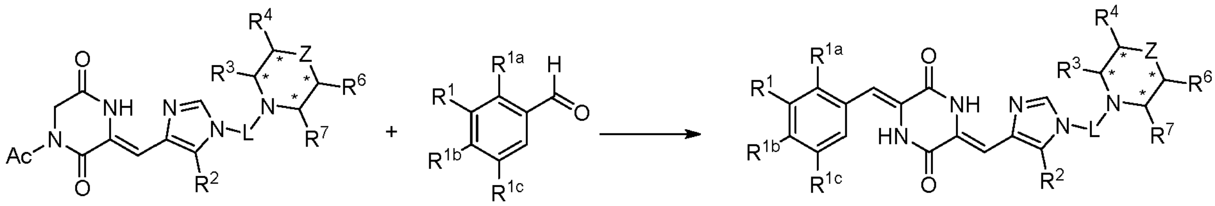

(II) (III) (I)

wherein *, L, Z, $R^1$, $R^{1a}$, $R^{1b}$, $R^{1c}$, $R^2$, $R^3$, $R^4$, $R^6$, and $R^7$ are as defined in any one of the preceding items.

The present disclosure also provides a compound of formula (II):

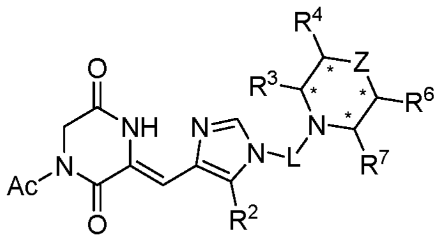

(II)

wherein *, $R^2$, $R^3$, $R^4$, $R^6$, $R^7$, L, and Z are as defined in any one of the preceding items.

Preferably, the compound of formula (II) is any one of the following compounds:

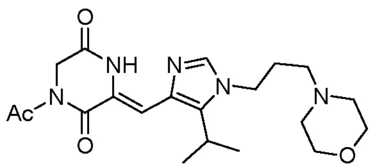

,

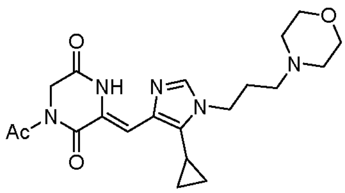

,

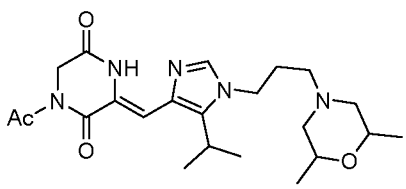

,

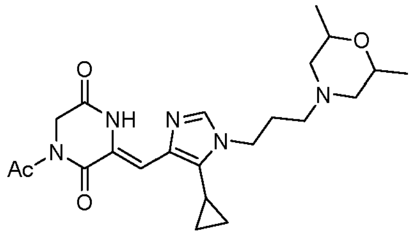,

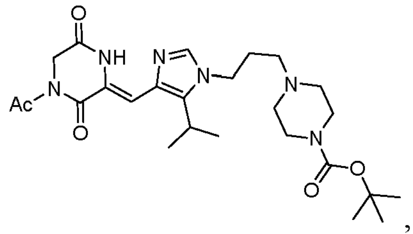,

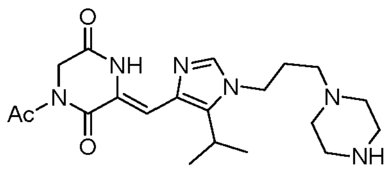,

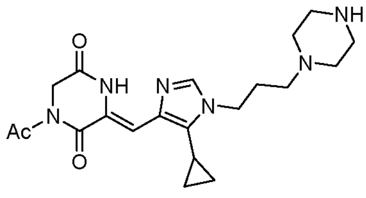,

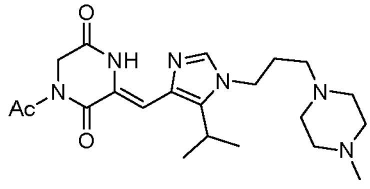,

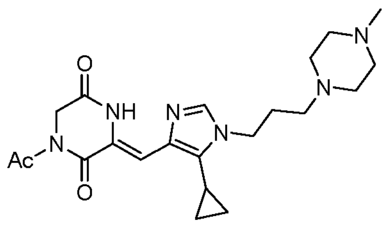,

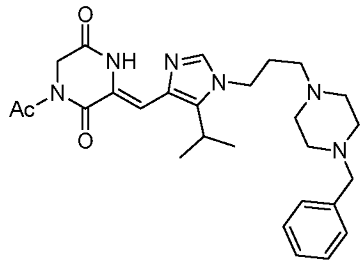,

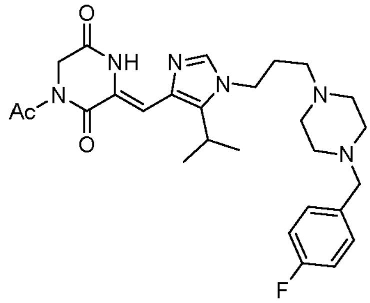,

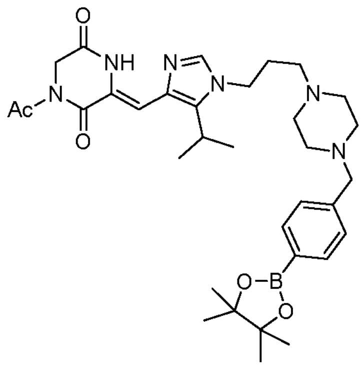,

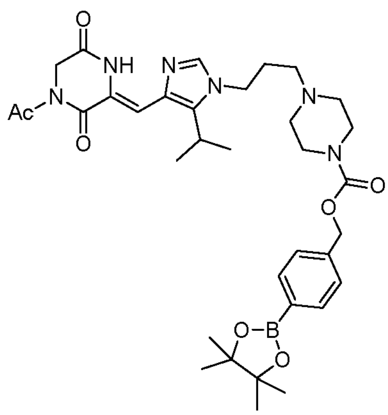,

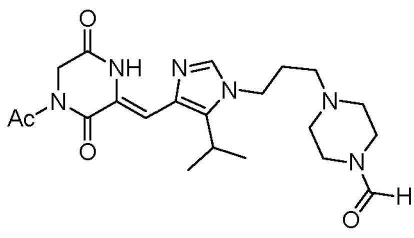,

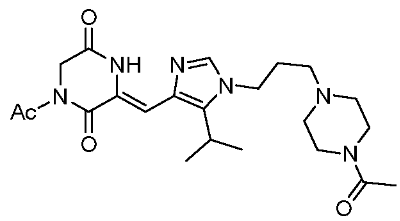,

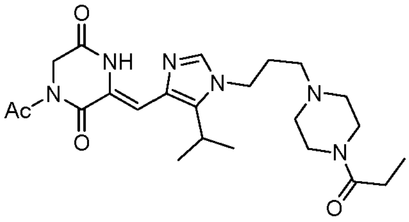
,
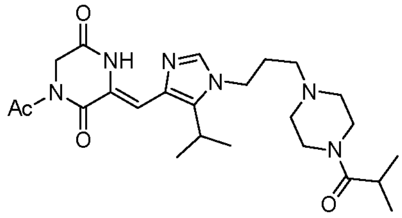
,
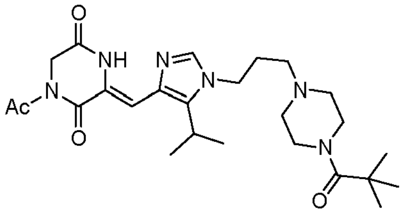
,
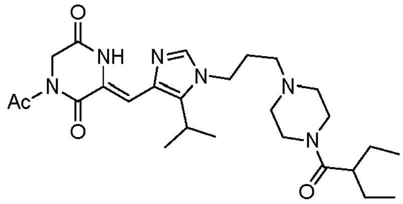
,
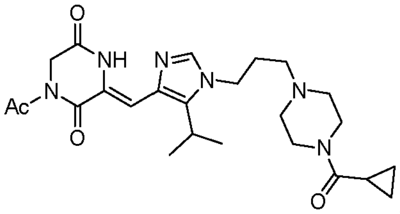
,
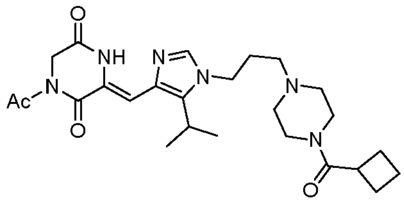
,
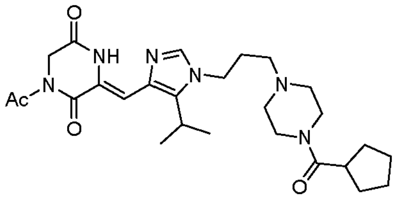
,
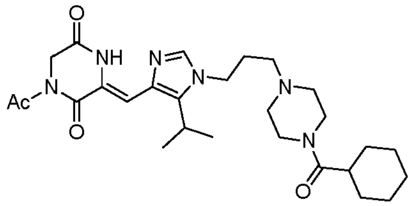
,
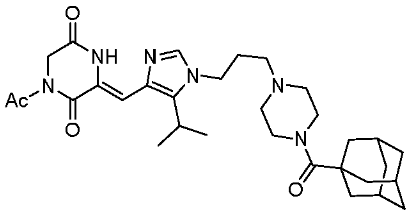
,
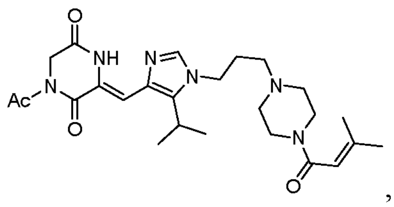
,
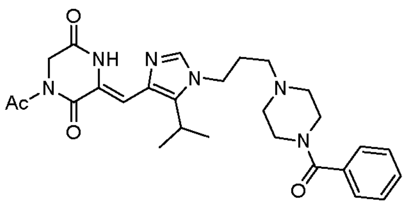
,
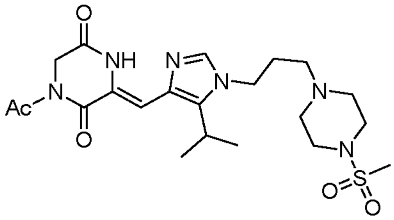
,

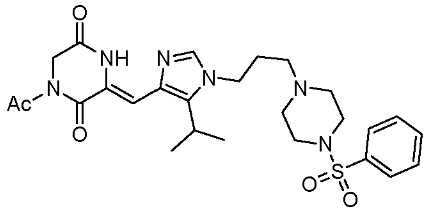,

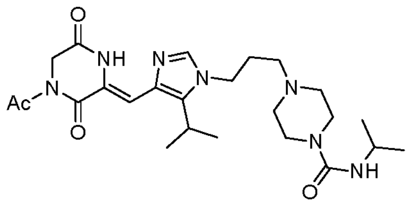,

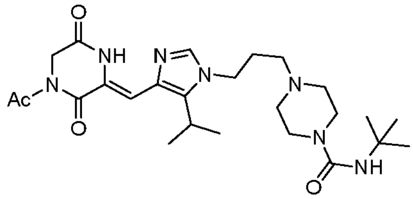,

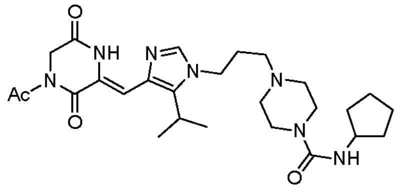,

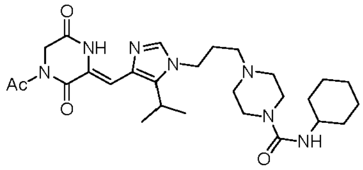,

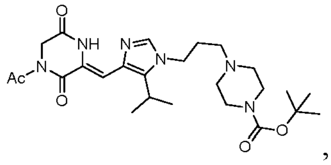,

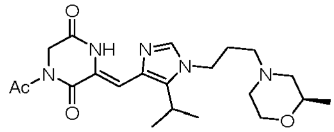,

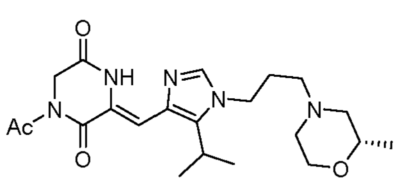,

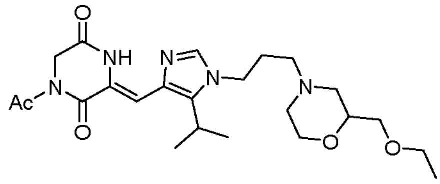,

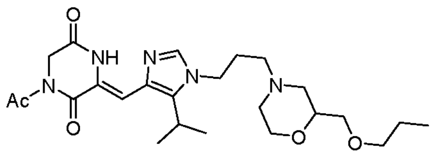,

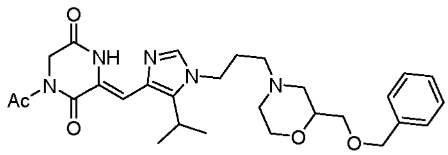,

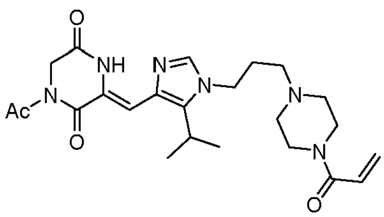,

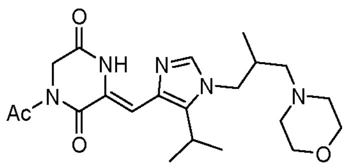,

or

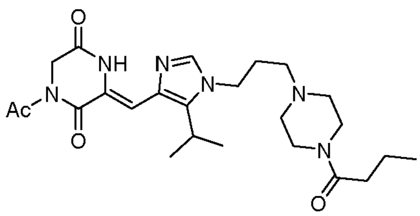.

The present disclosure also provides a preparation method for the compound of formula (II), which comprises the following steps: carrying out a substitution reaction as shown below between a 2,5-diketopiperazine derivative of formula

(A) and a compound of formula (B) to obtain the compound of formula (II);

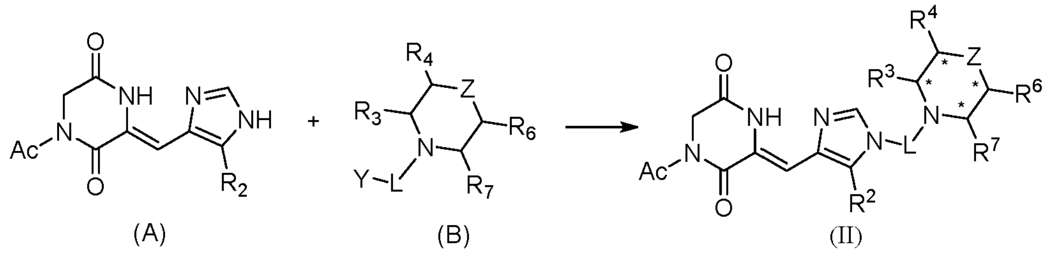

(A) (B) (II)

wherein Y is halogen or $-S(=O)_2-R^8$, $R^8$ is $C_1$-$C_6$ alkyl or $C_6$-$C_{10}$ aryl; *, $R^2$, $R^3$, $R^4$, $R^6$, $R^7$, L, and Z are as defined in any one of the preceding items.

Preferably, the substitution reaction is carried out in an aprotic solvent, and the aprotic solvent may be conventional in the art. Preferably, the aprotic solvent is one or more than one of acetonitrile, N,N-dimethylformamide, N,N-dimethylacetamide, dimethylsulfoxide, hexamethylphosphoric triamide, benzene, toluene, nitrobenzene, xylene, and carbon tetrachloride; for example, N,N-dimethylformamide.

Preferably, the substitution reaction is carried out in the presence of a base, and the base may be a base commonly used in the art for such reactions. Preferably, the base is one or more than one of sodium hydride, potassium hydroxide, potassium carbonate, cesium carbonate, and sodium bicarbonate, such as cesium carbonate.

Preferably, the substitution reaction is carried out in the presence of a catalyst, and the catalyst may be a catalyst commonly used in the art for such reactions. Preferably, the catalyst is one or more than one of iodine, potassium iodide, and sodium iodide, such as potassium iodide.

The present disclosure also provides a pharmaceutical composition, which comprises the above compound of formula (I), the stereoisomer thereof, the tautomer thereof, the pharmaceutically acceptable salt thereof, or the solvate of any one of the foregoing, and a pharmaceutical excipient.

In some embodiments, the pharmaceutical excipient does not comprise a cosolvent.

The present disclosure also provides the above compound of formula (I), the stereoisomer thereof, the tautomer thereof, the pharmaceutically acceptable salt thereof, or the solvate of any one of the foregoing, or the above pharmaceutical composition for use in preventing and/or treating cancer. The cancer is preferably one or more than one of lung cancer, pancreatic cancer, colon cancer, and liver cancer.

The compound, the stereoisomer thereof, the tautomer thereof, the pharmaceutically acceptable salt thereof or the solvate of any one of the foregoing, or the pharmaceutical composition of the present disclosure may be administered locally or systemically, for example, for enteral administration, such as rectal or oral administration, or for parenteral administration to mammals (especially humans). Exemplary combinations for rectal administration include a suppository, which may contain, for example, a suitable non-irritating excipient, such as cocoa butter, synthetic glycerides, or polyethylene glycol, which is a solid at room temperature, but melts and/or dissolves in the rectal cavity to release drugs. The compounds of the present disclosure may also be administered parenterally, for example, by inhalation, injection, or infusion, such as by intravenous, intraarterial, intraosseous, intramuscular, intracerebral, extraventricular, intrasynovial, intrasternal, intrathecal, intralesional, intralesional, intracranial, intratumoral, intradermal and subcutaneous injection, or infusion.

The therapeutically effective amount of the active ingredient is as defined in the context and depends on the species, weight, age, individual condition, individual pharmacokinetic parameters, diseases to be treated, and administration mode of the mammal. For enteral administration, such as oral administration, the compounds of the present disclosure can be formulated in a wide variety of dosage forms.

The effective amount of the compound, the stereoisomer thereof, the tautomer thereof, the pharmaceutically acceptable salt thereof or the solvate of any one of the foregoing, or the pharmaceutical composition of the present disclosure can be easily determined by routine experiments, and the most effective and convenient route of administration and the most appropriate preparation can also be determined by routine experiments.

The pharmaceutical excipients can be those widely used in the field of pharmaceutical production. Excipients are primarily used to provide a safe, stable, and functional pharmaceutical composition, and may also provide methods to enable the active ingredient to dissolve at a desired rate after the subject has received administration or to facilitate effective absorption of the active ingredient after the subject has received administration of the composition. The pharmaceutical excipients can be inert fillers or provide some functions, such as stabilizing the overall pH of the composition or preventing degradation of the active ingredients of the composition. The pharmaceutical excipients may include one or more than one of the following excipient: binder, suspending agent, emulsifying agent, diluent, filler, granulating agent, adhesive agent, disintegrating agent, lubricant, anti-adhesive agent, glidant, wetting agent, gelling agent, absorption retardant, dissolution inhibitor, enhancer, adsorbent, buffer, chelating agent, preservative, coloring

agent, flavoring agent, and sweetener.

Substances that may be used as pharmaceutically acceptable excipients include, but are not limited to, ion exchangers, aluminum stearate, lecithin, serum proteins such as human serum albumin, buffer substances such as phosphate, glycine, sorbic acid, potassium sorbate, a mixture of partial glycerides of saturated vegetable fatty acids, water, salts, or electrolytes such as protamine sulfate, disodium hydrogen phosphate, potassium hydrogen phosphate, sodium chloride, zinc salts, colloidal silicon, magnesium trisilicate, polyvinylpyrrolidone, polyacrylates, waxes, polyethylene-polyoxypropylene-block polymers, lanolin, sugars such as lactose, glucose, and sucrose; starches such as corn starch and potato starch; cellulose and its derivatives such as sodium carboxymethyl cellulose, ethyl cellulose, and cellulose acetate; gum powder; malt; gelatin; talc; excipients such as cocoa butter and suppository waxes; oils such as peanut oil, cottonseed oil, safflower oil, sesame oil, olive oil, corn oil, and soybean oil; glycol compounds such as propylene glycol and polyethylene glycol; esters such as ethyl oleate and ethyl laurate; agar; buffers such as magnesium hydroxide and aluminum hydroxide; alginic acid; pyrogen-free water; isotonic salt; ethanol, phosphate buffer solution, and other nontoxic suitable lubricants such as sodium lauryl sulfate and magnesium stearate, coloring agents, releasing agents, coating materials, sweeteners, flavoring agents and fragrances, preservatives, and antioxidants.

The pharmaceutical composition of the present disclosure can be prepared according to the disclosure using any method known to those skilled in the art. For example, conventional mixing, dissolving, granulating, emulsifying, levigating, encapsulating, embedding, or lyophilizing processes.

Pharmaceutical dosage forms of the compound of the present disclosure may be provided as immediate release, controlled release, sustained release, or targeted drug release systems. Common dosage forms include, for example, solutions and suspensions, (micro)emulsions, ointments, gels and patches, liposomes, tablets, sugar-coated pills, soft-shell or hard-shell capsules, suppositories, ovules, implants, amorphous or crystalline powder, aerosols, and lyophilized preparations. Depending on the route of administration, special devices, such as a syringe and needle, inhaler, pump, injection pen, applicator, or special flask, may be required to administer or give the drug. Pharmaceutical dosage forms often consist of a drug, an excipient, and a container/sealing system. One or more than one excipient (also known as inactive ingredients) may be added to the compound of the present disclosure to improve or facilitate the manufacture, stability, administration, and safety of the drug, and a method for obtaining the desired drug release curve can be provided. Therefore, the types of excipients added to the drug can depend on various factors, such as the physical and chemical properties of the drug, the route of administration, and the preparation steps. There are pharmaceutical excipients in this art, including those listed in various pharmacopoeias. (See U.S. Pharmacopeia (USP), Japanese Pharmacopoeia (JP), European Pharmacopoeia (EP), and British pharmacopoeia (BP); publications of Center for Drug Evaluation and Research (CEDR) of the U.S. Food and Drug Administration (www.fda.gov), such as *Inactive Ingredient Guide, 1996;* Handbook of Pharmaceutical Additives, 2002, by Ash and Ash, Synapse Information Resources, Inc. Endicott NY; etc.).

Pharmaceutical dosage forms of the compound of the present disclosure may be manufactured by any of the methods well known in the art, for example, by conventional mixing, sieving, dissolving, melting, granulating, making sugar-coated pills, tabletting, suspending, extruding, spray-drying, grinding, emulsification, (nano/micro) encapsulation, inclusion, or lyophilization processes. As described above, the composition of the present disclosure may include one or more than one physiologically acceptable inactive ingredient that facilitates processing of the active molecule into a preparation for pharmaceutical use.

The pharmaceutical composition and dosage forms may comprise one or more than one compound of the present disclosure, one or more than one pharmaceutically acceptable salt thereof, or one or more than one solvate of any one of the foregoing as active components. Pharmaceutically acceptable carriers may be a solid or liquid. Preparations in solid form include powders, tablets, pills, lozenges, capsules, cachets, suppositories, and dispersible granules. A solid carrier may also be one or more than one substance which acts as diluents, flavoring agents, solubilizers, lubricants, suspending agents, binders, preservatives, tablet disintegrating agents, or encapsulating materials. In powders, the carrier is usually a finely divided solid, which is a mixture with the finely divided active component. In tablets, the active component is usually mixed with a carrier with the necessary binding capacity in an appropriate proportion and compacted to the desired shape and size. Suitable carriers include, but are not limited to, magnesium carbonate, magnesium stearate, talc, sugar, lactose, pectin, dextrin, starch, gelatin, methyl cellulose, sodium carboxymethyl cellulose, low melting point wax, cocoa butter, etc. The preparation of the active compound may comprise an encapsulating material as a carrier, providing a capsule in which the active component, with or without carriers, is surrounded by carriers bound to the active component.

Other forms suitable for oral administration include liquid form preparations, including emulsions, syrups, elixirs, aqueous solutions, aqueous suspensions, or solid form preparations which are intended to be converted, shortly before use, to liquid form preparations. Emulsions may be prepared in a solution, for example, in a propylene glycol aqueous solution or may contain an emulsifying agent, such as lecithin, sorbitan monooleate, or acacia. Aqueous solutions may be prepared by dissolving the active component in water and adding suitable coloring agents, flavors, stabilizers, and thickeners. Aqueous suspensions may be prepared by dispersing the finely divided active ingredient in water with binders, such as natural or synthetic gums, resins, methyl cellulose, carboxymethyl cellulose, and other commonly used

suspending agents. Preparations in solid form include solutions, suspensions, and emulsions, which may contain, in addition to the active ingredient, coloring agents, flavors, stabilizers, buffers, artificial and natural sweeteners, dispersants, thickeners, solubilizers, etc.

For parenteral administration, the pharmaceutical composition of the present disclosure may be in the form of sterile injectable or infusible preparations, for example, as sterile aqueous or oleaginous suspensions. The suspension may be formulated according to techniques known in the art using a suitable dispersing or wetting agent (such as Tween 80) and a suspending agent. The sterile injectable or infusible preparation may also be a sterile injectable or infusible solution or suspension in a non-toxic parenterally acceptable diluent or solvent. Other examples of acceptable vehicles and solvents that can be used in the pharmaceutical composition of the present disclosure include, but are not limited to, mannitol, water, and isotonic sodium chloride solution. In addition, sterile non-volatile oils are commonly used as solvents or suspension media. Any mild non-volatile oil can be used for this purpose, including synthetic monoglycerides or diglycerides. Fatty acids such as oleic acid and its glyceride derivatives can be used to prepare injections, as well as natural pharmaceutically acceptable oils, such as olive oil or castor oil, especially in their polyethoxylated form. These oil solutions or suspensions may also contain a long-chain alcohol diluent or dispersant. Solutions for parenteral use may also include suitable stabilizers and, if necessary, buffers. Suitable stabilizers include antioxidants such as sodium bisulphate, sodium sulphite or ascorbic acid, citric acid and a salt thereof and EDTA sodium salt, alone or in combination. Parenteral solutions can also contain preservatives, such as benzalkonium chloride, p-hydroxybenzoic acid, or propylparaben and chlorobutanol.

A therapeutically effective amount can be estimated initially using various methods well known in the art. The initial amount for animal studies can be based on the effective concentration established in the cell culture assay. Dose ranges suitable for human subjects can be determined, for example, using data obtained from animal studies and cell culture assays. In certain embodiments, the compounds of the present disclosure can be prepared as medicaments for oral administration.

An effective or therapeutically effective amount or dose of a medicament (e.g., the compound of the present disclosure) refers to the amount of the medicament or compound that results in amelioration of symptoms or prolongation of survival in a subject. Toxicity and therapeutic efficacy of the molecules can be determined in cell culture or experimental animals by standard pharmaceutical procedures, for example, by measuring the $LD_{50}$ (the dose lethal to 50% of the population) and the $ED_{50}$ (the dose therapeutically effective in 50% of the population). The dose ratio of toxic effect to therapeutic effect is therapeutic index, which can be expressed as $LD_{50}/ED_{50}$. Agents exhibiting high therapeutic index are preferred.

An effective amount or therapeutically effective amount is an amount of a compound or pharmaceutical composition that will trigger a biological or medical response in a tissue, system, animal, or human being sought by a researcher, veterinarian, physician, or other clinician. The dose is preferably within the range of circulating concentration including minimal toxicity or no toxicity of $ED_{50}$. The dose may vary within this range, depending on the dosage form used and/or the route of administration used. The proper preparation, route of administration, dose, and interval of administration should be selected according to methods known in the art, taking into account the particularities of individual conditions.

The dose and interval may be individually adjusted to provide plasma levels of the active moiety sufficient to obtain the desired effect; i.e., a minimal effective concentration (MEC). The MEC varies for each compound, but may be estimated, for example, from in vitro data and animal experiments. The dose necessary to obtain MEC will depend on individual characteristics and route of administration. In the case of topical administration or selective uptake, the effective local concentration of the drug may be independent of the plasma concentration.

The amount of medicament or composition administered will depend on various factors, including the sex, age and weight of the individual being treated, the severity of the condition, the mode of administration, and the judgment of the prescribing physician.

Unless otherwise specified, the terms used in the present disclosure have the following meanings:
Those skilled in the art can understand that, according to the convention used in the art, the

" 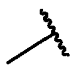 "

used in the structural formula of the group described in the present disclosure means that the corresponding group is connected to other moieties and groups in the compound through this site.

The term "more than one" refers to 2, 3, 4, or 5, preferably 2 or 3.

The term "pharmaceutically acceptable" refers to salts, solvents, excipients and the like that are generally non-toxic, safe, and suitable for patient use.

The term "solvate" refers to a substance formed by combining the compound of the present disclosure or a pharmaceutically acceptable salt thereof with a stoichiometric or non-stoichiometric amount of solvent. Solvent molecules

in the solvate can exist in an ordered or non-ordered arrangements. The solvent includes, but is not limited to, water, methanol, ethanol, etc.

The terms "compound", "pharmaceutically acceptable salt", "solvate" and "solvate of a pharmaceutically acceptable salt" may exist in the form of a single tautomer or a mixture thereof if tautomers exist, preferably in the form of relatively stable tautomers.

When an arbitrary variable (e.g., $R^{5-1}$) appears many times in the definition of a compound, the definition of each occurrence of the variable has nothing to do with the definitions of other occurrences, and their meanings are independent of each other and have no influence on each other. Therefore, if a group is substituted by 1, 2 or 3 $R^{5-1}$ groups, that is, the group may be substituted by up to 3 $R^{5-1}$ groups, the definition of $R^{5-1}$ at this position is independent of the definition of $R^{5-1}$ at the remaining positions. Additionally, combinations of substituents and/or variables are permissible only if such combinations result in stable compounds.

The term "halogen" refers to fluorine, chlorine, bromine, or iodine.

The term "alkyl" refers to a saturated linear or branched alkyl with a specified number of carbon atoms. Examples of alkyl include methyl, ethyl, n-propyl, isopropyl, n-butyl, tert-butyl, isobutyl, sec-butyl, n-pentyl, n-hexyl, n-heptyl, n-octyl, and similar alkyl.

The term "alkylene" refers to a subunit formed by formally eliminating two monovalent or one divalent atoms or groups from saturated linear or branched alkane with a specified number of carbon atoms. The two valences can be on the same carbon atom or on different carbon atoms (for example, two valences are on the carbon atoms at both ends). For example, methylene may be ($-CH_2-$), and ethylene may be $-CH_2CH_2-$, or $-CH(CH_3)-$.

The term "heteroalkylene" refers to a subunit formed by formally eliminating two monovalent or one divalent atoms or groups from saturated linear or branched heteroalkane. The two valences can be on the same atom or on different atoms (for example, two valences are on the atoms at both ends). For example, ethylene containing an oxygen atom can be - $CH_2OCH_2$- or $-CHO(CH_3)-$, etc.

The term "alkoxy" refers to the group $-O-R^X$, wherein $R^X$ is the alkyl as defined above.

The term "alkenyl" refers to a linear or branched alkene with a specific number of carbon atoms containing one or more than one carbon-carbon double bonds and without a carbon-carbon triple bond, and the one or more than one carbon-carbon double bond may be internal or terminal. Examples of alkene include vinyl, allyl, methylvinyl, propenyl, butenyl, pentenyl, 1,1-dimethyl-2-propenyl, hexenyl, etc.

The term "aryl" refers to $C_6$-$C_{10}$ aryl, such as phenyl or naphthyl.

The term "heteroaryl" refers to an aromatic group containing a heteroatom, preferably aromatic 3- to 6-membered monocyclic rings or 9- to 10-membered bicyclic rings containing 1, 2, or 3 heteroatoms independently selected from nitrogen, oxygen, and sulfur, and in the case of bicyclic rings, at least one ring is aromatic, such as furyl, pyridyl, pyridazinyl, pyrimidyl, pyrazinyl, thienyl, isoxazolyl, oxazolyl, diazolyl, imidazolyl, pyrrolyl, pyrazolyl, triazolyl, tetrazolyl, thiazolyl, isothiazolyl, thiadiazolyl, benzimidazolyl, indolyl, indazolyl, benzothiazolyl, benzisothiazolyl, benzazolyl, benzisoxazolyl, quinolinyl, isoquinolyl, etc.

The term "cycloalkyl" refers to monovalent saturated cyclic alkyl, preferably monovalent saturated cyclic alkyl having 3 to 6 carbon atoms, such as cyclopropyl, cyclobutyl, cyclopentyl, or cyclohexyl.

The term "heterocycloalkyl" refers to a saturated cyclic group having a heteroatom, preferably a 3- to 10-membered saturated monocyclic ring containing 1, 2 or 3 cyclic heteroatoms independently selected from N, O, and S. Examples of heterocycloalkyl are: tetrahydrofuranyl, tetrahydropyranyl, tetrahydrothienyl, tetrahydropyridyl, tetrahydropyrrolyl, azetidinyl, thiazolidinyl, azolidinyl, piperidinyl, morpholinyl, thiomorpholinyl, piperazinyl, azepanyl, diazepanyl, oxazepanyl, etc. Preferred heterocyclyl is morpholin-4-yl, piperidin-1-yl, pyrrolidin-1-yl, thiomorpholin-4-yl, and 1,1-dioxo-thiomorpholin-4-yl.

The term "treatment" refers to a therapeutic therapy. Regarding to a specific disease, the treatment refers to: (1) alleviation of one or more than one biological manifestation of a disorder or disease, (2) interfering with (a) one or more than one point in the biological cascade leading to or causing a disease or (b) one or more than one biological manifestation of the disease, (3) improvement of one or more than one symptom, effect, or side effect associated with the disease, or one or more than one symptom, effect, or side effect associated with the disease or treatment thereof, or (4) slowdown of the progression of a disease or one or more than one biological manifestation of the disease.

The term "prevention" refers to a reduced risk of acquiring or developing a disease or disorder.

The term "patient" refers to any animal, preferably a mammal, and most preferably a human, to whom the compound or composition will be or has been administered according to examples of the present disclosure. The term "mammal" includes any mammal. Examples of the mammal include, but are not limited to, cow, horse, sheep, pig, cat, dog, mouse, rat, rabbit, guinea pig, monkey, human, etc., and most preferably a human.

On the basis of not violating the common sense in the art, the above-mentioned preferred conditions may be arbitrarily combined to obtain the preferred embodiments of the present disclosure.

The reagents and raw materials used in the present disclosure are commercially available.

The positive progressive effect of the present disclosure is that:

The compounds involved in the present disclosure are brand new compounds obtained by the inventors of the present disclosure through structure and synthetic route design and chemical synthesis, which have not been reported in the literature. Compared with the control Plinabulin, some compounds have anti-tumor activities equivalent to or even better than that Plinabulin; some compounds have good water solubility when formed into salts, and can be administered by intravenous injection after dissolving with the commonly used clinical saline/5% glucose aqueous solution. Especially when Z in the compound of formula (I) is oxygen, the compounds of the present disclosure have an improved water solubility compared with Plinabulin, which have a good development prospect.

BRIEF DESCRIPTION OF THE DRAWINGS

FIG. 1 shows the immunofluorescence results of H460 cell line of a derivative of a 2,5-diketopiperazine compound.
FIG. 2 shows the immunofluorescence results of BxPC-3 cell line of a derivative of a 2,5-diketopiperazine compound.
FIG. 3 shows experimental results of NCI-H460 cell line apoptosis detected by a flow cytometer.
FIG. 4 shows experimental results of BxPC-3 cell line apoptosis detected by the flow cytometer.

DETAILED DESCRIPTION OF THE PREFERRED EMBODIMENT

The following examples further illustrate the present disclosure, but the present disclosure is not limited thereto. Experimental methods without specific conditions in the following examples are selected according to conventional methods and conditions, or according to the commercial specification.
Examples of some of the compounds involved in the following examples are shown below:

| Compound name | Structural formula |
|---|---|
| Plinabulin | 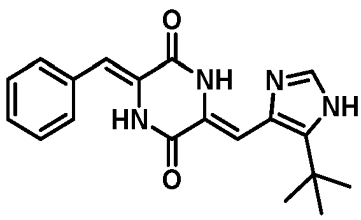 |
| PLN-2-1 | 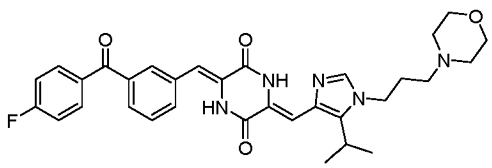 |
| PLN-2-1-1 | 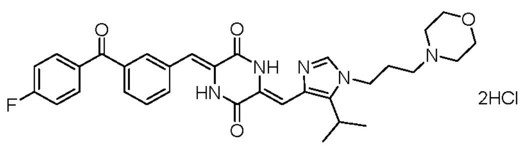 2HCl |
| PLN-2-1-2 | 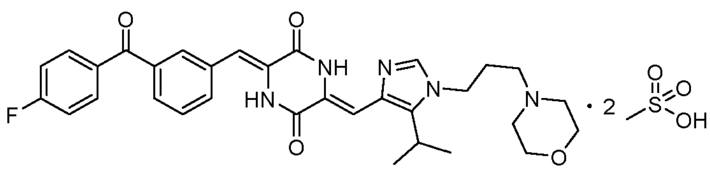 |
| PLN-2-2 | 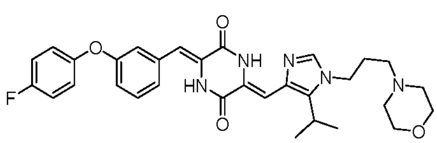 |

(continued)

| Compound name | Structural formula |
|---|---|
| PLN-2-2-1 | 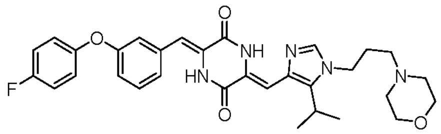 • 2HCl |
| PLN-2-3 | 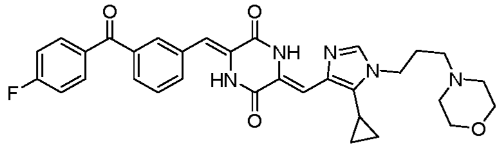 |
| PLN-2-3-1 | 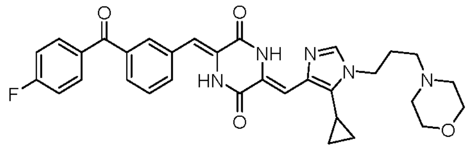 • 2HCl |
| PLN-2-4 | 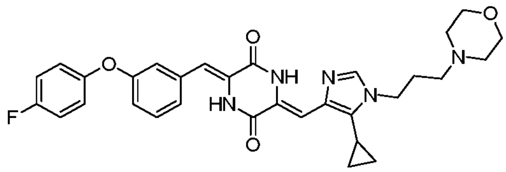 |
| PLN-2-4-1 | 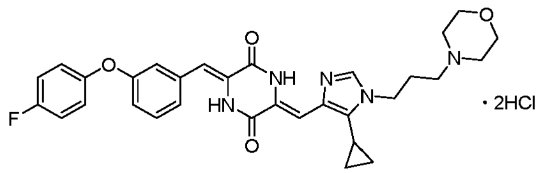 • 2HCl |
| PLN-2-5 | 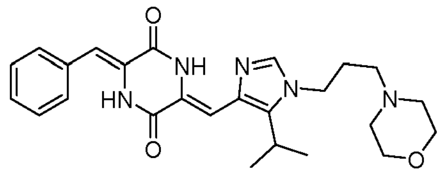 |
| PLN-2-5-1 | 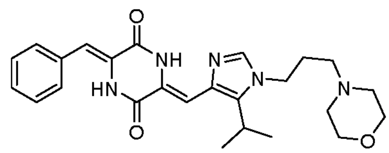 • 2HCl |
| PLN-2-6 | 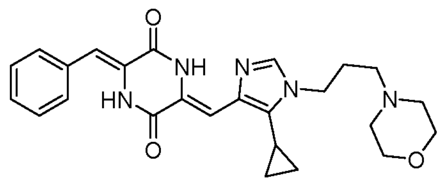 |
| PLN-2-7 | 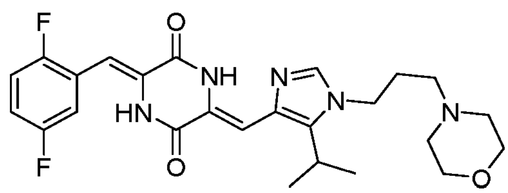 |

(continued)

| Compound name | Structural formula |
| --- | --- |
| PLN-2-7-1 | 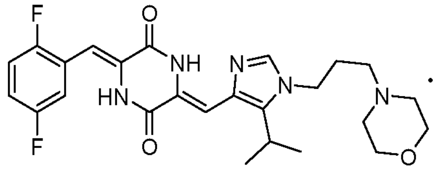 • 2HCl |
| PLN-2-8 | 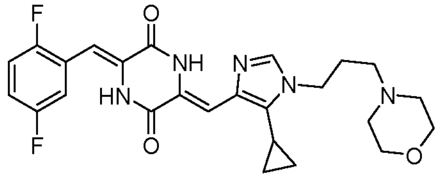 |
| PLN-2-9 | 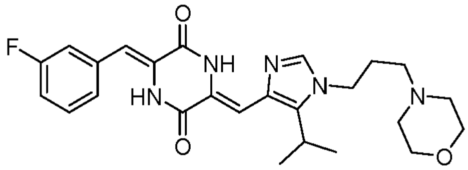 |
| PLN-2-9-1 | 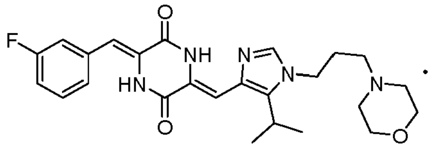 • 2HCl |
| PLN-2-9-2 | 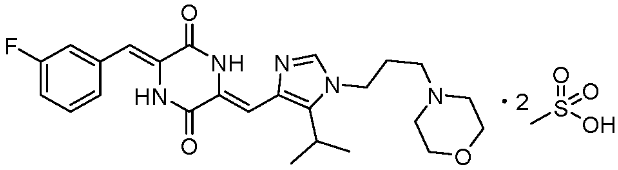 |
| PLN-2-10 | 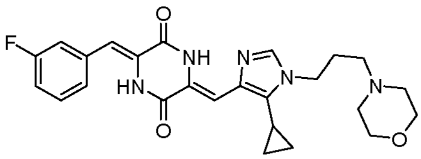 |
| PLN-2-10-1 | 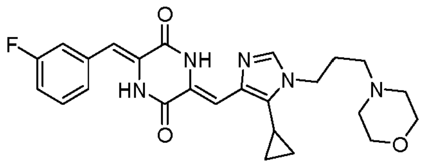 • 2HCl |
| PLN-2-10-2 | 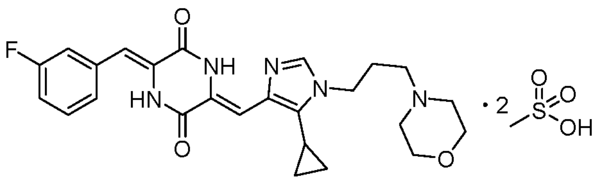 |
| PLN-2-11 | 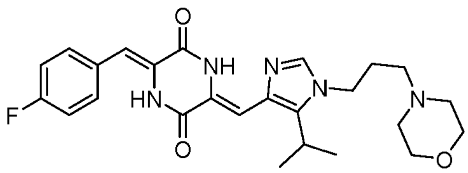 |

(continued)

| Compound name | Structural formula |
|---|---|
| PLN-2-12 | 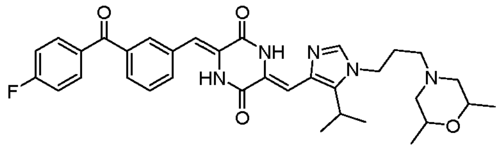 One stereoisomer of |
| PLN-2-13 | 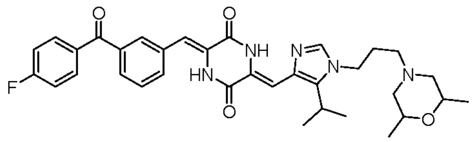 One stereoisomer of |
| PLN-2-14 | 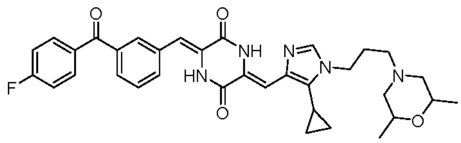 |
| PLN-2-15 | 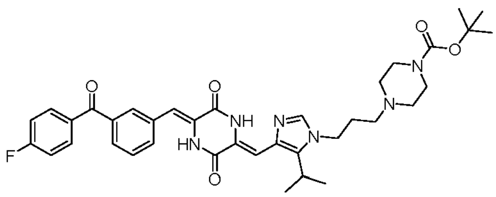 |
| PLN-2-16 | 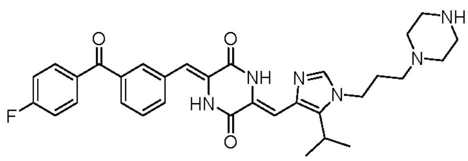 |
| PLN-2-17 | 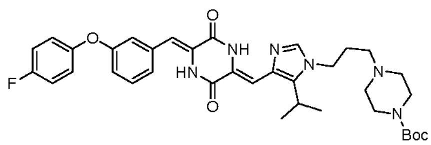 |
| PLN-2-18 | 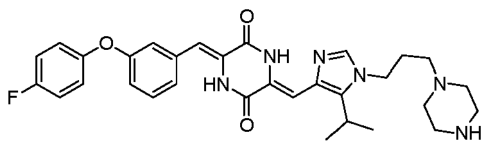 |
| PLN-2-19 | 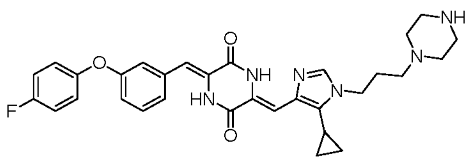 |
| PLN-2-19-1 | 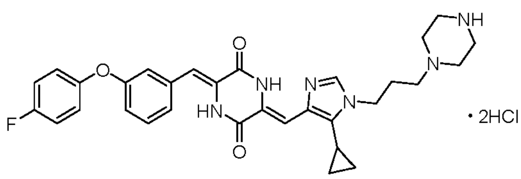 • 2HCl |

(continued)

| Compound name | Structural formula |
|---|---|
| PLN-2-20 | 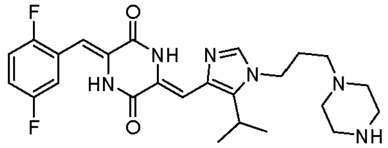 |
| PLN-2-21 | 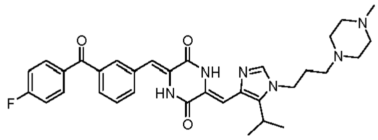 |
| PLN-2-22 | 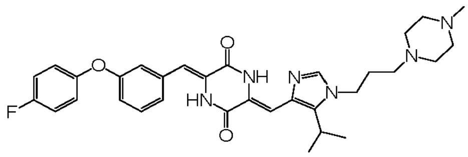 |
| PLN-2-23 | 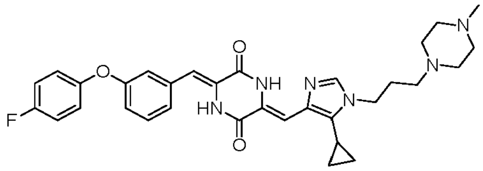 |
| PLN-2-24 | 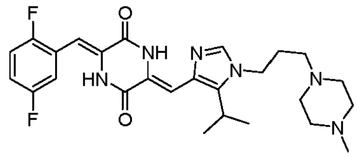 |
| PLN-2-25 | 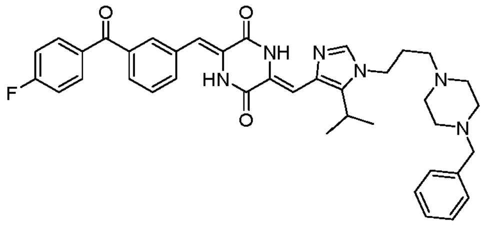 |
| PLN-2-26 | 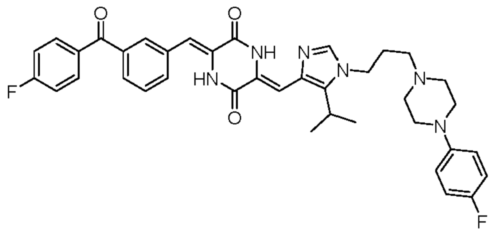 |
| PLN-2-27 | 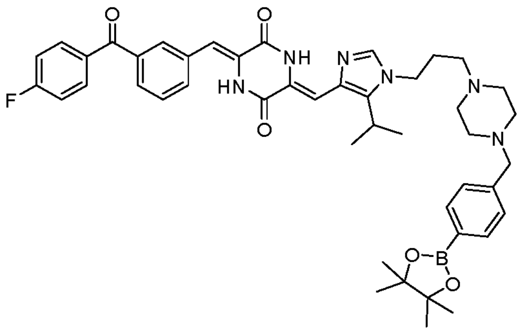 |

(continued)

| Compound name | Structural formula |
|---|---|
| PLN-2-28 | 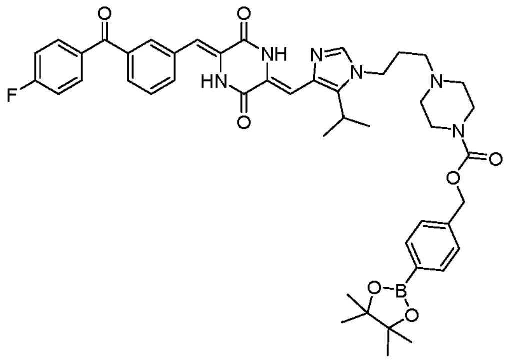 |
| PLN-2-29 | 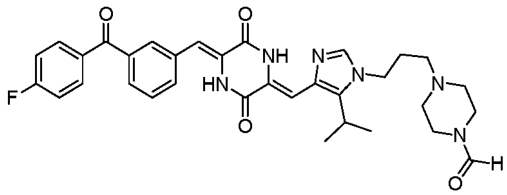 |
| PLN-2-30 | 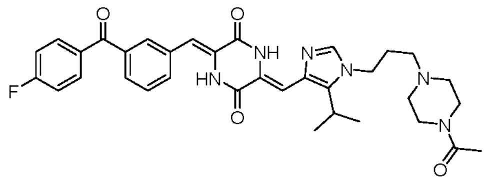 |
| PLN-2-31 | 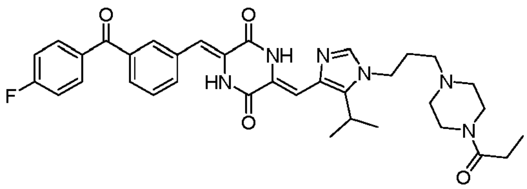 |
| PLN-2-32 | 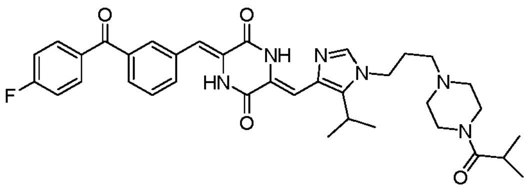 |
| PLN-2-33 | 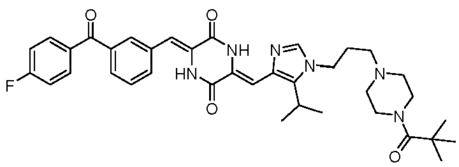 |
| PLN-2-34 | 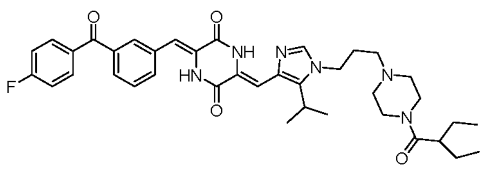 |

(continued)

| Compound name | Structural formula |
|---|---|
| PLN-2-35 | 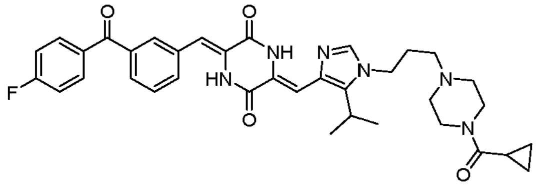 |
| PLN-2-36 | 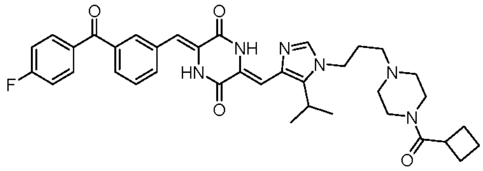 |
| PLN-2-37 | 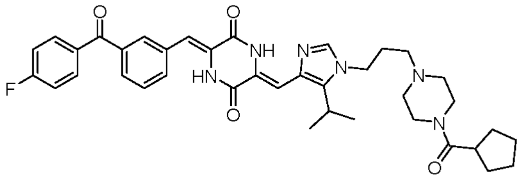 |
| PLN-2-38 | 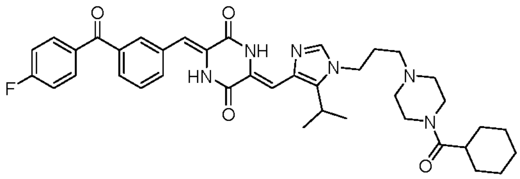 |
| PLN-2-39 | 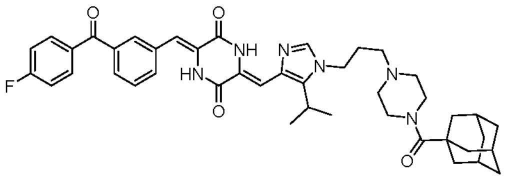 |
| PLN-2-40 | 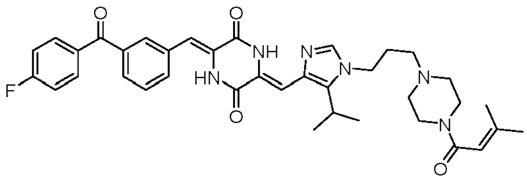 |
| PLN-2-41 | 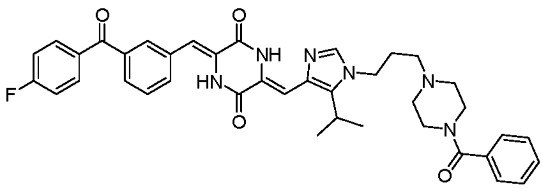 |
| PLN-2-42 | 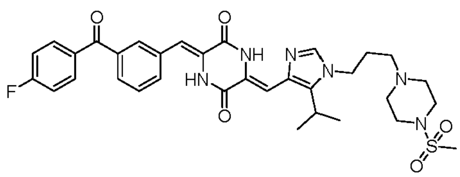 |

(continued)

| Compound name | Structural formula |
|---|---|
| PLN-2-43 | 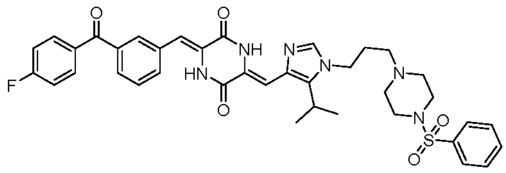 |
| PLN-2-44 | 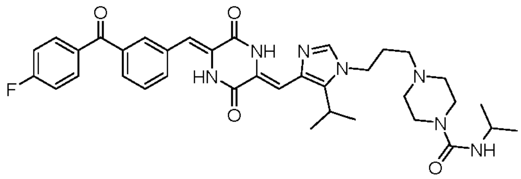 |
| PLN-2-45 | 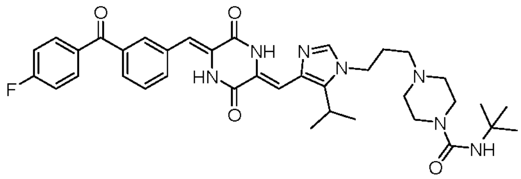 |
| PLN-2-46 | 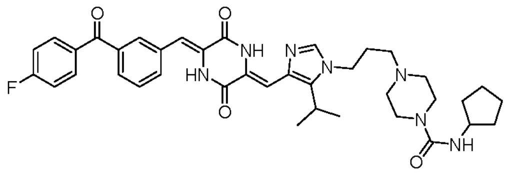 |
| PLN-2-47 | 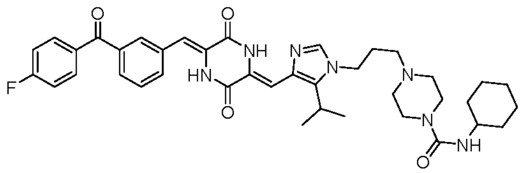 |
| PLN-2-48 | 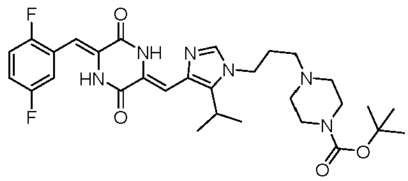 |
| PLN-2-49 | 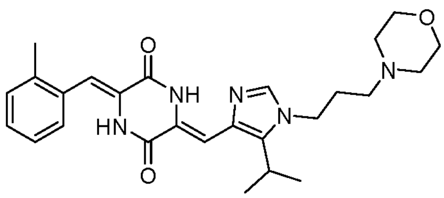 |
| PLN-2-50 | 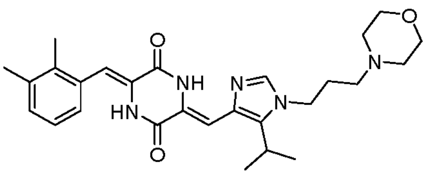 |

(continued)

| Compound name | Structural formula |
| --- | --- |
| PLN-2-51 | 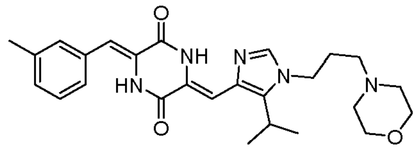 |
| PLN-2-52 | 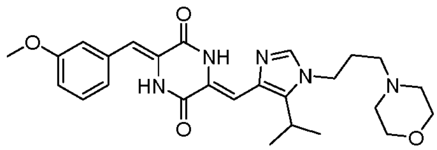 |
| PLN-2-53 | 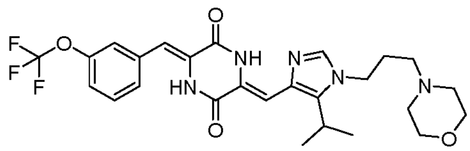 |
| PLN-2-54 | 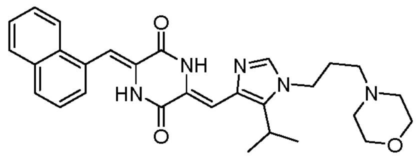 |
| PLN-2-55 | 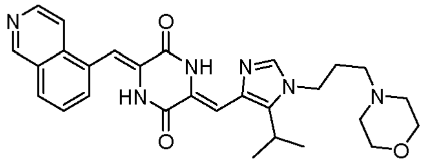 |
| PLN-2-56 | 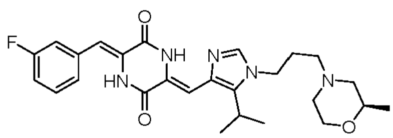 |
| PLN-2-56-1 | 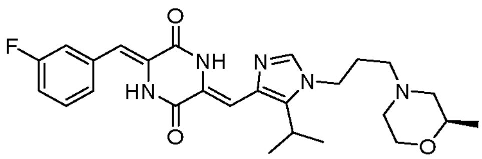 • 2HCl |
| PLN-2-57 | 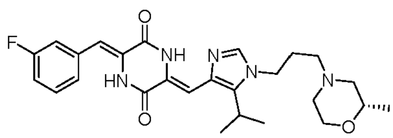 |
| PLN-2-57-1 | 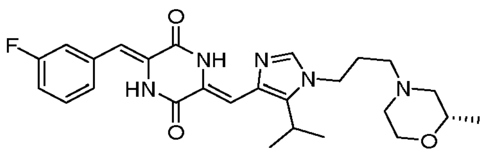 • 2HCl |
| PLN-2-58 | 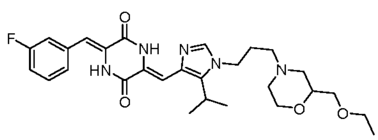 |

(continued)

| Compound name | Structural formula |
| --- | --- |
| PLN-2-59 | 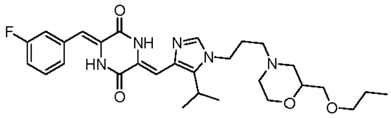 |
| PLN-2-60 | 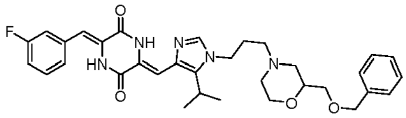 |
| PLN-2-61 | 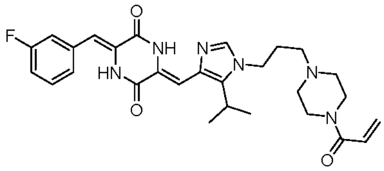 |
| PLN-2-61-1 | 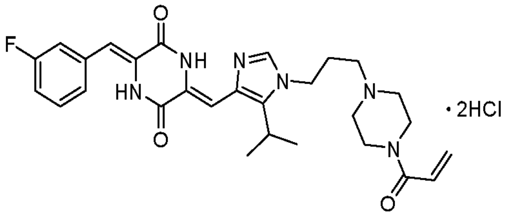 |
| PLN-2-62 | 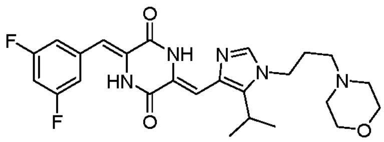 |
| PLN-2-62-1 | 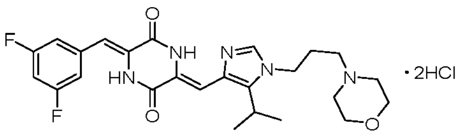 |
| PLN-2-62-2 | 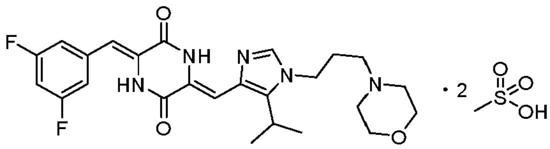 |
| PLN-2-63 | 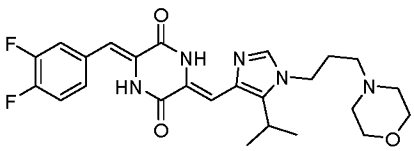 |
| PLN-2-63-1 | 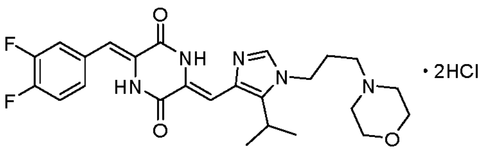 |
| PLN-2-63-2 | 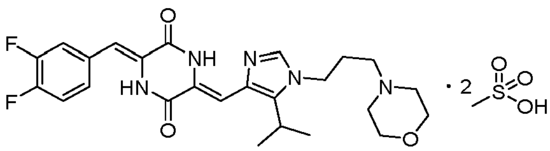 |

(continued)

| Compound name | Structural formula |
|---|---|
| PLN-2-64 | 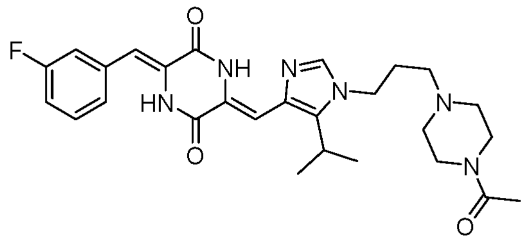 |
| PLN-2-65 | 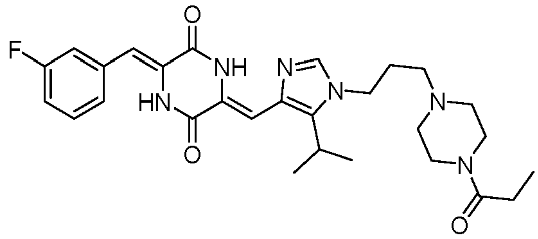 |
| PLN-2-66 | 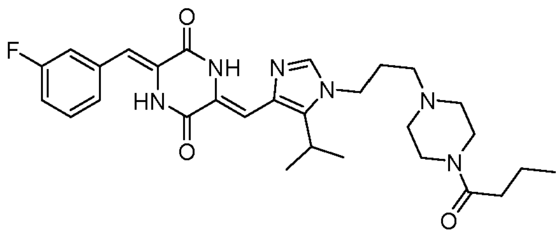 |
| PLN-2-67 | 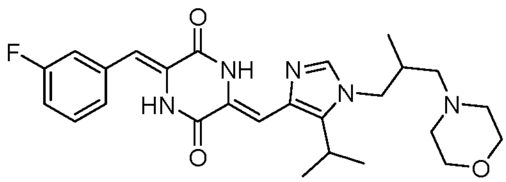 |

**Example 1: Synthesis of (3Z,6Z)-3-(3-(p-fluorobenzoyl)phenyl)methylene-6-((5-isopropyl-1-(3-morpholinyl)propylimidazol-4-yl)methylene)piperazine-2,5-dione (PLN-2-1)**

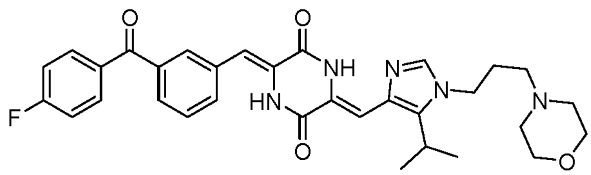

1. Preparation of 4-(3-chloropropyl)morpholine

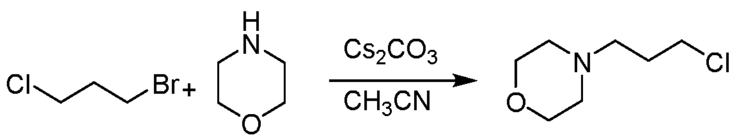


To a 250 mL dry round-bottom flask was sequentially added morpholine (2.0 g, 22.96 mmol), acetonitrile (50 mL),

cesium carbonate (8.98 g, 27.55 mmol), and 1-chloro-3-bromopropane (7.23 g, 45.91 mmol). The mixture was stirred and reacted in an oil bath at 25°C for 16.5 hours. The reaction was monitored by TLC (MeOH: DCM = 1:10), and the reaction was balanced. The mixture was filtered, and the filter cake was washed with EA, concentrated under reduced pressure, and saturated brine was added thereto. The mixture was extracted with EA (100 mL * 3), dried over anhydrous sodium sulfate, concentrated under reduced pressure, subjected to column chromatography (MeOH: DCM = 1:80, 1:40), concentrated under reduced pressure to obtain 3.07 g of a colorless oily liquid with a yield of 81.65%.

$^{1}H$ NMR (500 MHz, $CDCl_3$) δ = 3.75 - 3.67 (m, 4H), 3.61 (t, *J*=6.5, 2H), 2.49 (t, *J*=7.0, 2H), 2.44 (s, 4H), 1.95 (p, *J*=6.7, 2H).

2. Preparation of (Z)-1-acetyl-3-((5-isopropyl-1-(3-morpholinopropyl)-1H-imidazol-4-yl)methylene)piperazine-2,5-dione

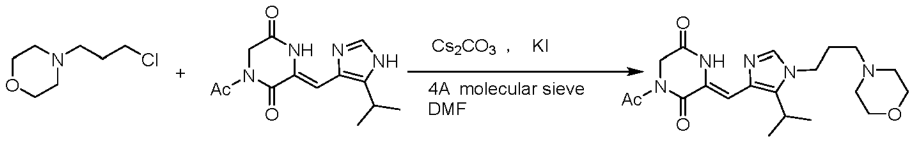


2) To a 50 mL dry round-bottom flask was sequentially added (Z)-1-acetyl-3-((5-isopropyl-1H-imidazol-4-yl) methylene)piperazine-2,5-dione (300 mg, 1.08 mmol), 1-chloro-3-morpholinopropane (355.37 mg, 2.17 mmol), cesium carbonate (707.55 mg, 2.17 mmol), potassium iodide (180.24 mg, 1.08 mmol), a 4A molecular sieve (500 mg), and DMF (6 mL). The mixture was degassed, placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with EtOH: DCM = 1:5, filtered under reduced pressure, and the filter cake was washed with EtOH: DCM = 1:5, concentrated under reduced pressure to obtain 350.48 mg of a brown solid crude product with a yield of 80.00%. The brown solid crude product was used directly in the next step without purification.

3) To a 25 mL dry brown round-bottom flask was sequentially added (Z)-1-acetyl-3-((5-isopropyl-1-(3-morpholinyl)propyl-imidazol-4-yl)methylene)piperazine-2,5-dione (350.48 mg, 0.87 mmol), 3-p-fluorobenzoylbenzaldehyde (257.71 mg, 1.13 mmol), cesium carbonate (424.54 mg, 1.30 mmol), anhydrous sodium sulfate (246.76 mg, 1.74 mmol), and DMF (6 mL). The mixture was degassed, placed in an oil bath at 45°C under nitrogen atmosphere, stirred and reacted for 18 hours. The reaction was monitored by LC-MS. After the reaction was completed, the reaction mixture was transferred to a 100 mL single-necked flask, washed with ethanol, concentrated under reduced pressure, redissolved with EtOH: DCM = 1:3, filtered under reduced pressure, and the filter cake was washed with EtOH: DCM = 1:3, concentrated under reduced pressure, subjected to flash reversed-phase column chromatography loaded with C18 using gradient elution, and concentrated under reduced pressure to obtain 87 mg of the target product with a yield of 17.52%.

$^{1}H$ NMR (500 MHz, DMSO-$d_6$) δ = 11.98 (s, 1H), 10.29 (s, 1H), 7.94-7.90 (m, 2H), 7.87 (d, J=5.9, 2H), 7.82 (d, *J*=7.3, 1H), 7.59 (dt, *J*=15.1, 7.6, 2H), 7.40 (t, *J*=8.8, 2H), 6.76 (s, 1H), 6.67 (s, 1H), 4.04 (t, *J*=7.1, 2H), 3.58 (t, *J*=4.3, 4H), 3.23 (dt, *J*=14.2, 7.1, 1H), 2.33 (s, 4H), 2.25 (t, *J*=6.7, 2H), 1.89-1.78 (m, 2H), 1.33 (d, *J*=7.1, 6H). MS (ESI) : m/z 572.25 $[M+H]^+$. Mp: 218-219°C.

**Example 2: Preparation of (3Z,6Z)-3-(4-fluorophenoxy)phenyl)methylene-6-((5-isopropyl-1-(3-morpholinyl)propyl)-imidazol-4-yl)methylene)piperazine-2,5-dione (PLN-2-2)**

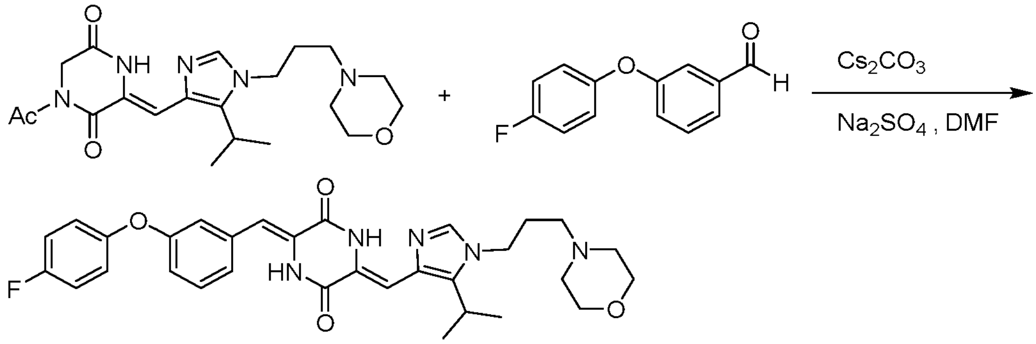


To a 25 mL dry reaction flask was sequentially added (Z)-1-acetyl-3-((5-isopropyl-1-(3-morpholinyl)propyl)-1H-imidazol-4-yl)methylene)piperazine-2,5-dione (250 mg, 0.62 mmol), 3-(4-fluorophenoxy)benzaldehyde (160 mg, 0.74 mmol), DMF (6 mL), cesium carbonate (303 mg, 0.93 mmol), and anhydrous sodium sulfate (176 mg, 1.24 mmol). The mixture was replaced with nitrogen, protected from light, and stirred and reacted in an oil bath at 45°C for 20 hours. The mixture was transferred to a 100 mL single-necked flask, rinsed with ethanol, concentrated under reduced pressure, redissolved with a mixed solvent of ethanol and dichloromethane (V ethanol: V dichloromethane = 1:3), filtered under reduced pressure, and the filter cake was rinsed with the mixed solvent (V ethanol: V dichloromethane = 1:3), and concentrated under reduced pressure. Methanol (3 mL) was added thereto, ultrasonically dispersed, and the mixture was left overnight at -20°C. The mixture was filtered and dried to obtain 120 mg of the target product with a yield of 29.7%.

$^1$H NMR (500 MHz, DMSO-d6) δ 11.99 (s, 1H), 10.12 (s, 1H), 7.88 (s, 1H), 7.40 (t, *J* = 7.9 Hz, 1H), 7.27 (d, *J* = 7.8 Hz, 1H), 7.23 (t, *J* = 8.7 Hz, 2H), 7.18 (s, 1H), 7.14-7.08 (m, 2H), 6.90 (dd, *J* = 8.1, 2.0 Hz, 1H), 6.70 (s, 2H), 4.04 (t, *J* = 7.0 Hz, 2H), 3.57 (t, *J* = 4.3 Hz, 4H), 3.25-3.20 (m, 1H), 2.32 (s, 4H), 2.24 (t, *J* = 6.7 Hz, 2H), 1.91-1.79 (m, 2H), 1.32 (d, *J* = 7.1 Hz, 6H). MS (ESI) : m/z 560.23 [M + H]$^+$. Mp: 180-182°C.

**Example 3: Synthesis of (3Z,6Z)-3-(3-(p-fluorobenzoyl)phenyl)methylene-6-((5-cyclopropyl-1-(3-morpholinyl)propylimidazol-4-yl)methylene)piperazine-2,5-dione (PLN-2-3)**

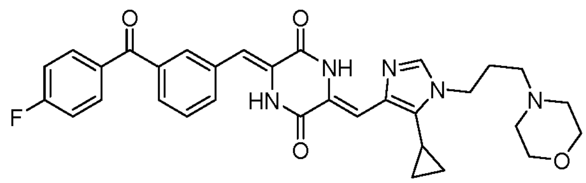

1) To a 50 mL dry round-bottom flask was sequentially added (Z)-1-acetyl-3-((5-cyclopropyl-1H-imidazol-4-yl) methylene)piperazine-2,5-dione (150 mg, 0.55 mmol), 1-chloro-3-morpholinopropane (179.00 mg, 1.09 mmol), cesium carbonate (356.38 mg, 1.09 mmol), potassium iodide (90.79 mg, 0.55 mmol), a 4A molecular sieve (300 mg), and DMF (4 mL). The mixture was degassed, placed in an oil bath at 70°C under nitrogen atmosphere, stirred and reacted for 24 hours. After the reaction was completed, the reaction mixture was transferred to a 100 mL single-necked flask, washed with ethanol, concentrated under reduced pressure, redissolved with EtOH: DCM = 1:5, filtered under reduced pressure, and the filter cake was washed with EtOH: DCM = 1:5, concentrated under reduced pressure to obtain 175.45 mg of a brown solid crude product with a yield of 79.91%. The brown solid crude product was used directly in the next step without purification.

2) To a 25 mL dry brown round-bottom flask was sequentially added (Z)-1-acetyl-3-((5-cyclopropyl-1-(3-morpholinyl) propylimidazol-4-yl)methylene)piperazine-2,5-dione (175.45 mg, 0.44 mmol), 3-p-fluorobenzoylbenzaldehyde (119.82 mg, 0.52 mmol), cesium carbonate (213.84 mg, 0.66 mmol), anhydrous sodium sulfate (124.29 mg, 0.88 mmol), and DMF (4 mL). The mixture was degassed, placed in an oil bath at 45°C under nitrogen atmosphere, stirred and reacted for 18 hours. The reaction was monitored by LC-MS. After the reaction was completed, the reaction mixture was dropped into cold water (40 mL) at 4°C, and filtered under reduced pressure. The filter cake was washed with cold water, dried under reduced pressure, dissolved in methanol and dichloromethane (1:3), filtered, concentrated under reduced pressure, ultrasonically slurried with methanol, left to stand overnight at -30°C, and filtered under reduced pressure. The filter cake was washed with cold methanol, detected by LC-MS showing many impurities, subjected to reversed-phase column chromatography loaded with C18, concentrated under reduced pressure to obtain 61.0 mg of the target product with a yield of 24.48%.

$^1$H NMR (500 MHz, DMSO-$d_6$) δ 11.92 (s, 1H), 10.34 (s, 1H), 7.90 (dd, *J* = 6.59, 9.51 Hz, 3H), 7.81 (s, 1H), 7.74 (d, *J* = 7.63 Hz, 1H), 7.63 (d, *J*= 7.67 Hz, 1H), 7.58 (t, *J* = 7.63 Hz, 1H), 7.40 (t, *J* = 8.77 Hz, 2H), 6.80 (s, 1H), 6.71 (s, 1H), 4.11 (t, *J* = 7.17 Hz, 2H), 3.57 (t, *J* = 4.28 Hz, 4H), 2.33 (s, 4H), 2.27 (t, *J* = 6.66 Hz, 2H), 1.94 (dd, *J* = 6.86, 13.95 Hz, 2H), 1.84-1.72 (m, 1H), 1.05 (dd, *J* = 1.59, 8.13 Hz, 2H), 0.65 (d, *J* = 3.93 Hz, 2H). MS (ESI): m/z 570.25 [M + H]$^+$. Mp: 194-195°C.

**Example 4: Synthesis of (3Z,6Z)-3-(3-p-fluorophenoxy)phenyl)methylene-6-((5-cyclopropyl-1-(3-morpholinyl) propylimidazol-4-yl)methylene)piperazine-2,5-dione (PLN-2-4)**

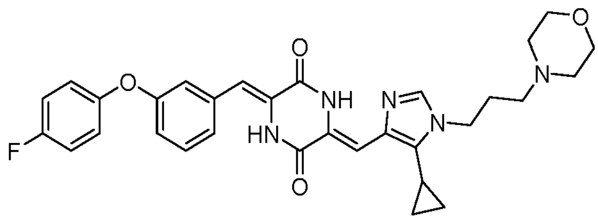

To a 25 mL dry brown round-bottom flask was sequentially added (Z)-1-acetyl-3-((5-cyclopropyl-1-(3-morpholinyl)propyl-imidazol-4-yl)methylene)piperazine-2,5-dione (228.84 mg, 0.57 mmol), 3-(4-fluorophenoxy)benzaldehyde (244 mg, 1.13 mmol), cesium carbonate (424.54 mg, 1.30 mmol), anhydrous sodium sulfate (246.76 mg, 1.74 mmol), and DMF (6 mL). The mixture was degassed, placed in an oil bath at 45°C under nitrogen atmosphere, stirred and reacted for 18 hours. The reaction was monitored by LC-MS. After the reaction was completed, the reaction mixture was transferred to a 100 mL single-necked flask, washed with ethanol, concentrated under reduced pressure, redissolved with EtOH: DCM = 1:3, filtered under reduced pressure, and the filter cake was washed with EtOH: DCM = 1:3, concentrated under reduced pressure, subjected to flash reversed-phase column chromatography loaded with C18 using gradient elution, and concentrated under reduced pressure to obtain 101 mg of the target product with a yield of 16.27%.

$^1$H NMR (500 MHz, DMSO-$d_6$) δ 11.75 (s, 1H), 10.19 (s, 1H), 8.14 (s, 1H), 7.41 (t, *J* = 7.9 Hz, 1H), 7.29 - 7.21 (m, 3H), 7.17 (s, 1H), 7.12 (dd, *J* = 8.9, 4.5 Hz, 2H), 6.91 (d, *J* = 8.3 Hz, 1H), 6.73 (s, 1H), 6.69 (s, 1H), 4.22 (t, *J* = 6.7 Hz, 2H), 3.94 (d, *J* = 12.1 Hz, 2H), 3.84 (dd, *J* = 21.8, 10.4 Hz, 2H), 3.49 - 3.34 (m, 2H), 3.05 (dd, *J* = 28.2, 17.9 Hz, 4H), 2.32 (d, *J* = 7.0 Hz, 2H), 1.85 (s, 1H), 1.07 (d, *J* = 6.9 Hz, 2H), 0.69 (d, *J* = 3.9 Hz, 2H). MS (ESI) : m/z 558.18 [M + H]$^+$. Mp: 197-199°C.

**Example 5: Preparation of (3Z,6Z)-3-benzylidene-6-((5-isopropyl-1-(-3-morpholinyl)propylimidazol-4-yl)methylene)piperazine-2,5-dione (PLN-2-5)**

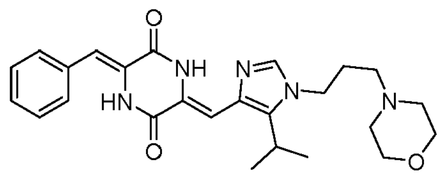

To a 50 mL dry round-bottom flask was sequentially added (Z)-1-acetyl-3-((5-isopropyl-1H-imidazol-4-yl) methylene)piperazine-2,5-dione (300 mg, 1.08 mmol), 1-chloro-3-morpholinopropane (355.37 mg, 2.17 mmol), cesium carbonate (707.55 mg, 2.17 mmol), potassium iodide (180 mg, 1.09 mmol), a 4A molecular sieve (800 mg), and DMF (6 mL). The mixture was degassed, placed in an oil bath at 70°C under nitrogen atmosphere, stirred and reacted for 24 hours. The temperature was naturally cooled to room temperature, then benzaldehyde (115.22 mg, 1.09 mmol) in DMF (2 mL) was added thereto, and the mixture was heated to 45°C in an oil bath, stirred and reacted for 12 hours. The reaction was monitored by LC-MS. After the reaction was completed, the reaction mixture was dropped into cold water (80 mL) at 4°C, and filtered under reduced pressure. The filter cake was washed with cold water, dried under reduced pressure, dissolved in methanol and dichloromethane (1:3), filtered, dried, chromatographed, concentrated under reduced pressure, dried, ultrasonically slurried with methanol, left to stand in a refrigerator at -30°C, and filtered under reduced pressure. The filter cake was washed with cold methanol, dried under vacuum at 50°C to obtain 26.1 mg of the target product with a yield of 5.35%.

$^1$H NMR (600 MHz, DMSO-$d_6$) δ 12.00 (s, 1H), 10.04 (s, 1H), 7.89 (s, 1H), 7.53 (d, *J* = 7.51 Hz, 2H), 7.42 (t, *J* = 7.51

Hz, 2H), 7.32 (t, $J$ = 7.25 Hz, 1H), 6.75 (s, 1H), 6.71 (s, 1H), 4.05 (t, $J$ = 6.88 Hz, 2H), 3.58 (s, 4H), 3.24 (dt, $J$ = 6.93, 13.98 Hz, 1H), 2.27 (dd, $J$ = 24.12, 30.58 Hz, 6H), 1.90-1.80 (m, 2H), 1.33 (d, $J$ = 7.00 Hz, 6H). MS (ESI) : m/z 450.19 $[M + H]^+$. Mp: 198-199°C.

**Example 6: Preparation of (3Z,6Z)-3-benzylidene-6-((5-cyclopropyl-1-(-3-morpholinyl)propylimidazol-4-yl) methylene)piperazine-2,5-dione (PLN-2-6)**

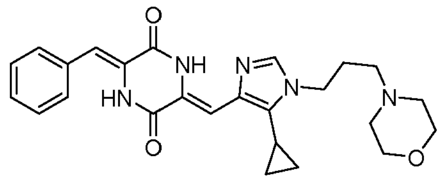

To a 50 mL dry round-bottom flask was sequentially added (Z)-1-acetyl-3-((5-cyclopropyl-1H-imidazol-4-yl) methylene)piperazine-2,5-dione (200 mg, 0.73 mmol), cesium carbonate (475.18 mg, 1.46 mmol), potassium iodide (121.05 mg, 0.73 mmol), a 4A molecular sieve (400 mg), a solution of 1-chloro-3-morpholinopropane (179.00 mg, 1.09 mmol ) in DMF (4 mL). The mixture was degassed, placed in an oil bath at 70°C under nitrogen atmosphere, stirred and reacted for 24 hours. The temperature was cooled to room temperature, then benzaldehyde (115.22 mg, 1.09 mmol) in DMF (2 mL) was added thereto, and the mixture was heated to 45°C in an oil bath, stirred and reacted for 18 hours. The reaction was monitored by LC-MS. After the reaction was completed, the reaction mixture was dropped into cold water (80 mL) at 4°C, and filtered under reduced pressure. The filter cake was washed with cold water, dried under reduced pressure, dissolved in methanol and dichloromethane (1:3), filtered, dried, chromatographed, concentrated under reduced pressure, dried, ultrasonically slurried with methanol, left to stand in a refrigerator at -30°C, and filtered under reduced pressure. The filter cake was washed with cold methanol, dried under vacuum at 50°C to obtain 38.3 mg of the target product with a yield of 11.74%.

$^1$H NMR (600 MHz, DMSO-$d_6$) δ 11.89 (s, 1H), 10.03 (s, 1H), 7.92 (s, 1H), 7.52 (d, $J$ = 7.57 Hz, 2H), 7.41 (t, $J$ = 7.72 Hz, 2H), 7.32 (t, $J$ = 7.38 Hz, 1H), 6.75 (s, 1H), 6.72 (s, 1H), 4.11 (t, $J$ = 7.25 Hz, 2H), 3.57 (t, $J$ = 4.39 Hz, 4H), 2.29 (dd, $J$ = 17.58, 24.30 Hz, 6H), 2.00-1.87 (m, 2H), 1.78 (tt, $J$ = 5.41, 8.29 Hz, 1H), 1.08-0.98 (m, 2H), 0.73-0.60 (m, 2H). MS (ESI) : m/z 448.17 $[M + H]^+$. Mp: 201-203°C.

**Example 7: Synthesis of (3Z,6Z)-3-(2,5-difluorophenyl)methylene-6-((5-isopropyl-1-(3-morpholinyl)propylimidazol-4-yl)methylene)piperazine-2,5-dione (PLN-2-7)**

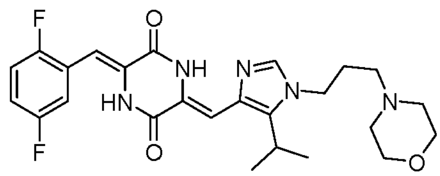

To a 25 mL dry brown round-bottom flask was sequentially added (Z)-1-acetyl-3-((5-isopropyl-1-(3-morpholinyl) propylimidazol-4-yl)methylene)piperazine-2,5-dione (202.75 mg, 0.50 mmol), 2,5-difluorobenzaldehyde (85.69 mg, 0.60 mmol), cesium carbonate (245.57 mg, 0.75 mmol), anhydrous sodium sulfate (142.75 mg, 1.00 mmol), and DMF (4 mL). The mixture was degassed, placed in an oil bath at 45°C under nitrogen atmosphere, stirred and reacted for 19 hours. The reaction was monitored by LC-MS. After the reaction was completed, the reaction mixture was dropped into cold water (40 mL) at 4°C, and filtered under reduced pressure. The filter cake was washed with cold water, dried under reduced pressure, dissolved in methanol and dichloromethane (1:3), filtered, concentrated under reduced pressure, ultrasonically slurried with methanol, left to stand overnight at -30°C, and filtered under reduced pressure. The filter cake was washed with cold methanol, dried under vacuum at 50°C to obtain 110.0 mg of the target product with a yield of 45.09%.

$^1$H NMR (500 MHz, DMSO-$d_6$) δ 12.06 (s, 1H), 10.45 (s, 1H), 7.89 (s, 1H), 7.40 (ddd, $J$ = 3.13, 5.71, 8.93 Hz, 1H), 7.28 (td, $J$ = 4.67, 9.30 Hz, 1H), 7.23-7.16 (m, 1H), 6.73 (s, 1H), 6.61 (s, 1H), 4.05 (t, $J$ = 7.10 Hz, 2H), 3.58 (t, $J$ = 4.43 Hz, 4H), 3.24 (dt, $J$ = 7.05, 14.14 Hz, 1H), 2.33 (s, 4H), 2.25 (t, $J$ = 6.73 Hz, 2H), 1.92-1.76 (m, 2H), 1.33 (d, $J$ = 7.10 Hz, 6H). MS (ESI) : m/z 486.20 $[M + H]^+$. Mp: 187-188°C.

**Example 8: Preparation of (3Z,6Z)-3-(2,5-difluorophenyl)methylene-6-((5-cyclopropyl-1-(-3-morpholinyl)propylimidazol-4-yl)methylene)piperazine-2,5-dione (PLN-2-8)**

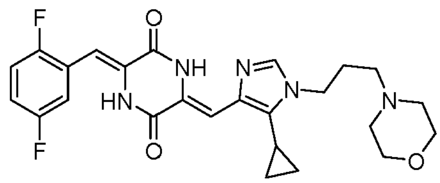

To a 25 mL dry brown round-bottom flask was sequentially added (Z)-1-acetyl-3-((5-cyclopropyl-1-(3-morpholinyl)propylimidazol-4-yl)methylene)piperazine-2,5-dione (585.50 mg, 1.46 mmol), 2,5-difluorobenzaldehyde (207.24 mg, 1.46 mmol), cesium carbonate (712.76 mg, 2.19 mmol), anhydrous sodium sulfate (414.30 mg, 2.92 mmol), and DMF (8 mL). The mixture was degassed, placed in an oil bath at 45°C under nitrogen atmosphere, stirred and reacted for 20 hours. The reaction was monitored by LC-MS. After the reaction was completed, the reaction mixture was dropped into cold water (80 mL) at 4°C, and filtered under reduced pressure. The filter cake was washed with cold water, dried under reduced pressure, dissolved in methanol and dichloromethane (1:3), filtered, concentrated under reduced pressure, dried, chromatographed, concentrated under reduced pressure, dried, ultrasonically slurried with methanol, left to stand in a refrigerator at -30°C, and filtered under reduced pressure. The filter cake was washed with cold methanol, dried under vacuum at 50°C to obtain 159.1 mg of the target product with a yield of 22.56%.

$^1$H NMR (600 MHz, DMSO-$d_6$) δ 11.96 (s, 1H), 10.45 (s, 1H), 7.93 (s, 1H), 7.40 (ddd, *J* = 3.14, 5.68, 8.95 Hz, 1H), 7.28 (td, *J* = 4.65, 9.30 Hz, 1H), 7.23-7.17 (m, 1H), 6.74 (s, 1H), 6.61 (s, 1H), 4.12 (dd, *J* = 9.38, 16.51 Hz, 2H), 3.57 (t, *J* = 4.33 Hz, 4H), 2.29 (dd, *J* = 17.31, 24.02 Hz, 6H), 1.98-1.88 (m, 2H), 1.79 (tt, *J* = 5.45, 8.26 Hz, 1H), 1.11-1.00 (m, 2H), 0.71-0.63 (m, 2H). MS (ESI) : m/z 484.16 [M + H]$^+$. Mp: 223-225°C.

**Example 9: Preparation of (3Z,6Z)-3-fluorophenylmethylene-6-((5-isopropyl-1-(-3-morpholinyl)propylimidazol-4-yl)methylene)piperazine-2,5-dione (PLN-2-9)**

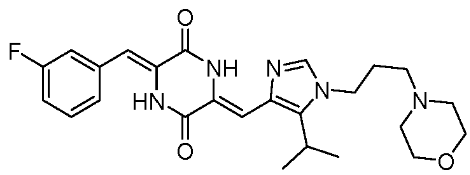

To a 25 mL dry brown round-bottom flask was sequentially added (Z)-1-acetyl-3-((5-isopropyl-1-(3-morpholinyl) propylimidazol-4-yl)methylene)piperazine-2,5-dione (730.14 mg, 1.81 mmol), 3-fluorobenzaldehyde (224.59 mg, 1.81 mmol), cesium carbonate (884.41 mg, 2.71 mmol), anhydrous sodium sulfate (514.08 mg, 3.62 mmol), and DMF (8 mL). The mixture was degassed, placed in an oil bath at 45°C under nitrogen atmosphere, stirred and reacted for 20 hours. The reaction was monitored by LC-MS. After the reaction was completed, the reaction mixture was dropped into cold water (80 mL) at 4°C, and filtered under reduced pressure. The filter cake was washed with cold water, dried under reduced pressure, dissolved in methanol and dichloromethane (1:3), filtered, concentrated under reduced pressure, dried, chromatographed, concentrated under reduced pressure, dried, ultrasonically slurried with methanol, left to stand in a refrigerator at -30°C, and filtered under reduced pressure. The filter cake was washed with cold methanol, dried under vacuum at 50°C to obtain 301.7 mg of the target product with a yield of 35.66%.

$^1$H NMR (600 MHz, DMSO-$d_6$) δ12.02 (s, 1H), 10.27 (s, 1H), 7.88 (d, *J* = 5.34 Hz, 1H), 7.46-7.40 (m, 1H), 7.36 (d, *J* = 10.26 Hz, 1H), 7.34 (dd, *J* = 9.02, 19.44 Hz, 1H), 7.13 (td, *J*= 2.37, 8.57 Hz, 1H), 6.73 (s, 1H), 6.71 (s, 1H), 4.04 (t, *J* = 7.13 Hz, 2H), 3.57 (t, *J* = 4.44 Hz, 4H), 3.24 (dt, *J* = 7.10, 14.24 Hz, 1H), 2.26 (dd, *J* = 24.07, 30.84 Hz, 6H), 1.84 (p, *J* = 6.86 Hz, 2H), 1.33 (d, *J* = 7.13 Hz, 6H). MS (ESI) : m/z 468.18 [M + H]$^+$. Mp: 208-210°C.

**Example 10: Preparation of (3Z,6Z)-3-m-fluorophenylmethylene-6-((5-cyclopropyl-1-(-3-morpholinyl)propylimidazol-4-yl)methylene)piperazine-2,5-dione (PLN-2-10)**

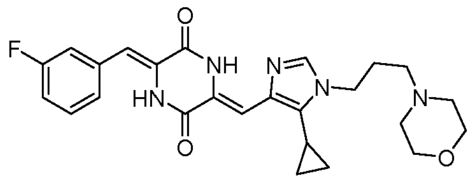

To a 25 mL dry brown round-bottom flask was sequentially added (Z)-1-acetyl-3-((5-cyclopropyl-1-(3-morpholinyl)propylimidazol-4-yl)methylene)piperazine-2,5-dione (585.50 mg, 1.46 mmol), 3-fluorobenzaldehyde (181.00 mg, 1.46 mmol), cesium carbonate (712.76 mg, 2.19 mmol), anhydrous sodium sulfate (414.30 mg, 2.92 mmol), and DMF (8 mL). The mixture was degassed, placed in an oil bath at 45°C under nitrogen atmosphere, stirred and reacted for 21 hours. The reaction was monitored by LC-MS. After the reaction was completed, the reaction mixture was dropped into cold water (80 mL) at 4°C, and filtered under reduced pressure. The filter cake was washed with cold water, dried under reduced pressure, dissolved in methanol and dichloromethane (1:3), filtered, concentrated under reduced pressure, dried, chromatographed, concentrated under reduced pressure, dried, ultrasonically slurried with methanol, left to stand in a refrigerator at -30°C, and filtered under reduced pressure. The filter cake was washed with cold methanol, dried under vacuum at 50°C to obtain 217.6 mg of the target product with a yield of 32.05%.

$^1$H NMR (600 MHz, DMSO-$d_6$) δ 11.92 (s, 1H), 10.27 (s, 1H), 7.92 (d, *J* = 4.20 Hz, 1H), 7.47-7.41 (m, 1H), 7.34 (dd, *J* = 9.06, 19.18 Hz, 2H), 7.14 (td, *J* = 2.34, 8.55 Hz, 1H), 6.73 (s, 2H), 4.11 (t, *J* = 7.24 Hz, 2H), 3.57 (t, *J* = 4.39 Hz, 4H), 2.29 (dd, *J* = 17.58, 24.29 Hz, 6H), 1.97-1.89 (m, 2H), 1.79 (tt, *J* = 5.42, 8.26 Hz, 1H), 1.10-1.00 (m, 2H), 0.67 (dd, *J* = 1.73, 5.38 Hz, 2H). MS (ESI) : m/z 466.15 [M + H]$^+$. Mp: 208-211°C.

**Example 11: Preparation of (3Z,6Z)-3-p-fluorobenzylidene-6-((S-isopropyl-1-(-3-morpholinyl)propylimidazol-4-yl)methylene)piperazine-2,5-dione (PLN-2-11)**

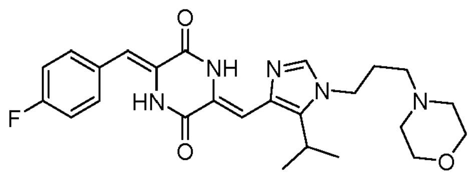

To a 50 mL dry round-bottom flask was sequentially added (Z)-1-acetyl-3-((5-isopropyl-1H-imidazol-4-yl) methylene)piperazine-2,5-dione (300 mg, 1.09 mmol), 1-chloro-3-morpholinopropane (355.37 mg, 2.17 mmol), cesium carbonate (707.55 mg, 2.17 mmol), potassium iodide (180 mg, 1.09 mmol), a 4A molecular sieve (800 mg), and DMF (6 mL). The mixture was degassed, placed in an oil bath at 70°C under nitrogen atmosphere, stirred and reacted for 24 hours. The temperature was naturally cooled to room temperature, then 4-fluorobenzaldehyde (134.76 mg, 1.09 mmol) in DMF (2 mL) was added thereto, and the mixture was heated to 45°C in an oil bath, stirred and reacted for 20 hours. The reaction was monitored by LC-MS. After the reaction was completed, the reaction mixture was dropped into cold water (80 mL) at 4°C, and filtered under reduced pressure. The filter cake was washed with cold water, dried under reduced pressure, dissolved in methanol and dichloromethane (1:3), and filtered. The aqueous phase was extracted with DCM (100 mL * 2), and the organic phases were combined, concentrated under reduced pressure, dried, chromatographed, concentrated under reduced pressure, dried, ultrasonically slurried with methanol, left to stand in a refrigerator at -30°C, and filtered under reduced pressure. The filter cake was washed with cold methanol, dried under vacuum at 50°C to obtain 25.4 mg of the target product with a yield of 5.00%.

$^1$H NMR (600 MHz, DMSO-$d_6$) δ11.99 (s, 1H), 10.16 (s, 1H), 7.89 (s, 1H), 7.57 (dd, *J* = 5.68, 8.47 Hz, 2H), 7.24 (t, *J* = 8.83 Hz, 2H), 6.74 (s, 1H), 6.71 (s, 1H), 4.09-4.01 (m, 2H), 3.58 (t, *J* = 4.18 Hz, 4H), 3.24 (dt, *J* = 7.08, 14.20 Hz, 1H), 2.27 (dd, *J* = 24.15, 30.88 Hz, 6H), 1.88-1.78 (m, 2H), 1.33 (d, *J* = 7.09 Hz, 6H). MS (ESI) : m/z 468.17 [M + H]$^+$. Mp: 195-198°C.

**Example 12: Preparation of (3Z,6Z)-3-(3-(p-fluorobenzoyl)phenyl)methylene-6-((5-isopropyl-1-((2,6-dimethyl)-3-morpholinyl)propylimidazol-4-yl)methylene)piperazine-2,5-dione (PLN-2-1-12)**

One stereoisomer of

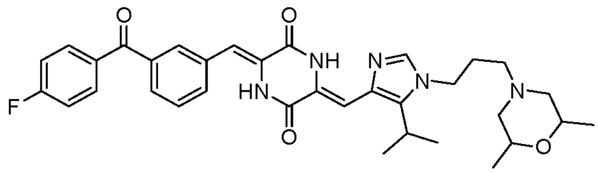

1) To a 100 mL dry round-bottom flask was sequentially added 2,6-dimethylmorpholine (1.0 g, 8.68 mmol), cesium carbonate (3.39 g, 10.42 mmol), potassium iodide (288.25 mg, 1.74 mmol), acetonitrile (40 mL), and 1-chloro-3-bromopropane (2.73 g, 17.4 mmol). The mixture was stirred and reacted in an oil bath at 25°C for 16 hours. The reaction was monitored by TLC (MeOH: DCM=1:10), and the reaction was completed. The mixture was filtered, and the filter cake was washed by EA and concentrated under reduced pressure to obtain 931.7 mg of a light brown oily liquid with a yield of 56.13%.

2) To a 50 mL dry round-bottom flask was sequentially added (Z)-1-acetyl-3-((5-isopropyl-1H-imidazol-4-yl)methylene)piperazine-2,5-dione (300 mg, 1.09 mmol), cesium carbonate (707.55 mg, 2.17 mmol), potassium iodide (180.24 mg, 1.09 mmol), a 4A molecular sieve (500 mg), and a solution of N-3-chloropropyl-2,6-dimethylmorpholine (312.22 mg, 1.63 mmol ) in DMF (6 mL). The mixture was degassed, placed in an oil bath at 70°C under nitrogen atmosphere, stirred and reacted for 24 hours. After the reaction was completed, the temperature was naturally cooled to room temperature, then 3-p-fluorobenzoylbenzaldehyde (198.24 mg, 0.87 mmol) in DMF (2 mL) was added thereto, and the mixture was heated to 45°C in an oil bath, stirred and reacted for 24 hours. The reaction was monitored by LC-MS. After the reaction was completed, the reaction mixture was dropped into cold water (80 mL) at 4°C, and filtered under reduced pressure. The filter cake was washed with cold water, dried under reduced pressure, dissolved in methanol and dichloromethane (1:3), filtered, dried, and chromatographed. The upper point of preparative thin-layer chromatography (MeOH: DCM = 1:20) was product 1, concentrated under reduced pressure, dried, ultrasonically slurried with methanol, left to stand in a refrigerator at -30°C, and filtered under reduced pressure. The filter cake was washed with cold methanol, dried under vacuum at 50°C to obtain 10.7 mg of the target product with a yield of 1.64%. MS (ESI) : m/z 600.29 $[M + H]^+$. Mp: 216-217°C.

HPLC test: chromatographic column: Acclaim™ 120, C18, 5 μm, 4.6 * 150 mm; mobile phase: mobile phase A: MeOH, mobile phase B: 0.1% formic acid aqueous solution, flow rate: 1 mL/min, gradient elution, the conditions are as shown in Table 1 below. The retention time was 6.48 min.

Table 1: Gradient table

| Time (min) | Mobile phase A (%V/V) | Mobile phase B (% V/V) |
|---|---|---|
| 0 | 40 | 60 |
| 0→6 | 40→100 | 60→0 |
| 6→10 | 100→40 | 0→60 |

**Example 13: Preparation of (3Z,6Z)-3-(3-(p-fluorobenzoyl)phenyl)methylene-6-((5-isopropyl-1-((2,6-dimethyl)-3-morpholinyl)propylimidazol-4-yl)methylene)piperazine-2,5-dione (PLN-2-13)**

One stereoisomer of

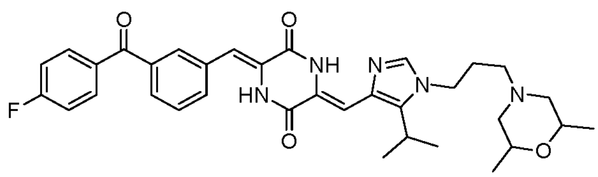

Preparation method referred to the synthesis of compound (3Z,6Z)-3-(3-(p-fluorobenzoyl)phenyl)methylene-6-((5-isopropyl-1-((2,6-dimethyl)-3-morpholinyl)propylimidazol-4-yl)methylene)piperazine-2,5-dione The lower point of preparative thin-layer chromatography (MeOH: DCM = 1:20) was product 2, concentrated under reduced pressure, dried, ultrasonically slurried with methanol, left to stand in a refrigerator at -30°C, and filtered under reduced pressure. The filter cake was washed with cold methanol, dried under vacuum at 50°C to obtain 51.6 mg of the target product with a yield of 7.92%.

$^1H$ NMR (600 MHz, DMSO-$d_6$) δ 12.03 (s, 1H), 10.35 (s, 1H), 7.90 (d, $J$ = 15.6 Hz, 3H), 7.82 (s, 1H), 7.75 (s, 1H), 7.61 (d, $J$ = 26.6 Hz, 2H), 7.41 (s, 2H), 6.81 (s, 1H), 6.71 (s, 1H), 4.04 (s, 2H), 3.55 (s, 1H), 2.76 - 2.63 (m, 2H), 2.23 (s, 2H), 1.98 (s, 2H), 1.85 (s, 2H), 1.56 (s, 2H), 1.32-1.23 (d, $J$ = 48.3 Hz, 12H).

MS (ESI) : m/z 600.30 $[M + H]^+$. Mp: 235-237°C.

HPLC test: chromatographic column: Acclaim™ 120, C18, 5 μm, 4.6 * 150 mm; mobile phase: mobile phase A: MeOH, mobile phase B: 0.1% formic acid aqueous solution, flow rate: 1 mL/min, gradient elution, the conditions are as shown in Table 2 below. The retention time was 6.29 min.

Table 2: Gradient table

| Time (min) | Mobile phase A (%V/V) | Mobile phase B (% V/V) |
|---|---|---|
| 0 | 40 | 60 |
| 0→6 | 40→100 | 60→0 |
| 6→10 | 100→40 | 0→60 |

**Example 14: Preparation of (3Z,6Z)-3-(3-(p-fluorobenzoyl)phenyl)methylene-6-((5-cyclopropyl-1-((2,6-dimethyl)-3-morpholinyl)propylimidazol-4-yl)methylene)piperazine-2,5-dione (PLN-2-14)**

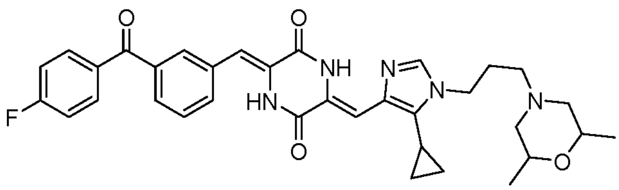

To a 50 mL dry round-bottom flask was sequentially added (Z)-1-acetyl-3-((5-cyclopropyl-1H-imidazol-4-yl) methylene)piperazine-2,5-dione (200 mg, 0.73 mmol), cesium carbonate (475.16 mg, 1.46 mmol), potassium iodide (121.05 mg, 0.73 mmol), a 4A molecular sieve (400 mg), and a solution of N-3-chloropropyl-2,6-dimethylmorpholine (209.68 mg, 1.09 mmol ) in DMF (4 mL). The mixture was degassed, placed in an oil bath at 70°C under nitrogen atmosphere, stirred and reacted for 24 hours. After the reaction was completed, the temperature was naturally cooled to room temperature, then 3-p-fluorobenzoylbenzaldehyde (133.13 mg, 0.58 mmol) in DMF (2 mL) was added thereto, and the mixture was heated to 45°C in an oil bath, stirred and reacted for 24 hours. The reaction was monitored by LC-MS. After the reaction was completed, the reaction mixture was dropped into cold water (80 mL) at 4°C, and filtered under reduced pressure. The filter cake was washed with cold water, dried under reduced pressure, dissolved in methanol and dichloromethane (1:3), filtered, dried, chromatographed, concentrated under reduced pressure, dried, ultrasonically slurried with methanol, left to stand in a refrigerator at -30°C, and filtered under reduced pressure. The filter cake was washed with cold methanol, dried under vacuum at 50°C to obtain 56.8 mg of the target product with a yield of 13.03%.

$^1H$ NMR (600 MHz, DMSO-$d_6$) δ11.93 (s, 1H), 10.35 (s, 1H), 7.94-7.89 (m, 3H), 7.82 (s, 1H), 7.76 (d, $J$ = 7.73 Hz, 1H), 7.64 (d, $J$ = 7.71 Hz, 1H), 7.59 (t, $J$ = 7.65 Hz, 1H), 7.41 (t, $J$ = 8.79 Hz, 2H), 6.81 (s, 1H), 6.72 (s, 1H), 4.11 (dd, $J$ = 8.40, 15.52 Hz, 2H), 3.61-3.53 (m, 2H), 2.73 (d, $J$ = 10.48 Hz, 2H), 2.26 (t, $J$ = 6.67 Hz, 2H), 1.98-1.89 (m, 2H), 1.82-1.74 (m, 1H), 1.55 (t, $J$ = 10.68 Hz, 2H), 1.17-1.10 (m, 1H), 1.05 (dd, $J$ = 6.73, 13.84 Hz, 7H), 0.66 (dt, $J$ = 5.2, 2.8 Hz, 2H). MS (ESI) : m/z 598.28 $[M + H]^+$. Mp: 217-219°C.

**Example 15: Synthesis of (3Z,6Z)-3-(3-(p-fluorobenzoyl)phenyl)methylene-6-((5-isopropyl-1-(3-N-tert-butoxycarbonylpiperazinyl)propylimidazol-4-yl)methylene)piperazine-2,5-dione (PLN-2-15)**

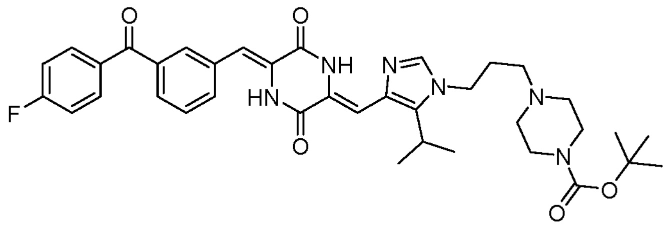

1. Preparation of 4-(3-chloropropyl)-1-tert-butoxycarbonylpiperazine

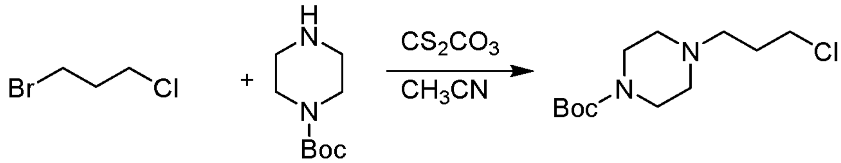


To a 250 mL dry round-bottom flask was sequentially added N-tert-butoxycarbonylpiperazine (1.5 g, 8.05 mmol), cesium carbonate (3.15 g, 9.66 mmol), acetonitrile (40 mL), and 1-chloro-3-bromopropane (2.54 g, 16.11 mmol). The mixture was stirred and reacted in an oil bath at 25°C for 17 hours. The completion of the reaction was monitored by TLC (MeOH: DCM = 1:10). The mixture was filtered, and the filter cake was washed with EA (100 mL * 5), and the mixture was filtered once repeatedly and concentrated under reduced pressure to obtain 1.49 g of a colorless oily liquid with a yield of 60.32%.

2. Preparation of (Z)-1-acetyl-3-((5-isopropyl-1-(3-(4-tert-butoxycarbonylpiperazin-1-yl)propyl)-1H-imidazol-4-yl) methylene)piperazine-2,5-dione

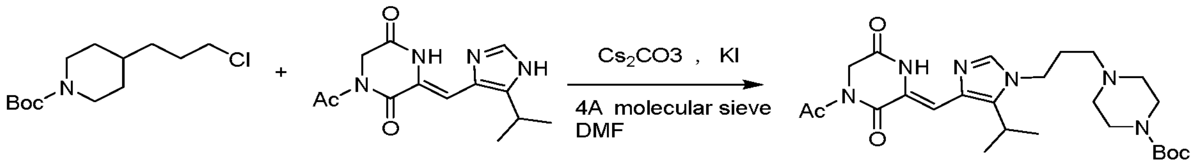


To a 50 mL dry round-bottom flask was sequentially added (Z)-1-acetyl-3-((5-isopropyl-1H-imidazol-4-yl) methylene)piperazine-2,5-dione (300 mg, 1.08 mmol), 4-(3-chloropropyl)-1-tert-butoxycarbonylpiperazine (428 mg, 1.62 mmol), cesium carbonate (707 mg, 2.16 mmol), potassium iodide (180.24 mg, 1.08 mmol), a 4A molecular sieve (500 mg), and DMF (6 mL). The mixture was degassed, placed in an oil bath at 70°C under nitrogen atmosphere, stirred and reacted for 24 hours. The mixture was transferred to a 100 mL single-necked flask, rinsed with ethanol, concentrated under reduced pressure obtain an orange oil. The orange oil was redissolved with a mixed solvent of ethanol and dichloromethane (V ethanol: V dichloromethane = 1:5), filtered under reduced pressure, and the filter cake was rinsed with the mixed solvent (V ethanol: V dichloromethane = 1:5), and concentrated under reduced pressure to obtain 900 mg of an orange oily mixture, which was used directly in the next step without purification.

3) To a 25 mL dry brown round-bottom flask was sequentially added (Z)-1-acetyl-3-((5-isopropyl-1-(3-N-tert-butoxycarbonylpiperazinyl)propyl-imidazol-4-yl)methylene)piperazine-2,5-dione (436.60 mg, 0.87 mmol), 3-p-fluorobenzoylbenzaldehyde (237.90 mg, 1.04 mmol), cesium carbonate (424.54 mg, 1.30 mmol), anhydrous sodium sulfate (246.76 mg, 1.30 mmol), and DMF (6 mL). The mixture was degassed, placed in an oil bath at 45°C under nitrogen atmosphere, stirred and reacted for 20 hours. The reaction was monitored by LC-MS. After the reaction was completed, the reaction mixture was transferred to a 100 mL single-necked flask, washed with ethanol, concentrated under reduced pressure, redissolved with EtOH: DCM = 1:3, filtered under reduced pressure, and the filter cake was washed with EtOH: DCM = 1:3, concentrated under reduced pressure, slurried with methanol, sonicated for 10 min, placed in a refrigerator at -30°C for 2 hours, filtered under reduced pressure. The filter cake was washed with cold methanol, detected by LC-MS, with a purity of 95% or more, and dried under vacuum at 50°C to obtain 326.5 mg of the target product with a yield of 56.04%.

$^1$H NMR (500 MHz, DMSO-$d_6$) δ 11.96 (s, 1H), 10.35 (s, 1H), 7.94-7.82 (m, 5H), 7.58 (dt, *J* = 7.60, 15.12 Hz, 2H), 7.40 (t, *J* = 8.78 Hz, 2H), 6.74 (s, 1H), 6.66 (s, 1H), 4.04 (t, *J* = 7.01 Hz, 2H), 3.23 (dd, *J* = 7.15, 14.23 Hz, 1H), 2.27 (dd, *J* =

5.82, 13.01 Hz, 6H), 1.93-1.79 (m, 2H), 1.39 (s, 9H), 1.33 (d, *J* = 7.06 Hz, 6H). MS (ESI) : m/z 671.22 [M + H]+. Mp: 168-170°C. (The lack of four hydrogens in the NMR spectrum may be contained in the water peak or solvent peak)

**Example 16: Synthesis of (3Z,6Z)-3-(3-(p-fluorobenzoyl)phenyl)methylene-6-((5-isopropyl-1-(3-piperazinyl)propylimidazol-4-yl)methylene)piperazine-2,5-dione (PLN-2-16)**

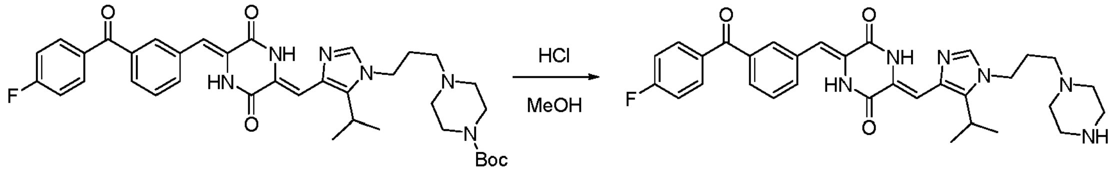


To a 100 mL dry round-bottom flask was sequentially added (3Z,6Z)-3-(3-(p-fluorobenzoyl)phenyl)methylene-6-((5-isopropyl-1-(3-N-tert-butoxycarbonylpiperazinyl)propylimidazol-4-yl)methylene)piperazine-2,5-dione (250 mg, 0.37 mmol), methanol (40 mL), and hydrochloric acid (1.5 mL, 12 mmol/mL), and the mixture was stirred and reacted in an oil bath at 40°C for 7 hours. The reaction was monitored by LC-MS. After the reaction was completed, the mixture was neutralized with saturated sodium carbonate aqueous solution, and the pH of the mixture was neutral, and the mixture was concentrated under reduced pressure to be an oil. The oil was dissolved with 1 mL of methanol, dropped into EA (40 mL), and a solid was precipitated. The mixture was placed in a cold trap at -15°C, stirred for 2 hours, filtered under reduced pressure, and the filter cake was washed with cold EA, dried under vacuum at 50°C to obtain 199 mg of the target product with a yield of 93.56%.

$^1$H NMR (500 MHz, DMSO-$d_6$) δ 12.02 (s, 1H), 7.95-7.85 (m, 3H), 7.82 (s, 1H), 7.75 (d, *J* = 7.41 Hz, 1H), 7.64 (d, *J* = 7.55 Hz, 1H), 7.59 (t, *J* = 7.57 Hz, 1H), 7.40 (t, *J* = 8.62 Hz, 2H), 6.80 (s, 1H), 6.70 (s, 1H), 4.04 (t, *J* = 6.23 Hz, 2H), 3.29-3.18 (m, 1H), 2.89 (s, 4H), 2.42 (s, 4H), 2.26 (t, *J* = 6.13 Hz, 2H), 1.86-1.77 (m, 2H), 1.33 (d, *J* = 6.95 Hz, 6H). MS (ESI): m/z 571.27 [M + H]+. Mp: 190-191°C. (The lack of 2 hydrogens in the NMR spectrum may be contained in the water peak or solvent peak)

**Example 17: (3Z,6Z)-3-(3-(4-Fluorophenoxy)phenyl)methylene-6-((5-isopropyl-1-(3-N-tert-butoxycarbonylpiperazinyl)propylimidazol-4-yl)methylene)piperazine-2,5-dione (PLN-2-17)**

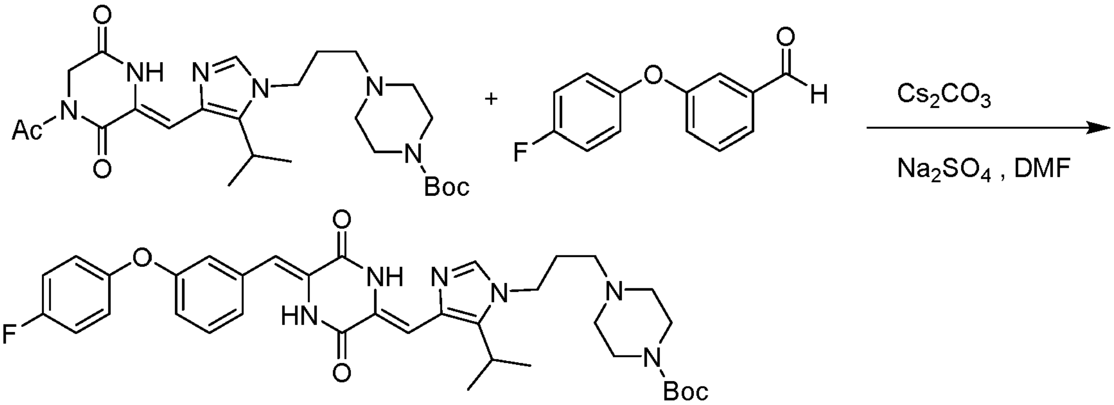


To a 25 mL dry brown round-bottom flask was sequentially added (Z)-1-acetyl-3-((5-isopropyl-1-(3-(4-tert-butoxycarbonylpiperazin-1-yl)propyl)-1H-imidazol-4-yl)methylene)piperazine-2,5-dione (400 mg, 0.79 mmol), 3-(4-fluorophenoxy)benzaldehyde (206 mg, 0.96 mmol), cesium carbonate (389 mg, 1.19 mmol), anhydrous sodium sulfate (226 mg, 1.59 mmol) and DMF (6 mL). The mixture was degassed, placed in an oil bath at 45°C under nitrogen atmosphere, stirred and reacted for 20 hours. The mixture was transferred to a 100 mL single-necked flask, rinsed with ethanol, concentrated under reduced pressure, redissolved with a mixed solvent of ethanol and dichloromethane (V ethanol: V dichloromethane = 1:3), filtered under reduced pressure, and the filter cake was rinsed with the mixed solvent (V ethanol: V dichloromethane = 1:3), concentrated under reduced pressure, subjected to flash column chromatography to obtain 170 mg of the target product with a yield of 32.47%.

$^1H$ NMR (500 MHz, DMSO-*d6*) δ 11.99 (s, 1H), 10.13 (s, 1H), 7.88 (s, 1H), 7.41 (t, *J* = 7.9 Hz, 1H), 7.27 (d, *J* = 7.8 Hz, 1H), 7.23 (t, *J* = 8.7 Hz, 2H), 7.19 (s, 1H), 7.14-7.09 (m, 2H), 6.90 (dd, *J* = 8.2, 1.9 Hz, 1H), 6.70 (s, 2H), 4.04 (t, *J* = 7.0 Hz, 2H), 3.26-3.20 (m, 1H), 2.29-2.25 (m, 6H), 1.91-1.79 (m, 2H), 1.39 (s, 9H), 1.33 (d, *J*= 7.1 Hz, 6H). MS (ESI) : m/z 659.39 [M + H]$^+$. Mp: 148-150°C. (The lack of four hydrogens in the NMR spectrum may be contained in the water peak or solvent peak)

**Example 18: Preparation of (3Z,6Z)-3-(4-fluorophenoxy)phenyl)methylene-6-((5-isopropyl-1-(3-(piperazin-1-yl) propyl)-imidazol-4-yl)methylene)piperazine-2,5-dione (PLN-2-18)**

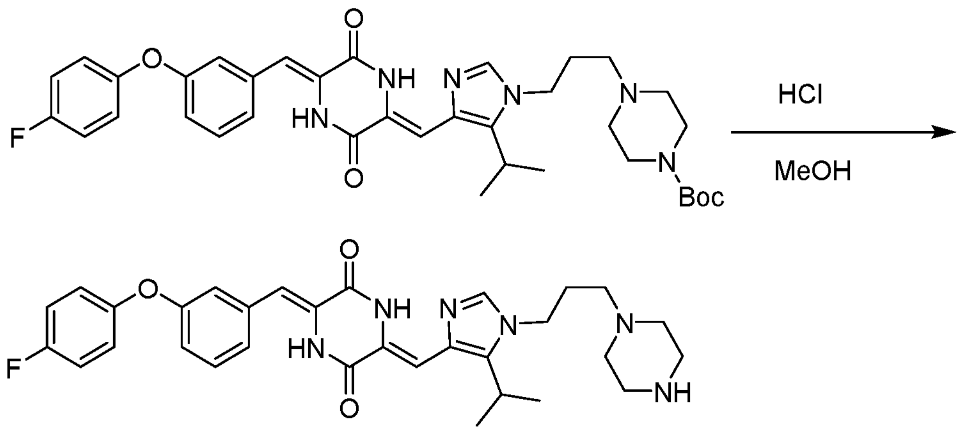


To a 100 mL dry round-bottom flask was sequentially added (3Z,6Z)-3-(3-(4-fluorophenoxy)phenyl)methylene-6-((5-isopropyl-1-(3-N-tert-butoxycarbonylpiperazinyl)propylimidazol-4-yl)methylene)piperazine-2,5-dione (135 mg, 0.20 mmol), methanol (20 mL), and hydrochloric acid (0.75 mL, 12 mmol/mL), and the mixture was stirred and reacted in an oil bath at 40°C for 7 hours. After the reaction was completed, the mixture was neutralized with saturated sodium carbonate aqueous solution, and the pH of the mixture was neutral, and the mixture was concentrated under reduced pressure to be an oil. The oil was dissolved with 1 mL of methanol, dropped into EA (20 mL), and a solid was precipitated. The mixture was placed in a cold trap at -15°C, stirred for 2 hours, filtered under reduced pressure, and the filter cake was washed with cold EA, dried to obtain 80 mg of the target product (3Z,6Z)-3-(4-fluorophenoxy)phenyl) methylene-6-((5-isopropyl-1-(3-(piperazin-1-yl)propyl)-imidazol-4-yl)methylene)piperazine-2,5-dione with a yield of 71.68%. MS (ESI) : m/z 559.25 [M + H]$^+$. Mp: 176-178°C.

**Example 19: Synthesis of (3Z,6Z)-3-(3-p-fluorophenoxy)phenyl)methylene-6-((5-cyclopropyl-1-(piperazinyl) propylimidazol-4-yl)methylene)piperazine-2,5-dione (PLN-2-19)**

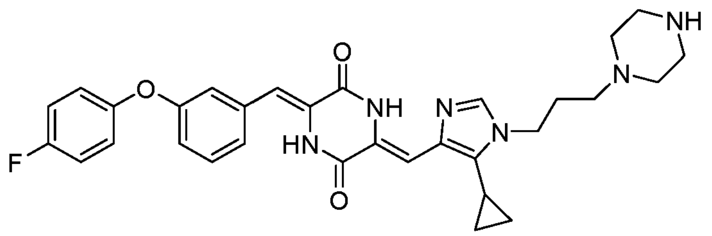

1) To a 250 mL dry round-bottom flask was sequentially added N-tert-butyloxycarbonylpiperazine (1.5 g, 8.05 mmol), acetonitrile (40 mL), cesium carbonate (3.15 g, 9.66 mmol), 1-chloro-3-bromopropane (2.54 g, 16.11 mmol), and the mixture was stirred and reacted in an oil bath at 25°C for 17 hours. The reaction was monitored by TLC (MeOH: DCM = 1:10), and the color changes were observed with an iodine chamber, and the reaction was balanced. The mixture was filtered, and silica gel was spread, and the filter cake was washed with EA (100 mL * 5), and the mixture was filtered once repeatedly to basically remove the raw materials, and concentrated under reduced pressure to obtain 1.49 g of a colorless oily liquid with a yield of 60.32%.

$^1H$ NMR (500 MHz, $CDCl_3$) δ 3.59 (t, J=6.5, 2H), 3.43 - 3.39 (m, 4H), 2.48 (t, J=7.0, 2H), 2.41 - 2.34 (m, 4H), 1.98 - 1.89 (m, 2H), 1.45 (d, J=4.9, 9H).

2) To a 50 mL two-necked dry round-bottom flask was sequentially added (Z)-1-acetyl-3-((5-cyclopropyl-1H-imidazol-4-yl)methylene)piperazine-2,5-dione (180 mg, 0.66 mmol), 1-chloro-3-N-tert-butoxycarbonylpiperazinylpropane (345 mg, 1.31 mmol), cesium carbonate (320 mg, 0.98 mmol), potassium iodide (109 mg, 0.66 mmol), a 4A molecular sieve (300 mg), and DMF (5 mL). The mixture was degassed, placed in an oil bath at 70°C under nitrogen atmosphere, stirred and reacted for 24 hours. After the reaction was completed, the reaction mixture was transferred to a 100 mL single-necked flask, washed with ethanol, concentrated to dryness under reduced pressure, added with 20 mL of dichloromethane and 10 mL of methanol, ultrasonically dispersed, filtered under reduced pressure, and the filter cake was washed with EtOH: DCM = 1:5, concentrated under reduced pressure to obtain a brown solid crude product which was used directly in the next step without purification.

3) To a 25 mL dry brown round-bottom flask was sequentially added the crude product of (Z)-1-acetyl-3-((5-cyclopropyl-1-(3-N-tert-butoxycarbonylpiperazinyl)propyl-imidazol-4-yl)methylene)piperazine-2,5-dione from the previous step, 3-(4-fluorophenoxy)benzaldehyde (155 mg, 0.72 mmol), cesium carbonate (320.6 mg, 0.98 mmol), anhydrous sodium sulfate (186.3 mg, 1.31 mmol) and DMF (4 mL). The mixture was degassed, placed in an oil bath at 45°C under nitrogen atmosphere, stirred and reacted for 18 hours. The reaction was monitored by LC-MS. After the reaction was completed, the reaction mixture was dropped into cold water (40 mL) at 4°C. The solution was a brown transparent liquid, extracted three times with ethyl acetate. The combined organic phases were concentrated to dryness, and 8 mL of methanol was added thereto, ultrasonically dispersed to obtain 152 mg of a yellow solid.

4) To a 25 mL dry flask was added the above yellow solid, methanol (10 mL), and hydrochloric acid (1 mL, 12 mmol/mL). The mixture was stirred and reacted in an oil bath at 40°C for 5 hours. The reaction was monitored by LC-MS. After the reaction was completed, the mixture was neutralized with saturated sodium carbonate aqueous solution, and the pH of the mixture was neutral, and the mixture was concentrated under reduced pressure to be an oil. The oil was dissolved with an appropriate amount of methanol, dropped into EA (40 mL), and a solid was precipitated. The mixture was placed in a cold trap at -15°C, stirred for 2 hours, filtered under reduced pressure, and the filter cake was washed with cold EA, dried under vacuum at 50°C to obtain 107 mg of the target product.

$^1H$ NMR (500 MHz, dmso) δ11.76 (s, 1H), 10.19 (s, 1H), 8.11 (s, 1H), 7.41 (t, *J* = 7.9 Hz, 1H), 7.31-7.20 (m, 3H), 7.17 (s, 1H), 7.12 (dt, *J* = 6.6, 4.1 Hz, 2H), 6.91 (dd, *J* = 8.2, 2.2 Hz, 1H), 6.73 (s, 1H), 6.70 (s, 1H), 4.22 (t, *J* = 6.9 Hz, 2H), 3.89 (s, 4H), 3.48 (d, *J* = 24.5 Hz, 4H), 3.23 - 3.13 (m, 2H), 2.34-2.24 (m, 2H), 1.84 (ddd, *J* = 13.8, 8.3, 5.6 Hz, 1H), 1.12-1.04 (m, 2H), 0.69 (q, *J* = 5.5 Hz, 2H). MS (ESI) : m/z 557.23 $[M + H]^+$. Mp: 183-186°C.

**Example 20: Synthesis of (3Z,6Z)-3-(2,5-difluorophenyl)methylene-6-((5-isopropyl-1-(3-piperazinyl)propylimidazol-4-yl)methylene)piperazine-2,5-dione (PLN-2-20)**

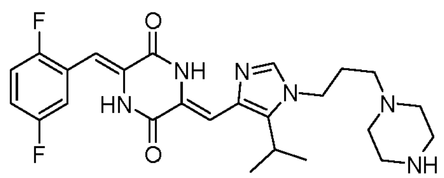

To a 100 mL dry round-bottom flask was sequentially added (3Z,6Z)-3-(2,5-difluorophenyl)methylene-6-((5-isopropyl-1-(3-N-tert-butoxycarbonylpiperazinyl)propylimidazol-4-yl)methylene)piperazine-2,5-dione (142.8 mg, 0.24 mmol), methanol (10 mL), hydrochloric acid (1 mL, 12 mmol/mL), and the mixture was stirred and reacted in an oil bath at 40°C for 5 hours. The reaction was monitored by LC-MS. After the reaction was completed, the mixture was neutralized with saturated sodium carbonate aqueous solution, and the pH of the mixture was neutral, and the mixture was concentrated under reduced pressure to be an oil. The oil was dissolved with methanol, dropped into EA (40 mL), and a solid was precipitated. The mixture was placed in a cold trap at -15°C, stirred for 2 hours, filtered under reduced pressure, and the filter cake was washed with cold EA, dried under vacuum at 50°C to obtain 112.7 mg of the target product with a yield of 95.24%.

$^1H$ NMR (500 MHz, DMSO-$d_6$) δ11.92 (s, 1H), 10.51 (s, 1H), 8.09 (s, 1H), 7.40 (s, 1H), 7.29 (td, *J* = 4.80, 9.37 Hz, 1H), 7.22 (d, *J* = 7.98 Hz, 1H), 6.71 (s, 1H), 6.63 (s, 1H), 4.18 (t, *J* = 6.79 Hz, 2H), 3.70 (s, 2H), 3.57-3.12 (m, 9H), 2.19 (s, 2H), 1.34 (d, *J* = 6.92 Hz, 6H). MS (ESI) : m/z 485.19 $[M + H]^+$. Mp: 214-215°C.

**Example 21: Synthesis of (3Z,6Z)-3-(3-(p-fluorobenzoyl)phenyl)methylene-6-((5-isopropyl-1-(3-N-methylpiperazinyl)propylimidazol-4-yl)methylene)piperazine-2,5-dione (PLN-2-21)**

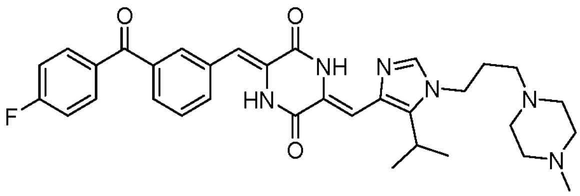

1) To a 250 mL dry round-bottom flask was sequentially added methylpiperazine (2.0 g, 19.97 mmol), acetonitrile (50 mL), cesium carbonate (6.29 g, 19.31 mmol), 1-chloro-3-bromopropane (6.29 g, 39.94 mmol), and the mixture was stirred and reacted in an oil bath at 25°C for 17 hours. The reaction was monitored by TLC (MeOH: DCM = 1:10), and the color changes were observed with an iodine chamber, and the reaction was balanced. The mixture was filtered, and silica gel was spread, and the filter cake was washed with EA. The mixture was filtered once repeatedly to basically remove the raw materials, and concentrated under reduced pressure to obtain 1.67 g of a colorless oily liquid with a yield of 47.31%.

$^1$H NMR (500 MHz, $CDCl_3$) δ 3.58 (t, J=6.6, 2H), 2.46 (dd, J=24.4, 17.2, 10H), 2.28 (d, J=8.9, 3H), 2.08 - 1.82 (m, 2H).

2) To a 50 mL dry round-bottom flask was sequentially added (Z)-1-acetyl-3-((5-isopropyl-1H-imidazol-4-yl)methylene)piperazine-2,5-dione (200 mg, 0.73 mmol), 1-chloro-3-N-methylpiperazinylpropane (255.79 mg, 1.45 mmol), cesium carbonate (471.69 mg, 1.45 mmol), potassium iodide (120.16 mg, 0.73 mmol), a 4A molecular sieve (300 mg), and DMF (4 mL). The mixture was degassed, placed in an oil bath at 70°C under nitrogen atmosphere, stirred and reacted for 24 hours. After the reaction was completed, the reaction mixture was transferred to a 100 mL single-necked flask, washed with ethanol, concentrated under reduced pressure, redissolved with EtOH: DCM = 1:5, filtered under reduced pressure, and the filter cake was washed with EtOH: DCM = 1:5, concentrated under reduced pressure to obtain 580 mg (241.22 mg) of a brown solid crude product with a yield of 80.00%. The brown solid crude product was used directly in the next step without purification.

3) To a 25 mL dry brown round-bottom flask was sequentially added (Z)-1-acetyl-3-((5-isopropyl-1-(3-N-methylpiperazinyl)propyl-imidazol-4-yl)methylene)piperazine-2,5-dione (241.22 mg, 0.58 mmol), 3-p-fluorobenzoylbenzaldehyde (158.59 mg, 0.69 mmol), cesium carbonate (283.04 mg, 0.87 mmol), anhydrous sodium sulfate (164.52 mg, 1.16 mmol), and DMF (4 mL). The mixture was degassed, placed in an oil bath at 45°C under nitrogen atmosphere, stirred and reacted for 20 hours. The reaction was monitored by LC-MS. After the reaction was completed, the reaction mixture was transferred to a 100 mL single-necked flask, washed with ethanol, concentrated under reduced pressure, redissolved with EtOH: DCM = 1:3, filtered under reduced pressure, and the filter cake was washed with EtOH: DCM = 1:3, concentrated under reduced pressure, subjected to flash reversed-phase column chromatography loaded with C18 using gradient elution to obtain a final product, and the eluent containing the final product was concentrated under reduced pressure to obtain 120 mg of the target product with a yield of 35.44%.

$^1$H NMR (500 MHz, DMSO-$d_6$) δ 11.94 (s, 1H), 10.38 (s, 1H), 8.00 (s, 1H), 7.91 (t, *J* = 6.62 Hz, 2H), 7.82 (s, 1H), 7.75 (d, *J* = 7.48 Hz, 1H), 7.64 (d, *J* = 7.67 Hz, 1H), 7.59 (t, *J* = 7.49 Hz, 1H), 7.41 (t, *J* = 8.48 Hz, 2H), 6.81 (s, 1H), 6.70 (s, 1H), 4.16 (s, 2H), 3.71-3.62 (m, 4H), 3.44 (s, 3H), 3.31-3.10 (m, 4H), 2.82 (s, 3H), 2.18 (s, 2H), 1.34 (d, *J* = 6.92 Hz, 6H). MS (ESI) : m/z 585.31 [M + H]$^+$. Mp: 102-104°C.

**Example 22: Synthesis of (3Z,6Z)-3-(3-(p-fluorophenoxy)phenyl)methylene-6-((5-isopropyl-1-(3-N-methylpiperazinyl)propylimidazol-4-yl)methylene)piperazine-2,5-dione (PLN-2-22)**

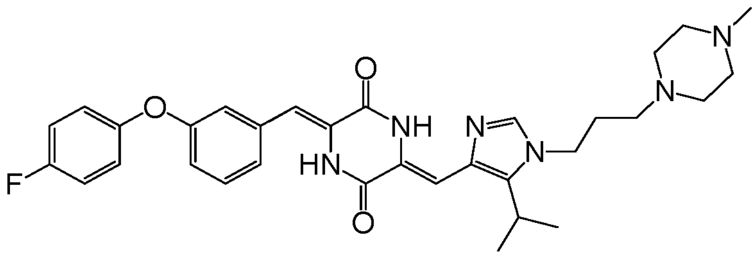

To a 25 mL dry brown round-bottom flask was sequentially added (Z)-1-acetyl-3-((5-isopropyl-1-(3-N-methylpiperazinyl)propyl-imidazol-4-yl)methylene)piperazine-2,5-dione (241.22 mg, 0.58 mmol), 3-p-fluorophenoxybenzaldehyde (150.24 mg, 0.69 mmol), cesium carbonate (283.04 mg, 0.87 mmol), anhydrous sodium sulfate (164.52 mg, 1.16 mmol), and DMF (4 mL). The mixture was degassed, placed in an oil bath at 45°C under nitrogen atmosphere, stirred and reacted for 20 hours. The reaction was monitored by LC-MS. After the reaction was completed, the reaction mixture was transferred to a 100 mL single-necked flask, washed with ethanol, concentrated under reduced pressure, redissolved with EtOH: DCM = 1:3, filtered under reduced pressure, and the filter cake was washed with EtOH: DCM = 1:3, concentrated under reduced pressure, subjected to flash reversed-phase column chromatography loaded with C18 using gradient elution to obtain a final product, and the eluent containing the final product was concentrated under reduced pressure to obtain 73 mg of the target product with a yield of 21.49%.

$^1$H NMR (500 MHz, DMSO-$d_6$) δ 12.00 (s, 1H), 10.13 (s, 1H), 7.87 (s, 1H), 7.41 (t, *J* = 7.93 Hz, 1H), 7.29 - 7.21 (m, 3H), 7.18 (s, 1H), 7.12 (dd, *J* = 4.46, 8.94 Hz, 2H), 6.90 (d, *J* = 7.99 Hz, 1H), 6.70 (d, *J* = 7.13 Hz, 2H), 4.03 (t, *J*= 6.98 Hz, 2H), 3.23 (dd, *J* = 7.17, 14.15 Hz, 1H), 2.26 (dd, *J* = 20.91, 27.56 Hz, 10H), 2.14 (s, 3H), 1.89 - 1.75 (m, 2H), 1.33 (d, *J* = 7.04 Hz, 6H). MS (ESI) : m/z 573.31 [M + H]$^+$. Mp: 181-183°C.

**Example 23: Synthesis of (3Z,6Z)-3-(3-p-fluorophenoxy)phenyl)methylene-6-((5-cyclopropyl-1-(3-N-methylpiperazinyl)propylimidazol-4-yl)methylene)piperazine-2,5-**dione (PLN-2-23)

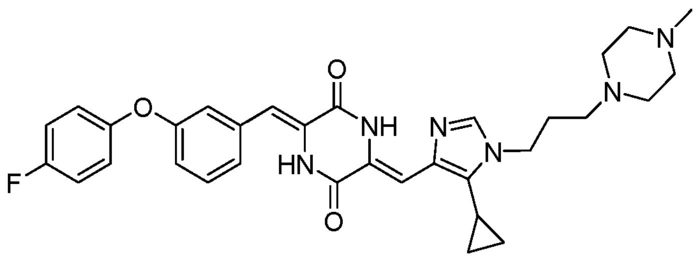

1) To a 50 mL dry round-bottom flask was sequentially added (Z)-1-acetyl-3-((5-cyclopropyl-1H-imidazol-4-yl) methylene)piperazine-2,5-dione (180 mg, 0.66 mmol), 1-chloro-3-N-methylpiperazinylpropane (232 mg, 1.31 mmol), cesium carbonate (427 mg, 1.31 mmol), potassium iodide (109 mg, 0.66 mmol), a 4A molecular sieve (300 mg), and DMF (5 mL). The mixture was degassed, placed in an oil bath at 70°C under nitrogen atmosphere, stirred and reacted for 24 hours. After the reaction was completed, the reaction mixture was transferred to a 100 mL single-necked flask, washed with ethanol, concentrated under reduced pressure, added with 20 mL of dichloromethane and 10 mL of methanol, ultrasonically dispersed, filtered under reduced pressure, and the filter cake was washed with EtOH: DCM = 1:5, concentrated under reduced pressure to obtain a brown solid crude product which was used directly in the next step without purification.

2) To a 25 mL dry brown round-bottom flask was sequentially added the crude product of (Z)-1-acetyl-3-((5-cyclopropyl-1-3-N-methylpiperazinyl)propyl-imidazol-4-yl)methylene)piperazine-2,5-dione from the previous step, 3-(4-fluorophenoxy)benzaldehyde (155 mg, 0.72 mmol), cesium carbonate (320.6 mg, 0.98 mmol), anhydrous sodium sulfate (186.3 mg, 1.31 mmol), and DMF (4 mL). The mixture was degassed, placed in an oil bath at 45°C under nitrogen atmosphere, stirred and reacted for 18 hours. The reaction was monitored by LC-MS. After the reaction was completed, the reaction mixture was dropped into cold water (40 mL) at 4°C. The solution was a brown transparent liquid, extracted three times with ethyl acetate. The combined organic phases were concentrated, and subjected to reverse-phase chromatography loaded with C18 to obtain 135 mg of the target product with a total yield of 35.19 %.

$^1H$ NMR (500 MHz, DMSO-$d_6$) δ 11.81 (s, 1H), 10.15 (s, 1H), 7.87 (s, 1H), 7.46-7.27 (m, 3H), 7.22 (t, *J* = 8.7 Hz, 2H), 7.10 (dd, *J* = 8.9, 4.4 Hz, 2H), 6.84 (d, *J* = 6.8 Hz, 1H), 6.63 (d, *J* = 15.1 Hz, 2H), 4.08 (t, *J* = 7.2 Hz, 2H), 2.31-2.25 (m, 10H), 2.14 (s, 3H), 1.92 (dt, *J* = 13.6, 6.7 Hz, 2H), 1.77 (ddd, *J* = 13.6, 8.0, 5.5 Hz, 1H), 1.04 (q, *J* = 5.5 Hz, 2H), 0.65 (d, *J* = 4.2 Hz, 2H). MS (ESI) : m/z 571.27 [M + H]$^+$. Mp: 214-216°C.

**Example 24: Synthesis of (3Z,6Z)-3-(3-(-(2,5-difluorophenyl)methylene-6-((5-isopropyl-1-(3-N-methylpiperazinyl)propylimidazol-4-yl)methylene)piperazine-2,5-dione (PLN-2-24)**

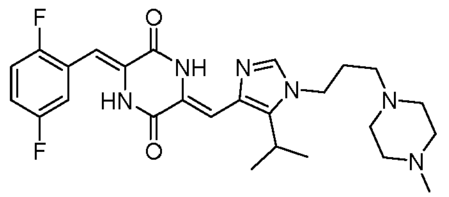

To a 25 mL dry brown round-bottom flask was sequentially added (Z)-1-acetyl-3-((5-isopropyl-1-(3-N-methylpiperazinyl)propyl-imidazol-4-yl)methylene)piperazine-2,5-dione (241.22 mg, 0.58 mmol), 2,5-difluorobenzaldehyde (98.75 mg, 0.69 mmol), cesium carbonate (283.04 mg, 0.87 mmol), anhydrous sodium sulfate (164.52 mg, 1.16 mmol), and DMF (4 mL). The mixture was degassed, placed in an oil bath at 45°C under nitrogen atmosphere, stirred and reacted for 21 hours. The reaction was monitored by LC-MS. After the reaction was completed, the reaction mixture was dropped into cold water (40 mL) at 4°C, and filtered under reduced pressure. The filter cake was washed with cold water, dried under reduced pressure, dissolved in methanol and dichloromethane (1:3), filtered, concentrated under reduced pressure, ultrasonically slurried with methanol, left to stand overnight at -30°C, and filtered under reduced pressure. The filter cake was washed with cold methanol, dried under vacuum at 50°C to obtain 48.6 mg of the target product with a yield of 16.83%.

$^1H$ NMR (500 MHz, DMSO-$d_6$) δ 12.06 (s, 1H), 10.45 (s, 1H), 7.88 (s, 1H), 7.41 (ddd, *J* = 3.11, 5.62, 8.87 Hz, 1H), 7.28 (td, *J* = 4.67, 9.30 Hz, 1H), 7.24-7.15 (m, 1H), 6.72 (s, 1H), 6.61 (s, 1H), ), 4.03 (t, *J* = 7.07 Hz, 2H), 3.24 (dt, *J* = 7.10, 14.22 Hz, 1H), 2.44-2.08 (m, 10H), 1.88-1.77 (m, 2H), 1.33 (d, *J* = 7.09 Hz, 6H). MS (ESI) : m/z 499.22 [M + H]$^+$. Mp: 204-206°C. (The lack of 3 hydrogens in the $^1H$ NMR spectrum contained in the water peak or solvent peak).

**Example 25: Preparation of (3Z,6Z)-3-(3-(p-fluorobenzoyl)phenyl)methylene-6-((5-isopropyl-1-(3-N-benzylpiperazinyl)propylimidazol-4-yl)methylene)piperazine-2,5-dione (PLN-2-25)**

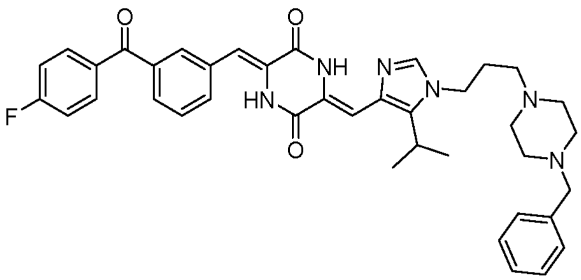

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (80.0 mg, 0.14 mmol), potassium carbonate (38.75 mg, 0.28 mmol), and DMF (1.5 mL), benzyl bromide (23.98 mg, 0.14 mmol) in DMF (1.5 mL) was added dropwise thereto at room temperature, and the mixture was continuously stirred and reacted for 3.0 hours. The reaction mixture was dropped into cold water at 4°C, and a solid was precipitated. The mixture was filtered under reduced pressure, and the filter cake was washed with water, and the mixture was dried under vacuum at 50°C, chromatographed, and concentrated under reduced pressure to obtain 50.2 mg of the target product with a yield of 54.22%.

$^1H$ NMR (600 MHz, DMSO-$d_6$) δ 12.02 (s, 1H), 10.35 (s, 1H), 7.91 (dd, *J* = 5.65, 8.17 Hz, 2H), 7.87 (s, 1H), 7.82 (s, 1H), 7.75 (d, *J* = 7.50 Hz, 1H), 7.64 (d, *J* = 7.54 Hz, 1H), 7.58 (t, *J* = 7.59 Hz, 1H), 7.40 (t, *J* = 8.65 Hz, 2H), 7.34-7.22 (m, 5H), 6.81 (s, 1H), 6.70 (s, 1H), 4.03 (t, *J* = 6.66 Hz, 2H), 3.45 (dd, *J* = 6.49, 11.66 Hz, 2H), 3.23 (dt, *J* = 7.00, 14.07 Hz, 1H), 2.32 (d, *J* = 75.64 Hz, 10H), 1.83 (s, 2H), 1.32 (d, *J* = 7.03 Hz, 6H). MS (ESI) : m/z 661.49 [M + H]$^+$. Mp: 199-203°C.

**Example 26: Preparation of (3Z,6Z)-3-(3-(p-fluorobenzoyl)phenyl)methylene-6-((5-isopropyl-1-(3-N-(p-fluorophenyl)piperazinyl)propylimidazol-4-yl)methylene)piperazine-2,5-dione (PLN-2-26)**

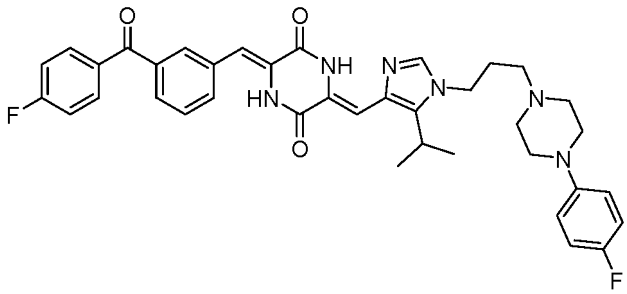

1) To a 100 mL dry round-bottom flask was sequentially added 1-(4-fluorophenyl)piperazine (1.0 g, 5.55 mmol), cesium carbonate (2.17 g, 6.66 mmol), potassium iodide (184.21 mg, 1.11 mmol), acetonitrile (40 mL), and 1-chloro-3-bromopropane (1.75 g, 11.10 mmol). The mixture was stirred and reacted in an oil bath at 25°C for 22 hours. The completion of the reaction was monitored by TLC (MeOH: DCM = 1:10). The mixture was filtered, and the filter cake was washed by EA, and the mixture was filtered once repeatedly and concentrated under reduced pressure to obtain 643.9 mg of a light brown oily liquid with a yield of 45.35%.

2) To a 50 mL dry round-bottom flask was sequentially added (Z)-1-acetyl-3-((5-isopropyl-1H-imidazol-4-yl)methylene)piperazine-2,5-dione (200 mg, 0.73 mmol), cesium carbonate (475.18 mg, 1.46 mmol), potassium iodide (121.05 mg, 0.73 mmol), a 4A molecular sieve (400 mg), and a solution of 4-(3-chloropropyl)-1-(4-fluorophenyl) piperazine (280.83 mg, 1.09 mmol) in DMF (5 mL). The mixture was degassed, placed in an oil bath at 70°C under nitrogen atmosphere, stirred and reacted for 24 hours. After the reaction was completed, the temperature was cooled to room temperature, then 3-p-fluorobenzoylbenzaldehyde (166.42 mg, 0.73 mmol) in DMF (2 mL) was added thereto, and the mixture was heated to 45°C in an oil bath, stirred and reacted for 20 hours. The reaction was monitored by LC-MS. After the reaction was completed, the reaction mixture was dropped into cold water (70 mL) at 4°C, and filtered under reduced pressure. The filter cake was washed with cold water, dried under reduced pressure, dissolved in methanol and dichloromethane (1:3), filtered, dried, ultrasonically slurried with methanol, left to stand in a refrigerator at -30°C, filtered under reduced pressure. The filter cake was washed with cold methanol, dried under vacuum at 50°C to obtain 70.0 mg of the target product with a yield of 14.44%.

$^1$H NMR (400 MHz, DMSO-$d_6$) ) δ 12.04 (s, 1H), 10.36 (s, 1H), 7.92 (d,*J* = 8.17 Hz, 3H), 7.82 (s, 1H), 7.75 (d, *J* = 7.63 Hz, 1H), 7.61 (dt, *J* = 7.32, 14.90 Hz, 2H), 7.40 (t, *J* = 8.05 Hz, 2H), 7.03 (t, *J* = 7.98 Hz, 2H), 6.95 (d, *J* = 2.61 Hz, 2H), 6.81 (s, 1H), 6.71 (s, 1H), 4.07 (s, 2H), 3.25 (d, *J* = 6.50 Hz, 1H), 3.08 (s, 4H), 2.31 (d, *J* = 5.54 Hz, 2H), 1.87 (d, *J* = 5.62 Hz, 2H), 1.33 (d, *J* = 6.62 Hz, 6H). (The lack of 4 hydrogens in the $^1$H NMR spectrum contained in the water peak or solvent peak). MS (ESI) : m/z 665.39 [M + H]$^+$. Mp: 247-250°C.

**Example 27: Preparation of (3Z,6Z)-3-(3-(p-fluorobenzoyl)phenyl)methylene-6-((5-isopropyl-1-(3-N-(4-(4,4,5,5-tetramethyl-1,3,2-dioxaborolan-2-yl))benzylpiperazinyl)propylimidazol-4-yl)methylene)piperazine-2,5-dione (PLN-2-27)**

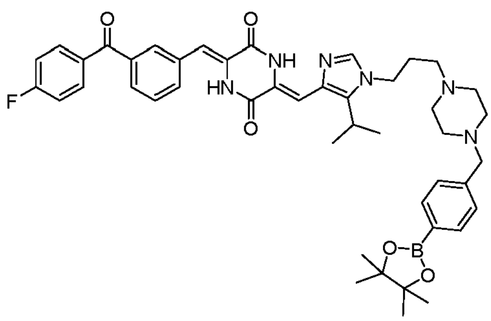

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (100.0 mg, 0.18 mmol), potassium carbonate (36.31 mg, 0.26 mmol), anhydrous sodium sulfate (49.77 mg, 0.35 mmol), and DMF (2 mL). The mixture was placed in an oil bath at 45°C, then 4-bromomethylphenylboronic acid pinacol ester (137.19 mg, 0.99 mmol) in DMF (2 mL) was added dropwise thereto, and the mixture was continuously stirred and reacted for 2.0 hours. The reaction mixture was dropped into cold water at 4°C, and a solid was precipitated. The mixture was filtered under reduced pressure, and the filter cake was washed with water, and the mixture was dried under vacuum at 50°C, chromatographed, and concentrated under reduced pressure to obtain 57.2 mg of the target product with a yield of 41.50%.

$^{1}H$ NMR (600 MHz, DMSO-*$d_6$*) δ12.02 (s, 1H), 10.35 (s, 1H), 7.94-7.89 (m, 2H), 7.87 (s, 1H), 7.82 (s, 1H), 7.75 (d, *J* = 7.75 Hz, 1H), 7.61 (dq, *J* = 7.80, 15.26 Hz, 4H), 7.40 (t, *J* = 8.78 Hz, 2H), 7.30 (d, *J* = 7.86 Hz, 2H), 6.81 (s, 1H), 6.70 (s, 1H), 4.02 (t, *J* = 6.99 Hz, 2H), 3.47 (s, 2H), 3.21 (d, *J* = 7.14 Hz, 1H), 2.27 (dd, *J* = 35.31, 41.86 Hz, 10H), 1.86-1.78 (m, 2H), 1.36-1.24 (m, 18H). MS (ESI) : m/z 787.41 $[M + H]^+$. Mp: 148-150°C.

**Example 28: Preparation of (3Z,6Z)-3-(3-(p-fluorobenzoyl)phenyl)methylene-6-((5-isopropyl-1-(3-N-p-(4,4,5,5-tetramethyl-1,3,2-dioxaborolan-2-yl)benzyloxycarbonylpiperazinyl)propylimidazol-4-yl)methylene)piperazine-2,5-dione** (PLN-2-28)

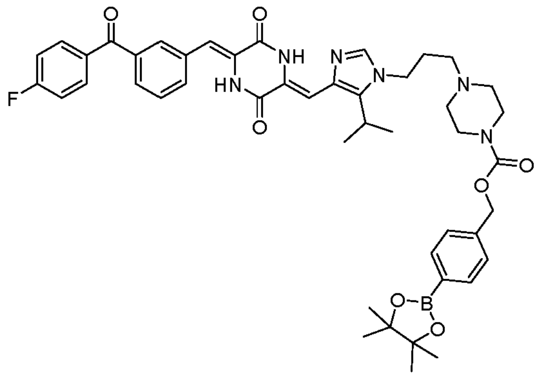

1) To a 25 mL dry round-bottom flask was sequentially added p-nitrophenyl chloroformate (516.60 mg, 2.56 mmol), THF (10 mL), 4-(hydroxymethyl)phenylboronic acid pinacol ester (500 mg, 2.14 mmol). The mixture was placed in a cold trap at 0°C and stirred for 5 min, then a solution of triethylamine (432.3 mg, 4.27 mmol) in THF (10 mL) was added dropwise thereto. When the dropwise addition was completed for 5 min, the mixture was moved to room temperature, stirred and reacted for 1 hour. The reaction was monitored by TLC (EA: PE = 1:1). After the reaction was completed, the mixture was diluted, washed with 1 M hydrochloric acid (20 mL * 2), washed with sodium bicarbonate (20 mL * 2), and the aqueous phase was back-extracted with EA (50 mL * 1). The organic phase was dried over anhydrous sodium sulfate and subjected to column chromatography to obtain 489.6 mg of a white solid with a yield of 57.42%.

2) To a 25 mL dry brown round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (100 mg, 0.18 mmol), phenyl p-nitrocarboxylate p-boronic acid pinacol ester (104.9 mg, 0.26 mmol), HOBt (4.74 mg, 0.035 mmol), and then DIPEA (45.29 mg, 0.35 mmol) in DMF (4 mL) was added thereto, and the mixture was stirred and reacted at room temperature for 0.5 hours. The reaction was monitored by LC-MS, and the reaction was completed. The reaction mixture was dropped into PE (40 mL), and a solid was precipitated. The mixture was filtered under reduced pressure, and the filter cake was washed with PE to obtain a yellow solid, and then dried. The mixture was ultrasonically slurried with methanol, left to stand in a refrigerator at -30°C for 2 hours or more, filtered under reduced pressure. The filter cake was washed with cold methanol to obtain 95 mg of the target product with a yield of 65.27%.

$^{1}H$ NMR (600 MHz, DMSO-*$d_6$*) δ 12.03 (s, 1H), 10.35 (s, 1H), 7.96-7.88 (m, 3H), 7.96-7.88 (m, 1H), 7.82 (s, 1H), 7.76 (d, *J* = 7.46 Hz, 3H), 7.66 (dd, *J* = 7.62, 18.61 Hz, 1H), 7.59 (t, *J* = 7.59 Hz, 2H), 7.45-7.33 (m, 2H), 6.81 (s, 1H), 6.71 (s, 1H), 5.10 (s, 2H), 4.05 (t, *J* = 6.64 Hz, 2H), 3.44 (dd, *J* = 6.85, 12.20 Hz, 4H), 3.24 (dt, *J* = 6.85, 13.96 Hz, 1H), 2.29 (dd, *J* = 16.05, 22.01 Hz, 6H), 1.91-1.79 (m, 2H), 1.41-1.25 (m, 18H). MS (ESI) : m/z 831.49 $[M + H]^+$. Mp: 153-155°C.

**Example 29: Synthesis of (3Z,6Z)-3-(3-(p-fluorobenzoyl)phenyl)methylene-6-((5-isopropyl-1-(3-N-formylpiperazinyl)propylimidazol-4-yl)methylene)piperazine-2,5-dione (PLN-2-29)**

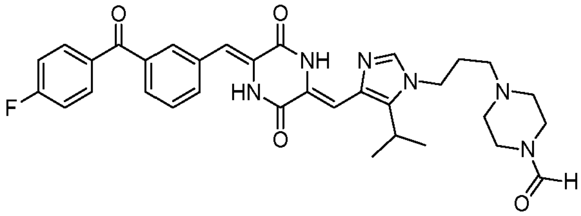

To a 100 mL dry round-bottom flask was sequentially added (3Z,6Z)-3-(3-(p-fluorobenzoyl)phenyl)methylene-6-((5-isopropyl-1-(3-N-tert-butoxycarbonylpiperazinyl)propylimidazol-4-yl)methylene)piperazine-2,5-dione (250 mg, 0.37 mmol), methanol (40 mL), and hydrochloric acid (1.5 mL, 12 mmol/mL). The mixture was stirred and reacted in an oil bath at 40°C for 7 hours. The reaction was monitored by LC-MS. After the reaction was completed, the mixture was concentrated under reduced pressure, subjected to reversed-phase column chromatography (MeOH: $H_2O$ (5% formic acid)) loaded with C18, concentrated under reduced pressure, slurried with methanol, filtered under reduced pressure, and the filter cake was washed with cold methanol and dried under vacuum at 50°C to obtain 150 mg of the target product with a yield of 67.23%.

$^1H$ NMR (500 MHz, DMSO-$d_6$) δ 12.02 (s, 1H), 10.34 (s, 1H), 7.99 (s, 1H), 7.95 - 7.88 (m, 3H), 7.82 (s, 1H), 7.75 (d, *J* = 7.59 Hz, 1H), 7.64 (d, *J* = 7.67 Hz, 1H), 7.59 (t, *J* = 7.61 Hz, 1H), 7.40 (t, *J* = 8.69 Hz, 2H), 6.81 (s, 1H), 6.71 (s, 1H), 4.06 (t, *J* = 6.93 Hz, 2H), 3.44-3.35 (m, 4H), 3.24 (dt, *J* = 6.95, 14.02 Hz, 1H), 2.35 (s, 2H), 2.30 (d, *J* = 5.97 Hz, 4H), 1.86 (dd, *J* = 6.66, 13.40 Hz, 2H), 1.33 (d, *J* = 7.05 Hz, 6H). MS (ESI) : m/z 599.29 $[M + H]^+$. Mp: 244-246°C.

**Example 30: Synthesis of (3Z,6Z)-3-(3-(p-fluorobenzoyl)phenyl)methylene-6-((5-isopropyl-1-(3-N-acetylpiperazinyl)propylimidazol-4-yl)methylene)piperazine-2,5-dione (PLN-2-30)**

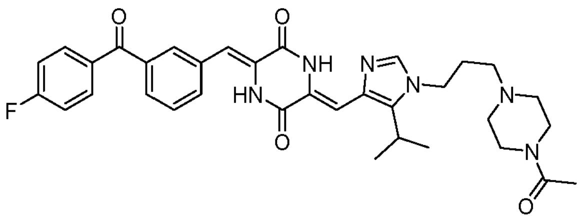

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (150 mg, 0.26 mmol), dichloromethane (2.5 mL), and triethylamine (39.90 mg, 0.39 mmol). The mixture was placed in a cold trap at 0°C and stirred for 5 min, then a solution of acetyl chloride (20.63 mg, 0.26 mmol) in dichloromethane (2.5 mL) was added dropwise thereto, and the mixture was stirred and reacted for 1 hour. The reaction was monitored by LC-MS. After the reaction was completed, the mixture was quenched with methanol, concentrated under reduced pressure, subjected to reversed-phase column chromatography loaded with C18 using gradient elution, concentrated under reduced pressure, ultrasonically slurried with methanol, left to stand in a refrigerator at -30°C for 2 hours or more, filtered under reduced pressure. The filter cake was washed with cold methanol to obtain 135.4 mg of the target product with a yield of 84.09%.

$^1H$ NMR (500 MHz, DMSO-$d_6$) δ 12.02 (s, 1H), 10.34 (s, 1H), 7.91 (m, 3H), 7.82 (s, 1H), 7.75 (d, *J* = 7.66 Hz, 1H), 7.64 (d, *J* = 7.67 Hz, 1H), 7.58 (t, *J* = 7.63 Hz, 1H), 7.40 (t, *J* = 8.76 Hz, 2H), 6.80 (s, 1H), 6.70 (s, 1H), 4.05 (t, *J* = 7.03 Hz, 2H), 3.46-3.39 (m, 4H), 3.23 (dd, *J* = 7.02, 14.11 Hz, 1H), 2.39-2.32 (m, 2H), 2.27 (t, *J* = 5.32 Hz, 4H), 1.97 (s, 3H), 1.89-1.80 (m, 2H), 1.33 (d, *J* = 7.08 Hz, 6H). MS (ESI) : m/z 613.36 $[M + H]^+$. Mp: 195-197°C.

**Example 31: Preparation of (3Z,6Z)-3-(3-(p-fluorobenzoyl)phenyl)methylene-6-((5-isopropyl-1-(3-N-propionylpiperazinyl)propylimidazol-4-yl)methylene)piperazine-2,5-dione (PLN-2-31)**

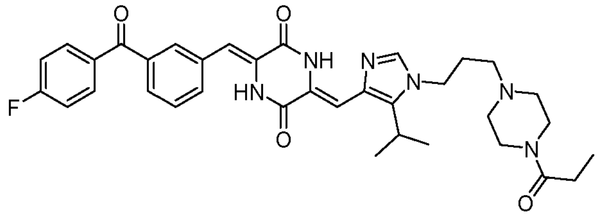

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (70 mg, 0.12 mmol), a solution of triethylamine (18.62 mg, 0.18 mmol) in dichloromethane (2 mL). The mixture was placed in a cold trap at 0°C and stirred for 5 min, then a solution of propionyl chloride (13.62 mg, 0.15 mmol) in dichloromethane (2 mL) was added dropwise thereto, and the mixture was stirred and reacted for 1 hour. The reaction was monitored by LC-MS. After the reaction was completed, the mixture was quenched with methanol, concentrated under reduced pressure, dried, concentrated under reduced pressure, dried, ultrasonically slurried with EA, left to stand in a refrigerator at -30°C, filtered under reduced pressure. The filter cake was washed with cold EA and dried under vacuum at 50°C to obtain 27.8 mg of the target product with a yield of 36.15%.

$^1$H NMR (600 MHz, DMSO-$d_6$) δ12.02 (s, 1H), 10.34 (s, 1H), 7.93-7.87 (m, 3H), 7.82 (s, 1H), 7.75 (d, *J* = 7.69 Hz, 1H), 7.63 (d, *J* = 7.67 Hz, 1H), 7.58 (t, *J* = 7.64 Hz, 1H), 7.40 (t, *J*= 8.75 Hz, 2H), 6.80 (s, 1H), 6.70 (s, 1H), 4.05 (t, *J* = 7.02 Hz, 2H), 3.47-3.38 (m, 4H), 3.24 (dt, *J* = 7.10, 14.24 Hz, 1H), 2.36-2.24 (m, 8H), 1.85 (dd, *J* = 6.78, 13.63 Hz, 2H), 1.32 (d, *J* = 7.10 Hz, 6H), 0.97 (t, *J* = 7.41 Hz, 3H). MS (ESI) : m/z 627.36 [M + H]$^+$. Mp: 207-209°C.

**Example 32: Preparation of (3Z,6Z)-3-(3-(p-fluorobenzoyl)phenyl)methylene-6-((5-isopropyl-1-(3-N-isobutyrylpiperazinyl)propylimidazol-4-yl)methylene)piperazine-2,5-dione (PLN-2-32)**

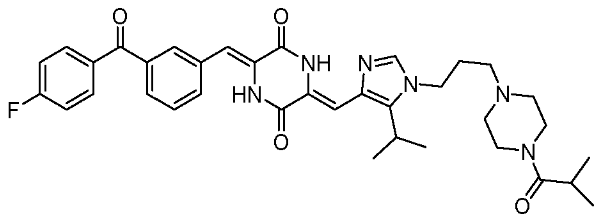

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (90 mg, 0.16 mmol) and a solution of triethylamine (23.94 mg, 0.24 mmol) in dichloromethane (15 mL). A solution of isobutyryl chloride (16.80 mg, 0.16 mmol) in dichloromethane (3 mL) was added dropwise thereto at room temperature, and the mixture was stirred and reacted for 1 hour. The reaction was monitored by LC-MS. After the reaction was completed, the mixture was quenched with methanol, concentrated under reduced pressure, dried, chromatographed, concentrated under reduced pressure, dried, ultrasonically slurried with EA, left to stand in a refrigerator at -30°C, filtered under reduced pressure. The filter cake was washed with cold EA and dried under vacuum at 50°C to obtain 49.4 mg of the target product with a yield of 48.89%.

$^1$H NMR (600 MHz, DMSO-$d_6$) δ12.03 (s, 1H), 10.35 (s, 1H), 7.91 (dd, *J* = 6.58, 9.63 Hz, 3H), 7.82 (s, 1H), 7.75 (d, *J* = 7.70 Hz, 1H), 7.64 (d, *J* = 7.68 Hz, 1H), 7.59 (t, *J* = 7.64 Hz, 1H), 7.40 (t, *J* = 8.77 Hz, 2H), 6.81 (s, 1H), 6.71 (s, 1H), 4.06 (t, *J* = 7.02 Hz, 2H), 3.47 (d, *J* = 17.54 Hz, 4H), 3.24 (dt, *J* = 7.09, 14.25 Hz, 1H), 2.85 (dt, *J* = 6.73, 13.46 Hz, 1H), 2.29 (dd, *J* = 20.95, 27.64 Hz, 6H), 1.90-1.82 (m, 2H), 1.33 (d, *J* = 7.09 Hz, 6H), 0.98 (d, *J* = 6.73 Hz, 6H). MS (ESI) : m/z 641.40 [M + H]$^+$. Mp: 215-217°C.

**Example 33: Preparation of (3Z,6Z)-3-(3-(p-fluorobenzoyl)phenyl)methylene-6-((5-isopropyl-1-(3-N-tert-butyrylpiperazinyl)propylimidazol-4-yl)methylene)piperazine-2,5-dione (PLN-2-33)**

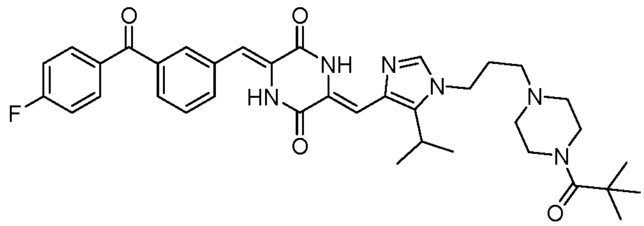

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (80 mg, 0.14 mmol) and a solution of triethylamine (21.28 mg, 0.21 mmol) in dichloromethane (15 mL). A solution of trimethylacetyl chloride (16.91 mg, 0.14 mmol) in dichloromethane (3 mL) was added dropwise thereto at room temperature, and the mixture was stirred and reacted for 1 hour. The reaction was monitored by LC-MS. After the reaction was completed, the mixture was quenched with methanol, concentrated under reduced pressure, dried, chromatographed, concentrated under reduced pressure, dried, ultrasonically slurried with EA, left to stand in a refrigerator at -30°C, filtered under reduced pressure. The filter cake was washed with cold EA and dried under vacuum at 50°C to obtain 60.9 mg of the target product with a yield of 66.34%.

$^1$H NMR (600 MHz, DMSO-$d_6$) $\delta$12.03 (s, 1H), 10.35 (s, 1H), 7.94-7.88 (m, 3H), 7.82 (s, 1H), 7.75 (d, $J$ = 7.69 Hz, 1H), 7.64 (d, $J$ = 7.67 Hz, 1H), 7.59 (t, $J$ = 7.63 Hz, 1H), 7.40 (t, $J$ = 8.76 Hz, 2H), 6.81 (s, 1H), 6.71 (s, 1H), 4.06 (t, $J$ = 7.02 Hz, 2H), 3.54 (s, 4H), 3.24 (dt, $J$ = 7.08, 14.25 Hz, 1H), 2.27 (dd, $J$ = 16.93, 23.59 Hz, 6H), 1.89-1.81 (m, 2H), 1.33 (d, $J$ = 7.10 Hz, 6H), 1.18 (s, 9H). MS (ESI) : m/z 655.43 [M + H]$^+$. Mp: 167-169°C.

**Example 34: Preparation of (3Z,6Z)-3-(3-(p-fluorobenzoyl)phenyl)methylene-6-((5-isopropyl-1-(3-N-(2-ethylbutyryl)piperazinyl)propylimidazol-4-yl)methylene)piperazine-2,5-dione (PLN-2-34)**

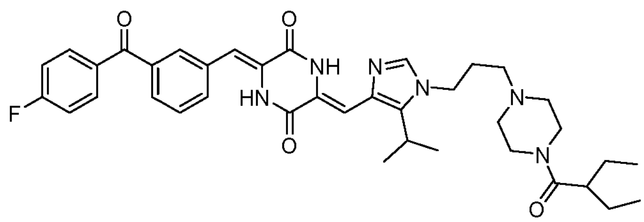

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (80 mg, 0.14 mmol) and a solution of triethylamine (21.28 mg, 0.21 mmol) in dichloromethane (15 mL). A solution of 2-ethylbutyryl chloride (18.87 mg, 0.14 mmol) in dichloromethane (3 mL) was added dropwise thereto at room temperature, and the mixture was stirred and reacted for 1 hour. The reaction was monitored by LC-MS. After the reaction was completed, the mixture was quenched with methanol, concentrated under reduced pressure, dried, chromatographed, concentrated under reduced pressure, dried, ultrasonically slurried with EA, left to stand in a refrigerator at -30°C, filtered under reduced pressure. The filter cake was washed with cold EA and dried under vacuum at 50°C to obtain 71.1 mg of the target product with a yield of 75.82%.

$^1$H NMR (600 MHz, DMSO-$d_6$) $\delta$12.03 (s, 1H), 10.36 (s, 1H), 7.92 (dd, $J$ = 6.39, 8.96 Hz, 3H), 7.82 (s, 1H), 7.76 (d, $J$ = 7.53 Hz, 1H), 7.64 (d, $J$ = 7.58 Hz, 1H), 7.59 (t, $J$ = 7.60 Hz, 1H), 7.41 (t, $J$ = 8.67 Hz, 2H), 6.81 (s, 1H), 6.71 (s, 1H), 4.06 (t, $J$ = 6.80 Hz, 2H), 3.53 (d, $J$= 17.04 Hz, 4H), 3.24 (dd, $J$ = 7.05, 14.13 Hz, 1H), 2.66-2.57 (m, 1H), 2.29 (dd, $J$ = 15.97, 22.71 Hz, 6H), 1.90-1.82 (m, 2H), 1.49 (dt, $J$ = 7.60, 14.88 Hz, 2H), 1.34 (d, $J$ = 7.02 Hz, 8H), 0.78 (t, $J$ = 7.35 Hz, 6H). MS (ESI) : m/z 669.51 [M + H]$^+$. Mp: 153-155°C.

**Example 35: Preparation of (3Z,6Z)-3-(3-(p-fluorobenzoyl)phenyl)methylene-6-((5-isopropyl-1-(3-N-cyclopropanecarbonylpiperazinyl)propylimidazol-4-yl)methylene)piperazine-2,5-dione (PLN-2-35)**

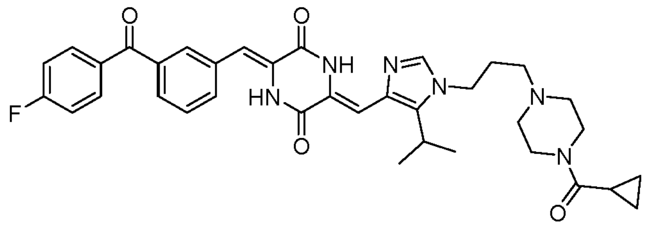

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (80 mg, 0.14 mmol) and a solution of triethylamine (21.28 mg, 0.21 mmol) in dichloromethane (15 mL). A solution of cyclopropanecarbonyl chloride (14.66 mg, 0.14 mmol) in dichloromethane (3 mL) was added dropwise thereto at room temperature, and the mixture was stirred and reacted for 1 hour. The reaction was monitored by LC-MS. After the reaction was completed, the mixture was quenched with methanol, concentrated under reduced pressure, dried, chromatographed, concentrated under reduced pressure, dried, ultrasonically slurried with EA, left to stand in a refrigerator at -30°C, filtered under reduced pressure. The filter cake was washed with cold EA and dried under vacuum at 50°C to obtain 65.1 mg of the target product with a yield of 72.70%.

$^1$H NMR (600 MHz, DMSO-$d_6$) δ 12.02 (s, 1H), 10.35 (s, 1H), 7.91 (dd, $J$=4.90, 8.01 Hz, 3H), 7.82 (s, 1H), 7.75 (d, $J$=7.71 Hz, 1H), 7.63 (d, $J$=7.69 Hz, 1H), 7.58 (t, $J$=7.64 Hz, 1H), 7.40 (t, $J$=8.77 Hz, 2H), 6.80 (s, 1H), 6.70 (s, 1H), 4.06 (t, $J$=7.04 Hz, 2H), 3.66 (s, 2H), 3.48 (d, $J$=27.53 Hz, 2H), 3.24 (dt, $J$=7.10, 14.27 Hz, 1H), 2.31 (dd, $J$=28.27, 34.84 Hz, 6H), 1.95 (ddd, $J$=4.80, 7.84, 12.79 Hz, 1H), 1.89-1.82 (m, 2H), 1.33 (d, $J$=7.09 Hz, 6H), 0.73 - 0.65 (m, 4H). MS (ESI) : m/z 639.41 $[M + H]^+$. Mp: 219-221°C.

**Example 36: Preparation of (3Z,6Z)-3-(3-(p-fluorobenzoyl)phenyl)methylene-6-((5-isopropyl-1-(3-N-cyclobutanoylpiperazinyl)propylimidazol-4-yl)methylene)piperazine-2,5-dione (PLN-2-36)**

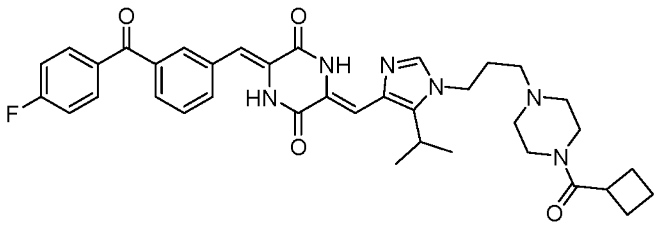

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (80 mg, 0.14 mmol) and a solution of triethylamine (21.28 mg, 0.21 mmol) in dichloromethane (15 mL). A solution of cyclobutanecarbonyl chloride (16.62 mg, 0.14 mmol) in dichloromethane (3 mL) was added dropwise thereto at room temperature, and the mixture was stirred and reacted for 1 hour. The reaction was monitored by LC-MS. After the reaction was completed, the mixture was quenched with methanol, concentrated under reduced pressure, dried, chromatographed, concentrated under reduced pressure, dried, ultrasonically slurried with EA, left to stand in a refrigerator at -30°C, filtered under reduced pressure. The filter cake was washed with cold EA and dried under vacuum at 50°C to obtain 55.6 mg of the target product with a yield of 60.75%.

$^1$H NMR (600 MHz, DMSO-$d_6$) δ12.02 (s, 1H), 10.35 (s, 1H), 7.91 (dd, $J$ = 6.03, 13.80 Hz, 3H), 7.82 (s, 1H), 7.75 (d, $J$ = 7.34 Hz, 1H), 7.64 (d, $J$ = 7.40 Hz, 1H), 7.59 (t, $J$ = 7.54 Hz, 1H), 7.40 (t, $J$ = 8.51 Hz, 2H), 6.81 (s, 1H), 6.70 (s, 1H), 4.05 (t, $J$ = 6.52 Hz, 2H), 3.43 (s, 2H), 3.31 (d, $J$ = 8.13 Hz, 2H), 3.24 (dd, $J$ = 6.94, 13.92 Hz, 1H), 2.32-2.23 (m, 6H), 2.17-1.81 (m, 8H), 1.72 (d, $J$ = 9.52 Hz, 1H), 1.33 (d, $J$ = 6.91 Hz, 6H). MS (ESI) : m/z 653.41 $[M + H]^+$. Mp: 208-210°C.

**Example 37: Preparation of (3Z,6Z)-3-(3-(p-fluorobenzoyl)phenyl)methylene-6-((5-isopropyl-1-(3-N-cyclopentanecarbonylpiperazinyl)propylimidazol-4-yl)methylene)piperazine-2,5-dione (PLN-2-37)**

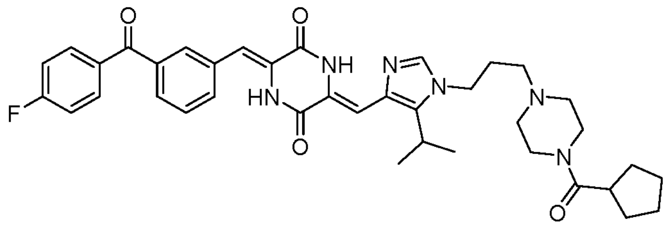

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (80 mg, 0.14 mmol) and a solution of triethylamine (21.28 mg, 0.21 mmol) in dichloromethane (15 mL). A solution of cyclopentanecarbonyl chloride (18.59 mg, 0.14 mmol) in dichloromethane (3 mL) was added dropwise thereto at room temperature, and the mixture was stirred and reacted for 1 hour. The reaction was monitored by LC-MS. After the reaction was completed, the mixture was quenched with methanol, concentrated under reduced pressure, dried, chromatographed, concentrated under reduced pressure, dried, ultrasonically slurried with EA, left to stand in a refrigerator at -30°C, filtered under reduced pressure. The filter cake was washed with cold EA and dried under vacuum at 50°C to obtain 65.5 mg of the target product with a yield of 70.06%.

$^1H$ NMR (400 MHz,DMSO-$d_6$) δ 12.03 (s, 1H), 10.39 (s, 1H), 7.92 (dd, *J* = 2.01, 9.13 Hz, 3H), 7.82 (s, 1H), 7.75 (d, *J* = 7.60 Hz, 1H), 7.60 (dt, *J* = 7.57, 15.06 Hz, 2H), 7.46-7.36 (m, 2H), 6.80 (s, 1H), 6.70 (s, 1H), 4.05 (t, *J* = 6.58 Hz, 2H), 3.47 (d, *J* = 12.06 Hz, 4H), 3.29-3.19 (m, 1H), 2.37-2.22 (m, 6H), 1.87-1.81 (m, 2H), 1.72 (d, *J* = 7.15 Hz, 2H), 1.66-1.46 (m, 7H), 1.33 (d, *J* = 6.95 Hz, 6H). MS (ESI) : m/z 667.42 $[M + H]^+$. Mp: 211-214°C.

### Example 38: Preparation of (3Z,6Z)-3-(3-(p-fluorobenzoyl)phenyl)methylene-6-((5-isopropyl-1-(3-N-cyclohexanecarbonylpiperazinyl)propylimidazol-4-yl)methylene)piperazine-2,5-dione (PLN-2-38)

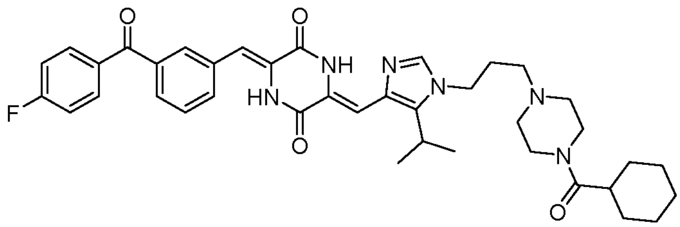

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (80 mg, 0.14 mmol) and a solution of triethylamine (21.28 mg, 0.21 mmol) in dichloromethane (15 mL). A solution of cyclohexanecarboxylic acid chloride (20.55 mg, 0.14 mmol) in dichloromethane (3 mL) was added dropwise thereto at room temperature, and the mixture was stirred and reacted for 1 hour. The reaction was monitored by LC-MS. After the reaction was completed, the mixture was quenched with methanol, concentrated under reduced pressure, dried, chromatographed, concentrated under reduced pressure, dried, ultrasonically slurried with EA, left to stand in a refrigerator at -30°C, filtered under reduced pressure. The filter cake was washed with cold EA and dried under vacuum at 50°C to obtain 64.3 mg of the target product with a yield of 67.37%.

$^1H$ NMR (600 MHz, DMSO-$d_6$) δ12.03 (s, 1H), 10.35 (s, 1H), 7.90 (s, 3H), 7.82 (s, 1H), 7.75 (s, 1H), 7.61 (d, *J* = 26.87 Hz, 2H), 7.40 (s, 2H), 6.81 (s, 1H), 6.71 (s, 1H), 4.06 (s, 2H), 3.45 (d, *J* = 17.08 Hz, 4H), 3.24 (s, 1H), 2.31 (d, *J* = 35.10 Hz, 6H), 1.85 (s, 2H), 1.64 (d, *J* = 48.67 Hz, 6H), 1.30 (dd, *J* = 21.19, 40.78 Hz, 11H). MS (ESI) : m/z 681.46 $[M + H]^+$. Mp: 170-172°C.

### Example 39: Preparation of (3Z,6Z)-3-(3-(p-fluorobenzoyl)phenyl)methylene-6-((5-isopropyl-1-(3-N-adamantanecarbonylpiperazinyl)propylimidazol-4-yl)methylene)piperazine-2,5-dione (PLN-2-39)

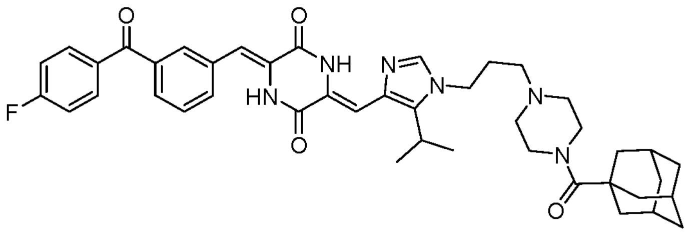

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (80 mg, 0.14 mmol) and a solution of triethylamine (21.28 mg, 0.21 mmol) in dichloromethane (15 mL). A solution of 1-adamantanecarbonyl chloride (27.86 mg, 0.14 mmol) in dichloromethane (3 mL) was added dropwise thereto at room temperature, and the mixture was stirred and reacted for 1 hour. The reaction was monitored by LC-MS. After the reaction was completed, the mixture was quenched with methanol, concentrated under reduced pressure, dried, chromatographed, concentrated under reduced pressure, dried, ultrasonically slurried with EA, left to stand in a refrigerator at -30°C, filtered under reduced pressure. The filter cake was washed with cold EA and dried under vacuum at 50°C to obtain 65.7 mg of the target product with a yield of 63.94%.

$^1$H NMR (600 MHz, DMSO-*d*$_6$) δ12.03 (s, 1H), 10.35 (s, 1H), 7.95-7.88 (m, 3H), 7.82 (s, 1H), 7.75 (d, *J* = 7.73 Hz, 1H), 7.64 (d, *J* = 7.69 Hz, 1H), 7.59 (t, *J* = 7.64 Hz, 1H), 7.40 (t, *J* = 8.77 Hz, 2H), 6.81 (s, 1H), 6.71 (s, 1H), 4.06 (t, *J* = 7.00 Hz, 2H), 3.58 (s, 4H), 3.22 (d, *J* = 7.14 Hz, 1H), 2.35-2.24 (m, 6H), 1.96 (s, 3H), 1.91-1.81 (m, 8H), 1.67 (q, *J* = 12.05 Hz, 6H), 1.33 (d, *J* = 7.09 Hz, 6H). MS (ESI) : m/z 733.46 [M + H]$^+$. Mp: 181-183°C.

**Example 40: Preparation of (3Z,6Z)-3-(3-(p-fluorobenzoyl)phenyl)methylene-6-((5-isopropyl-1-(3-N-(3,3-dimethyl)acryloylpiperazinyl)propylimidazol-4-yl)methylene)piperazine-2,5-dione (PLN-2-40)**

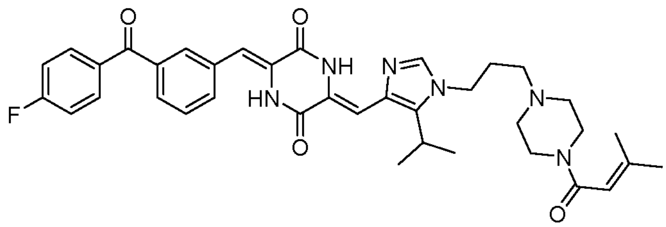

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (80 mg, 0.14 mmol) and a solution of triethylamine (21.28 mg, 0.21 mmol) in dichloromethane (15 mL). A solution of 3-methylcrotonoyl chloride (16.62 mg, 0.14 mmol) in dichloromethane (3 mL) was added dropwise thereto at room temperature, and the mixture was stirred and reacted for 1 hour. The reaction was monitored by LC-MS. After the reaction was completed, the mixture was quenched with methanol, concentrated under reduced pressure, dried, chromatographed, concentrated under reduced pressure, dried, ultrasonically slurried with EA, left to stand in a refrigerator at -30°C, filtered under reduced pressure. The filter cake was washed with cold EA and dried under vacuum at 50°C to obtain 70.3 mg of the target product with a yield of 76.81%.

$^1$H NMR (600 MHz, DMSO-*d*$_6$) δ12.02 (s, 1H), 10.35 (s, 1H), 7.94-7.89 (m, 3H), 7.82 (s, 1H), 7.75 (d, *J* = 7.62 Hz, 1H), 7.64 (d, *J* = 7.64 Hz, 1H), 7.59 (t, *J* = 7.62 Hz, 1H), 7.40 (t, *J* = 8.73 Hz, 2H), 6.81 (s, 1H), 6.70 (s, 1H), 5.88 (s, 1H), 4.06 (t, *J* = 6.87 Hz, 2H), 3.44 (dt, *J* = 6.92, 11.94 Hz, 4H), 3.24 (dt, *J* = 7.07, 14.20 Hz, 1H), 2.29 (d, *J* = 19.70 Hz, 6H), 1.81 (t, *J* = 20.17 Hz, 8H), 1.33 (d, *J* = 7.06 Hz, 6H). MS (ESI) : m/z 653.41 [M + H]$^+$. Mp: 160-162°C.

**Example 41: Synthesis of (3Z,6Z)-3-(3-(p-fluorobenzoyl)phenyl)methylene-6-((5-isopropyl-1-(3-N-benzoylpiperazinyl)propylimidazol-4-yl)methylene)piperazine-2,5-dione (PLN-2-41)**

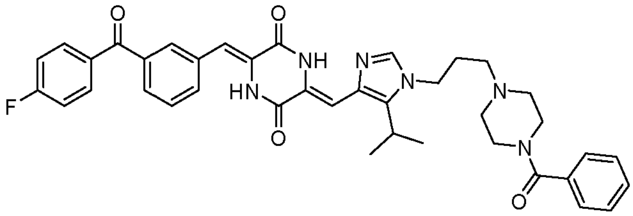

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (150 mg, 0.26 mmol), dichloromethane (2.5 mL), and triethylamine (39.90 mg, 0.39 mmol). The mixture was placed in a cold trap at 0°C and stirred for 5 min, then a solution of benzoyl chloride (36.94 mg, 0.26 mmol) in dichloromethane (2.5 mL) was added dropwise thereto, and the mixture was stirred and reacted for 1 hour. The reaction was monitored by LC-MS. After the reaction was completed, the mixture was quenched with methanol, concentrated under reduced pressure, subjected to reversed-phase column chromatography loaded with C18 using gradient elution, concentrated under reduced pressure, ultrasonically slurried with methanol, left to stand in a refrigerator at -30°C for 2 hours or more, filtered under reduced pressure. The filter cake was washed with cold methanol to obtain 78.8 mg of the target product with a yield of 44.44%.

$^1$H NMR (500 MHz, DMSO-$d_6$) δ 12.02 (s, 1H), 10.34 (s, 1H), 7.91 (dd, *J* = 6.44, 9.37 Hz, 3H), 7.82 (s, 1H), 7.75 (d, *J* = 7.67 Hz, 1H), 7.64 (d, *J* = 7.66 Hz, 1H), 7.59 (t, *J* = 7.61 Hz, 1H), 7.48-7.35 (m, 7H), 6.81 (s, 1H), 6.70 (s, 1H), 4.06 (t, *J* = 6.98 Hz, 2H), 3.63 (s, 2H), 3.23 (dd, *J* = 7.15, 14.27 Hz, 1H), 2.33 (dd, *J* = 27.41, 34.09 Hz, 6H), 1.86 (dd, *J* = 6.69, 13.56 Hz, 2H), 1.33 (d, *J* = 7.07 Hz, 6H). (The lack of two hydrogens may be contained in the water peak or solvent peak). MS (ESI) : m/z 675.50 [M + H]$^+$. Mp: 219-221°C.

**Example 42: Synthesis of (3Z,6Z)-3-(3-(p-fluorobenzoyl)phenyl)methylene-6-((5-isopropyl-1-(3-N-methylsulfonylpiperazinyl)propylimidazol-4-yl)methylene)piperazine-2,5-dione (PLN-2-42)**

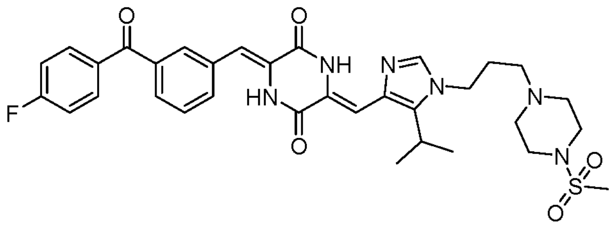

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (150 mg, 0.26 mmol), dichloromethane (2.5 mL), and triethylamine (39.90 mg, 0.39 mmol). The mixture was placed in a cold trap at 0°C and stirred for 5 min, then a solution of methanesulfonyl chloride (30.10 mg, 0.26 mmol) in dichloromethane (2.5 mL) was added dropwise thereto, and the mixture was stirred and reacted for 1 hour. The reaction was monitored by LC-MS. After the reaction was completed, the mixture was quenched with methanol, concentrated under reduced pressure, subjected to reversed-phase column chromatography loaded with C18 using gradient elution, concentrated under reduced pressure, ultrasonically slurried with methanol, left to stand in a refrigerator at -30°C for 2 hours or more, filtered under reduced pressure. The filter cake was washed with cold methanol to obtain 133.1 mg of the target product with a yield of 78.07%.

$^1$H NMR (500 MHz, DMSO-$d_6$) δ 12.01 (s, 1H), 10.35 (s, 1H), 7.90 (dt, *J* = 7.42, 14.58 Hz, 3H), 7.82 (s, 1H), 7.75 (d, *J* = 7.58 Hz, 1H), 7.64 (d, *J* = 7.67 Hz, 1H), 7.58 (t, *J* = 7.62 Hz, 1H), 7.40 (t, *J* = 8.76 Hz, 2H), 6.81 (s, 1H), 6.71 (s, 1H), 4.06 (s, 2H), 3.29-3.18 (m, 1H), 3.12 (s, 4H), 2.89 (s, 2H), 2.48 (s, 3H), 1.86 (s, 2H), 1.33 (d, *J* = 7.00 Hz, 6H). (The lack of four hydrogens may be contained in the water peak or solvent peak). MS (ESI) : m/z 649.36 [M + H]$^+$. Mp: 175-177°C.

**Example 43: Synthesis of (3Z,6Z)-3-(3-(p-fluorobenzoyl)phenyl)methylene-6-((5-isopropyl-1-(3-N-phenylmethylsulfonylpiperazinyl)propylimidazol-4-yl)methylene)piperazine-2,5-dione (PLN-2-43)**

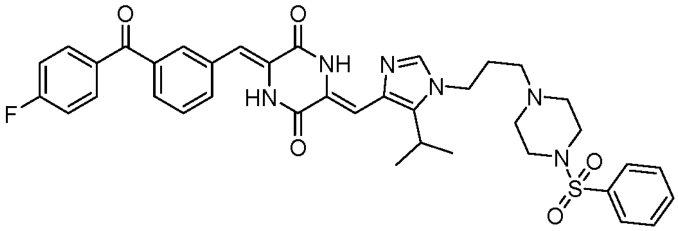

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (150 mg, 0.26 mmol), dichloromethane (2.5 mL), and triethylamine (39.90 mg, 0.39 mmol). The mixture was placed in a cold trap at 0°C and stirred for 5 min, then a solution of benzenesulfonyl chloride (46.41 mg, 0.26 mmol) in dichloromethane (2.5 mL) was added dropwise thereto, and the mixture was stirred and reacted for 1 hour. The reaction was monitored by LC-MS. After the reaction was completed, the mixture was quenched with methanol, concentrated under reduced pressure, subjected to reversed-phase column chromatography loaded with C18 using gradient elution, concentrated under reduced pressure, ultrasonically slurried with methanol, left to stand in a refrigerator at -30°C for 2 hours or more, filtered under reduced pressure. The filter cake was washed with cold methanol to obtain 87.3 mg of the target product with a yield of 46.73%.

$^1$H NMR (500 MHz, DMSO-$d_6$) δ 11.98 (s, 1H), 10.34 (s, 1H), 7.91 (dd, *J*= 5.61, 8.54 Hz, 2H), 7.81 (d, *J* = 4.96 Hz, 2H), 7.77-7.71 (m, 4H), 7.65 (dd, *J* = 7.64, 15.78 Hz, 3H), 7.59 *(t, J= 7.62* Hz, 1H), 7.40 (t, *J* = 8.76 Hz, 2H), 6.81 (s, 1H), 6.66 (s, 1H), 3.95 (t, *J* = 6.94 Hz, 2H), 3.18-3.10 (m, 1H), 2.88 (s, 4H), 2.41 (s, 4H), 2.25 (t, *J* = 6.46 Hz, 2H), 1.81-1.73 (m, 2H), 1.24 (d, *J* = 7.08 Hz, 6H). MS (ESI) : m/z 711.38 [M + H]$^+$. Mp: 164-166°C.

**Example 44: Preparation of (3Z,6Z)-3-(3-(p-fluorobenzoyl)phenyl)methylene-6-((5-isopropyl-1-(3-N-isopropylaminocarbonylpiperazinyl)propylimidazol-4-yl)methylene)piperazine-2,5-dione (PLN-2-44)**

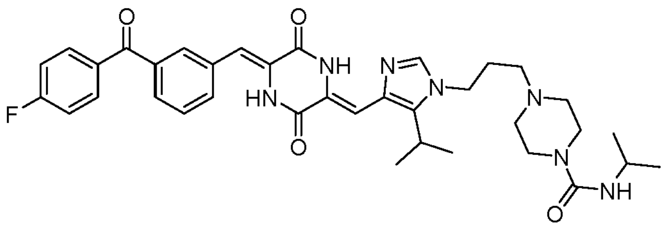

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (60 mg, 0.10 mmol) and a solution of triethylamine (31.92 mg, 0.32 mmol) in dichloromethane (3 mL). A solution of isopropyl isocyanate (10.74 mg, 0.13 mmol) in dichloromethane (3 mL) was added dropwise thereto at room temperature, and the mixture was stirred and reacted for 1 hour. The reaction was monitored by LC-MS. After the reaction was completed, the mixture was concentrated under reduced pressure, subjected to reversed-phase column chromatography loaded with C18 using gradient elution, concentrated under reduced pressure, dried, ultrasonically slurried with methanol, left to stand in a refrigerator at -30°C, filtered under reduced pressure. The filter cake was washed with cold methanol and dried under vacuum at 50°C to obtain 41.8 mg of the target product with a yield of 60.65%.

$^1$H NMR (400 MHz, DMSO-$d_6$)) δ 12.03 (s, 1H), 10.35 (s, 1H), 7.91 (dd, *J* = 5.80, 7.84 Hz, 3H), 7.82 (s, 1H), 7.75 (d, *J* = 7.41 Hz, 1H), 7.61 (dt, *J* = 7.61, 15.20 Hz, 2H), 7.40 *(t, J= 8.57* Hz, 2H), 6.81 (s, 1H), 6.70 (s, 1H), 6.14 (d, *J* = 7.42 Hz, 1H), 4.05 (t, *J* = 6.40 Hz, 2H), 3.73 (dq, *J* = 6.62, 13.42 Hz, 2H), 3.26 (s, 5H), 2.28 (s, 6H), 1.86 (s, 2H), 1.33 (d, *J* = 6.93 Hz, 6H), 1.09-1.00 (m, 6H). MS (ESI) : m/z 656.44 [M + H]$^+$. Mp: 240-242°C.

**Example 45: (3Z,6Z)-3-(3-(p-Fluorobenzoyl)phenyl)methylene-6-((5-isopropyl-1-(3-N-tert-butylaminocarbonylpiperazinyl)propylimidazol-4-yl)methylene)piperazine-2,5-dione** (PLN-2-45)

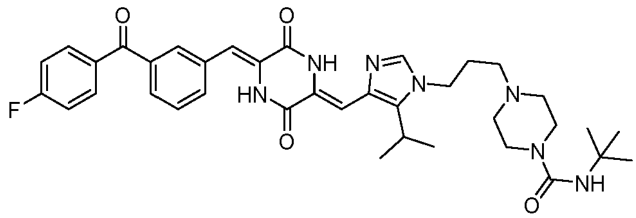

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (60 mg, 0.10 mmol) and a solution of triethylamine (31.92 mg, 0.32 mmol) in dichloromethane (3 mL). A solution of tert-butyl isocyanate (12.51 mg, 0.13 mmol) in dichloromethane (3 mL) was added dropwise thereto at room temperature, and the mixture was stirred and reacted for 1 hour. The reaction was monitored by LC-MS. After the reaction was completed, the mixture was concentrated under reduced pressure, subjected to reversed-phase column chromatography loaded with C18 using gradient elution, concentrated under reduced pressure, dried, ultrasonically slurried with methanol, left to stand in a refrigerator, filtered under reduced pressure. The filter cake was washed with cold methanol and dried under vacuum at 50°C to obtain 42.0 mg of the target product with a yield of 59.66%.

$^1H$ NMR (500 MHz, DMSO-$d_6$) δ 12.02 (s, 1H), 10.34 (s, 1H), 7.96 - 7.88 (m, 3H), 7.82 (s, 1H), 7.75 (d, *J* = 7.45 Hz, 1H), 7.67 - 7.55 (m, 2H), 7.40 (t, *J* = 8.42 Hz, 2H), 6.81 (s, 1H), 6.71 (s, 1H), 5.73 (s, 1H), 4.05 (s, 2H), 3.24 (s, 5H), 2.27 (s, 6H), 1.85 (s, 2H), 1.33 (d, *J* = 6.76 Hz, 6H), 1.24 (s, 9H). MS (ESI) : m/z 670.49 $[M + H]^+$. Mp: 203-205°C.

**Example 46: Preparation of (3Z,6Z)-3-(3-(p-fluorobenzoyl)phenyl)methylene-6-((5-isopropyl-1-(3-N-cyclopentylaminocarbonylpiperazinyl)propylimidazol-4-yl)methylene)piperazine-2,5-dione (PLN-2-46)**

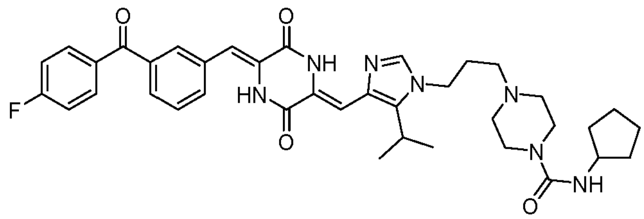

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (60 mg, 0.10 mmol) and a solution of triethylamine (31.92 mg, 0.32 mmol) in dichloromethane (3 mL). A solution of cyclopentyl isocyanate (15.80 mg, 0.13 mmol) in dichloromethane (3 mL) was added dropwise thereto at room temperature, and the mixture was stirred and reacted for 1 hour. The reaction was monitored by LC-MS. After the reaction was completed, the mixture was concentrated under reduced pressure, subjected to reversed-phase column chromatography loaded with C18 using gradient elution, concentrated under reduced pressure, dried, ultrasonically slurried with methanol, left to stand in a refrigerator at -30°C, filtered under reduced pressure. The filter cake was washed with cold methanol and dried under vacuum at 50°C to obtain 55.9 mg of the target product with a yield of 78.01%. MS (ESI) : m/z 682.48 $[M + H]^+$. Mp: 176-178°C.

**Example 47: Preparation of (3Z,6Z)-3-(3-(p-fluorobenzoyl)phenyl)methylene-6-((5-isopropyl-1-(3-N-cyclohexylaminocarbonylpiperazinyl)propylimidazol-4-yl)methylene)piperazine-2,5-dione (PLN-2-47)**

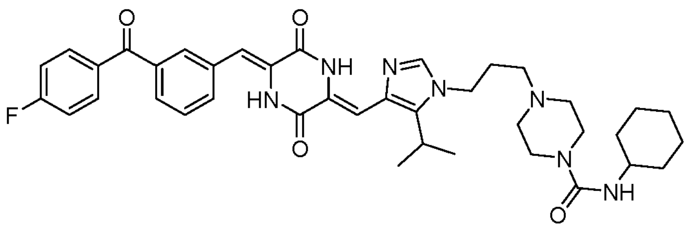

To a 25 mL dry round-bottom flask was sequentially added (3Z,6Z)-3-(3-(p-fluorobenzoyl)phenyl)methylene-6-((5-isopropyl-1-(3-piperazinyl)propylimidazol-4-yl)methylene)piperazine-2,5-dione (60 mg, 0.10 mmol) and a solution of triethylamine (31.92 mg, 0.32 mmol) in dichloromethane (3 mL). A solution of cyclohexyl isocyanate (14.03 mg, 0.13 mmol) in dichloromethane (3 mL) was added dropwise thereto at room temperature, and the mixture was stirred and reacted for 1 hour. The reaction was monitored by LC-MS. After the reaction was completed, the mixture was concentrated under reduced pressure, subjected to reversed-phase column chromatography loaded with C18 using gradient elution, concentrated under reduced pressure, dried, ultrasonically slurried with methanol, left to stand in a refrigerator at -30°C, filtered under reduced pressure. The filter cake was washed with cold methanol and dried under vacuum at 50°C to obtain 45.2 mg of the target product with a yield of 61.81%. MS (ESI) : m/z 696.49 $[M + H]^+$. Mp: 184-186°C.

**Example 48: Synthesis of (3Z,6Z)-3-(3-(2,5-difluorophenyl)methylene-6-((5-isopropyl-1-(3-N-tert-butoxycarbonylpiperazinyl)propylimidazol-4-yl)methylene)piperazine-2,5-dione (PLN-2-48)**

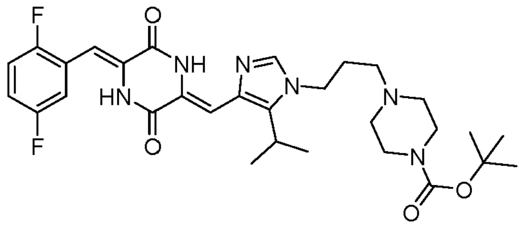

To a 25 mL dry brown round-bottom flask was sequentially added (Z)-1-acetyl-3-((5-isopropyl-1-(3-N-tert-butoxycarbonylpiperazinyl)propyl-imidazol-4-yl)methylene)piperazine-2,5-dione (436.60 mg, 0.87 mmol), 2,5-difluorobenzaldehyde (148.13 mg, 1.04 mmol), cesium carbonate (424.54 mg, 1.30 mmol), anhydrous sodium sulfate (246.76 mg, 1.30 mmol), and DMF (6 mL). The mixture was degassed, placed in an oil bath at 45°C under nitrogen atmosphere, stirred and reacted for 19 hours. The reaction was monitored by LC-MS. After the reaction was completed, the reaction mixture was dropped into cold water (60 mL) at 4°C, and filtered under reduced pressure. The filter cake was washed with cold water, dried under reduced pressure, dissolved in methanol and dichloromethane (1:3), filtered, concentrated under reduced pressure, ultrasonically slurried with methanol, left to stand overnight at -30°C, filtered under reduced pressure. The filter cake was washed with cold methanol, dried under vacuum at 50°C to obtain 367.2 mg of the target product with a yield of 72.31%.

$^1H$ NMR (500 MHz, DMSO-$d_6$) δ 12.06 (s, 1H), 10.45 (s, 1H), 7.89 (s, 1H), 7.40 (ddd, *J* = 3.07, 5.57, 8.82 Hz, 1H), 7.28 (td, *J* = 4.67, 9.31 Hz, 1H), 7.19 (ddd, *J* = 3.52, 8.19, 12.09 Hz, 1H), 6.72 (s, 1H), 6.61 (s, 1H), 4.04 (t, *J* = 7.07 Hz, 2H), 3.23 (dt, *J* = 7.09, 14.26 Hz, 1H), 2.33-2.20 (m, 6H), 1.88-1.78 (m, 2H), 1.43-1.29 (m, 15H). The lack of four hydrogens may be contained in the water peak or solvent peak. MS (ESI) : m/z 585.21 $[M + H]^+$. Mp: 209-211°C.

**Example 49: (3Z,6Z)-3-(2-Methylphenyl)methylene-6-((5-isopropyl-1-(3-morpholinyl)propylimidazol-4-yl) methylene)piperazine-2,5-dione (PLN-2-49)**

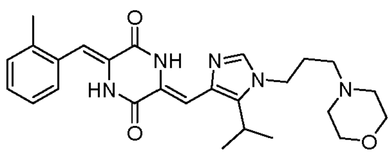

1) To a 50 mL two-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-chloropropyl)morpholine (0.59 g, 3.62 mmol), cesium carbonate (1.18 g, 3.62 mmol), potassium iodide (0.30 g, 1.81 mmol), a 4A molecular sieve (1.00 g), and DMF (10 mL). The mixture was placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with 60 mL of a mixed solvent (EtOH: DCM = 1:5), filtered under reduced pressure, and the filter cake was washed with EtOH: DCM = 1:5 and concentrated under reduced pressure to obtain 0.72 g of a crude product as a brown oil with a yield of 99%. The crude product was used directly in the next step

without purification.

2) To a 25 mL dry brown round-bottom flask was sequentially added (Z)-1-acetyl-3-((5-isopropyl-1-(3-morpholinyl) propyl-imidazol-4-yl)methylene)piperazine-2,5-dione (0.72 g, 1.78 mmol), 2-methylbenzaldehyde (261.0 mg, 2.17 mmol), cesium carbonate (424.5 mg, 2.71 mmol), anhydrous sodium sulfate (514.0 mg, 3.62 mmol), and DMF (10 mL). The mixture was placed in an oil bath at 45°C under nitrogen atmosphere, stirred and reacted for 18 hours. The completion of the reaction was monitored by TLC. After the reaction was completed, the reaction mixture was dropped into 100 mL of ice water, and a yellow solid was precipitated. The mixture was filtered, and the filter cake was dissolved by adding 40 mL of DCM, concentrated and purified to obtain 300.0 mg of the target product with a yield of 24%.

$^1H$ NMR (500 MHz, DMSO-$d_6$) $\delta$ 12.00 (s, 1H), 9.82 (s, 1H), 7.90 (s, 1H), 7.45-7.34 (m, 1H), 7.30-7.17 (m, 3H), 6.78 (s, 1H), 6.70 (s, 1H), 4.05 (t, *J* = 7.1 Hz, 2H), 3.58 (t, *J* = 4.2 Hz, 4H), 3.28-3.19 (m, 1H), 2.34 (s, 4H), 2.26-2.25 (m, 5H), 1.89-1.78 (m, 2H), 1.33 (d, *J* = 7.1 Hz, 6H). MS (ESI) : m/z 464.4 $[M + H]^+$. Mp 169-171°C.

**Example 50: (3Z,6Z)-3-(2,3-Dimethylphenyl)methylene-6-((5-isopropyl-1-(3-morpholinyl)propylimidazol-4-yl) methylene)piperazine-2,5-dione (PLN-2-50)**

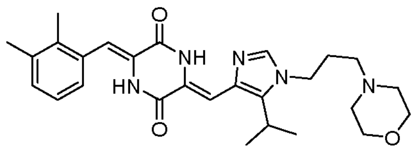

1) To a 50 mL two-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-chloropropyl)morpholine (0.59 g, 3.62 mmol), cesium carbonate (1.18 g, 3.62 mmol), potassium iodide (0.30 g, 1.81 mmol), a 4A molecular sieve (1.00 g), and DMF (10 mL). The mixture was placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with 60 mL of a mixed solvent (EtOH: DCM = 1:5), filtered under reduced pressure, and the filter cake was washed with EtOH: DCM = 1:5 to obtain 0.72 g of a crude product as a brown oil with a yield of 99%. The crude product was used directly in the next step without purification.

2) To a 25 mL dry brown round-bottom flask was sequentially added (Z)-1-acetyl-3-((5-isopropyl-1-(3-morpholinyl) propyl-imidazol-4-yl)methylene)piperazine-2,5-dione (0.72 g, 1.78 mmol), 2,3-dimethylbenzaldehyde (291.0 mg, 2.17 mmol), cesium carbonate (424.5 mg, 2.71 mmol), anhydrous sodium sulfate (514.0 mg, 3.62 mmol), and DMF (10 mL). The mixture was degassed, placed in an oil bath at 45°C under nitrogen atmosphere, stirred and reacted for 18 hours. The completion of the reaction was monitored by TLC. The reaction mixture was dropped into 100 mL of ice water, and a yellow solid was precipitated. The mixture was filtered, and the filter cake was dissolved by adding 40 mL of DCM, concentrated and then purified to obtain 180.0 mg of the target product with a yield of 21%.

$^1H$ NMR (500 MHz, DMSO-$d_6$) $\delta$ 12.00 (s, 1H), 9.61 (s, 1H), 7.89 (s, 1H), 7.19-7.17 (m, 1H), 7.15-7.12 (m, 2H), 6.82 (s, 1H), 6.69 (s, 1H), 4.05 (t, *J* = 7.1 Hz, 2H), 3.58 (t, *J* = 4.4 Hz, 4H), 3.28-3.19 (m, 1H), 2.33 (s, 4H), 2.29-2.23 (m, 5H), 2.15 (s, 3H), 1.84 (p, *J* = 6.9 Hz, 2H), 1.33 (d, *J* = 7.1 Hz, 6H). MS (ESI) : m/z 478.5 $[M + H]^+$. Mp 164-166°C.

**Example 51: (3Z,6Z)-3-(3-Methylphenyl)methylene-6-((5-isopropyl-1-(3-morpholinyl)propylimidazol-4-yl) methylene)piperazine-2,5-dione (PLN-2-51)**

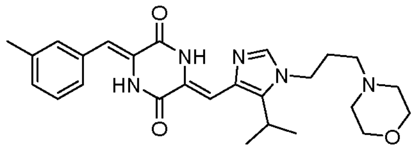

1) To a 50 mL two-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-chloropropyl)morpholine (0.59 g, 3.62 mmol), cesium carbonate (1.18 g, 3.62 mmol), potassium iodide (0.30 g, 1.81 mmol), a 4A molecular sieve (1.00 g), and DMF (10 mL).

The mixture was placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with 60 mL of a mixed solvent (EtOH: DCM = 1:5), filtered under reduced pressure, and the filter cake was washed with EtOH: DCM = 1:5, concentrated under reduced pressure to obtain 0.72 g of a crude product as a brown oil with a yield of 99%. The crude product was used directly in the next step without purification.

2) To a 25 mL dry brown round-bottom flask was sequentially added (Z)-1-acetyl-3-((5-isopropyl-1-(3-morpholinyl) propyl-imidazol-4-yl)methylene)piperazine-2,5-dione (0.72 g, 1.78 mmol), 3-methylbenzaldehyde (261.0 mg, 2.17 mmol), cesium carbonate (424.5 mg, 2.71 mmol), anhydrous sodium sulfate (514.0 mg, 3.62 mmol) and DMF (10 mL). The mixture was degassed, placed in an oil bath at 45°C under nitrogen atmosphere, stirred and reacted for 18 hours. The completion of the reaction was monitored by TLC. The reaction mixture was dropped into 100 mL of ice water, and a yellow solid was precipitated. The mixture was filtered, and the filter cake was dissolved by adding 40 mL of DCM, concentrated and then purified to obtain 200.0 mg of the target product with a yield of 24%.

$^1$H NMR (500 MHz, DMSO-$d_6$) δ 11.99 (s, 1H), 10.04 (s, 1H), 7.89 (s, 1H), 7.36 (s, 1H), 7.30 (d, *J* = 4.8 Hz, 2H), 7.14 (t, *J* = 3.9 Hz, 1H), 6.72 (s, 1H), 6.71 (s, 1H), 4.05 (t, *J* = 7.1 Hz, 2H), 3.58 (t, *J* = 4.4 Hz, 4H), 3.28-3.21 (m, 1H), 2.34 (s, 7H), 2.25 (t, *J* = 6.7 Hz, 2H), 1.84 (p, *J* = 6.9 Hz, 2H), 1.34 (d, *J* = 7.1 Hz, 6H). MS (ESI) : m/z 464.5 [M + H]$^+$. Mp 178-180°C.

**Example 52: (3Z,6Z)-3-(3-Methoxyphenyl)methylene-6-((5-isopropyl-1-(3-morpholinyl)propylimidazol-4-yl) methylene)piperazine-2,5-dione (PLN-2-52)**

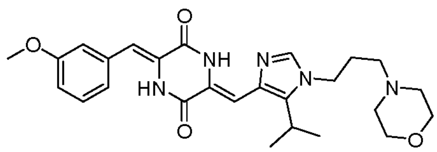

1) To a 50 mL two-necked dry round-bottom flask was sequentially added (Z)-1-acetyl-3-((5-isopropyl-1H-imidazol-4-yl)methylene)piperazine-2,5-dione (0.50 g, 1.81 mmol), 1-chloro-3-morpholinopropane N-(3-chloropropyl)morpholine (0.59 g, 3.62 mmol), cesium carbonate (1.18 g, 3.62 mmol), potassium iodide (0.30 g, 1.81 mmol), a 4A molecular sieve (1.00 g), and DMF (10 mL). The mixture was placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with 60 mL of a mixed solvent (EtOH: DCM = 1:5), filtered under reduced pressure, and the filter cake was washed with EtOH: DCM = 1:5, concentrated under reduced pressure to obtain 0.72 g of a crude product as a brown oil with a yield of 99%. The crude product was used directly in the next step without purification.

2) To a 25 mL dry brown round-bottom flask was sequentially added (Z)-1-acetyl-3-((5-isopropyl-1-(3-morpholinyl) propyl-imidazol-4-yl)methylene)piperazine-2,5-dione (0.72 g, 1.78 mmol), 3-methoxybenzaldehyde (296.0 mg, 2.17 mmol), cesium carbonate (424.5 mg, 2.71 mmol), anhydrous sodium sulfate (514.0 mg, 3.62 mmol), and DMF (10 mL). The mixture was degassed, placed in an oil bath at 45°C under nitrogen atmosphere, stirred and reacted for 18 hours. The completion of the reaction was monitored by TLC. The reaction mixture was dropped into 100 mL of ice water, and a yellow solid was precipitated. The mixture was filtered, and the filter cake was dissolved by adding 40 mL of DCM, concentrated and then purified to obtain 280.0 mg of the target product with a yield of 32%.

$^1$H NMR (500 MHz, DMSO-$d_6$) δ 12.00 (s, 1H), 10.05 (s, 1H), 7.89 (s, 1H), 7.33 (t, *J* = 7.9 Hz, 1H), 7.12-7.02 (m, 2H), 6.90 (dd, *J* = 8.2, 2.4 Hz, 1H), 6.73 (s, 1H), 6.71 (s, 1H), 4.04 (t, *J* = 7.1 Hz, 2H), 3.79 (s, 3H), 3.58 (t, *J* = 4.4 Hz, 4H), 3.32-3.21 (m, 1H), 2.33(s, 4H), 2.25 (t, *J*= 6.7 Hz, 2H), 1.84(p, *J* = 6.8 Hz, 2H), 1.33 (d, *J* = 7.1 Hz, 6H). MS (ESI) : m/z 480.4 [M + H]$^+$. Mp 184-186°C.

**Example 53: (3Z,6Z)-3-(3-Trifluoromethoxyphenyl)methylene-6-((5-isopropyl-1-(3-morpholinyl)propylimidazol-4-yl)methylene)piperazine-2,5-dione (PLN-2-53)**

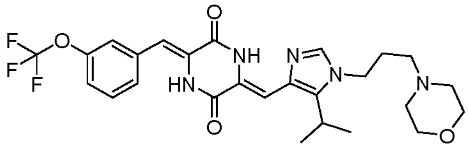

1) To a 50 mL two-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-chloropropyl)morpholine (0.59 g, 3.62 mmol), cesium carbonate (1.18 g, 3.62 mmol), potassium iodide (0.30 g, 1.81 mmol), a 4A molecular sieve (1.00 g), and DMF (10 mL). The mixture was placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with 60 mL of a mixed solvent (EtOH: DCM = 1:5), filtered under reduced pressure, and the filter cake was washed with EtOH: DCM = 1:5, concentrated under reduced pressure to obtain 0.72 g of a crude product as a brown oil with a yield of 99%. The crude product was used directly in the next step without purification.

2) To a 25 mL dry brown round-bottom flask was sequentially added (Z)-1-acetyl-3-((5-isopropyl-1-(3-morpholinyl) propyl-imidazol-4-yl)methylene)piperazine-2,5-dione (0.72 g, 1.78 mmol), 3-trifluoromethoxybenzaldehyde (412.7 mg, 2.17 mmol), cesium carbonate (424.5 mg, 2.71 mmol), anhydrous sodium sulfate (514.0 mg, 3.62 mmol), and DMF (10 mL). The mixture was degassed, placed in an oil bath at 45°C under nitrogen atmosphere, stirred and reacted for 18 hours. The completion of the reaction was monitored by TLC. The reaction mixture was transferred into 100 mL of ice water, and a yellow solid was precipitated. The mixture was filtered, and the filter cake was dissolved by adding 40 mL of DCM, concentrated and then purified to obtain 190.0 mg of the target product with a yield of 20%.

$^1H$ NMR (500 MHz, $CDCl_3$) δ 12.29 (s, 1H), 8.00 (s, 1H), 7.53-7.46 (m, 2H), 7.34 (d, *J* = 7.7 Hz, 1H), 7.21-7.19 (m, 2H), 6.94 (s, 1H), 6.91 (s, 1H), 4.03 (t, *J* = 6.9 Hz, 2H), 3.74 (t, *J= 4.3* Hz, 4H), 3.22-3.10 (m,1H), 2.43(s, 4H), 2.31 (t, *J* = 6.5 Hz, 2H),1.90 (p, *J* = 6.7 Hz, 2H), 1.41 (d, *J* = 7.1 Hz, 6H). (One active hydrogen not shown in NMR), MS (ESI) : m/z 534.4 $[M + H]^+$. Mp 251-253°C.

**Example 54: (3Z,6Z)-3-(1-Naphthalenyl)methylene-6-((5-isopropyl-1-(3-morpholinyl)propylimidazol-4-yl)methylene)piperazine-2,5-dione (PLN-2-54)**

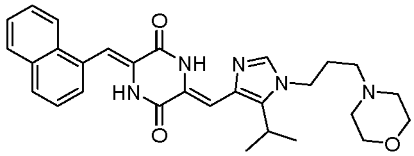

1) To a 50 mL two-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-chloropropyl)morpholine (0.59 g, 3.62 mmol), cesium carbonate (1.18 g, 3.62 mmol), potassium iodide (0.30 g, 1.81 mmol), a 4A molecular sieve (1.00 g), and DMF (10 mL). The mixture was placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with 60 mL of a mixed solvent (EtOH: DCM = 1:5), filtered under reduced pressure, and the filter cake was washed with EtOH: DCM = 1:5, concentrated under reduced pressure to obtain 0.72 of a crude product as a brown oil with a yield of 99%. The crude product was used directly in the next step without purification.

2) To a 25 mL dry brown round-bottom flask was sequentially added (Z)-1-acetyl-3-((5-isopropyl-1-(3-morpholinyl) propyl-imidazol-4-yl)methylene)piperazine-2,5-dione (0.72 g, 1.78 mmol), 1-naphthaldehyde (339.0 mg, 2.17 mmol), cesium carbonate (424.5 mg, 2.71 mmol), anhydrous sodium sulfate (514.0 mg, 3.62 mmol), and DMF (10 mL). The mixture was degassed, placed in an oil bath at 45°C under nitrogen atmosphere, stirred and reacted for 18 hours. The completion of the reaction was monitored by TLC. The reaction mixture was dropped into 100 mL of ice water, and a yellow solid was precipitated. The mixture was filtered, and the filter cake was dissolved by adding 40 mL of DCM, concentrated and then purified to obtain 260.0 mg of the target product with a yield of 29%.

$^1H$ NMR (500 MHz, $CDCl_3$) δ 12.30 (s, 1H), 8.02-7.97 (m, 1H), 7.91-7.85 (m, 3H), 7.57-7.51 (m, 4H), 7.47 (s, 1H),

6.88 (s, 1H), 4.04 (t, $J$ = 6.9 Hz, 2H), 3.74 (t, $J$ = 4.5 Hz, 4H), 3.20-3.11 (m,1H), 2.43 (s, 4H), 2.32 (t, $J$ = 6.6 Hz, 2H),1.91 (p, $J$ = 6.7 Hz, 2H), 1.40 (d, $J$ = 7.2 Hz, 6H). (One active hydrogen not shown in NMR) MS (ESI) : m/z 500.4 $[M + H]^+$. Mp 248-250 °C.

**Example 55: (3Z,6Z)-3-(4-Isoquinolinyl)methylene-6-((5-isopropyl-1-(3-morpholinyl)propylimidazol-4-yl)methylene)piperazine-2,5-dione (PLN-2-55)**

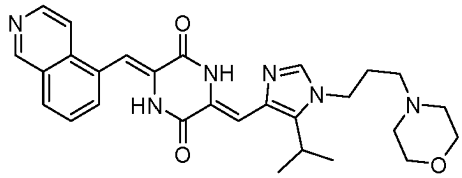

1) To a 50 mL two-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-chloropropyl)morpholine (0.59 g, 3.62 mmol), cesium carbonate (1.18 g, 3.62 mmol), potassium iodide (0.30 g, 1.81 mmol), a 4A molecular sieve (1.00 g), and DMF (10 mL). The mixture was placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with 60 mL of a mixed solvent (EtOH: DCM = 1:5), filtered under reduced pressure, and the filter cake was washed with EtOH: DCM = 1:5, concentrated under reduced pressure to obtain 0.72 of a crude product as a brown oil with a yield of 99%. The crude product was used directly in the next step without purification.

2) To a 25 mL dry brown round-bottom flask was sequentially added (Z)-1-acetyl-3-((5-isopropyl-1-(3-morpholinyl) propyl-imidazol-4-yl)methylene)piperazine-2,5-dione (0.72 g, 1.78 mmol), isoquinoline-4-carbaldehyde (339.0 mg, 2.17 mmol), cesium carbonate (424.5 mg, 2.71 mmol), anhydrous sodium sulfate (514.0 mg, 3.62 mmol), and DMF (10 mL). The mixture was degassed, placed in an oil bath at 45°C under nitrogen atmosphere, stirred and reacted for 18 hours. The completion of the reaction was monitored by TLC. The reaction mixture was dropped into 100 mL of ice water, and a yellow solid was precipitated. The mixture was filtered, and the filter cake was dissolved by adding 40 mL of DCM, concentrated and then purified to obtain 260.0 mg of the target product with a yield of 29%.

$^1H$ NMR (500 MHz, $CDCl_3$) δ 12.36 (s, 1H), 8.60 (d, $J$ = 5.9 Hz, 1H), 8.00 (d, $J$ = 8.2 Hz, 1H), 7.85 (s, 1H), 7.80 (d, $J$ = 5.9 Hz, 1H), 7.75 (d, $J$ = 7.1 Hz, 1H), 7.71 - 7.64 (m, 1H), 7.51 (s, 1H), 7.37 (s, 1H), 6.90 (s, 1H), 4.04 (t, $J$ = 6.9 Hz, 2H), 3.74 (t, $J$ = 4.5 Hz, 4H), 3.22-3.06 (m,1H), 2.43 (s, 4H), 2.32 (t, $J$ = 6.5 Hz, 2H), 1.90 (p, $J$ = 6.7 Hz, 2H), 1.41 (d, $J$ = 7.2 Hz, 6H). (One active hydrogen not shown in NMR), MS (ESI) : m/z 501.4 $[M + H]^+$. Mp 250-252°C.

**Example 56: (3Z,6Z)-3-(3-Fluorophenyl)methylene-6-(5-isopropyl-1-(3-((R)-2-methylmorpholinyl)propylimidazol-4-yl)methylene)piperazine-2,5-dione (PLN-2-56)**

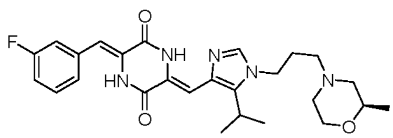

1) To a 50 mL dry round-bottom flask was sequentially added (R)-2-methylmorpholine (1.00 g, 9.89 mmol), cesium carbonate (6.44 g, 19.77 mmol), acetonitrile (25 mL), and 1-chloro-3-bromopropane (2.33 g, 14.83 mmol). The mixture was stirred and reacted at room temperature (25°C) for 23 hours. The reaction was monitored by TLC (MeOH: DCM = 1:10), and the trace amount of raw materials remained and was not further converted. The reaction was stopped and filtered with silica gel, and the filter cake was washed with EA until there was no product, and concentrated under reduced pressure to obtain 1.41 g of (R)-N-(3-chloropropyl)-2-methylmorpholine as a colorless oily liquid with a yield of 80%.

2) To a 100 mL dry round-bottom flask was sequentially added (Z)-1-acetyl-3-((5-isopropyl-1H-imidazol-4-yl)methylene)piperazine-2,5-dione (1.10 g, 3.98 mmol), (*R*)-N-(3-chloropropyl)-2-methylmorpholine (1.41 g, 7.96 mmol),

cesium carbonate (2.59 g, 7.96 mmol), potassium iodide (0.46 g, 3.98 mmol), a 4Å molecular sieve (2.50 g), and DMF (30 mL). The mixture was placed in an oil bath at 70°C under nitrogen atmosphere, stirred and reacted for 20 hours. After the reaction was completed, the reaction mixture was concentrated under reduced pressure, redissolved with 100 mL of a mixed solvent (EtOH: DCM = 1:5), filtered under reduced pressure, and the filter cake was washed with EtOH: DCM = 1:5, concentrated under reduced pressure to obtain 1.66 g of a crude product as a brown oil with a yield of 99%. The crude product was used directly in the next step without purification.

3) To a 100 mL dry brown round-bottom flask was sequentially added (Z)-1-acetyl-3-((5-isopropyl-1-(3-((*R*)-2-methylmorpholinyl))propyl-imidazol-4-yl)methylene)piperazine-2,5-dione (1.66 g, 3.98 mmol), 3-fluorobenzaldehyde (0.59 g, 4.78 mmol), cesium carbonate (1.94 g, 5.97 mmol), anhydrous sodium sulfate (1.13 g, 7.96 mmol), and DMF (30 mL). The mixture was degassed, placed in an oil bath at 45°C under nitrogen atmosphere, stirred and reacted for 18 hours. The completion of the reaction was monitored by TLC. After the reaction was completed, the reaction mixture was dropped into 300 mL of ice water, and a yellow solid was precipitated. The mixture was filtered, and the filter cake was dissolved by adding 120 mL of DCM, concentrated and then subjected to column chromatography to obtain 300.0 mg of a yellow solid with a yield of 16%.

$^1$H NMR (500 MHz, $CDCl_3$) δ 13.32 (s, 1H), 8.08 (s, 1H), 7.53 (s, 1H), 7.48-7.44 (m, 1H),7.22 (d, *J* = 7.7 Hz, 1H),7.13-7.11 (m, 1H), 7.10-7.01 (m, 1H), 6.98 (s, 1H), 6.94 (s, 1H),4.07 (t, *J* = 6.9 Hz, 2H), 3.94-3.82 (m, 1H),3.72-3.66 (m,2H),3.24-3.17 (m,1H), 2.73-2.71 (m, 1H),2.69-2.67 (m, 1H) , 2.35-2.31 (m, 2H) ,2.19-2.14 (m, 1H) ,1.98-1.91 (m,2H) , 1.88-1.84 (m,1H) ,1.45 (d, *J* = 7.2 Hz, 6H),1.21 (d, *J* = 6.3 Hz, 3H). MS (ESI) : m/z 482.2 [M + H]$^+$. Mp 212-214°C.

**Example 57: (3Z,6Z)-3-(3-Fluorophenyl)methylene-6-(5-isopropyl-1-(3-((S)-2-methylmorpholinyl)propylimidazol-4-yl)methylene)piperazine-2,5-dione (PLN-2-57)**

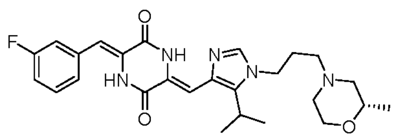

1) To a 50 mL dry round-bottom flask was sequentially added (*S*)-2-methylmorpholine (1.00 g, 9.89 mmol), cesium carbonate (6.44 g, 19.77 mmol), acetonitrile (25 mL), 1-chloro-3-bromopropane (2.33 g, 14.83 mmol). The mixture was stirred and reacted at room temperature (25°C) for 23 hours. The reaction was monitored by TLC (MeOH: DCM = 1: 10), and the trace amount of raw materials remained and was not further converted. The reaction was stopped and filtered with silica gel, and the filter cake was washed with EA until there was no product, and concentrated under reduced pressure to obtain 1.63 g of (S)-N-(3-chloropropyl)-2-methylmorpholine as a colorless oily liquid with a yield of 93%.

2) To a 100 mL dry round-bottom flask was sequentially added (Z)-1-acetyl-3-((5-isopropyl-1H-imidazol-4-yl)methylene)piperazine-2,5-dione (1.27 g, 4.60 mmol), (S)-N-(3-chloropropyl)-2-methylmorpholine (1.63 g, 9.19 mmol), cesium carbonate (2.99 g, 9.19 mmol), potassium iodide (0.53 g, 4.60 mmol), a 4Å molecular sieve (2.50 g), and DMF (30 mL). The mixture was placed in an oil bath at 70°C under nitrogen atmosphere, stirred and reacted for 20 hours. After the reaction was completed, the reaction mixture was concentrated under reduced pressure, redissolved with 100 mL of a mixed solvent (EtOH: DCM = 1:5), filtered under reduced pressure, and the filter cake was washed with EtOH: DCM = 1:5, concentrated under reduced pressure to obtain 1.92 g of a crude product as a brown oil with a yield of 99%. The crude product was used directly in the next step without purification.

3) To a 100 mL dry brown round-bottom flask was sequentially added (Z)-1-acetyl-3-((5-isopropyl-1-(3-((*S*)-2-methylmorpholinyl))propyl-imidazol-4-yl)methylene)piperazine-2,5-dione (1.92 g, 4.60 mmol), 3-fluorobenzaldehyde (0.68 g, 5.52 mmol), cesium carbonate (2.25 g, 6.89 mmol), anhydrous sodium sulfate (1.31 g, 9.19 mmol), and DMF (30 mL). The mixture was degassed, placed in an oil bath at 45°C under nitrogen atmosphere, stirred and reacted for 18 hours. The completion of the reaction was monitored by TLC. After the reaction was completed, the reaction mixture was dropped into 300 mL of ice water, and a yellow solid was precipitated. The mixture was filtered, and the filter cake was dissolved by adding 120 mL of DCM, concentrated and then subjected to column chromatography to obtain 500.0 mg of a yellow solid with a yield of 23%.

$^1$H NMR (500 MHz, $CDCl_3$) δ 13.32 (s, 1H), 8.08 (s, 1H), 7.53 (s, 1H), 7.48-7.44 (m, 1H),7.22 (d, *J* = 7.7 Hz, 1H),7.13-7.11 (m, 1H), 7.10-7.01 (m, 1H), 6.98 (s, 1H), 6.94 (s, 1H),4.07 (t, *J* = 6.9 Hz, 2H), 3.94-3.82 (m, 1H),3.72-3.66 (m,2H),3.24-3.17 (m,1H), 2.73-2.71 (m, 1H), 2.69-2.67 (m, 1H) 2.35-2.31 (m, 2H) ,2.19-2.14 (m, 1H) ,1.98-1.91 (m,2H) ,

1.88-1.84 (m,1H) ,1.45 (d, *J* = 7.2 Hz, 6H),1.21 (d, *J* = 6.3 Hz, 3H). MS (ESI) : m/z 482.2 [M + H]$^+$. Mp 211-213°C.

**Example 58: (3Z,6Z)-3-(3-Fluorophenyl)methylene-6-(5-isopropyl-1-(3-(2-(ethoxymethyl)morpholinyl)propylimidazol-4-yl)methylene)piperazine-2,5-dione (PLN-**2-58)

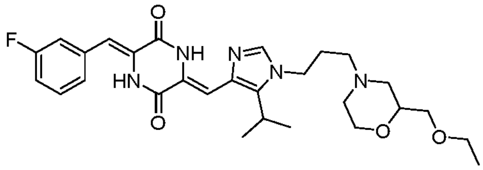

1) To a 100 mL three-necked dry round-bottom flask was sequentially added 2-(hydroxymethyl)-4-benzylmorpholine (2.00 g, 9.65 mmol) and 20 mL of DMF. The mixture was cooled to 0°C under $N_2$ atmosphere, and a suspension of NaH (0.57 g, 14.48 mmol) in 10 mL of DMF was added dropwise thereto. After the addition, the temperature was kept and the mixture was reacted for 0.5 hours, then ethyl iodide (1.81 g, 11.58 mmol) was added dropwise thereto, and the reaction was stirred at 25°C for 20 hours after the addition. After the reaction was completed, the reaction mixture was dropped into 80 mL of water, extracted by adding 30 mL * 2 EA. The organic phases were combined, washed by adding 30 mL of saturated brine, dried, concentrated, mixed with silica gel and subjected to column chromatography (PE: EA = 20:1 to 10:1) to obtain 1.70 g of a colorless liquid with a yield of 75%.

2) To a 50 mL dry round-bottom flask was sequentially added 2-(ethoxymethyl)-4-benzylmorpholine (1.65 g, 7.01 mmol), MeOH (16 mL), and 10% Pd/C (0.17 g). After the addition, the mixture was replaced with hydrogen and reacted for 24 hours at room temperature (25°C) under hydrogen atmosphere. The completion of the reaction was monitored by TLC. The reaction mixture was filtered with diatomite, and the filtrate was concentrated to obtain 0.95 g of a light yellow oil with a yield of 93%.

3) To a 50 mL dry round-bottom flask was sequentially added 2-(ethoxymethyl)morpholine (0.90 g, 6.20 mmol), cesium carbonate (4.04 g, 12.40 mmol), acetonitrile (20 mL), 1-chloro-3-bromopropane (1.46 g, 9.30 mmol). The mixture was stirred and reacted at room temperature (25°C) for 18 hours. The reaction was monitored by TLC (MeOH: DCM = 1: 10), and the trace amount of raw materials remained and was not further converted. The reaction was stopped and filtered with silica gel, and the filter cake was washed with EA until there was no product, and concentrated under reduced pressure to obtain 1.10 g of N-(3-chloropropyl)-2-(ethoxymethyl)morpholine as a colorless oily liquid with a yield of 80%.

4) tautomer To a 50 mL two-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-chloropropyl)-2-(ethoxymethyl)morpholine (0.80 g, 3.62 mmol), cesium carbonate (1.18 g, 3.62 mmol), potassium iodide (0.30 g, 1.81 mmol), a 4Å molecular sieve (1.00 g), and DMF (10 mL). The mixture was placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with 60 mL of a mixed solvent (EtOH: DCM = 1:5), filtered under reduced pressure, and the filter cake was washed with EtOH: DCM = 1:5 to obtain 0.83 g of a crude product as a brown oil with a yield of 99%. The crude product was used directly in the next step without purification.

5) To a 25 mL dry brown round-bottom flask was sequentially added (Z)-1-acetyl-3-((5-isopropyl-1-(3-(2-ethoxymethyl)morpholinyl)propyl-imidazol-4-yl)methylene)piperazine-2,5-dione (0.83 g, 1.81 mmol), 3-fluorobenzaldehyde (0.27 g, 2.17 mmol), cesium carbonate (0.88 g, 2.71 mmol), anhydrous sodium sulfate (0.51 g, 3.62 mmol), and DMF (12 mL). The mixture was degassed, placed in an oil bath at 45°C under nitrogen atmosphere, stirred and reacted for 18 hours. The completion of the reaction was monitored by TLC. The reaction mixture was dropped into 100 mL of ice water, and a yellow solid was precipitated. The mixture was filtered, and the filter cake was dissolved by adding 60 mL of DCM, concentrated and then purified by preparative thin-layer chromatography to obtain 180.0 mg of a yellow solid with a yield of 19%.

$^1$H NMR (500 MHz, $CDCl_3$) $\delta$ 13.32 (s, 1H), 8.06 (s, 1H), 7.53 (s, 1H), 7.48-7.44 (m 1H),7.22 (d, *J* = 7.7 Hz, 1H),7.13-7.11 (m, 1H), 7.10-7.01 (m, 1H), 6.98 (s, 1H), 6.94 (s, 1H),4.07 (t, *J* = 6.9 Hz, 2H), 3.96-3.89 (m, 1H),3.75-3.63 (m,2H),3.26-3.11 (m,1H), 2.73-2.67 (m, 1H), 2.68 (d, *J* = 11.3 Hz, 1H),2.33 (td, *J* = 6.5, 2.4 Hz, 2H) , 2.18-2.14 (m, 1H), 1.98-1.90 (m,2H), 1.89-1.81 (m,1H) ,1.45 (d, *J*= 7.2 Hz, 6H),1.21 (d, *J* = 6.3 Hz, 3H). MS (ESI) : m/z 526.3 [M + H]$^+$. Mp 202-204°C.

**Example 59: (3Z,6Z)-3-(3-fluorophenyl)methylene-6-(5-isopropyl-1-(3-(2-(propoxymethyl)morpholinyl)propylimidazol-4-yl)methylene)piperazine-2,5-dione (PLN-2-59)**

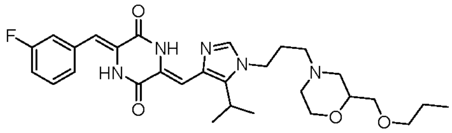

1) To a 100 mL three-necked dry round-bottom flask was sequentially added 2-(hydroxymethyl)-4-benzylmorpholine (2.00 g, 9.65 mmol) and 20 mL of DMF. The mixture was cooled to 0°C under $N_2$ atmosphere, and a suspension of NaH (0.57 g, 14.48 mmol) in 10 mL of DMF was added dropwise thereto. After the addition, the temperature was kept and the mixture was reacted for 0.5 hours, then 1-iodopropane (1.97 g, 11.58 mmol) was added dropwise thereto, and the reaction was stirred at 25°C for 20 hours after the addition. After the reaction was completed, the reaction mixture was dropped into 80 mL of water, extracted by adding 30 mL * 2 EA. The organic phases were combined, washed by adding 30 mL of saturated brine, dried, concentrated, mixed with silica gel and subjected to column chromatography (PE: EA = 20:1 to 10:1) to obtain 1.00 g of a colorless liquid with a yield of 42%.
2) To a 50 mL dry round-bottom flask was sequentially added 2-(propoxymethyl)-4-benzylmorpholine (1.00 g, 4.01 mmol), MeOH (10 mL), and 10% Pd/C (0.10 g). After the addition, the mixture was replaced with hydrogen and reacted for 24 hours at room temperature (25°C) under hydrogen atmosphere. The completion of the reaction was monitored by TLC. The reaction mixture was filtered with diatomite, and the filtrate was concentrated to obtain 0.64 g of a light yellow oil with a yield of 97%.
3) To a 50 mL dry round-bottom flask was sequentially added 2-(propoxymethyl)morpholine (0.60 g, 3.77 mmol), cesium carbonate (2.46 g, 7.54 mmol), acetonitrile (20 mL), 1-chloro-3-bromopropane (0.42 g, 5.65 mmol). The mixture was stirred and reacted at room temperature (25°C) for 18 hours. The reaction was monitored by TLC (MeOH: DCM = 1:10), and the trace amount of raw materials remained and was not further converted. The reaction was stopped and filtered with silica gel, and the filter cake was washed with EA until there was no product, and concentrated under reduced pressure to obtain 0.76 g of N-(3-chloropropyl)-2-(propoxymethyl)morpholine as a colorless oily liquid with a yield of 85%.
4) To a 50 mL two-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-chloropropyl)-2-(propoxymethyl)morpholine (0.76 g, 3.22 mmol), cesium carbonate (1.18 g, 3.62 mmol), potassium iodide (0.30 g, 1.81 mmol), a 4Å molecular sieve (1.00 g), and DMF (10 mL). The mixture was placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with 60 mL of a mixed solvent (EtOH: DCM = 1:5), filtered under reduced pressure, and the filter cake was washed with EtOH: DCM = 1:5 to obtain 0.84 g of a crude product as a brown oil with a yield of 99%. The crude product was used directly in the next step without purification.
5) To a 25 mL dry brown round-bottom flask was sequentially added (Z)-1-acetyl-3-((5-isopropyl-1-(3-(2-propoxymethyl)morpholinyl)propyl-imidazol-4-yl)methylene)piperazine-2,5-dione (0.84 g, 1.81 mmol), 3-fluorobenzaldehyde (0.27 g, 2.17 mmol), cesium carbonate (0.88 g, 2.71 mmol), anhydrous sodium sulfate (0.51 g, 3.62 mmol), and DMF (12 mL). The mixture was degassed, placed in an oil bath at 45°C under nitrogen atmosphere, stirred and reacted for 18 hours. The completion of the reaction was monitored by TLC. The reaction mixture was dropped into 100 mL of ice water, and a yellow solid was precipitated. The mixture was filtered, and the filter cake was dissolved by adding 60 mL of DCM, concentrated and then purified by preparative thin-layer chromatography to obtain 160.0 mg of a yellow solid with a yield of 16%.

$^1$H NMR (500 MHz, $CDCl_3$) $\delta$ 13.31 (s, 1H), 8.09 (s, 1H), 7.52 (s, 1H), 7.49-7.43 (m 1H),7.22 (d, *J* = 7.7 Hz, 1H),7.13-7.11 (m, 1H), 7.10-7.01 (m, 1H), 6.97 (s, 1H), 6.94 (s, 1H), 4.07 (t, *J* = 6.9 Hz, 2H), 3.98-3.96 (m, 1H),3.78-3.71 (m,2H), 3.55-3.52 (m,1H), 3.47-3.44 (m,3H), 3.26-3.11 (m,1H), 2.80 *(d, J*= 11.1 Hz, 1H), 2.68 *(d, J*= 11.3 Hz, 1H),2.37-2.35 (m, 2H) ,2.23-2.18 (m,1H) , 2.00-1.93 (m,3H) ,1.67-1.62 (m,2H),1.45 (d, *J* = 7.2 Hz, 6H),0.96 (t, *J* = 7.4 Hz, 3H). MS (ESI) : m/z 539.3 $[M + H]^+$. Mp 178-180°C.

**Example 60: (3Z,6Z)-3-(3-Fluorophenyl)methylene-6-(5-isopropyl-1-(3-(2-(benzyloxymethyl)morpholinyl)propylimidazol-4-yl)methylene)piperazine-2,5-dione** (PLN-2-60)

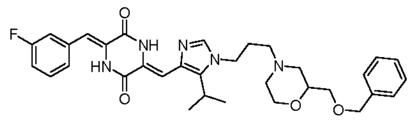

1) To a 100 mL three-necked dry round-bottom flask was sequentially added 2-(hydroxymethyl)-4-benzylmorpholine (2.00 g, 9.65 mmol) and 20 mL of DMF. The mixture was cooled to 0°C under $N_2$ atmosphere, and a suspension of NaH (0.57 g, 14.48 mmol) in 10 mL of DMF was added dropwise thereto. After the addition, the temperature was kept and the mixture was reacted for 0.5 hours, then benzyl bromide (1.73 g, 10.13 mmol) was added dropwise thereto, and the reaction was stirred at 25°C for 20 hours after the addition. After the reaction was completed, the reaction mixture was dropped into 80 mL of water, extracted by adding 30 mL * 2 EA. The organic phases were combined, washed by adding 30 mL of saturated brine, dried, concentrated, mixed with silica gel and subjected to column chromatography (PE: EA = 20:1 to 10:1) to obtain 2.30 g of a colorless liquid with a yield of 84%.
2) To a 50 mL dry round-bottom flask was sequentially added 2-(benzyloxymethyl)-4-benzylmorpholine (2.30 g, 8.10 mmol), MeOH (20 mL), and 10% Pd/C (0.23 g). After the addition, the mixture was replaced with hydrogen and reacted for 24 hours at room temperature (25°C) under hydrogen atmosphere. The completion of the reaction was monitored by TLC. The reaction mixture was filtered with diatomite, and the filtrate was concentrated to obtain 1.40 g of a light yellow oil with a yield of 83%.
3) To a 50 mL dry round-bottom flask was sequentially added 2-(benzyloxymethyl)morpholine (1.40 g, 6.75 mmol), cesium carbonate (3.31 g, 10.15 mmol), acetonitrile (30 mL), 1-chloro-3-bromopropane (2.12 g, 13.50 mmol). The mixture was stirred and reacted at room temperature (25°C) for 18 hours. The reaction was monitored by TLC (MeOH: DCM = 1:10), and the trace amount of raw materials remained and was not further converted. The reaction was stopped and filtered with silica gel, and the filter cake was washed with EA, and concentrated under reduced pressure to obtain 1.42 g of N-(3-chloropropyl)-2-(propoxymethyl)morpholine as a colorless oily liquid with a yield of 74%.
4) To a 50 mL two-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-chloropropyl)-2-(benzyloxymethyl)morpholine (1.03 g, 3.22 mmol), cesium carbonate (1.18 g, 3.62 mmol), potassium iodide (0.30 g, 1.81 mmol), a 4Å molecular sieve (1.00 g), and DMF (10 mL). The mixture was placed in an oil bath at 70°C under nitrogen atmosphere, stirred and reacted for 20 hours. After the reaction was completed, the reaction mixture was transferred to a 100 mL single-necked flask, washed with ethanol, concentrated under reduced pressure, redissolved with 60 mL of a mixed solvent (EtOH: DCM = 1:5), filtered under reduced pressure, and the filter cake was washed with EtOH: DCM = 1:5 to obtain 0.95 g of a crude product as a brown oil with a yield of 99%. The crude product was used directly in the next step without purification.
5) To a 25 mL dry brown round-bottom flask was sequentially added (Z)-1-acetyl-3-((5-isopropyl-1-(3-(2-benzyloxymethyl)morpholinyl)propyl-imidazol-4-yl)methylene)piperazine-2,5-dione (0.95 g, 1.81 mmol), 3-fluorobenzaldehyde (0.27 g, 2.17 mmol), cesium carbonate (0.88 g, 2.71 mmol), anhydrous sodium sulfate (0.51 g, 3.62 mmol), and DMF (12 mL). The mixture was degassed, placed in an oil bath at 45°C under nitrogen atmosphere, stirred and reacted for 18 hours. The completion of the reaction was monitored by TLC. The reaction mixture was dropped into 100 mL of ice water, and a yellow solid was precipitated. The mixture was filtered, and the filter cake was dissolved by adding 60 mL of DCM, concentrated and then purified by preparative thin-layer chromatography to obtain 230.0 mg of a yellow solid with a yield of 22%.

$^1$H NMR (500 MHz, $CDCl_3$) $\delta$ 13.31 (s, 1H), 8.08 (s, 1H), 7.51 (s, 1H), 7.48-7.45 (m 1H),7.43 -7.36 (m, 3H),7.35 - 7.32 (m, 1H), 7.22 (d, *J* = 7.7 Hz, 1H),7.13-7.11 (m, 1H), 7.10-7.07 (m, 1H), 6.98 (s, 1H), 6.94 (s, 1H), 4.66-4.57 (m, 2H), 4.07-4.04 (m, 2H), 3.99-3.96 (m, 1H),3.82-3.79 (m,1H),3.75-3.71 (m,1H), 3.58-5.56 (m,1H), 3.51-3.48 (m,1H), 3.23-3.16 (m,1H), 2.79 (d, *J* = 11.1 Hz, 1H), 2.68 (d, *J* = 11.2 Hz, 1H), 2.41-2.31 (m, 2H),2.37-2.35 (m, 2H) ,2.24-2.19 (m,1H) , 2.01-1.92 (m,3H) ,1.44 (d, *J* = 7.2 Hz, 6H). (One active hydrogen not shown in NMR) MS (ESI): m/z 588.3 $[M + H]^+$. Mp 157-159°C.

**Example 61: (3Z,6Z)-3-(3-Fluorophenyl)methylene-6-(5-isopropyl-1-(3-(4-(acryloyl)piperazinyl)propylimidazol-4-yl)methylene)piperazine-2,5-dione (PLN-2-61)**

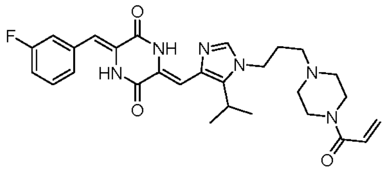

1) To a 500 mL dry round-bottom flask was sequentially added N-Boc-piperazine (10.00 g, 53.69 mmol), cesium carbonate (16.91 g, 107.40 mmol), acetonitrile (200 mL), 1-chloro-3-bromopropane (20.99 g, 64.43 mmol). The mixture was stirred and reacted at room temperature (25°C) for 23 hours. The reaction was monitored by TLC (MeOH: DCM = 1: 10), and the trace amount of raw materials remained and was not further converted. The reaction was stopped and filtered with silica gel, and the filter cake was washed with EA until there was no product, and concentrated under reduced pressure to obtain 12.00 g of 4-(3-chloropropyl)-1-tert-butoxycarbonylpiperazine as a colorless oily liquid with a yield of 85%.
2) To a 100 mL dry round-bottom flask was sequentially added (Z)-1-acetyl-3-((5-isopropyl-1H-imidazol-4-yl)methylene)piperazine-2,5-dione (1.50 g, 5.43 mmol), 4-(3-chloropropyl)-1-tert-butoxycarbonylpiperazine (2.14 g, 8.14 mmol), cesium carbonate (3.54 g, 10.86 mmol), potassium iodide (0.90 g, 5.43 mmol), a 4Å molecular sieve (3.00 g), and DMF (30 mL). The mixture was placed in an oil bath at 70°C under nitrogen atmosphere, stirred and reacted for 20 hours. After the reaction was completed, the reaction mixture was concentrated under reduced pressure, redissolved with 60 mL of a mixed solvent (EtOH: DCM = 1:5), filtered under reduced pressure, and the filter cake was washed with EtOH: DCM = 1:5, concentrated under reduced pressure to obtain 2.73 g of a crude product as a brown oil with a yield of 99%. The crude product was used directly in the next step without purification.
3) To a 100 mL dry brown round-bottom flask was sequentially added (Z)-1-acetyl-3-((5-isopropyl-1-(3-(4-tert-butoxycarbonylpiperazin-1-yl)propyl)-1H-imidazol-4-yl)methylene)piperazine-2,5-dione (2.73 g, 5.43 mmol), 3-fluorobenzaldehyde (0.80 g, 6.52 mmol), cesium carbonate (2.65 g, 8.14 mmol), anhydrous sodium sulfate (1.54 g, 10.86 mmol), and DMF (40 mL). The mixture was degassed, placed in an oil bath at 45°C under nitrogen atmosphere, stirred and reacted for 18 hours. The completion of the reaction was monitored by TLC. The reaction mixture was dropped into 100 mL of ice water, and a yellow solid was precipitated. The mixture was filtered, and the filter cake was dissolved by adding 160 mL of DCM, concentrated, then purified by column chromatography to obtain 1.30 g of a yellow solid with a yield of 42%.
4) To a 100 mL dry brown round-bottom flask was sequentially added (3Z,6Z)-3-(3-fluorophenyl)methylene-6-((5-isopropyl-1-(3-piperazinyl)propylimidazol-4-yl)methylene)piperazine-2,5-dione (1.30 g, 2.29 mmol) and 30 mL of MeOH at 0°C, and 4 mol/L HCl/MeOH (23 mL, 91.6 mmol) was added dropwise thereto. After the addition, the mixture was naturally warmed to 25°C and reacted for 20 hours. The mixture was directly concentrated to obtain 1.32 g of an off-white solid with a yield of 99%.
5) To a 100 mL dry brown round-bottom flask was sequentially added (3Z,6Z)-3-(3-fluorophenyl)methylene-6-((5-isopropyl-1-(3-piperazinyl)propylimidazol-4-yl)methylene)piperazine-2,5-dione (0.60 g, 1.04 mmol), 20 mL of DCM, TEA (0.53 g, 5.20 mmol), and acryloyl chloride (0.11 g, 1.25 mmol) at 0°C, and after the addition, the mixture was naturally warmed to 25°C and reacted for 16 hours. The completion of reaction was detected by TLC. The mixture was directly concentrated and subjected to column chromatography to obtain 0.32 g of a white solid with a yield of 59%.

$^1$H NMR (600 MHz, $CDCl_3$) δ 12.36 (s, 1H), 8.03 (s, 1H), δ 7.49 (s, 1H), 7.44-7.41 (m, 1H), 7.18 (d, *J* = 7.7 Hz, 1H), 7.06 (m, 2H), 6.94 (s, 1H), 6.90 (s, 1H), 6.57 (dd, *J* = 16.8, 10.6 Hz, 1H), 6.30 (dd, *J* = 16.8, 1.6 Hz, 1H), 5.72 (dd, *J* = 10.6, 1.6 Hz, 1H), 4.04 (t, *J* = 6.9 Hz, 2H), 3.71-3.60 (m, 4H), 3.20-3.13 (m, 1H), 2.44 (s, 4H), 2.34 (t, *J* = 6.6 Hz, 2H), 1.94-1.90 (m, 2H), 1.41 (d, *J* = 7.2 Hz, 6H). MS (ESI): m/z 521.3 $[M + H]^+$. Mp 182-184°C.

**Example 62: (3Z,6Z)-3-(3,5-Difluorophenyl)methylene-6-((5-isopropyl-1-(3-morpholinyl)propylimidazol-4-yl) methylene)piperazine-2,5-dione (PLN-2-62)**

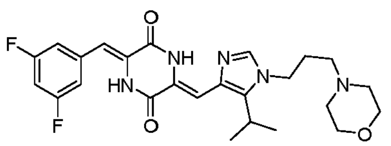

1) To a 25 mL single-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-ethanesulfonylpropyl)morpholine (1.29 g, 5.43 mmol), cesium carbonate (1.18 g, 3.62 mmol), potassium iodide (0.33 g, 1.99 mmol), and DMF (15 mL). The mixture was placed in an oil bath at 70°C under nitrogen atmosphere, stirred and reacted for 30 hours. After the reaction was completed, the reaction mixture was concentrated under reduced pressure, redissolved with 60 mL of DCM, filtered under reduced pressure, and the filtrate was concentrated under reduced pressure to obtain 0.72 g of a crude product as a brown oil with a yield of 99%. The crude product was used directly in the next step without purification.

2) To a 25 mL dry round-bottom flask was sequentially added (Z)-1-acetyl-3-((5-isopropyl-1-(3-morpholinyl)propyl-imidazol-4-yl)methylene)piperazine-2,5-dione (0.72 g, 1.78 mmol), 3,5-difluorobenzaldehyde (304.2 mg, 2.14 mmol), cesium carbonate (871.9 mg, 2.68 mmol), anhydrous sodium sulfate (514.0 mg, 3.62 mmol), and DMF (10 mL). The mixture was degassed, placed in an oil bath at 45°C under nitrogen atmosphere, stirred and reacted for 18 hours. The completion of the reaction was monitored by TLC. After the reaction was completed, the reaction mixture was dropped into 100 mL of ice water, and a yellow solid was precipitated. The mixture was filtered, and the filter cake was dissolved by adding 40 mL of DCM, concentrated, then purified by column chromatography to obtain 200.0 mg of a yellow solid with a yield of 23%.

$^1$H NMR (500 MHz, $CDCl_3$) δ 12.33 (s, 1H), 8.38 (s, 1H), 7.53 (s, 1H), 6.96-6.95 (m, 3H), 6.92 (s, 1H), 6.84-6.81 (m, 1H), 4.06 (t, *J*= 7.0 Hz, 2H), 3.82-3.72 (m, 4H), 3.24-3.14 (m, 1H), 2.49 (s, 4H), 2.38 (t, *J* = 6.7 Hz, 2H), 1.98-1.94 (m, 2H), 1.44 (d, *J*= 7.2 Hz, 6H). MS (ESI): m/z 485.2 $[M + H]^+$.

**Example 63: (3Z,6Z)-3-(3,4-Difluorophenyl)methylene-6-((5-isopropyl-1-(3-morpholinyl)propylimidazol-4-yl) methylene)piperazine-2,5-dione (PLN-2-63)**

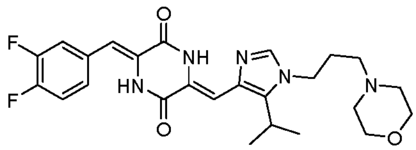

1) To a 25 mL single-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-ethanesulfonylpropyl)morpholine (1.29 g, 5.43 mmol), cesium carbonate (1.18 g, 3.62 mmol), potassium iodide (0.33 g, 1.99 mmol), and DMF (15 mL). The mixture was placed in an oil bath at 70°C under nitrogen atmosphere, stirred and reacted for 30 hours. After the reaction was completed, the reaction mixture was concentrated under reduced pressure, redissolved with 60 mL of DCM, filtered under reduced pressure, and the filtrate was concentrated under reduced pressure to obtain 0.72 g of a crude product as a brown oil with a yield of 99%. The crude product was used directly in the next step without purification.

2) To a 25 mL dry round-bottom flask was sequentially added (Z)-1-acetyl-3-((5-isopropyl-1-(3-morpholinyl)propyl-imidazol-4-yl)methylene)piperazine-2,5-dione (0.72 g, 1.78 mmol), 3,4-difluorobenzaldehyde (304.2 mg, 2.14 mmol), cesium carbonate (871.9 mg, 2.67 mmol), anhydrous sodium sulfate (514.0 mg, 3.62 mmol), and DMF (10 mL). The mixture was degassed, placed in an oil bath at 45°C under nitrogen atmosphere, stirred and reacted for 18 hours. The completion of the reaction was monitored by TLC. After the reaction was completed, the reaction mixture was dropped into 100 mL of ice water, and a yellow solid was precipitated. The mixture was filtered, and the filter cake was dissolved by adding 40 mL of DCM, concentrated, then purified by column chromatography to obtain 170 mg of a yellow solid with a yield of 20%.

$^1$H NMR (500 MHz, $CDCl_3$) δ 12.32 (s, 1H), 8.07 (s, 1H), 7.53 (s, 1H), 7.29-7.24 (m, 2H), 7.19-7.18 (m, 1H), 6.95-6.92 (m, 2H),4.07 (t, *J* = 7.0 Hz, 2H), 3.78-3.77 (m, 4H), 3.23-3.18 (m, 1H), 2.48 (s, 4H), 2.36 (t, *J* = 6.7 Hz, 2H), 1.97-1.93 (m, 2H), 1.44 (d, *J* = 7.2 Hz, 6H). MS (ESI): m/z 485.2 $[M + H]^+$.

**Example 64: (3Z,6Z)-3-(3-Fluorophenyl)methylene-6-(5-isopropyl-1-(3-(4-(acetyl)piperazinyl)propylimidazol-4-yl)methylene)piperazine-2,5-dione (PLN-2-64)**

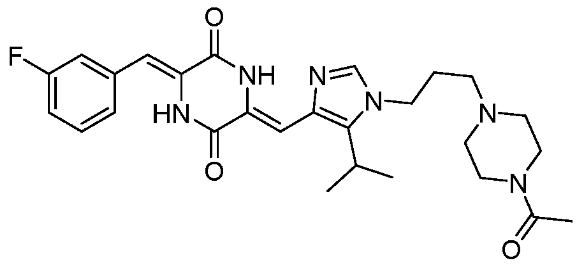

1) To a 500 mL dry round-bottom flask was sequentially added N-Boc-piperazine (10.00 g, 53.69 mmol), cesium carbonate (16.91 g, 107.40 mmol), acetonitrile (200 mL), and 1-chloro-3-bromopropane (20.99 g, 64.43 mmol). The mixture was stirred and reacted at room temperature (25°C) for 23 hours. The reaction was monitored by TLC (MeOH: DCM = 1:10), and the trace amount of raw materials remained and was not further converted. The reaction was stopped and filtered with silica gel, and the filter cake was washed with EA until there was no product, and concentrated under reduced pressure to obtain 12.00 g of 4-(3-chloropropyl)-1-tert-butoxycarbonylpiperazine as a colorless oily liquid with a yield of 85%.

2) To a 100 mL dry round-bottom flask was sequentially added (Z)-1-acetyl-3-((5-isopropyl-1H-imidazol-4-yl)methylene)piperazine-2,5-dione (1.50 g, 5.43 mmol), 4-(3-chloropropyl)-1-tert-butoxycarbonylpiperazine (2.14 g, 8.14 mmol), cesium carbonate (3.54 g, 10.86 mmol), potassium iodide (0.90 g, 5.43 mmol), a 4Å molecular sieve (3.00 g), and DMF (30 mL). The mixture was placed in an oil bath at 70°C under nitrogen atmosphere, stirred and reacted for 20 hours. After the reaction was completed, the reaction mixture was concentrated under reduced pressure, redissolved with 60 mL of a mixed solvent (EtOH: DCM = 1:5), filtered under reduced pressure, and the filter cake was washed with EtOH: DCM = 1:5, concentrated under reduced pressure to obtain 2.73 g of a crude product as a brown oil with a yield of 99%. The crude product was used directly in the next step without purification.

3) To a 100 mL dry brown round-bottom flask was sequentially added (Z)-1-acetyl-3-((5-isopropyl-1-(3-(4-tert-butoxycarbonylpiperazin-1-yl)propyl)-1H-imidazol-4-yl)methylene)piperazine-2,5-dione (2.73 g, 5.43 mmol), 3-fluorobenzaldehyde (0.80 g, 6.52 mmol), cesium carbonate (2.65 g, 8.14 mmol), anhydrous sodium sulfate (1.54 g, 10.86 mmol), and DMF (40 mL). The mixture was degassed, placed in an oil bath at 45°C under nitrogen atmosphere, stirred and reacted for 18 hours. The completion of the reaction was monitored by TLC. The reaction mixture was dropped into 100 mL of ice water, and a yellow solid was precipitated. The mixture was filtered, and the filter cake was dissolved by adding 160 mL of DCM, concentrated, then purified by column chromatography to obtain 1.30 g of a yellow solid with a yield of 42%.

4) To a 100 mL dry brown round-bottom flask was sequentially added (3Z,6Z)-3-(3-fluorophenyl)methylene-6-((5-isopropyl-1-(3-(4-tert-butoxycarbonylpiperazinyl)propylimidazol-4-yl)methylene)piperazine-2,5-dione (1.30 g, 2.29 mmol) and 30 mL of MeOH at 0°C, and 4 mol/L HCl/MeOH (23 mL, 91.6 mmol) was added dropwise thereto. After the addition, the mixture was naturally warmed to 25°C and reacted for 20 hours. The mixture was directly concentrated to obtain 1.32 g of an off-white solid with a yield of 99%.

5) To a 100 mL dry brown round-bottom flask was sequentially added (3Z,6Z)-3-(3-fluorophenyl)methylene-6-((5-isopropyl-1-(3-piperazinyl)propylimidazol-4-yl)methylene)piperazine-2,5-dione (0.20 g, 0.35 mmol), 20 mL of DCM, TEA (0.18 g, 1.75 mmol), and acetyl chloride (0.04 g, 0.42 mmol) at 0°C, and after the addition, the mixture was naturally warmed to 25°C and reacted for 16 hours. The completion of reaction was detected by TLC. The mixture was directly concentrated and subjected to column chromatography to obtain 126 mg of a white solid with a yield of 71%.

$^1$H NMR (600 MHz, DMSO-$d_6$) δ 12.26 (s, 1H), 8.09 (s, 1H), 7.48 (s, 1H), 7.44-7.41 (m, 1H), 7.18 (d, $J$ = 7.6 Hz, 1H), 7.08 (d, $J$ = 9.4 Hz, 1H),7.06-7.03 (m, 1H), 6.94 (s, 1H), 6.90 (s, 1H), 4.04 (t, $J$ = 6.8 Hz, 2H), 3.65 (s, 2H), 3.51-3.49 (m, 2H), 3.20-3.14 (m, 1H), 2.42-2.40(m, 4H), 2.34 (t, $J$ = 6.5 Hz, 2H), 2.11 (s, 3H), 1.94-1.91(m, 2H), 1.41 (d, $J$ = 7.1 Hz, 6H). MS (ESI): m/z 508.3 $[M + H]^+$.

**Example 65: (3Z,6Z)-3-(3-Fluorophenyl)methylene-6-(5-isopropyl-1-(3-(4-(propionyl)piperazinyl)propylimidazol-4-yl)methylene)piperazine-2,5-dione (PLN-2-65)**

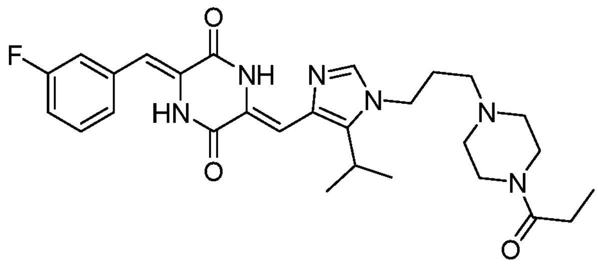

To a 100 mL dry brown round-bottom flask was sequentially added (3Z,6Z)-3-(3-fluorophenyl)methylene-6-((5-isopropyl-1-(3-piperazinyl)propylimidazol-4-yl)methylene)piperazine-2,5-dione (0.20 g, 0.35 mmol), 20 mL of DCM, TEA (0.18 g, 1.75 mmol), and propionyl chloride (0.04 g, 0.42 mmol) at 0°C, and after the addition, the mixture was naturally warmed to 25°C and reacted for 16 hours. The completion of reaction was detected by TLC. The mixture was directly concentrated and subjected to column chromatography to obtain 138 mg of a white solid with a yield of 75%.

$^1H$ NMR (600 MHz, DMSO-$d_6$) δ 12.26 (s, 1H), 8.10 (s, 1H), 7.49 (s, 1H), 7.44-7.40 (m, 1H), 7.18 (d, *J* = 7.6 Hz, 1H), 7.09 (d, *J* = 9.4 Hz, 1H), 7.06-7.03 (m, 1H), 6.94 (s, 1H), 6.90 (s, 1H), 4.04 (t, *J* = 6.8 Hz, 2H), 3.65 (s, 2H), 3.51-3.49 (m, 2H), 3.18-3.14 (m, 1H), 2.42-2.40(m, 4H), 2.37-2.32 (m, 4H), 1.93-1.91(m, 2H), 1.41 (d, *J* = 7.1 Hz, 6H),1.16 (t, *J* = 7.4 Hz, 3H). MS (ESI): m/z 522.3 $[M + H]^+$.

**Example 66: (3Z,6Z)-3-(3-Fluorophenyl)methylene-6-(5-isopropyl-1-(3-(4-(n-butyryl)piperazinyl)propylimidazol-4-yl)methylene)piperazine-2,5-dione (PLN-2-66)**

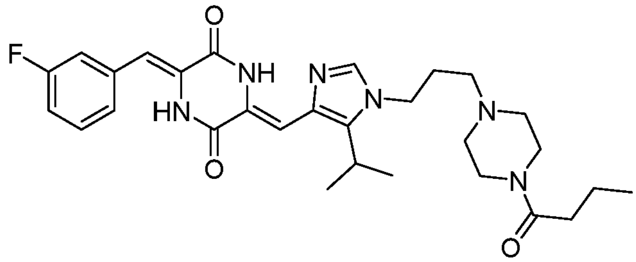

To a 100 mL dry brown round-bottom flask was sequentially added (3Z,6Z)-3-(3-fluorophenyl)methylene-6-((5-isopropyl-1-(3-piperazinyl)propylimidazol-4-yl)methylene)piperazine-2,5-dione (0.20 g, 0.35 mmol), 20 mL of DCM, TEA (0.18 g, 1.75 mmol), and n-butyryl chloride (0.04 g, 0.42 mmol) at 0°C, and after the addition, the mixture was naturally warmed to 25°C and reacted for 16 hours. The completion of reaction was detected by TLC. The mixture was directly concentrated and subjected to column chromatography to obtain 122 mg of a white solid with a yield of 65%.

$^1H$ NMR (600 MHz, DMSO-$d_6$) δ 12.25 (s, 1H), 8.24 (s, 1H), 7.48 (s, 1H), 7.44-7.40 (m, 1H), 7.18 (d, *J* = 7.7 Hz, 1H), 7.09 (d, *J* = 9.5 Hz, 1H), 7.06-7.04 (m, 1H), 6.94 (s, 1H), 6.90 (s, 1H), 4.03 (t, *J* = 6.8 Hz, 2H), 3.66 (s, 2H), 3.52-3.50 (m, 2H), 3.19-3.12 (m, 1H), 2.44-2.40(m, 4H), 2.35-2.30 (m, 4H), 1.95-1.90(m, 2H),1.69-1.63(m, 2H) 1.41 (d, *J* = 7.1 Hz, 6H), 0.97 (t, *J* = 7.4 Hz, 3H). MS (ESI): m/z 536.3 $[M + H]^+$.

**Example 67: (3Z,6Z)-3-(3-Fluorophenyl)methylene-6-((5-isopropyl-1-(3-morpholinyl)-2-methylpropylimidazol-4-yl)methylene)piperazine-2,5-dione** (PLN-2-67)

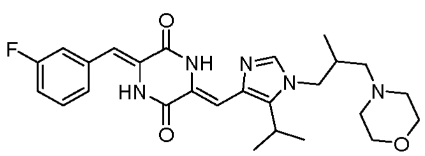

1) To a 250 mL dry round-bottom flask was sequentially added morpholine (0.75 g, 8.75 mmol), acetonitrile (50 mL), cesium carbonate (4.28 g, 13.13 mmol), and 1-chloro-3-bromo-2-methylpropane (3.00 g, 17.50 mmol), and the mixture was stirred and reacted at room temperature (25°C) for 18 hours. The reaction was monitored by TLC (MeOH:

DCM = 1:10), and the reaction was balanced. The mixture was filtered with diatomite, eluted with a mixed solvent (PE: EA-1:1), and concentrated under reduced pressure to obtain 3.00 g of N-(3-chloro-2-methylpropyl)morpholine as a colorless oily liquid with a yield of 48%.

2) To a 25 mL single-necked dry round-bottom flask was sequentially added (Z)-1-acetyl-3-((5-isopropyl-1H-imidazol-4-yl)methylene)piperazine-2,5-dione (0.50 g, 1.81 mmol), N-(3-chloro-2-methylpropyl)morpholine (0.64 g, 3.62 mmol), cesium carbonate (1.18 g, 3.62 mmol), potassium iodide (0.30 g, 1.81 mmol), and DMF (10 mL). The mixture was placed in an oil bath at 70°C under nitrogen atmosphere, stirred and reacted for 20 hours. After the reaction was completed, the reaction mixture was concentrated under reduced pressure, redissolved with 60 mL of DCM, filtered under reduced pressure, and the filtrate was concentrated under reduced pressure to obtain 0.72 g of a crude product as a brown oil with a yield of 99%. The crude product was used directly in the next step without purification.

3) To a 25 mL dry round-bottom flask was sequentially added (Z)-1-acetyl-3-((5-isopropyl-1-(3-morpholinyl))-2-methylpropyl-imidazol-4-yl)methylene)piperazine-2,5-dione (0.72 g, 1.78 mmol), 3-fluorobenzaldehyde (0.30 g, 2.14 mmol), cesium carbonate (0.87 g, 2.68 mmol), anhydrous sodium sulfate (0.51 g, 3.62 mmol), and DMF (10 mL). The mixture was degassed, placed in an oil bath at 45°C under nitrogen atmosphere, stirred and reacted for 18 hours. The completion of the reaction was monitored by TLC. After the reaction was completed, the reaction mixture was dropped into 100 mL of ice water, and a small amount of yellow solid was precipitated. The mixture was filtered, and the filter cake was dissolved by adding 40 mL of DCM, concentrated and then purified by preparative thin-layer chromatography to obtain 10.0 mg of a yellow solid with a yield of 1%.

$^1$H NMR (600 MHz, $CDCl_3$) δ 12.28 (s, 1H), 8.04 (s, 1H), 7.46 (s, 1H), 7.44-7.42 (m, 1H), 7.19-7.17 (m, 1H), 7.09-7.07 (m, 1H), 7.06-7.03 (m, 1H),6.94 (s, 1H), 6.92 (s, 1H), 4.17-4.14 (m, 1H), 3.74-3.63 (m, 5H), 3.17-3.09 (m, 1H), 2.49 (s, 2H),2.39 (s, 2H),2.26-2.17 (m, 2H), 2.05 (s, 1H), 1.41 (d, *J* = 7.2 Hz, 6H),0.91 (d, *J* = 6.6 Hz, 3H). MS (ESI): m/z 482.3 $[M + H]^+$. M.p 196-198°C.

**I. Preparation of hydrochloride of derivatives of 2,5-diketopiperazine compounds**

**(I) Preparation of hydrochloride of derivatives of 2,5-diketopiperazine compounds**

General procedure: A compound (1.0 eq) was placed in a dry round-bottom flask, dissolved with an appropriate amount of methanol, and concentrated hydrochloric acid (10.0 eq) was added dropwise thereto, and the mixture was stirred for 2 hours at room temperature in the dark. After the reaction was completed, the mixture was concentrated under reduced pressure until there was no solvent, dissolved with a small amount of methanol, dropped into ethyl acetate or acetone to precipitate a solid, and the mixture was placed in a cold trap at 0°C and stirred for 2 hours, filtered under reduced pressure. The filter cake was washed with cold ethyl acetate or acetone, dried under vacuum at 50°C to obtain a white solid, which was formed into a salt with hydrochloric acid in a quantitative molar ratio (compound: hydrochloric acid = 1:2).

**Example 1: (3Z,6Z)-3-(3-(p-Fluorobenzoyl)phenyl)methylene-6-((5-isopropyl-1-(3-morpholinyl)propylimidazol-4-yl)methylene)piperazine-2,5-dione, hydrochloride (PLN-2-1-1)**

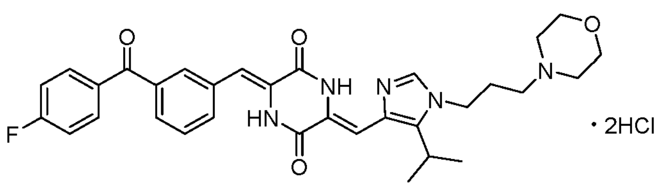

To a 25 mL single-necked dry round-bottom flask was sequentially added (3Z,6Z)-3-(3-(p-fluorobenzoyl)phenyl) methylene-6-((5-isopropyl-1-(3-morpholinyl)propylimidazol-4-yl)methylene)piperazine-2,5-dione (120.0 mg, 0.21 mmol) and MeOH (7 mL), and HCl/MeOH (4 mol/L, 8 mL) was added dropwise thereto at 0°C, and the whole process was protected from light. After the addition, the mixture was naturally warmed to room temperature and reacted for 2 hours. The reaction mixture was concentrated, and a small amount of MeOH was added thereto to just dissolve the product, and the mixture was added dropwise to 12 mL of EA to precipitate a solid, stirred for 10 min, and then filtered. The filter cake was dried to obtain 128.8 mg of a light yellow solid with a yield of 95%.

$^1$H NMR (600 MHz, $CDCl_3$) δ 11.83 (s, 1H), 10.43 (s, 1H), 8.16 (s, 1H), 7.93-7.90 (m, 2H), 7.83 (s, 1H), 7.76 (d, *J* = 7.6 Hz, 1H), 7.65 (d, *J* = 7.6 Hz, 1H), 7.60 (t, *J* = 7.6 Hz, 1H), 7.41 (t, *J* = 8.7 Hz, 2H), 6.83 (s, 1H), 6.68 (s, 1H), 4.19 (t, *J* = 6.9

Hz, 2H), 3.94 (d, *J* = 12.3 Hz, 3H), 3.96-3.93 (m, 3H), 3.42 (d, *J* = 12.0 Hz, 2H), 3.28-3.21 (m, 1H), 3.17-3.09 (m, 2H), 3.07-3.02 (m, 2H), 2.23-2.18 (m, 2H), 1.34 (d, *J* = 7.1 Hz, 6H).

**Example 2: (3Z,6Z)-3-(3-(p-Fluorophenoxy)phenyl)methylene-6-((5-isopropyl-1-(3-morpholinyl)propylimidazol-4-yl)methylene)piperazine-2,5-dione, hydrochloride (PLN-2-2-1)**

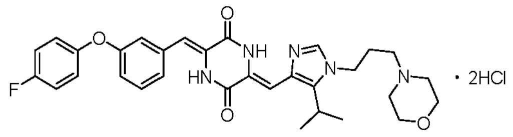

To a 50 mL single-necked dry round-bottom flask was sequentially added (3Z,6Z)-3-(3-(p-fluorophenoxy)phenyl) methylene-6-((5-isopropyl-1-(3-morpholinyl)propylimidazol-4-yl)methylene)piperazine-2,5-dione (173.5 mg, 0.31 mmol), MeOH (20 mL), and concentrated hydrochloric acid (1.26 mL, 15.5 mmol), and the whole process was protected from light. The mixture was reacted at room temperature for 2 hours, a solid was precipitated, and the mixture was stirred at 0°C for 2 hours, filtered, and the filter cake was dried to obtain 74.5 mg of a white solid with a yield of 75%.

**Example 3: (3Z,6Z)-3-(3-(p-Fluorobenzoyl)phenyl)methylene-6-((5-cyclopropyl-1-(3-morpholinyl)propylimidazol-4-yl)methylene)piperazine-2,5-dione, hydrochloride (PLN-2-3-1)**

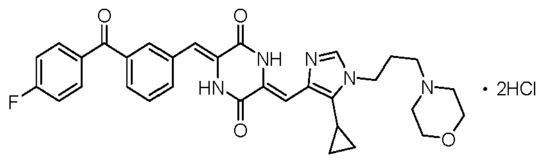

To a 100 mL single-necked dry round-bottom flask was sequentially added (3Z,6Z)-3-(3-(p-fluorobenzoyl) phenyl)methylene-6-((5-cyclopropyl-1-(3-**morpholinyl)propylimidazol-4-yl)methylene)piperazine-2,5-dione** (360.0 mg, 0.63 mmol) and MeOH (20 mL), and HCl/MeOH (4 mol/L, 30 mL) was added dropwise thereto at 0°C, and the whole process was protected from light. After the addition, the mixture was naturally warmed to room temperature and reacted for 2 hours. The reaction mixture was concentrated, a small amount of MeOH was added thereto to just dissolve the product, and the mixture was added dropwise to 35 mL of EA to precipitate a solid, stirred for 10 min, and then filtered. The filter cake was dried to obtain 401.0 mg of a light yellow solid with a yield of 99%.

**Example 4: (3Z,6Z)-3-(3-(p-Fluorophenoxy)phenyl)methylene-6-((5-cyclopropyl-1-(3-morpholinyl)propylimidazol-4-yl)methylene)piperazine-2,5-dione, hydrochloride (PLN-2-4-1)**

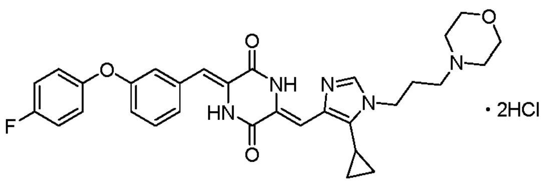

To a 50 mL single-necked dry round-bottom flask was sequentially added (3Z,6Z)-3-(3-(p-fluorophenoxy)phenyl) methylene-6-((5-cyclopropyl-1-(3-morpholinyl)propylimidazol-4-yl)methylene)piperazine-2,5-dione (127 mg, 0.23 mmol), MeOH (15 mL), and concentrated hydrochloric acid (0.94 mL, 11.5 mmol), and the whole process was protected from light. The mixture was reacted at room temperature for 2 hours, a solid was precipitated, and the mixture was stirred at 0°C for 2 hours, filtered. The filter cake was dried to obtain 121.7 mg of a white solid with a yield of 75%.

**Example 5: (3Z,6Z)-3-Benzylidene-6-((5-isopropyl-1-(3-morpholinyl)propylimidazol-4-yl)methylene)piperazine-2,5-dione, hydrochloride (PLN-2-5-1)**

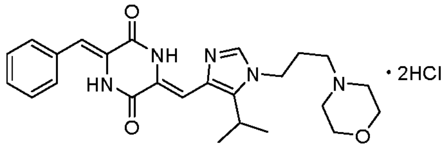

To a 25 mL single-necked dry round-bottom flask was sequentially added (3Z,6Z)-3-benzylidene-6-((5-isopropyl-1-(3-morpholinyl)propylimidazol-4-yl)methylene)piperazine-2,5-dione (50.0 mg, 0.11 mmol) and MeOH (3 mL), and HCl/MeOH (4 mol/L, 3 mL) was added dropwise thereto at 0°C, and the whole process was protected from light. After the addition, the mixture was naturally warmed to room temperature and reacted for 2 hours. The reaction mixture was concentrated, and a small amount of MeOH was added thereto to just dissolve the product, and the mixture was added dropwise to 5 mL of EA to precipitate a solid, stirred for 10 min, and then filtered. The filter cake was dried to obtain 55.2 mg of a light yellow solid with a yield of 95%.

$^1$H NMR (600 MHz, DMSO-$d_6$) δ 11.81 (s, 1H), 10.14 (s, 1H), 8.15 (s, 1H),7.53 (d, *J* = 7.6 Hz, 2H), 7.43 (t, *J* = 7.6 Hz, 2H), 7.33 (t, *J* = 7.3 Hz, 1H), 6.77 (s, 1H), 6.68 (s, 1H), 4.19 (t, *J* = 6.9 Hz, 2H), 3.94 (d, *J* = 10.9 Hz, 2H), 3.87 (t, *J* = 11.9 Hz, 2H), 3.42 (d, *J* = 12.0 Hz, 2H), 3.25-3.22 (m, 1H), 3.14-3.10 (m, 2H),3.07-3.02 (m, 2H),2.22-2.20 (m, 2H),1.34 (d, *J* = 7.0 Hz, 6H).

**Example 6: (3Z,6Z)-3-(2,5-Difluorophenyl)methylene-6-((5-isopropyl-1-(3-morpholinyl)propylimidazol-4-yl) methylene)piperazine-2,5-dione, hydrochloride (PLN-**2-7-1)

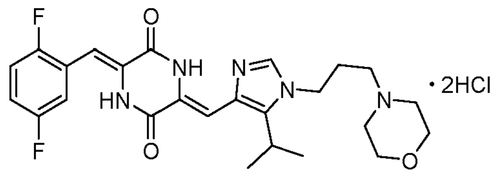

To a 25 mL single-necked dry round-bottom flask was sequentially added (3Z,6Z)-3-(3-(p-fluorobenzoyl)phenyl) methylene-6-((5-isopropyl-1-(3-morpholinyl)propylimidazol-4-yl)methylene)piperazine-2,5-dione (200.0 mg, 0.41 mmol) and MeOH (15 mL), and HCl/MeOH (4 mol/L, 15 mL) was added dropwise thereto at 0°C, and the whole process was protected from light. After the addition, the mixture was naturally warmed to room temperature and reacted for 2 hours. The reaction mixture was concentrated, and a small amount of MeOH was added thereto to just dissolve the product, and the mixture was added dropwise to 9 mL of EA to precipitate a solid, stirred for 10 min, and then filtered. The filter cake was dried to obtain 218.0 mg of a light yellow solid with a yield of 95%.

$^1$H NMR (600 MHz, DMSO-$d_6$) δ 11.92 (s, 1H), 10.53 (s, 1H), 8.12 (s, 1H), 7.43-7.40 (m, 1H),7.32-7.28 (m, 1H),7.24-7.20 (m,1H), 6.71 (s, 1H),6.63 (s, 1H), 4.20-4.17 (m, 2H) 3.95 (d, J = 10.3 Hz, 2H), 3.86 (t, J = 11.8 Hz, 2H), 3.42 (d, J = 12.0 Hz, 2H), 3.27-3.22 (m, 1H),3.17-3.10 (m, 2H),3.07-3.02 (m, 2H), 2.12-2.09 (m, 2H), 1.34 (d, J = 7.1 Hz, 6H).

**Example 7: (3Z,6Z)-3-(3-Fluorophenyl)methylene-6-((5-isopropyl-1-(3-morpholinyl)propylimidazol-4-yl)methylene)piperazine-2,5-dione, hydrochloride (PLN-2-9-1)**

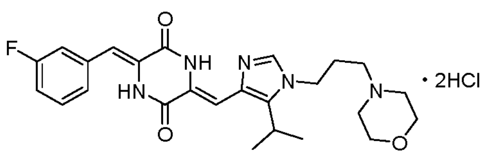

To a 100 mL single-necked dry round-bottom flask was sequentially added (3Z,6Z)-3-(3-fluorophenyl)methylene-6-((5-isopropyl-1-(3-morpholinyl)propylimidazol-4-yl)methylene)piperazine-2,5-dione (200.0 mg, 0.43 mmol) and

MeOH (10 mL), and HCl/MeOH (4 mol/L, 15 mL) was added dropwise thereto at 0°C, and the whole process was protected from light. After the addition, the mixture was naturally warmed to room temperature and reacted for 2 hours, and the reaction mixture was concentrated, a small amount of MeOH was added thereto to just dissolve the product. The mixture was added dropwise to 15 mL of EA to precipitate a solid, stirred for 10 min, then filtered, dried, and the filter cake was taken to obtain 218.0 mg of a light yellow solid with a yield of 94%.

$^1$H NMR (600 MHz, DMSO-$d_6$) δ 11.84 (s, 1H), 10.36 (s, 1H), 8.15 (s, 1H), 7.49 - 7.40 (m, 1H), 7.37 (d, *J* = 10.2 Hz, 1H), 7.34 (d, *J* = *7.8* Hz, 1H), 7.17-7.14 (m, 1H), 6.75 (s, 1H), 6.69 (s, 1H), 4.19 (t, *J* = 7.2 Hz, 2H), 3.96-3.93 (m, 2H), 3.86 (t, *J* = 11.5 Hz, 2H), 3.42 (d, J = 12.2 Hz, 2H), 3.28-3.21 (m, 1H), 3.14-3.10 (m, 2H), 3.08-3.02 (m, 2H), 2.23-2.18 (m, 2H), 1.34 (d, *J* = 7.1 Hz, 6H).

**Example 8: (3Z,6Z)-3-(3-Fluorophenyl)methylene-6-((5-cyclopropyl-1-(3-morpholinyl)propylimidazol-4-yl) methylene)piperazine-2,5-dione, hydrochloride (PLN-2-10-1)**

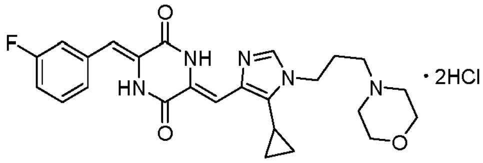

To a 100 mL single-necked dry round-bottom flask was sequentially added (3Z,6Z)-3-(3-fluorophenyl)methylene-6-((5-cyclopropyl-1-(3-morpholinyl)propylimidazol-4-yl)methylene)piperazine-2,5-dione (200.0 mg, 0.43 mmol) and MeOH (10 mL), and HCl/MeOH (4 mol/L, 15 mL) was added dropwise thereto at 0°C, and the whole process was protected from light. After the addition, the mixture was naturally warmed to room temperature and reacted for 2 hours. The reaction mixture was concentrated, and a small amount of MeOH was added thereto to just dissolve the product. The mixture was added dropwise to 15 mL of EA to precipitate a solid, stirred for 10 min, then filtered, dried, and the filter cake was taken to obtain 222.8 mg of a light yellow solid with a yield of 98%.

$^1$H NMR (600 MHz, DMSO-$d_6$) δ 11.80 (s, 1H), 10.37 (s, 1H), 8.27 (s, 1H), 7.45 - 7.30 (m, 3H), 7.15( s, 1H), 6.75 (s, 1H), 6.69 (s, 1H), 4.25 (s, 2H), 3.94-3.89 (m, 4H), 3.43 (s, 2H), 3.13-3.06 (m, 4H), 2.35 (s, 2H), 1.87 (s, 1H), 1.08 (s, 2H), 0.70 (s, 2H).

**Example 9: (3Z,6Z)-3-(3-Fluorophenyl)methylene-6-(S-isopropyl-1-(3-(R)-(2-methylmorpholinyl)propylimidazol-4-yl)methylene)piperazine-2,5-dione, hydrochloride (PLN-2-56-1)**

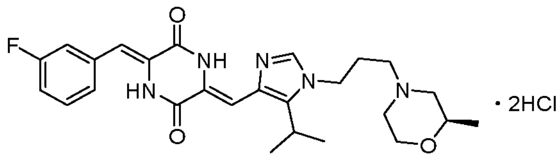

To a 25 mL single-necked dry round-bottom flask was sequentially added (3Z,6Z)-3-(3-(fluorophenyl)methylene-6-(5-isopropyl-1-(3-(*R*)-(2-methylmorpholinyl)propylimidazol-4-yl)methylene)piperazine-2,5-dione (100.0 mg, 0.21 mmol) and MeOH (6 mL), and HCl/MeOH (4 mol/L, 7 mL) was added dropwise thereto at 0°C, and the whole process was protected from light. After the addition, the mixture was naturally warmed to room temperature and reacted for 2 hours. The reaction mixture was concentrated, and a small amount of MeOH was added thereto to just dissolve the product, and the mixture was added dropwise to 9 mL of EA to precipitate a solid, stirred for 10 min, and then filtered. The filter cake was dried to obtain 106.0 mg of a light yellow solid with a yield of 92%.

$^1$H NMR (600 MHz, DMSO-$d_6$) δ 11.97 (s, 1H), 10.32 (s, 1H), 8.00 (s, 1H), 7.48-7.40 (m, 2H),7.38-7.33 (m, 2H),7.16-7.13 (m, 1H),6.74 (s, 1H),6.72 (s, 1H), 4.16 (t, *J* = 7.2 Hz, 2H), 3.99-3.92 (m, 2H), 3.90-3.86 (m, 1H), 3.45-3.39 (m, 1H), 3.28-3.21 (m, 1H),3.12-3.05 (m, 2H),2.98-2.92 (m, 1H),2.71-2.63 (m, 2H),2.22-2.18 (m, 2H),1.35 (d, *J* = 7.1 Hz, 6H),1.12 (d, *J* = 6.3 Hz, 3H).

**Example 10: (3Z,6Z)-3-(3-Fluorophenyl)methylene-6-(5-isopropyl-1-(3-5)-(-2-methylmorpholinyl)propylimidazol-4-yl)methylene)piperazine-2,5-dione, hydrochloride (PLN-2-57-1)**

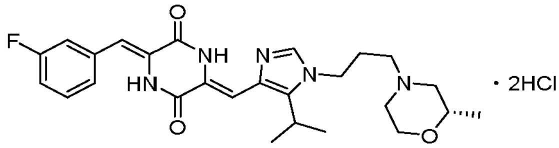

To a 25 mL single-necked dry round-bottom flask was sequentially added (3Z,6Z)-3-(3-fluorophenyl)methylene-6-(5-isopropyl-1-(3-(R)-(2-methylmorpholinyl)propylimidazol-4-yl)methylene)piperazine-2,5-dione (100.0 mg, 0.21 mmol) and MeOH (6 mL), and HCl/MeOH (4 mol/L, 7 mL) was added dropwise thereto at 0°C, and the whole process was protected from light. After the addition, the mixture was naturally heated to room temperature and reacted for 2 hours. The reaction mixture was concentrated, and a small amount of MeOH was added thereto to just dissolve the product, and the mixture was added dropwise to 9 mL of EA to precipitate a solid, stirred for 10 min, and then filtered. The filter cake was dried to obtain 107.0 mg of a light yellow solid with a yield of 93%.

$^1$H NMR (600 MHz, DMSO-$d_6$) $\delta$ 11.93 (s, 1H), 10.33 (s, 1H), 8.06 (s, 1H), 7.49-7.41 (m, 2H),7.38-7.33 (m, 2H),7.17-7.13 (m, 1H),6.74 (s, 1H),6.71 (s, 1H), 4.17 (t, *J* = 7.2 Hz, 2H), 4.01-3.86 (m, 3H), 3.46 *(d, J*= 11.8 Hz, 1H), 3.40 *(d, J*= 12.2 Hz, 1H), 3.28-3.19 (m, 1H),3.11-3.07 (m, 2H),2.98-2.93 (m, 1H),2.71-2.63 (m, 1H),2.22-2.18 (m, 2H),1.35 (d, *J* = 7.1 Hz, 6H),1.12 (d, *J* = 6.3 Hz, 3H).

**Example 11: (3Z,6Z)-3-(3,5-Difluorophenyl)methylene-6-((5-isopropyl-1-(3-morpholinyl)propylimidazol-4-yl) methylene)piperazine-2,5-dione, hydrochloride (PLN-2-62-1)**

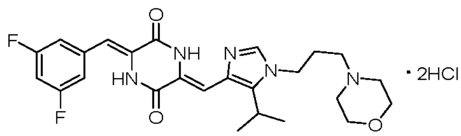

To a 25 mL single-necked dry round-bottom flask was sequentially added (3Z,6Z)-3-(3,5-difluorophenyl)phenyl) methylene-6-((5-isopropyl-1-(3-morpholinyl)propylimidazol-4-yl)methylene)piperazine-2,5-dione (80.0 mg, 0.16 mmol) and MeOH (5 mL), and HCl/MeOH (4 mol/L, 6 mL) was added dropwise thereto at 0°C, and the whole process was protected from light. After the addition, the mixture was naturally warmed to room temperature and reacted for 2 hours. The reaction mixture was concentrated, and a small amount of MeOH was added thereto to just dissolve the product, and the mixture was added dropwise to 9 mL of EA to precipitate a solid, stirred for 10 min, and then filtered. The filter cake was dried to obtain 85.4 mg of a light yellow solid with a yield of 93%.

$^1$H NMR (600 MHz, DMSO-$d_6$) $\delta$ 11.91 (s, 1H), 10.49 (s, 1H), 8.10 (s, 1H), 7.23 (d, *J* = 6.8 Hz, 2H),7.19-7.16 (m,1H), 6.71 (s, 2H), 4.18 (t, *J* = 7.1 Hz, 2H), 3.95-3.84 (m, 4H), 3.42 (d, *J* = 12.1 Hz, 2H), 3.28-3.21 (m, 1H),3.13-3.10 (m, 2H), 3.07-3.02 (m, 2H), 2.22-2.17 (m, 2H),1.34 (d, *J = 7.1* Hz, 6H).

**Example 12: (3Z,6Z)-3-(3,4-Difluorophenyl)methylene-6-((5-isopropyl-1-(3-morpholinyl)propylimidazol-4-yl) methylene)piperazine-2,5-dione, hydrochloride (PLN-2-63-1)**

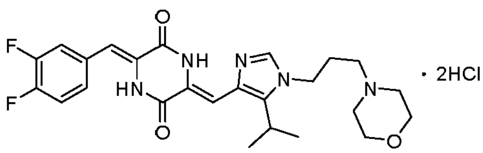

To a 25 mL single-necked dry round-bottom flask was sequentially added (3Z,6Z)-3-(3,4-difluorophenyl) methylene-6-((5-isopropyl-1-(3-morpholinyl)propylimidazol-4-yl)methylene)piperazine-2,5-dione (80.0 mg, 0.16 mmol) and MeOH (5 mL), and HCl/MeOH (4 mol/L, 6 mL) was added dropwise thereto at 0°C, and the whole process was

protected from light. After the addition, the mixture was naturally warmed to room temperature and reacted for 2 hours. The reaction mixture was concentrated, and a small amount of MeOH was added thereto to just dissolve the product, and the mixture was added dropwise to 9 mL of EA to precipitate a solid, stirred for 10 min, and then filtered. The filter cake was dried to obtain 87.3 mg of a light yellow solid with a yield of 95%.

$^1$HNMR (600 MHz, DMSO-$d_6$) δ 11.81 (s, 1H), 10.43 (s, 1H), 8.18 (s, 1H), 7.62-7.59 (m,1H), 7.49-7.44 (m, 1H), 7.35-7.34 (m, 1H), 6.72 (s, 1H), 6.69 (s, 1H),4.19 (t, *J* = 7.1 Hz, 2H), 3.96-3.89 (m, 2H), 3.89-3.85 (m, 2H), 3.42 (d, *J* = 12.1 Hz, 2H), 3.28-3.21 (m, 1H), 3.14-3.11 (m, 2H), 3.08-3.02 (m, 2H), 2.24-2.19 (m, 2H),1.34 (d, *J* = 7.1 Hz, 6H).

**(II) Content test of hydrochloride of derivatives of 2,5-diketopiperazine compounds**

**Content test and result analysis of pure hydrochloric acid in hydrochloride of derivatives of 2,5-diketopiperazine compounds**

1. Test drug and reagent

Sodium hydroxide (analytically pure), potassium hydrogen phthalate (reference substance); phenolphthalein indicator solution: 1 g of phenolphthalein and 100 mL of ethanol.

2. Configuration

An appropriate amount of sodium hydroxide was taken, added with water and shaken to dissolve into a saturated solution. After cooling, the saturated solution was put in a polyethylene plastic bottle, allowed to stand for several days, and clarified for later use.

Sodium hydroxide titration solution (0.1 mol/L): 5.6 mL of clear saturated sodium hydroxide solution was taken, added with freshly boiled cold water to make 1000 mL, and shaken evenly.

3. Calibration

3.1 Operation steps

Sodium hydroxide titration solution (0.1 mol/L): About 0.6 g of standard potassium hydrogen phthalate dried at 105°C to constant weight was taken, weighed accurately, added with 50 mL of freshly boiled cold water, and shaken to be dissolved as much as possible; 2 drops of phenolphthalein indicator solution was added thereto, and titrated with this solution; when approaching the end point, potassium hydrogen phthalate should be completely dissolved, and titrated until the solution was pink. Every 1 mL of sodium hydroxide titration solution (0.1 mol/L) was equivalent to 20.42 mg of potassium hydrogen phthalate.

3.2 Calculation formula

The concentration C (mol/L) of the sodium hydroxide titration solution was calculated according to the following formula:

$$C\ (mol/L) = (m * 1.000)/(V * 204.2)$$

In the formula: m is the weighed amount of standard potassium hydrogen phthalate (mg);

V is the consumption of this titration solution (mL);
204.2 is the number of milligrams of potassium hydrogen phthalate equivalent per 1 mL of sodium hydroxide solution (1.000 mol/L).

4. Titration

The test drug (about 10 mg) was precisely weighed, added with 50 mL of freshly boiled cold water, shaken to be dissolved; 2 drops of phenolphthalein indicator solution was added thereto, and titrated with the calibrated sodium hydroxide solution, reached the end point until the solution was pink. The volume $V_{consumption}$ of sodium hydroxide solution was recorded.

5. Result analysis

$$n_{(\text{sodium hydroxide})} = C\ (\text{mol/L}) * V_{\text{consumption}}$$

$$n_{(\text{test drug})} = m_{(\text{test drug})}/M_{(\text{test drug})}$$

The salt-forming ratio is: $n_{(\text{sodium hydroxide})}/n_{(\text{test drug})}$

The experimental results show that: Through detection and calculation, the salt-forming ratio of Plinabulin morpholine derivatives and hydrochloric acid in the present disclosure is 1:2, that is, one molecule of the derivative is combined with two molecules of hydrochloric acid.

**II. Preparation and content test of mesylate of derivatives of 2,5-diketopiperazine compounds**

**(I) Preparation of mesylate of derivatives of 2,5-diketopiperazine compounds**

A compound (1.0 eq) was placed in a dry round-bottom flask, dissolved with an appropriate amount of methanol, and methanesulfonic acid (2.5 eq) was added dropwise thereto, and the mixture was stirred and reacted for 2 hours at room temperature in the dark. After the reaction was completed, the mixture was concentrated under reduced pressure until there was no solvent, dissolved with a small amount of methanol, dropped into ethyl acetate to precipitate a solid, and the mixture was placed in a cold trap at 0°C and stirred for 2 hours, filtered under reduced pressure. The filter cake was washed with cold ethyl acetate or acetone, dried under vacuum at 50°C to obtain a white solid, which was formed into a salt with methanesulfonic acid in a quantitative molar ratio (compound: methanesulfonic acid = 1:2).

**Example 1: (3Z,6Z)-3-(3-(p-Fluorobenzoyl)phenyl)methylene-6-((5-isopropyl-1-(3-morpholinyl)propylimidazol-4-yl)methylene)piperazine-2,5-dione, mesylate (PLN-2-1-2)**

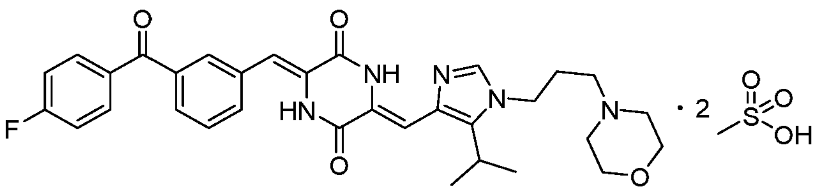

To a 100 mL single-necked dry round-bottom flask was sequentially added (3Z,6Z)-3-(3-(p-fluorobenzoyl) phenyl)methylene-6-((5-isopropyl-1-(3-morpholinyl)propylimidazol-4-yl)methylene)piperazine-2,5-dione (320.0 mg, 0.56 mmol), MeOH (20 mL), and MsOH (134.5 mg, 1.40 mmol), and the whole process was protected from light. After the addition, the mixture was naturally warmed to room temperature and reacted for 2 hours. The reaction mixture was concentrated, and a small amount of MeOH was added thereto to just dissolve the product. The mixture was added dropwise to 35 mL of EA to precipitate a solid, stirred for 10 min, then filtered, dried, and the filter cake was taken to obtain 415.0 mg of a light yellow solid with a yield of 97%.

$^1$H NMR (600 MHz, DMSO-$d_6$) δ 11.81 (s, 1H), 10.44 (s, 1H), 8.12 (s, 1H), 7.93-7.91 (m, 2H), 7.83 (s, 1H), 7.76 (d, *J* = 7.7 Hz, 1H), 7.65 (d, *J* = 7.7 Hz, 1H), 7.60 (t, *J* = 7.6 Hz, 1H), 7.41 (t, *J* = 8.8 Hz, 2H), 6.83 (s, 1H), 6.68 (s, 1H), 4.15 (t, *J* = 7.2 Hz, 2H), 3.99 (d, *J* = 10.8 Hz, 2H), 3.68 (t, *J* = 11.7 Hz, 2H), 3.46 (d, *J* = 12.2 Hz, 2H), 3.26-3.18 (m, 3H), 3.12-3.07 (m, 2H), 2.41 (s, 6H), 2.15-2.10 (m, 2H), 1.34 (d, *J* = 7.1 Hz, 6H).

**Example 2: (3Z,6Z)-3-(3-Fluorophenyl)methylene-6-((5-isopropyl-1-(3-morpholinyl)propylimidazol-4-yl)methylene)piperazine-2,5-dione, mesylate (PLN-2-9-2)**

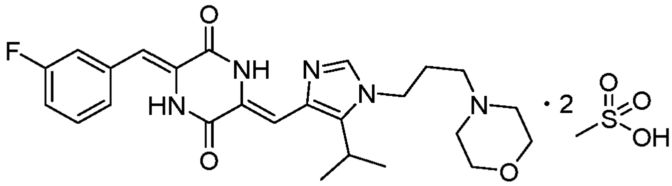

To a 100 mL single-necked dry round-bottom flask was sequentially added (3Z,6Z)-3-(3-fluorophenyl)methylene-6-((5-isopropyl-1-(3-morpholinyl)propylimidazol-4-yl)methylene)piperazine-2,5-dione (200.0 mg, 0.43 mmol), MeOH (10 mL), and MsOH (102.8 mg, 1.07 mmol), and the whole process was protected from light. After the addition, the mixture was naturally warmed to room temperature and reacted for 2 hours. The reaction mixture was concentrated, and a small amount of MeOH was added thereto to just dissolve the product, and the mixture was added dropwise to 15 mL of EA to precipitate a solid, stirred for 10 min, then filtered, dried, and the filter cake was taken to obtain 268.0 mg of a light yellow solid with a yield of 95%.

$^1$H NMR (600 MHz, DMSO-$d_6$) δ 11.76 (s, 1H), 10.39 (s, 1H), 8.18 (s, 1H), 7.47-7.43 (m, 1H), 7.38-7.35 (m, 1H), 7.34 (d, *J* = 7.8 Hz, 1H), 7.17-7.14 (m, 1H), 6.76 (s, 1H), 6.68 (s, 1H), 4.16 (t, *J* = 7.3 Hz, 2H), 4.01-3.98 (m, 2H), 3.69 (t, *J* = 11.6 Hz, 2H), 3.46 (d, *J* = 12.2 Hz, 2H), 3.26-3.19 (m, 3H), 3.14-3.06 (m, 2H), 2.42 (s, 6H), 2.16-2.11 (m, 2H), 1.34 (d, *J* = 7.1 Hz, 6H).

**Example 3: (3Z,6Z)-3-(3-Fluorophenyl)methylene-6-((5-cyclopropyl-1-(3-morpholinyl)propylimidazol-4-yl) methylene)piperazine-2,5-dione, mesylate (PLN-2-10-2)**

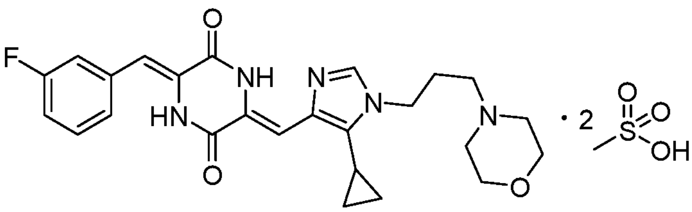

To a 100 mL single-necked dry round-bottom flask was sequentially added (3Z,6Z)-3-(3-fluorophenyl)methylene-6-((5-cyclopropyl-1-(3-morpholinyl)propylimidazol-4-yl)methylene)piperazine-2,5-dione (120.0 mg, 0.26 mmol), MeOH (6 mL), and MsOH (61.5 mg, 0.64 mmol), and the whole process was protected from light. After the addition, the mixture was naturally warmed to room temperature and reacted for 2 hours. The reaction mixture was concentrated, and a small amount of MeOH was added thereto to just dissolve the product, and the mixture was added dropwise to 12 mL of EA to precipitate a solid, stirred for 10 min, then filtered, dried, and the filter cake was taken to obtain 161.0 mg of a light yellow solid with a yield of 95%.

$^1$H NMR (600 MHz, DMSO-$d_6$) δ 11.76 (s, 1H), 10.36 (s, 1H), 8.11 (s, 1H), 7.50 - 7.41 (m, 1H), 7.37 (d, *J* = 10.2 Hz, 1H), 7.34 (d, *J* = 7.8 Hz, 1H), 7.19 - 7.08 (m, 1H), 6.75 (s, 1H), 6.71 (s, 1H), 4.20 (t, *J* = 7.1 Hz, 2H), 3.99 (d, *J* = 10.4 Hz, 2H), 3.67 (t, *J* = 11.6 Hz, 2H), 3.46 (d, *J* = 12.2 Hz, 2H), 3.20-3.16 (m, 2H), 3.12-3.06 (m, 2H), 2.39 (s, 6H), 2.28-2.16 (m, 2H), 1.86-1.79 (m, 1H), 1.13-0.97 (m, 2H), 0.76- 0.57 (m, 2H).

**Example 4: (3Z,6Z)-3-(3,5-Difluorophenyl)methylene-6-((5-isopropyl-1-(3-morpholinyl)propylimidazol-4-yl) methylene)piperazine-2,5-dione, mesylate (PLN-2-62-2)**

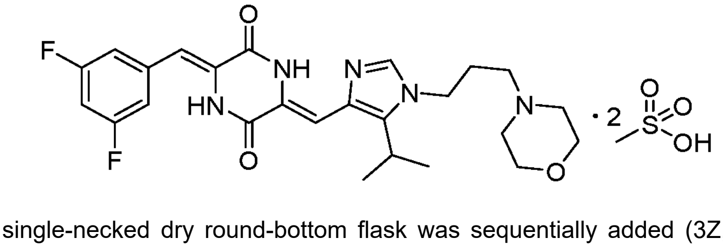

To a 100 mL single-necked dry round-bottom flask was sequentially added (3Z,6Z)-3-(3,5-difluorophenyl) methylene-6-((5-isopropyl-1-(3-morpholinyl)propylimidazol-4-yl)methylene)piperazine-2,5-dione (110.0 mg, 0.23

mmol), MeOH (6 mL), and MsOH (54.7 mg, 0.57 mmol), and the whole process was protected from light. After the addition, the mixture was naturally warmed to room temperature and reacted for 2 hours. The reaction mixture was concentrated, and a small amount of MeOH was added thereto to just dissolve the product, and the mixture was added dropwise to 12 mL of EA to precipitate a solid, stirred for 10 min, then filtered, dried, and the filter cake was taken to obtain 138.0 mg of a light yellow solid with a yield of 90%.

$^1$H NMR (600 MHz, DMSO-$d_6$) δ 11.89 (s, 1H), 10.50 (s, 1H), 8.07 (s, 1H), 7.25-7.23 (m, 2H), 7.20-7.16 (m, 1H), 6.71 (d, *J* = 1.6 Hz, 2H), 4.14 (t, *J* = 7.2 Hz, 2H), 3.99 (d, *J* = 12.4 Hz, 2H), 3.68 (t, *J* = 11.7 Hz, 2H), 3.45 (d, *J* = 12.2 Hz, 2H), 3.26-3.22 (m, 1H), 3.20-3.17 (m, 2H), 3.12-3.06 (m, 2H), 2.40 (s, 6H), 2.14-2.09 (m, 2H), 1.35 (d, *J* = 7.1 Hz, 6H).

**Example 5: (3Z,6Z)-3-(3,4-Difluorophenyl)methylene-6-((5-isopropyl-1-(3-morpholinyl)propylimidazol-4-yl) methylene)piperazine-2,5-dione, mesylate (PLN-2-63-2)**

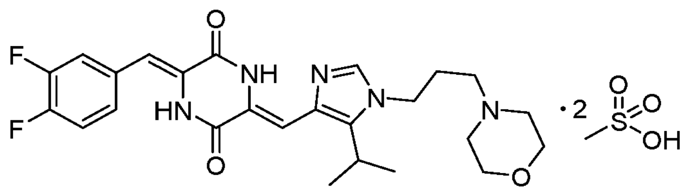

To a 100 mL single-necked dry round-bottom flask was sequentially added (3Z,6Z)-3-(3,4-difluorophenyl) methylene-6-((5-isopropyl-1-(3-morpholinyl)propylimidazol-4-yl)methylene)piperazine-2,5-dione (170.0 mg, 0.35 mmol), MeOH (12 mL), and MsOH (84.1 mg, 0.88 mmol), and the whole process was protected from light. After the addition, the mixture was naturally heated to room temperature and reacted for 2 hours. The reaction mixture was concentrated, and a small amount of MeOH was added thereto to just dissolve the product, and the mixture was added dropwise to 15 mL of EA to precipitate a solid, stirred for 10 min, then filtered, dried, and the filter cake was taken to obtain 225.1 mg of a light yellow solid with a yield of 95%.

$^1$H NMR (600 MHz, DMSO-$d_6$) δ 11.84 (s, 1H), 10.42 (s, 1H), 8.09 (s, 1H), 7.65-7.51 (m, 1H), 7.48-7.41 (m, 1H), 7.35-7.34 (m,1H), 6.72 (s, 1H), 6.70 (s, 1H), 4.14 (t, *J*= 7.3 Hz, 2H), 3.99 (d, *J* = 12.5 Hz, 2H), 3.68 (t, *J* = 11.7 Hz, 2H), 3.45 (d, *J* = 12.2 Hz, 2H), 3.29-3.14 (m, 3H), 3.12-3.06 (m, 2H), 2.40 (s, 6H), 2.14-2.09 (m, 2H), 1.35 (d, *J*= 7.1 Hz, 6H).

**(II) Content test of mesylate of derivatives of 2,5-diketopiperazine compounds**

**Content test and result analysis of pure methanesulfonic acid in mesylate of derivatives of 2,5-diketopiperazine compounds**

1. Test drug and reagent

Sodium hydroxide (analytically pure), potassium hydrogen phthalate (reference substance); phenolphthalein indicator solution: 1 g of phenolphthalein and 100 mL of ethanol.

2. Configuration

An appropriate amount of sodium hydroxide was taken, added with water and shaken to dissolve into a saturated solution. After cooling, the saturated solution was put in a polyethylene plastic bottle, allowed to stand for several days, and clarified for later use.

Sodium hydroxide titration solution (0.1 mol/L): 5.6 mL of clear saturated sodium hydroxide solution was taken, added with freshly boiled cold water to make 1000 mL, and shaken evenly.

3. Calibration

3.1 Operation steps

Sodium hydroxide titration solution (0.1 mol/L): About 0.6 g of standard potassium hydrogen phthalate dried at 105°C to constant weight was taken, weighed accurately, added with 50 mL of freshly boiled cold water, and shaken to be dissolved as much as possible; 2 drops of phenolphthalein indicator solution were added thereto, and titrated with this solution; when approaching the end point, potassium hydrogen phthalate should be completely dissolved, and titrated until

the solution was pink. Every 1 mL of sodium hydroxide titration solution (0.1 mol/L) was equivalent to 20.42 mg of potassium hydrogen phthalate.

3.2 Calculation formula

The concentration C (mol/L) of the sodium hydroxide titration solution was calculated according to the following formula:

$$C\ (mol/L) = (m * 1.000)/(V * 204.2)$$

In the formula: m is the weighed amount of standard potassium hydrogen phthalate (mg);

V is the consumption of this titration solution (mL);
204.2 is the number of milligrams of potassium hydrogen phthalate equivalent per 1 mL of sodium hydroxide solution (1.000 mol/L).

4. Titration

The test drug (about 15 mg) was precisely weighed, added with 50 mL of freshly boiled cold water, shaken to be dissolved; 2 drops of phenolphthalein indicator solution were added thereto, and titrated with the calibrated sodium hydroxide solution, reached the end point until the solution was pink. The volume $V_{consumption}$ of sodium hydroxide solution was recorded.

5. Result analysis

$$n_{(sodium\ hydroxide)} = C\ (mol/L) * V_{consumption}$$

$$n_{(test\ drug)} = m_{(test\ drug)}/M_{(test\ drug)}$$

The salt-forming ratio is: $n_{(sodium\ hydroxide)}/n_{(test\ drug)}$

The experimental results show that: Through detection and calculation, the salt-forming ratio of Plinabulin morpholine derivatives and methanesulfonic acid in the present disclosure is 1:2, that is, one molecule of the derivative is combined with two molecules of methanesulfonic acid.

**III. Summary of melting points of some compounds in the examples**

The test compound was taken, ground evenly, put into a melting point measuring tube, The sample was compacted, and the melting point was measured by using a melting point detector (WRS-3), and the melting point was determined according to the initial melting and final melting temperatures, and recorded. The specific results are shown in Table 3 below.

Table 3 Melting points of some 2,5-diketopiperazine compounds

| Serial number | Compound No. | Melting point (°C) |
|---|---|---|
| | Plinabulin | |
| | PLN-2-1 | 218-219 |
| | PLN-2-1-1 | 106.8-108.8 |
| | PLN-2-1-2 | 137.0-139.0 |
| | PLN-2-2 | 194-195 |
| | PLN-2-2-1 | 173.0-175.0 |
| | PLN-2-5 | 199.1-201.1 |

(continued)

| Serial number | Compound No. | Melting point (°C) |
|---|---|---|
| | PLN-2-5-1 | 220.0-222.0 |
| | PLN-2-7 | 211.6-213.6 |
| | PLN-2-7-1 | 174-176 |
| | PLN-2-9 | 208-210 |
| | PLN-2-9-1 | 141.8-143.8 |
| | PLN-2-9-2 | 195.5-197.5 |
| | PLN-2-10 | 208-211 |
| | PLN-2-10-1 | 148.8-150.8 |
| | PLN-2-10-2 | 113.6-115.6 |
| | PLN-2-49 | 169-171 |
| | PLN-2-50 | 164-166 |
| | PLN-2-51 | 178-180 |
| | PLN-2-52 | 184-186 |
| | PLN-2-53 | 251-253 |
| | PLN-2-54 | 248-250 |
| | PLN-2-55 | 250-252 |
| | PLN-2-56 | 212-214 |
| | PLN-2-56-1 | 232.8-234.8 |
| | PLN-2-57 | 211-213 |
| | PLN-2-57-1 | 232.2-234.2 |
| | PLN-2-58 | 202-204 |
| | PLN-2-59 | 178-180 |
| | PLN-2-60 | 157-159 |
| | PLN-2-61 | 182-184 |
| | PLN-2-61-1 | 238.6-240.6 |
| | PLN-2-62 | 187.0-189.0 |
| | PLN-2-62-1 | 148.1-150.1 |
| | PLN-2-62-2 | 177.0-179.0 |
| | PLN-2-63 | 188.0-190.0 |
| | PLN-2-63-1 | 145.7-147.7 |
| | PLN-2-63-2 | 160.7-162.7 |

**IV. Effect examples**

**Effect example 1: Compound solubility experiment**

1) Experimental method

Method 1: A 1.5 mL brown EP tube was taken, about 1 mg of compound was weighed respectively, and 1 mL of ultrapure water was added thereto. The mixture was vortexed, and sonicated until the compound was no longer dissolved (the solution was turbid or had suspended particles). The mixture was put into an incubation shaker, maintained at a temperature of (37 ± 1°C), shaken at 100 r/min for 24 hours, so as to achieve full dissolution equilibrium. After 24 hours, the

supernatant was quickly filtered through a 0.45 μm microporous membrane, and the initial filtrate was discarded. 200 μL of the renewed filtrate was taken and diluted with 200 μL of methanol. The assay was repeated at least three times. According to the chromatographic conditions, the sample was injected by LC-MS, and the peak area was measured and the equilibrium solubility of each 2,5-diketopiperazine derivative in pure water was calculated.

Method 2: A 0.5 mL brown EP tube was taken, about 2 mg of hydrochloride of derivatives of 2,5-diketopiperazine compounds was weighed respectively, and 0.2 mL of ultrapure water was added thereto. The mixture was vortexed, put into an incubation shaker, maintained at a temperature of (37 ± 1°C), shaken at 100 r/min for 24 hours, so as to achieve full dissolution equilibrium. The dissolution of the mixture was observed.

2) Experimental results

The results of the solubility experiments of the compounds are shown in Table 4 below:

Table 4 Solubility of 2,5-diketopiperazine compounds, hydrochloride thereof, and methanesulfonate thereof

| Compound name | Salt type | Solubility |
|---|---|---|
| Plinabulin | Non-salified | < 250 ng/mL |
| PLN-2-1 | Non-salified | <500 ng/mL |
| PLN-2-2 | Non-salified | <500 ng/mL |
| PLN-2-3 | Non-salified | <250 ng/mL |
| PLN-2-4 | Non-salified | <500 ng/mL |
| PLN-2-5 | Non-salified | <500 ng/mL |
| PLN-2-7 | Non-salified | <500 ng/mL |
| PLN-2-9 | Non-salified | <500 ng/mL |
| PLN-2-10 | Non-salified | <500 ng/mL |
| PLN-2-62 | Non-salified | <500 ng/mL |
| PLN-2-63 | Non-salified | <500 ng/mL |
| Plinabulin hydrochloride | Hydrochloride | 7654 ng/mL |
| PLN-2-1-1 | PLN-2-1 hydrochloride | >10 mg/mL |
| PLN-2-1-2 | PLN-2-1 mesylate | >10 mg/mL |
| PLN-2-2-1 | PLN-2-2 hydrochloride | >10 mg/mL |
| PLN-2-3-1 | PLN-2-3 hydrochloride | >10 mg/mL |
| PLN-2-4-1 | PLN-2-4 hydrochloride | >10 mg/mL |
| PLN-2-5-1 | PLN-2-5 hydrochloride | >10 mg/mL |
| PLN-2-7-1 | PLN-2-7 hydrochloride | >10 mg/mL |
| PLN-2-9-1 | PLN-2-9 hydrochloride | >10 mg/mL |
| PLN-2-9-2 | PLN-2-9 mesylate | >10 mg/mL |
| PLN-2-10-1 | PLN-2-10 hydrochloride | >10 mg/mL |
| PLN-2-10-2 | PLN-2-10 mesylate | >10 mg/mL |
| PLN-2-62-1 | PLN-2-62 hydrochloride | >10 mg/mL |
| PLN-2-62-2 | PLN-2-62 mesylate | >10 mg/mL |
| PLN-2-63-1 | PLN-2-63 hydrochloride | >10 mg/mL |
| PLN-2-63-2 | PLN-2-63 mesylate | >10 mg/mL |

**Effect example 2:** Inhibition test of compound or salt thereof on cell proliferation

Tumor cells (NCI-H460, BxPC-3, HT-29, HCC-LM3) in the logarithmic growth phase were digested, centrifuged,

and the supernatants were removed. Cells were resuspended by adding fresh culture medium, and counted using a cell counting plate. 100 μL of culture medium (containing cells) was added to a 96-well plate according to the standard of 2000 to 6000 cells per well and incubated in an incubator (37°C, 5% $CO_2$) for 24 hours. After the cells were completely adhered to the wall, the test sample and Plinabulin were diluted to different concentrations using fresh culture medium, and 4 wells were set for each concentration. After 72 hours of drug treatment, the culture medium was removed, and 20 μL of MTT at a concentration of 5 mg/mL was added to each well. After 3-4 hours in the incubator, the MTT was discarded, and 100 μL of DMSO was added to each well, and placed in a 96-well plate shaker to completely dissolve the purple formazan. Subsequently, the absorbance (OD value) was detected at a wavelength of 490 nm using a microplate reader. The experiment also required setting up zero-setting wells (blank culture medium, MTT, DMSO). Cell inhibition rate = 1 - (OD value of drug-dosed group - OD value of zero-setting group)/(OD value of blank group - OD value of zero-setting group)×100%.

The inhibition test results of the compounds or the hydrochloride thereof of the present disclosure on cell proliferation are shown in Table 5 below:

Table 5 Inhibition test of the compounds or the salts thereof of the present disclosure on cell proliferation

| Compound name | $IC_{50}$ (nM) NCI-H460 | $IC_{50}$ (nM) BxPC-3 | $IC_{50}$ (nM) HCC-LM3 | $IC_{50}$ (nM) HT-29 |
|---|---|---|---|---|
| Plinabulin | 11.56±0.96 | 5.41±0.76 | 8.31±0.23 | 5.74±0.73 |
| PLN-2-1 | 3.45±0.33 | 2.29±0.82 | 4.20±0.12 | 1.03±0.11 |
| PLN-2-1-1 | 2.28±1.24 | 1.77±0.03 | 2.04±0.57 | 1.5 6±0.47 |
| PLN-2-1-2 | 1.95±0.08 | 1.33±0.19 | 1.85±0.27 | 1.42±0.46 |
| PLN-2-2 | 5.25±0.49 | 4.63±0.16 | 5.58±1.03 | 3.83±0.43 |
| PLN-2-2-1 | 2.80±1.15 | 1.21±0.21 | 1.76±0.30 | 1.50±0.58 |
| PLN-2-3 | 4.90±0.61 | 1.43±0.95 | 6.31±0.16 | 2.15±0.33 |
| PLN-2-3-1 | 5.60:1:0.41 | 1.43:1:0.95 | | |
| PLN-2-4 | 25.66±2.62 | | | |
| PLN-2-4-1 | 23.81±1.36 | | | |
| PLN-2-5 | 19.35±1.63 | 12.52±1.47 | 9.78±2.21 | 11.85±1.31 |
| PLN-2-5-1 | 17.75±0.55 | 9.73±0.30 | 12.41±2.68 | 11.79±5.52 |
| PLN-2-6 | 33.45±0.46 | 27.74±0.94 | 16.5 6±0.66 | 25.26±3.54 |
| PLN-2-7 | 6.12±0.43 | 4.92±1.48 | 3.30±1.88 | 4.03±0.19 |
| PLN-2-7-1 | 6.93±4.85 | 4.83±0.20 | 4.00±1.50 | 5.61±1.96 |
| PLN-2-8 | 15.23±2.82 | 8.80±0.16 | | 13.45±2.08 |
| PLN-2-9 | 8.51±0.10 | 5.47±0.57 | 5.13±0.12 | 4.37±0.62 |
| PLN-2-9-1 | 8.87±0.05 | 5.76±1.00 | 6.99±0.59 | 6.22±1.61 |
| PLN-2-9-2 | 8.49±0.72 | 5.14±0.52 | 6.08±0.51 | 5.84±1.66 |
| PLN-2-10 | 17.69±5.18 | 8.97±0.74 | 14.9±0.22 | 10.58±1.27 |
| PLN-2-10-1 | 18.37±0.13 | 12.01±1.27 | 11.97±2.67 | 11.08±1.39 |
| PLN-2-10-2 | 11.21±2.04 | 12.59±0.96 | 13.35±4.02 | 13.17±2.15 |
| PLN-2-11 | 234.13±25.70 | | | |
| PLN-2-12 | 3.77±0.62 | 2.48±2.12 | | |
| PLN-2-13 | 8.20±0.54 | 14.89±2.62 | | |
| PLN-2-14 | 9.33±0.54 | 9.33±0.45 | | |
| PLN-2-15 | 8.01±1.24 | 6.77±0.08 | | |
| PLN-2-16 | 25.65±9.64 | 5.92±3.74 | | 11.10±1.87 |
| PLN-2-17 | 41.77±2.27 | 21.05±2.41 | | |

(continued)

| Compound name | $IC_{50}$ (nM) NCI-H460 | $IC_{50}$ (nM) BxPC-3 | $IC_{50}$ (nM) HCC-LM3 | $IC_{50}$ (nM) HT-29 |
|---|---|---|---|---|
| PLN-2-18 | 99.72±0.82 | | | |
| PLN-2-19 | 212.62±12.76 | | | |
| PLN-2-19-1 | 203.6±4.24 | | | |
| PLN-2-20 | 147.00±14.50 | 91.63±0.62 | | |
| PLN-2-21 | 23.88±1.02 | 15.31±1.83 | | 13.84±3.08 |
| PLN-2-22 | 85.71±10.63 | 66.16±1.45 | | |
| PLN-2-23 | 157.87±25.68 | | | |
| PLN-2-24 | 28.55±1.40 | 13.19±0.07 | | |
| PLN-2-25 | 18.44±2.95 | | | |
| PLN-2-26 | 50.14±0.69 | | | |
| PLN-2-27 | 23.86±4.76 | 17.90±5.90 | | |
| PLN-2-28 | 15.36±1.68 | 9.22±3.93 | | |
| PLN-2-29 | 3.46±0.25 | 1.40±0.04 | | |
| PLN-2-30 | 3.98±0.33 | 2.30±0.08 | | |
| PLN-2-31 | 6.93±0.96 | 2.48±0.57 | | |
| PLN-2-32 | 7.95±1.29 | 4.16±0.78 | | |
| PLN-2-33 | 9.91±2.00 | 6.39±2.51 | | |
| PLN-2-34 | 20.14±3.67 | 17.49±0.27 | | |
| PLN-2-35 | 5.28±0.71 | 2.23±2.2 | | |
| PLN-2-36 | 4.31±0.59 | 2.25±1.22 | | 2.97±0.51 |
| PLN-2-37 | 7.86±1.37 | 6.23±0.15 | | |
| PLN-2-38 | 7.30±0.44 | | | |
| PLN-2-39 | 37.19±3.61 | | | |
| PLN-2-40 | 7.56±0.33 | 4.40±3.1 | | |
| PLN-2-41 | 5.55±1.68 | 3.25±2.13 | | |
| PLN-2-42 | 6.59±1.29 | 3.38±0.53 | | |
| PLN-2-43 | 11.13±0.90 | | | |
| PLN-2-44 | 8.68±0.78 | 3.84±0.12 | | |
| PLN-2-45 | 6.43±1.98 | 1.79±0.49 | | |
| PLN-2-46 | 21.62±0.52 | 9.09±0.89 | | |
| PLN-2-47 | 6.56±0.32 | 3.62±0.48 | | |
| PLN-2-48 | 39.22±1.85 | 33.09±0.98 | | |
| PLN-2-49 | 33.70±0.70 | 27.27±2.75 | 18.41±1.42 | 35.23±2.15 |
| PLN-2-50 | 41.33±1.25 | 23.03±1.56 | 20.84±2.56 | 37.07±3.15 |
| PLN-2-51 | 21.68±3.04 | 13.86±3.21 | 7.49±0.12 | 9.03±0.29 |
| PLN-2-52 | 16.47±3.48 | 13.91±0.89 | 7.54±0.68 | 8.49±0.54 |
| PLN-2-53 | 11.62±1.11 | 7.17±0.52 | 6.55±1.39 | 9.71±0.74 |
| PLN-2-54 | 8.28±0.91 | 5.74:1:2.19 | 3.48±2.60 | 6.55±0.33 |
| PLN-2-55 | 22.87±3.19 | 16.63±1.18 | 35.03±11.46 | 24.73±0.51 |

(continued)

| Compound name | $IC_{50}$ (nM) NCI-H460 | $IC_{50}$ (nM) BxPC-3 | $IC_{50}$ (nM) HCC-LM3 | $IC_{50}$ (nM) HT-29 |
|---|---|---|---|---|
| PLN-2-56 | 5.59±1.40 | 3.24±0.73 | 4.26±0.25 | 2.62±0.91 |
| PLN-2-56-1 | 7.69±0.28 | 4.99±0.19 | 4.72±0.73 | 4.43±0.13 |
| PLN-2-57 | 16.64±4.94 | 8.56±0.01 | 13.87±2.50 | 5.07±2.12 |
| PLN-2-57-1 | 17.52±0.71 | 14.05±1.23 | 12.89±1.61 | 10.59±3.68 |
| PLN-2-58 | 11.76±1.82 | 8.10±1.44 | 16.67±0.98 | |
| PLN-2-59 | 27.41±0.52 | 15.51±0.74 | 23.31±2.49 | |
| PLN-2-60 | 59.67±0.30 | 27.19±1.42 | 49.20±1.30 | |
| PLN-2-61 | 19.61±2.15 | 8.00±1.15 | 31.16±6.92 | 14.05±0.22 |
| PLN-2-61-1 | 16.86±1.29 | 9.37±0.78 | 16.52±5.66 | 13.96±0.58 |
| PLN-2-62 | 4.17±0.75 | 3.44±0.12 | 3.89±0.16 | 3.49±0.23 |
| PLN-2-62-1 | 6.87±0.47 | 3.75±0.06 | 5.32±1.38 | 3.79±0.04 |
| PLN-2-62-2 | 4.60±1.33 | 3.66±1.15 | 3.99±0.08 | 3.55±0.04 |
| PLN-2-63 | 72. 16±4.86 | 55.26±3.42 | 64.92±5.12 | |
| PLN-2-63-1 | 94.69±9.48 | 50.1±7.79 | 66.61±4.98 | |
| PLN-2-63-2 | 95.91±3.66 | 50.85±6.75 | 69.53±4.26 | |
| PLN-2-64 | 18.54±0.30 | 7.70±0.39 | 26.13±2.14 | |
| PLN-2-65 | 17.39±1.45 | 10.26±2.18 | 20.94±0.97 | |
| PLN-2-66 | 40.34±3.72 | 30.42±0.35 | 43.89±7.94 | |
| PLN-2-67 | 8.39±0.58 | 3.73±0.06 | 7.70±0.04 | |

Note: NCI-H460 is a human non-small cell lung cancer cell line, BxPC-3 is a human pancreatic cancer cell line, HT-29 is a human colon cancer cell line, and HCC-LM3 is a human liver cancer cell line. Plinabulin was used as a positive control. The control group was DMSO without adding samples.

**Effect example 4: Immunofluorescence experiment**

1) Experimental method

1. Cell membrane rupture: After the cover slip was shaken to slight dryness, a circle was drawn using a histochemical pen at the position where the cells were evenly distributed in the middle of the cover slip (to prevent the antibody from flowing away). 50 to 100 μL of membrane rupture working solution was added, and incubated at room temperature for 20 min, washed 3 times with PBS, 5 min each time.
2. Serum blocking: BSA was added dropwise in the circle to evenly cover the tissue and block at room temperature for 30 min. (The primary antibody was from goat and blocked with 10% normal rabbit serum, and the primary antibody from other sources was blocked with 3% BSA)
3. Adding the primary antibody: The blocking solution was shaken off gently, and the primary antibody was added dropwise in the cell well plate (prepared in a certain proportion using PBS), and the cell culture plate was placed flat in a humid box and incubated overnight at 4°C.
4. Adding secondary antibody: PBS was added to the cell culture plate, placed on a decolorizing shaker, shaken and washed for 3 times, 5 min each time. After shaking to slight dryness, a secondary antibody corresponding to the species of the primary antibody in the histochemical kit was added dropwise in the circle to cover the tissue, and incubated at room temperature for 50 min.
5. DAPI restaining nucleus: The cover slip was placed in PBS (pH 7.4), shaken and washed 3 times on the decolorizing shaker, 5 min each time. After the cover slip was shaken to slight dryness, DAPI staining solution was added dropwise in the circle, and incubated at room temperature for 10 min in the dark.

6. Sealing the cover slip: The cover slip was placed in PBS (pH 7.4), shaken and washed 3 times on the decolorizing shaker, 5 min each time. After the cover slip was shaken to slight dryness, the cover slip was sealed with antifade mounting medium.
7. Microscopic examination and photographing: The cover slip was observed under a fluorescent microscope and images were collected.

**2) Experimental results**

The immunofluorescence results of NCI-H460 cell line of the derivative of the 2,5-diketopiperazine compound are shown in FIG. 1 and Table 6.

Table 6

| **Compound name** | Control | Plinabulin | PLN-2-1-1 | PLN-2-3-1 |
|---|---|---|---|---|
| **β-tubulin fluorescence value** | 20330±1233 | 8133±799 | 5344±671 | 7822±733 |

The immunofluorescence results of BxPC-3 cell line of the derivative of the 2,5-diketopiperazine compound are shown in FIG. 2 and Table 7.

Table 7

| **Compound name** | Control | Plinabulin | PLN-2-1-1 | PLN-2-3-1 |
|---|---|---|---|---|
| **β-tubulin fluorescence value** | 29873±4013 | 11000±1176 | 9533±984 | 9000±1000 |

Immunofluorescence experiments showed that after treatment with the derivatives PLN-2-1-1 and PLN-2-3-1, no matter for NCI-H460 or BxPC-3 cells, the cell morphology changed from the original shuttle shape to round-like, polygonal, or irregular shape, and the nuclei appeared ruptured and irregular, and the number of tubulin distributed radially was significantly reduced, and the microtubules were atrophied. The effects of the derivatives PLN-2-1-1 and PLN-2-3-1 were more pronounced compared with the positive control of Punabulin.

**Effect example 5: Cell apoptosis experiment**

1) Experimental method (Annexin V/PI double staining method)

1. Cell collection: The cell culture dish was taken out, the culture medium was washed with PBS, digested with EDTA-free trypsin, collected into a centrifuge tube. The number of cells per sample was about $1\times10^6$/mL, and the centrifuge tube was centrifuged at 800 r/min for 3 min, and the culture medium was discarded.
2. The residue was washed twice with frozen PBS, centrifuged at 800 r/min for 3 min each time, and the supernatant was discarded.
3. Blank group, single-stained group and double-stained group were set, and 100 μL of binding buffer was added thereto. 5 μL of FITC-annexin V and 5 μL of PI were chosen according to different groups, mixed gently, incubated at room temperature in the dark for 15 min, then 400 μL of binding buffer was added thereto, and the cells were gently mixed.
4. After filtering the cell suspension with a 300-mesh nylon membrane, the cell suspension was detected by a flow cytometer.

2) Experimental results

The apoptosis experiment of NCI-H460 cell line detected by a flow cytometer is shown in FIG. 3 and Table 8.

Table 8

| **Compound name** | NTC (blank control group) | Plinabulin | PLN-2-3-1 | PLN-2-1-1 |
|---|---|---|---|---|
| **Proportion of early apoptotic cells** | 1.84% | 1.90% | 3.08% | 3.55% |

(continued)

| Compound name | NTC (blank control group) | Plinabulin | PLN-2-3-1 | PLN-2-1-1 |
|---|---|---|---|---|
| **Proportion of late apoptotic cells** | 0.54 % | 5.18 % | 6.76 % | 9.70% |

The apoptosis experiment of BxPC-3 cell line detected by flow cytometer is shown in FIG. 4 and Table 9.

Table 9

| Compound name | NTC (blank control group) | Plinabulin | PLN-2-3-1 | PLN-2-1-1 |
|---|---|---|---|---|
| **Proportion of early apoptotic cells** | 1.11 % | 1.66 % | 2.75 % | 3.82 % |
| **Proportion of late apoptotic cells** | 0.94 % | 3.85 % | 5.83 % | 6.79 % |

Results and discussion: In NCI-H460 and BxPC-3, the derivatives **PLN-2-1-1 and PLN-2-3-1** both significantly increased the number of apoptosis, and the sum of the number of early apoptosis and late apoptosis was significant higher than that of Plinabulin, where the derivative **PLN-2-1-1** causes the sum of the number of early apoptosis and late apoptosis to be the highest.

## Claims

**1.** A compound of formula (I), a stereoisomer thereof, a tautomer thereof, a pharmaceutically acceptable salt thereof, or a solvate of any one of the foregoing:

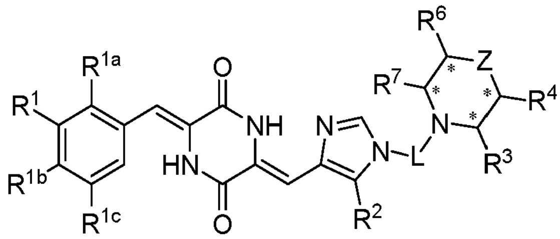

(I)

a carbon atom with "*" is a carbon atom or a chiral carbon atom, and when the carbon atom with "*" is the chiral carbon atom, it is in an S configuration and/or an R configuration;
$R^1$ is hydrogen, deuterium, halogen, $C_1$-$C_8$ alkyl, $C_1$-$C_8$ alkyl substituted by one or more than one halogen, $C_1$-$C_8$ alkoxy, $C_1$-$C_8$ alkoxy substituted by one or more than one halogen, benzoyl, benzoyl substituted by one or more than one halogen, phenoxy, or phenoxy substituted by one or more than one halogen;
$R^{1a}$, $R^{1b}$, and $R^{1c}$ are independently hydrogen, deuterium, halogen, or $C_1$-$C_8$ alkyl;
or, $R^1$, $R^{1a}$ together with the carbon atoms to which they are attached form a $C_6$-$C_{10}$ aryl or a 3- to 10-membered heteroaryl with 1-5 heteroatoms selected from one or more than one of N, O, and S;
or, $R^1$, $R^{1b}$ together with the carbon atoms to which they are attached form a $C_6$-$C_{10}$ aryl or a 3- to 10-membered heteroaryl with 1-5 heteroatoms selected from one or more than one of N, O, and S;
or, $R^{1b}$, $R^{1c}$ together with the carbon atoms to which they are attached form a $C_6$-$C_{10}$ aryl or a 3- to 10-membered heteroaryl with 1-5 heteroatoms selected from one or more than one of N, O, and S;
$R^2$ is $C_1$-$C_8$ alkyl or $C_3$-$C_{10}$ cycloalkyl;
L is $C_1$-$C_8$ heteroalkylene with 1-4 heteroatoms selected from one or more than one of N, O, S, and Se or $C_1$-$C_8$ alkylene;
$R^3$, $R^4$, $R^6$, and $R^7$ are independently hydrogen, deuterium, $C_1$-$C_8$ alkyl, $C_1$-$C_8$ alkyl substituted by one or more than one $R^{3-1}$, $C_1$-$C_8$ alkoxy, $C_3$-$C_{10}$ cycloalkyl, $C_6$-$C_{10}$ aryl, or 3- to 10-membered heterocycloalkyl with 1-5 heteroatoms selected from one or more than one of N, O, and S;
$R^{3-1}$ is independently O-$R^{3-1-1}$, $R^{3-1-1}$ is $C_1$-$C_8$ alkyl or $C_1$-$C_8$ alkyl substituted by one or more than one $R^{3-1-2}$;
$R^{3-1-2}$ is independently $C_6$-$C_{10}$ aryl;
Z is O or N($R^5$);

$R^5$ is hydrogen, deuterium, $C_1$-$C_8$ alkyl, $C_6$-$C_{10}$ aryl substituted by one or more than one $R^{5-1}$, benzyl, benzyl substituted by one or more than one $R^{5-2}$, -C(=O)-$R^{5-3}$, or -S(=O)$_2$-$R^{5-4}$;
$R^{5-1}$ is independently halogen;
$R^{5-2}$ is independently

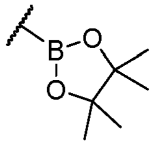
;

$R^{5-3}$ is hydrogen, $C_1$-$C_8$ alkoxy, benzyloxy, benzyloxy substituted by one or more than one $R^{5-2}$, $C_1$-$C_8$ alkyl, $C_3$-$C_{10}$ cycloalkyl, $C_2$-$C_8$ alkenyl, $C_6$-$C_{10}$ aryl, or $NR^{5-3-1}R^{5-3-2}$; $R^{5-3-1}$ and $R^{5-3-2}$ are independently hydrogen, $C_1$-$C_8$ alkyl, or $C_3$-$C_{10}$ cycloalkyl;
$R^{5-4}$ is $C_1$-$C_8$ alkyl or $C_6$-$C_{10}$ aryl.

**2.** The compound of formula (I) as claimed in claim 1, the stereoisomer thereof, the tautomer thereof, the pharmaceutically acceptable salt thereof, or the solvate of any one of the foregoing, wherein when $R^1$ is halogen, the halogen is fluorine, chlorine, bromine, or iodine;

or, when $R^1$ is $C_1$-$C_8$ alkyl, the $C_1$-$C_8$ alkyl is $C_1$-$C_4$ alkyl;
or, when $R^1$ is $C_1$-$C_8$ alkyl substituted by one or more than one halogen, the $C_1$-$C_8$ alkyl substituted by one or more than one halogen is $C_1$-$C_4$ alkyl substituted by one or more than one halogen;
or, when $R^1$ is $C_1$-$C_8$ alkoxy, the $C_1$-$C_8$ alkoxy is $C_1$-$C_4$ alkoxy;
or, when $R^1$ is $C_1$-$C_8$ alkoxy substituted by one or more than one halogen, the $C_1$-$C_8$ alkoxy substituted by one or more than one halogen is $C_1$-$C_4$ alkoxy substituted by one or more than one halogen;
or, when $R^1$ is benzoyl substituted by one or more than one halogen, the halogen is fluorine, chlorine, bromine, or iodine;
or, when $R^1$ is phenoxy substituted by one or more than one halogen, the halogen is fluorine, chlorine, bromine, or iodine;
or, when $R^{1a}$, $R^{1b}$, and $R^{1c}$ are independently halogen, the halogen is fluorine, chlorine, bromine, or iodine;
or, when $R^{1a}$, $R^{1b}$, and $R^{1c}$ are independently $C_1$-$C_8$ alkyl, the $C_1$-$C_8$ alkyl is $C_1$-$C_4$ alkyl;
or, when $R^1$, $R^{1a}$ together with the carbon atoms to which they are attached form the $C_6$-$C_{10}$ aryl, the $C_6$-$C_{10}$ aryl is phenyl;
or, when $R^1$, $R^{1a}$ together with the carbon atoms to which they are attached form the 3- to 10-membered heteroaryl with 1-5 heteroatoms selected from one or more than one of N, O, and S, the 3- to 10-membered heteroaryl with 1-5 heteroatoms selected from one or more than one of N, O, and S is 4- to 8-membered heteroaryl with 1-2 heteroatoms selected from one or more than one of N, O, and S;
or, when $R^2$ is $C_1$-$C_8$ alkyl, the $C_1$-$C_8$ alkyl is $C_1$-$C_4$ alkyl;
or, when $R^2$ is $C_3$-$C_{10}$ cycloalkyl, the $C_3$-$C_{10}$ cycloalkyl is $C_3$-$C_6$ cycloalkyl;
or, when L is $C_1$-$C_8$ alkylene, the $C_1$-$C_8$ alkylene is $C_3$-$C_8$ alkylene;
or, when $R^3$, $R^4$, $R^6$, and $R^7$ are independently $C_1$-$C_8$ alkyl, the $C_1$-$C_8$ alkyl is $C_1$-$C_4$ alkyl;
or, when $R^3$, $R^4$, $R^6$, and $R^7$ are independently $C_1$-$C_8$ alkyl substituted by one or more than one $R^{3-1}$, the $C_1$-$C_8$ alkyl is $C_1$-$C_4$ alkyl;
or, when $R^{3-1-1}$ is $C_1$-$C_8$ alkyl, the $C_1$-$C_8$ alkyl is $C_1$-$C_4$ alkyl;
or, when $R^{3-1-1}$ is $C_1$-$C_8$ alkyl substituted by one or more than one $R^{3-1-2}$, the $C_1$-$C_8$ alkyl is $C_1$-$C_4$ alkyl;
or, when $R^{3-1-2}$ is independently $C_6$-$C_{10}$ aryl, the $C_6$-$C_{10}$ aryl is phenyl;
or, when $R^5$ is $C_1$-$C_8$ alkyl, the $C_1$-$C_8$ alkyl is $C_1$-$C_4$ alkyl;
or, when $R^5$ is $C_6$-$C_{10}$ aryl substituted by one or more than one $R^{5-1}$, the $C_6$-$C_{10}$ aryl is phenyl or naphthyl;
or, when $R^{5-1}$ is halogen, the halogen is fluorine, chlorine, bromine, or iodine;
or, when $R^{5-3}$ is $C_1$-$C_8$ alkoxy, the $C_1$-$C_8$ alkoxy is $C_1$-$C_4$ alkoxy;
or, when $R^{5-3}$ is $C_1$-$C_8$ alkyl, the $C_1$-$C_8$ alkyl is methyl, ethyl, n-propyl, isopropyl, tert-butyl, or $-CH(C_2H_5)CH_2CH_3$;
or, when $R^{5-3}$ is $C_3$-$C_{10}$ cycloalkyl, the $C_3$-$C_{10}$ cycloalkyl is cyclopropyl, cyclobutyl, cyclopentyl, cyclohexyl, or adamantyl;
or, when $R^{5-3}$ is $C_2$-$C_8$ alkenyl, the $C_2$-$C_8$ alkenyl is $C_2$-$C_4$ alkenyl;
or, when $R^{5-3}$ is $C_6$-$C_{10}$ aryl, the $C_6$-$C_{10}$ aryl is phenyl or naphthyl;
or, when $R^{5-3-1}$ and $R^{5-3-2}$ are independently $C_1$-$C_8$ alkyl, the $C_1$-$C_8$ alkyl is $C_1$-$C_4$ alkyl;
or, when $R^{5-3-1}$ and $R^{5-3-2}$ are independently $C_3$-$C_{10}$ cycloalkyl, the $C_3$-$C_{10}$ cycloalkyl is $C_3$-$C_6$ cycloalkyl;

or, when $R^{5-4}$ is $C_1$-$C_8$ alkyl, the $C_1$-$C_8$ alkyl is $C_1$-$C_4$ alkyl;
or, when $R^{5-4}$ is $C_6$-$C_{10}$ aryl, the $C_6$-$C_{10}$ aryl is phenyl or naphthyl.

**3.** The compound of formula (I) as claimed in claim 1, the stereoisomer thereof, the tautomer thereof, the pharmaceutically acceptable salt thereof, or the solvate of any one of the foregoing, wherein when $R^1$ is halogen, the halogen is fluorine;

or, when $R^1$ is $C_1$-$C_8$ alkyl, the $C_1$-$C_8$ alkyl is methyl;
or, when $R^1$ is $C_1$-$C_8$ alkyl substituted by one or more than one halogen, the $C_1$-$C_8$ alkyl substituted by one or more than one halogen is trifluoromethyl;
or, when $R^1$ is $C_1$-$C_8$ alkoxy, the $C_1$-$C_8$ alkoxy is methoxy;
or, when $R^1$ is $C_1$-$C_8$ alkoxy substituted by one or more than one halogen, the $C_1$-$C_8$ alkoxy substituted by one or more than one halogen is trifluoromethoxy;
or, when $R^1$ is benzoyl substituted by one or more than one halogen, the halogen is fluorine; preferably, the benzoyl substituted by one or more than one halogen is

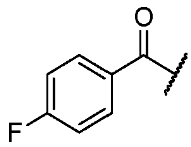
;

or, when $R^1$ is phenoxy substituted by one or more than one halogen, the halogen is fluorine; preferably, the phenoxy substituted by one or more than one halogen is

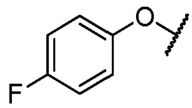
;

or, when $R^{1a}$, $R^{1b}$, and $R^{1c}$ are independently halogen, the halogen is fluorine;
or, when $R^{1a}$, $R^{1b}$, and $R^{1c}$ are independently $C_1$-$C_8$ alkyl, the $C_1$-$C_8$ alkyl is methyl;
or, when $R^1$, $R^{1a}$ together with the carbon atoms to which they are attached form the 3- to 10-membered heteroaryl with 1-5 heteroatoms selected from one or more than one of N, O, and S, the 3- to 10-membered heteroaryl with 1-5 heteroatoms selected from one or more than one of N, O, and S is pyridyl; preferably, the 3- to 10-membered heteroaryl with 1-5 heteroatoms selected from one or more than one of N, O, and S is

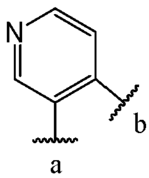
,

with an a-terminal attached to the carbon atom to which $R^1$ is attached and a b-terminal attached to the carbon atom to which $R^{1a}$ is attached;
or, when $R^2$ is $C_1$-$C_8$ alkyl, the $C_1$-$C_8$ alkyl is methyl, ethyl, isopropyl, or tert-butyl;
or, when $R^2$ is $C_3$-$C_{10}$ cycloalkyl, the $C_3$-$C_{10}$ cycloalkyl is cyclopropyl;
or, when L is $C_1$-$C_8$ alkylene, the $C_1$-$C_8$ alkylene is

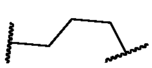
,
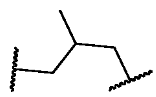
,

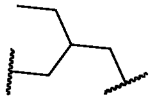, 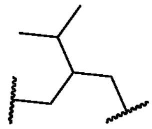, 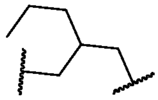, or 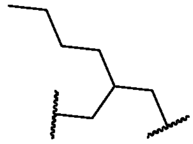,

such as

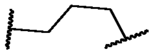;

or, when $R^3$, $R^4$, $R^6$, and $R^7$ are independently $C_1$-$C_8$ alkyl, the $C_1$-$C_8$ alkyl is methyl or ethyl, such as methyl;
or, when $R^3$, $R^4$, $R^6$, and $R^7$ are independently $C_1$-$C_8$ alkyl substituted by one or more than one $R^{3-1}$, the $C_1$-$C_8$ alkyl is methyl;
or, when $R^{3-1-1}$ is $C_1$-$C_8$ alkyl, the $C_1$-$C_8$ alkyl is methyl, ethyl, n-propyl, or n-butyl;
or, when $R^{3-1-1}$ is $C_1$-$C_8$ alkyl substituted by one or more than one $R^{3-1-2}$, the $C_1$-$C_8$ alkyl is methyl;
or, when $R^5$ is $C_1$-$C_8$ alkyl, the $C_1$-$C_8$ alkyl is methyl, ethyl, n-propyl, isopropyl, n-butyl, or 2-methylpropyl, such as methyl;
or, when $R^5$ is $C_6$-$C_{10}$ aryl substituted by one or more than one $R^{5-1}$, the $C_6$-$C_{10}$ aryl is phenyl;
or, when $R^{5-1}$ is halogen, the halogen is fluorine;
or, when $R^{5-3}$ is $C_1$-$C_8$ alkoxy, the $C_1$-$C_8$ alkoxy is tert-butoxy;
or, when $R^{5-3}$ is $C_1$-$C_8$ alkyl, the $C_1$-$C_8$ alkyl is $C_1$-$C_4$ alkyl;
or, when $R^{5-3}$ is $C_3$-$C_{10}$ cycloalkyl, the $C_3$-$C_{10}$ cycloalkyl is $C_3$-$C_6$ cycloalkyl;
or, when $R^{5-3}$ is $C_2$-$C_8$ alkenyl, the $C_2$-$C_8$ alkenyl is

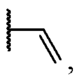, 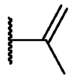, 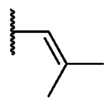,

or

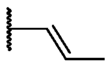,

such as

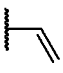 or 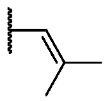;

or, when $R^{5-3}$ is $C_6$-$C_{10}$ aryl, the $C_6$-$C_{10}$ aryl is phenyl;
or, when $R^{5-3-1}$ and $R^{5-3-2}$ are independently $C_1$-$C_8$ alkyl, the $C_1$-$C_8$ alkyl is isopropyl or tert-butyl;
or, when $R^{5-3-1}$ and $R^{5-3-2}$ are independently $C_3$-$C_{10}$ cycloalkyl, the $C_3$-$C_{10}$ cycloalkyl is cyclopentyl or cyclohexyl;
or, when $R^{5-4}$ is $C_1$-$C_8$ alkyl, the $C_1$-$C_8$ alkyl is methyl;
or, when $R^{5-4}$ is $C_6$-$C_{10}$ aryl, the $C_6$-$C_{10}$ aryl is phenyl.

**4.** The compound of formula (I) as claimed in claim 1, the stereoisomer thereof, the tautomer thereof, the pharmaceutically acceptable salt thereof, or the solvate of any one of the foregoing, wherein $R^1$ is hydrogen, halogen, $C_1$-$C_8$ alkyl, $C_1$-$C_8$ alkoxy, $C_1$-$C_8$ alkoxy substituted by one or more than one halogen, benzoyl, benzoyl substituted by one or more than one halogen, phenoxy, or phenoxy substituted by one or more than one halogen;

or, $R^{1b}$ is hydrogen or halogen; such as hydrogen or fluorine;
or, $R^{1c}$ is hydrogen or halogen, such as hydrogen or fluorine;
or, $R^2$ is $C_1$-$C_8$ alkyl;
or, L is $C_1$-$C_8$ alkylene;
or, $R^3$, $R^4$, $R^6$, and $R^7$ are independently hydrogen or $C_1$-$C_8$ alkyl;

or, $R^5$ is benzyl, -C(=O)-$R^{5-3}$, or -S(=O)$_2$-$R^{5-4}$;
or, $R^{5-3}$ is hydrogen, $C_1$-$C_4$ alkyl, $C_3$-$C_6$ cycloalkyl, $C_2$-$C_8$ alkenyl, benzyloxy substituted by one or more than one $R^{5-2}$, $C_6$-$C_{10}$ aryl, or $NR^{5-3-1}R^{5-3-2}$;
or, the pharmaceutically acceptable salt of the compound of formula (I) is a salt prepared from the compound of formula (I) and a pharmaceutically acceptable acid, the pharmaceutically acceptable acid is an inorganic acid or an organic acid, the inorganic acid is preferably hydrochloric acid, and the organic acid is preferably methanesulfonic acid.

**5.** The compound of formula (I) as claimed in claim 1, the stereoisomer thereof, the tautomer thereof, the pharmaceutically acceptable salt thereof, or the solvate of any one of the foregoing, wherein

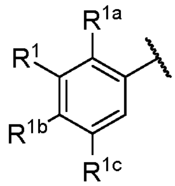

is

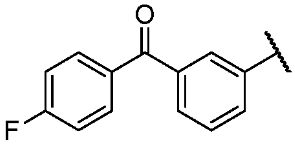,

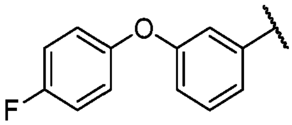, 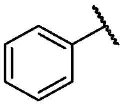, 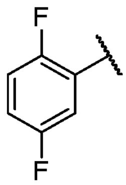, 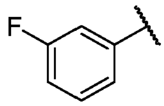, 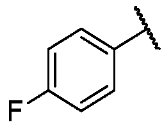,

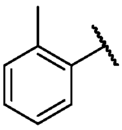, 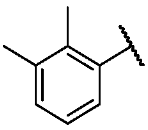, 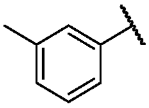, 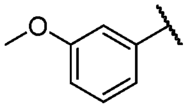, 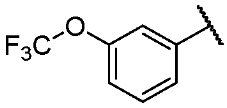,

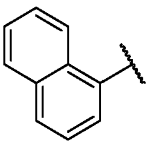, 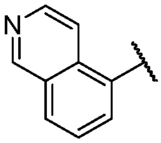, 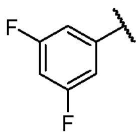, or 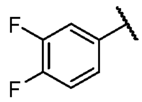;

or, $R^2$ is isopropyl or cyclopropyl;
or, L is

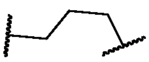 or 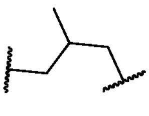;

or,

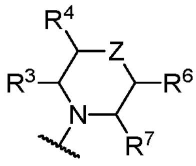

is

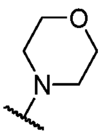 , 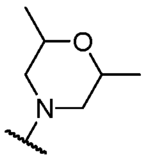 , 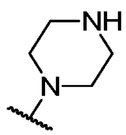 , 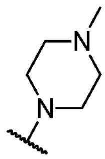 ,

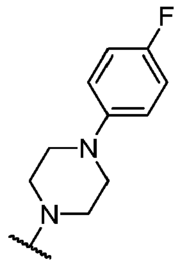 , 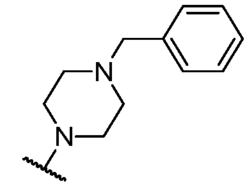 , 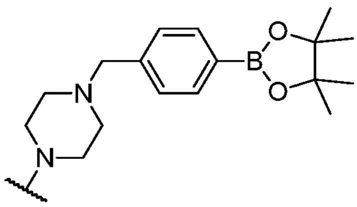 , 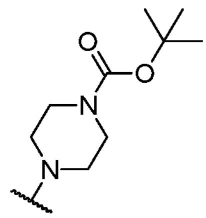 ,

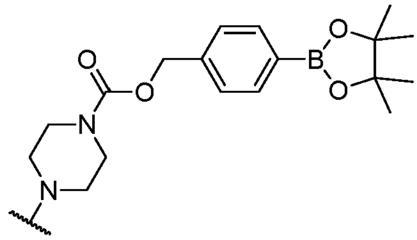 , 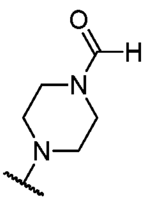 , 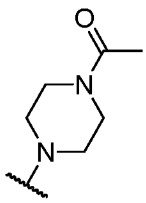 , 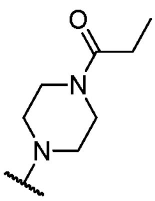 ,

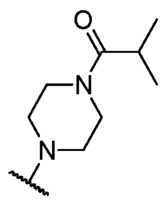 , 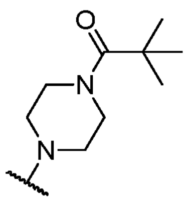 , 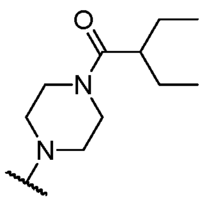 , 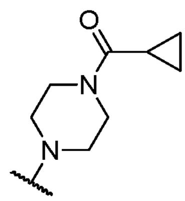 , 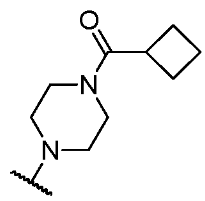 ,

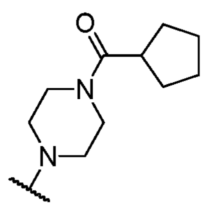 , 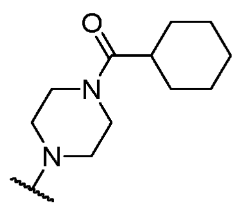 , 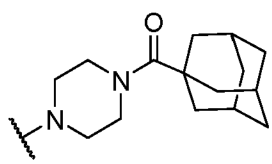 , 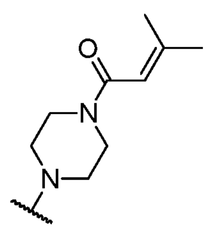 ,

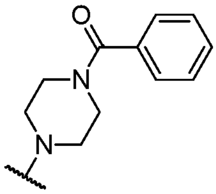, 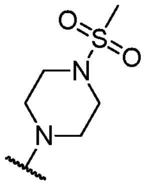, 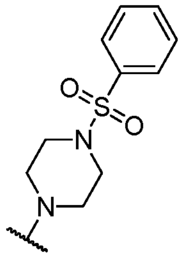, 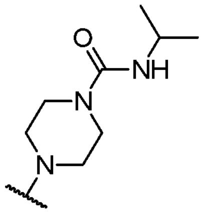, 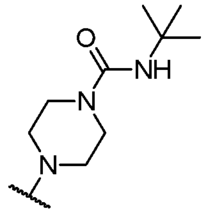,

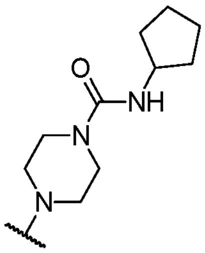, 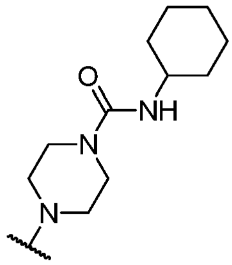, 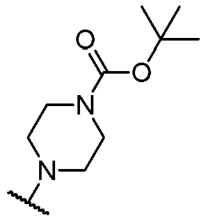, 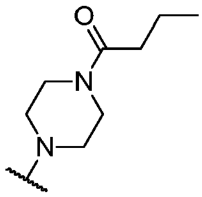, 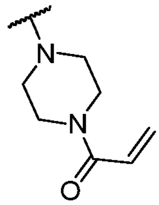,

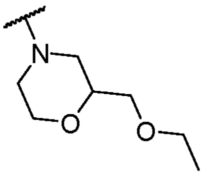, 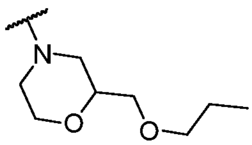, 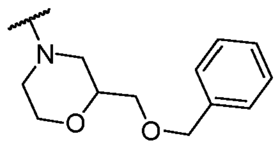, 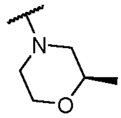,

or

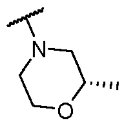;

or, the pharmaceutically acceptable salt of the compound of formula (I) is a salt prepared from the compound of formula (I) and a pharmaceutically acceptable acid, the pharmaceutically acceptable acid is hydrochloric acid or methanesulfonic acid;

or, the pharmaceutically acceptable salt of the compound of formula (I) is a salt formed by the compound of formula (I) and the pharmaceutically acceptable acid in a molar ratio of 1:2.

**6.** The compound of formula (I) as claimed in claim 1, the stereoisomer thereof, the tautomer thereof, the pharmaceutically acceptable salt thereof, or the solvate of any one of the foregoing, wherein the compound of formula (I) is any one of the following compounds:

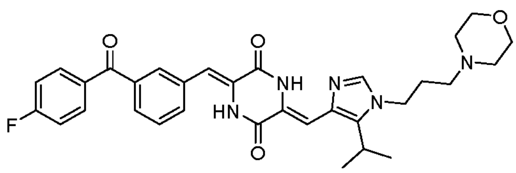, 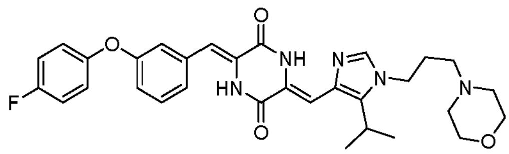,

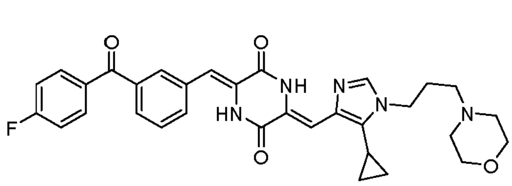, 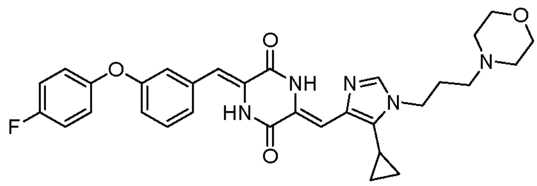,

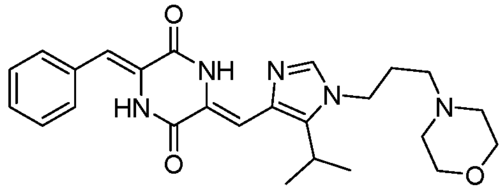,

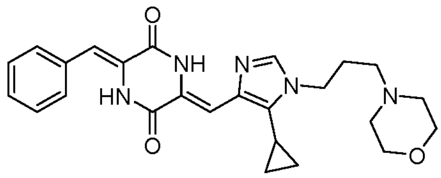,

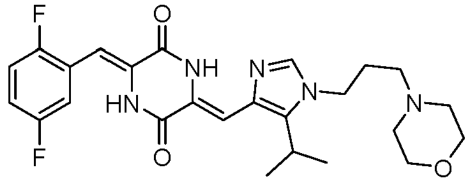,

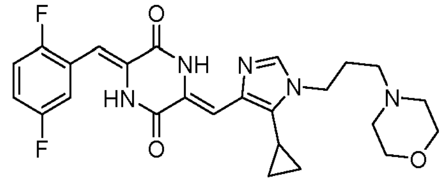,

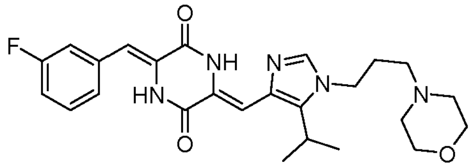,

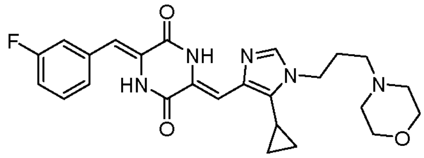,

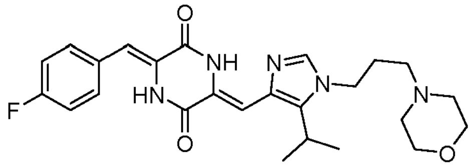,

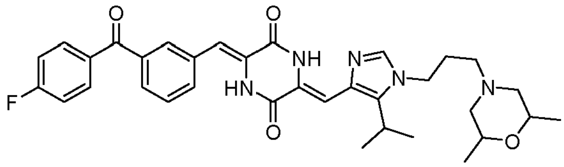,

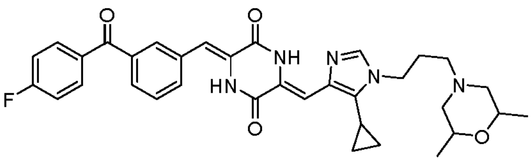,

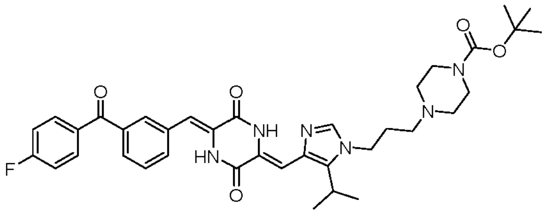,

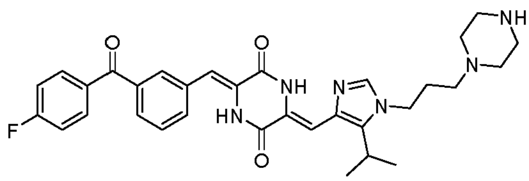,

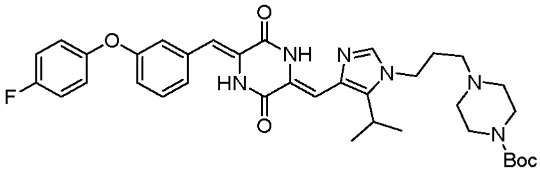,

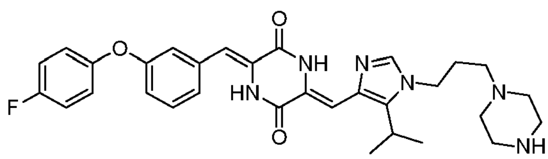,

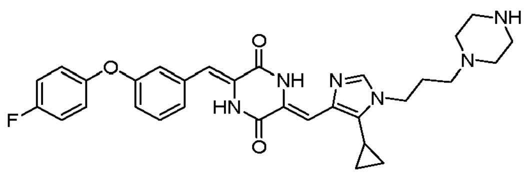,

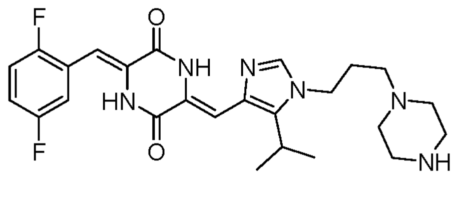,

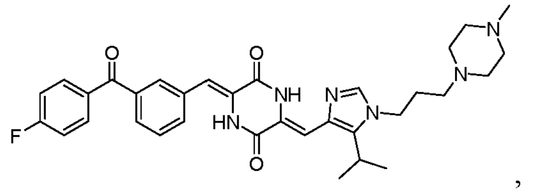,

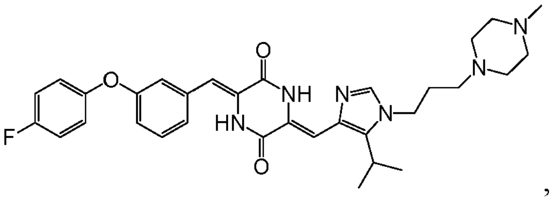,

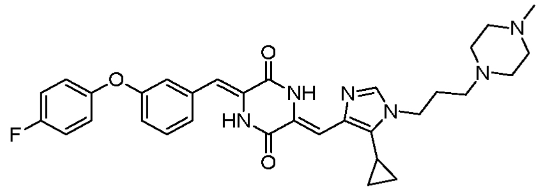,

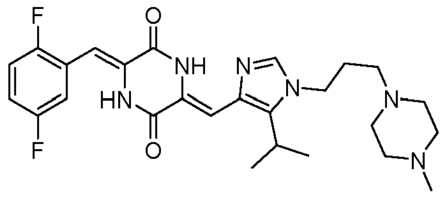,

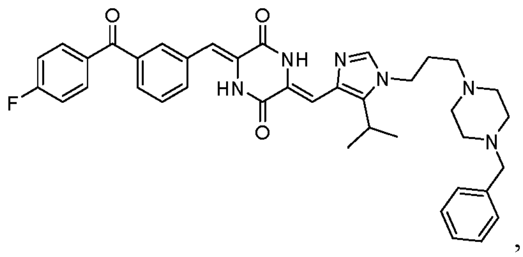,

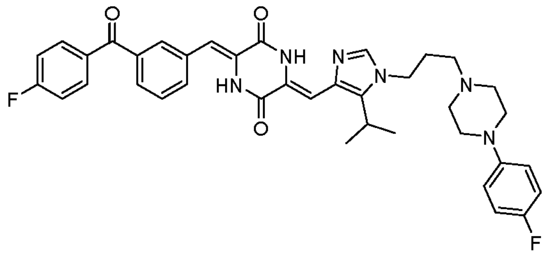,

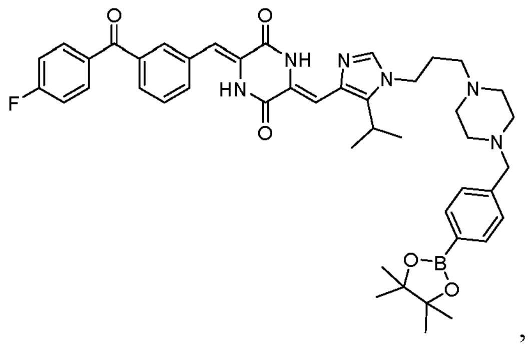,

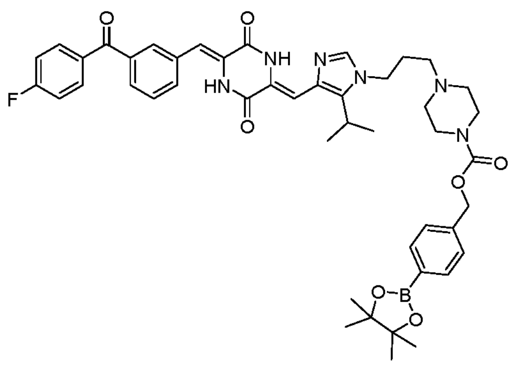,

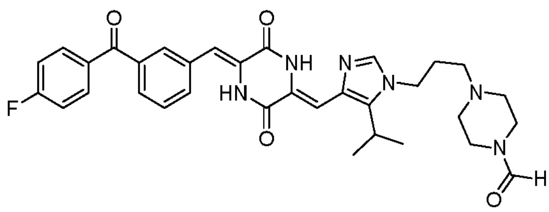,

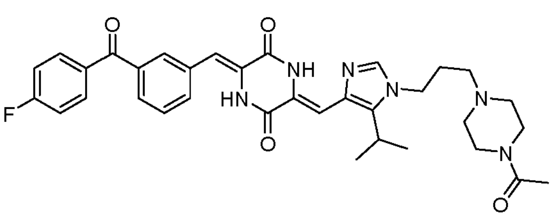,

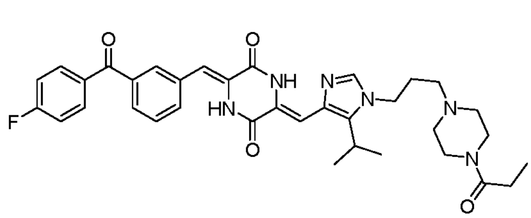,

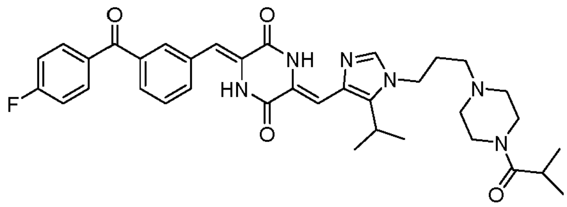,

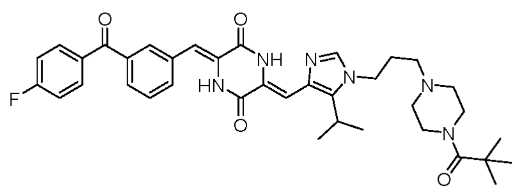,

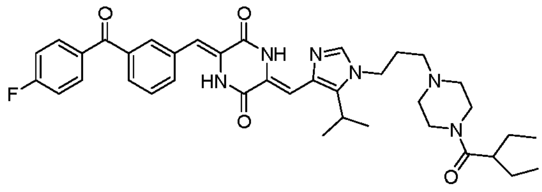,

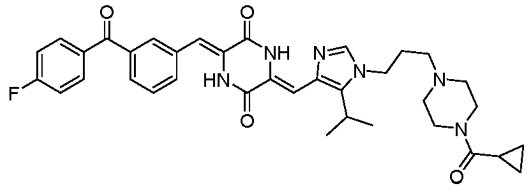,

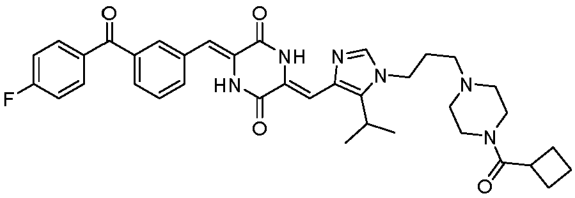,

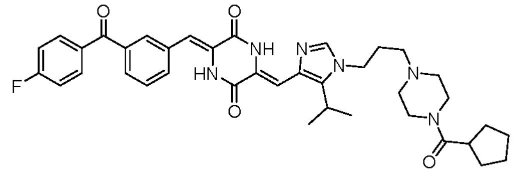,

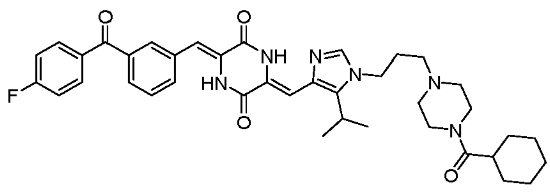,

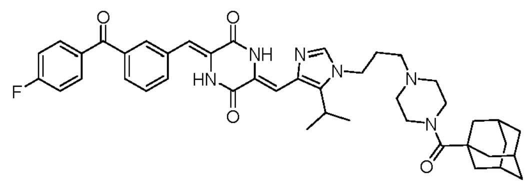,

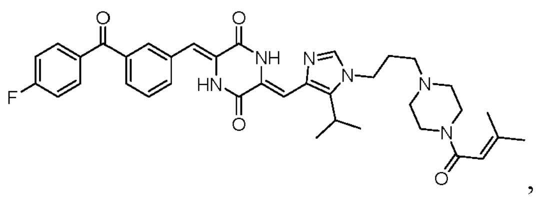,

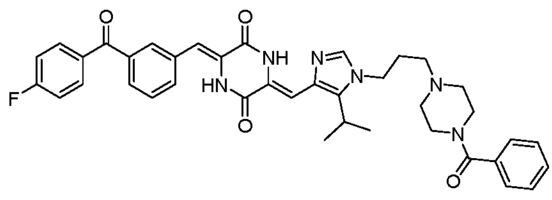,

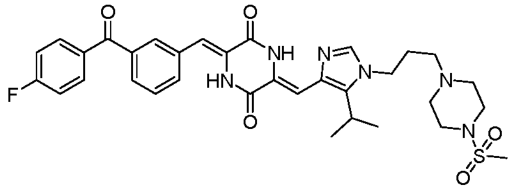,

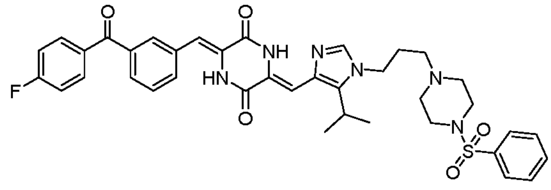,

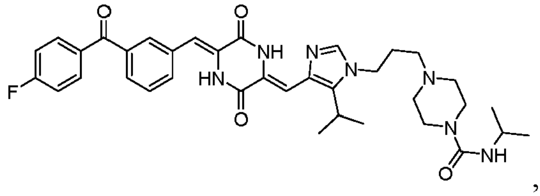,

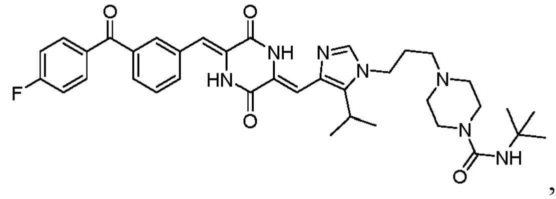,

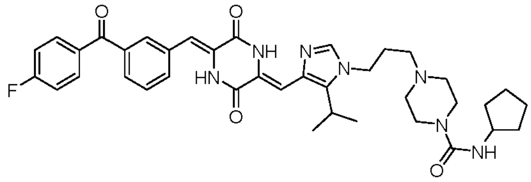
,
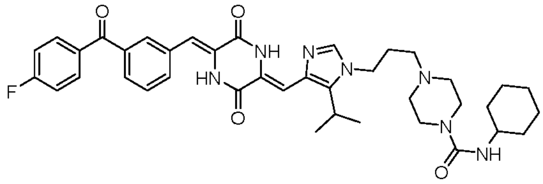
,
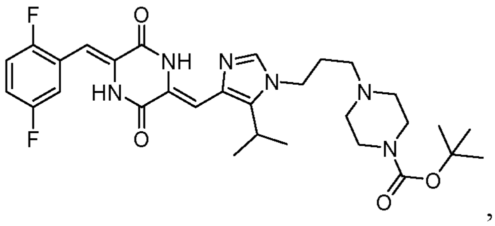
,
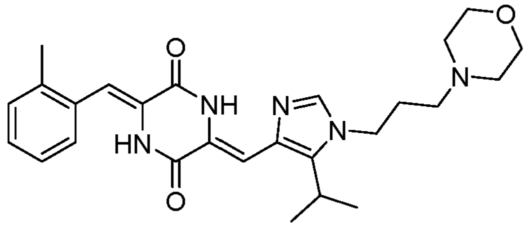
,
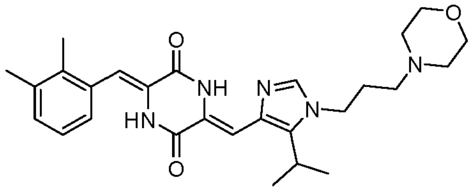
,
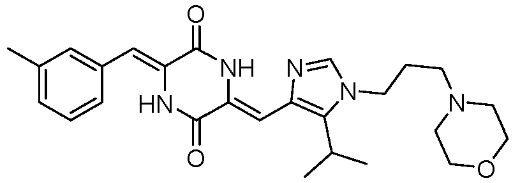
,
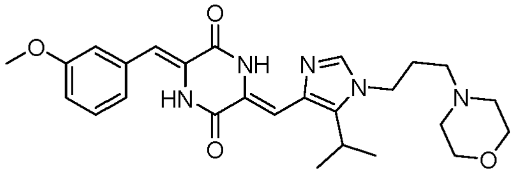
,
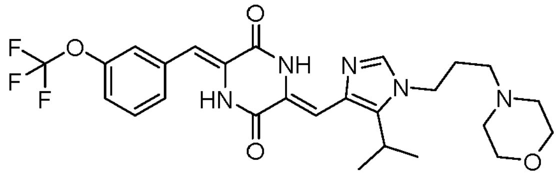
,
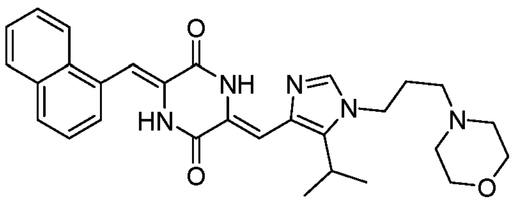
,
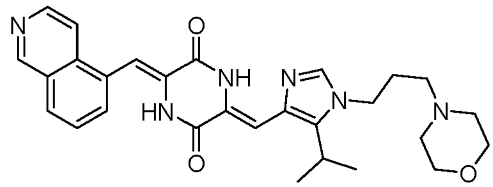
,
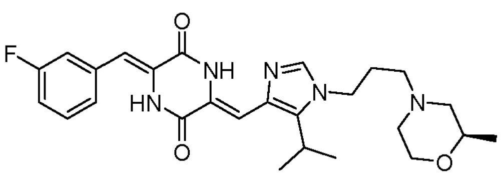
,
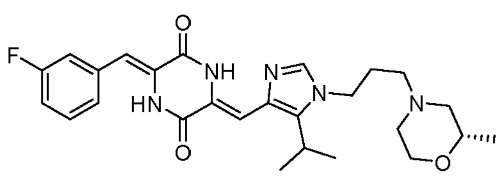
,
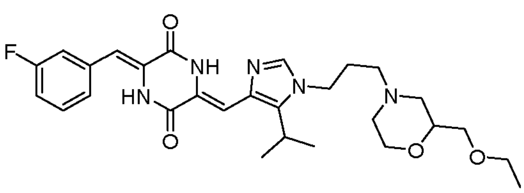
,
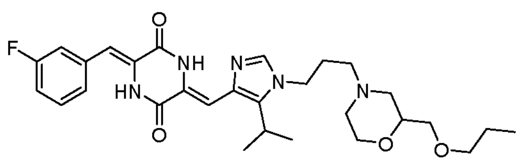
,

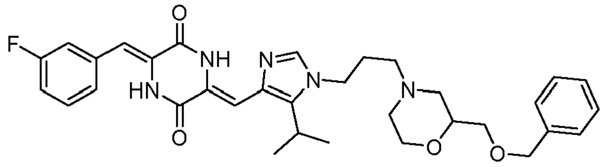,

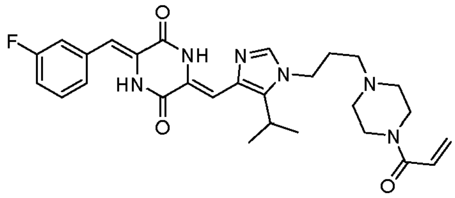,

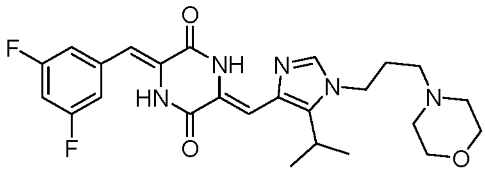,

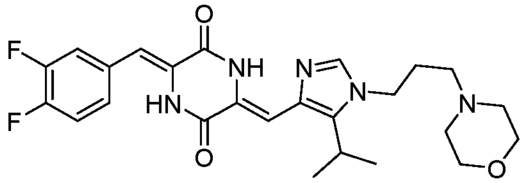,

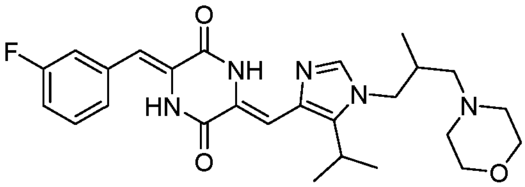,

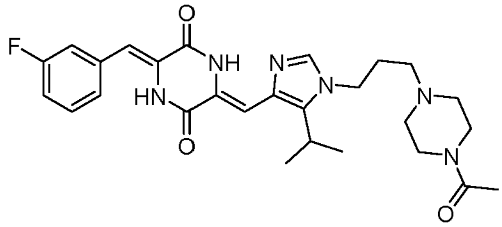,

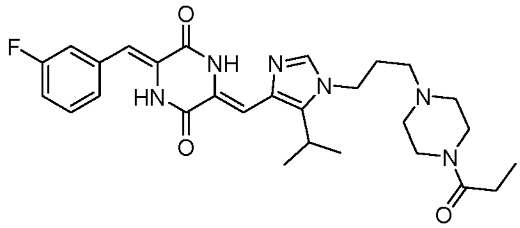,

or

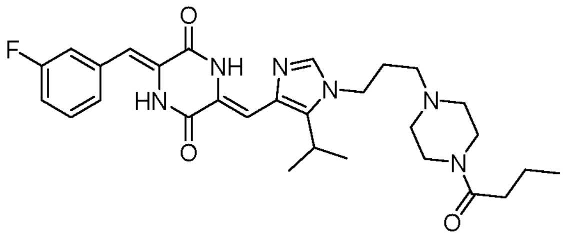;

the pharmaceutically acceptable salt of the compound of formula (I) is any one of the following compounds:

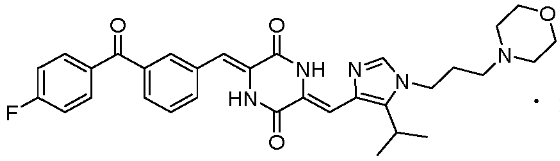 · 2HCl,

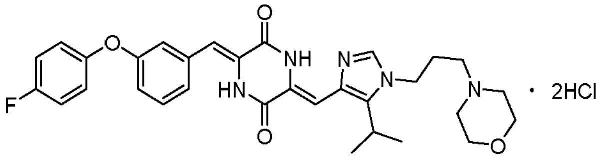

,

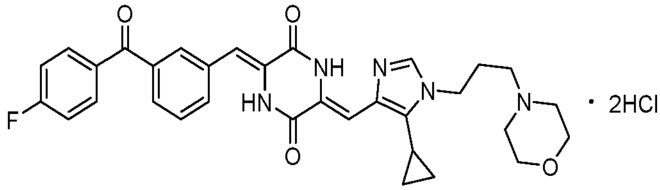

,

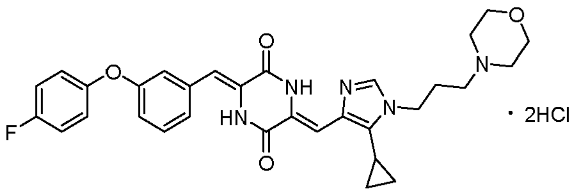

,

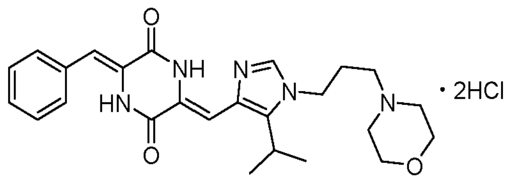

,

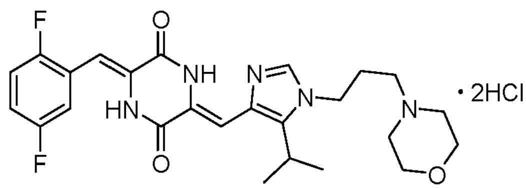

,

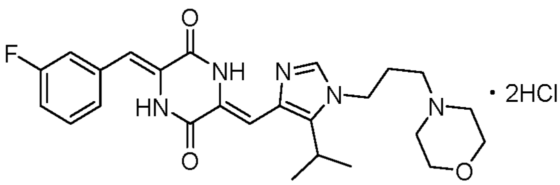

,

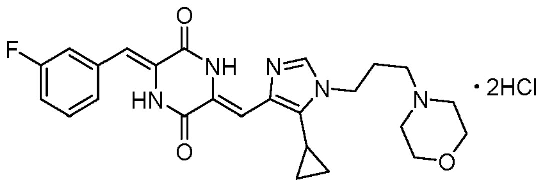

,

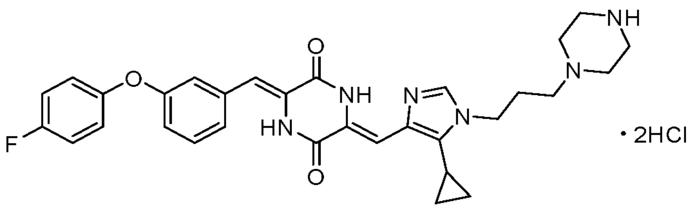

,

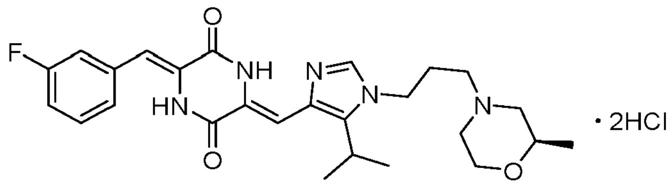
,

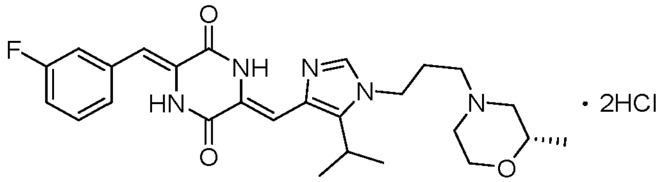
,

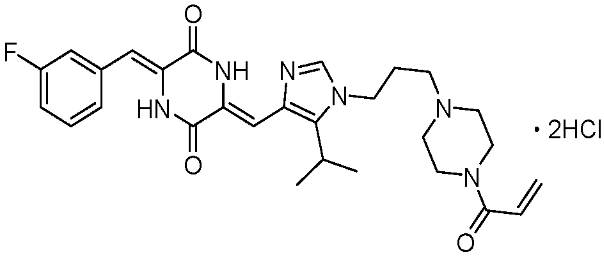
,

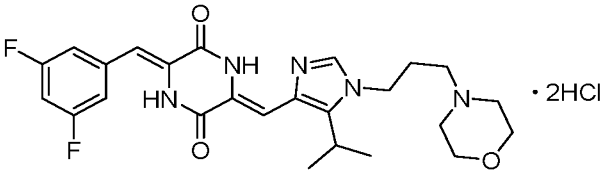
,

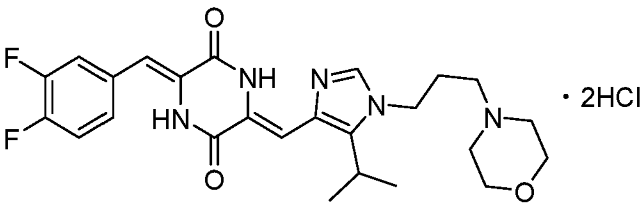
,

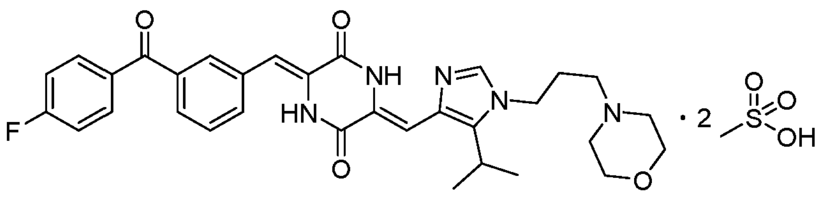
,

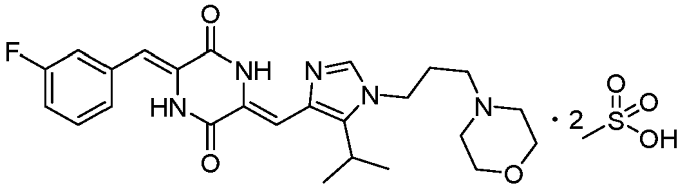
,

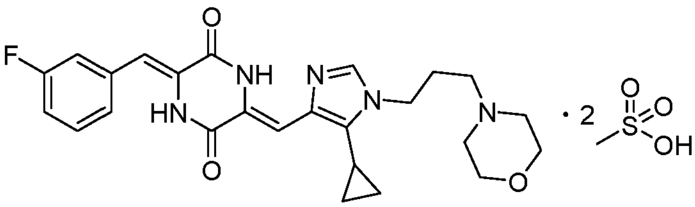

,

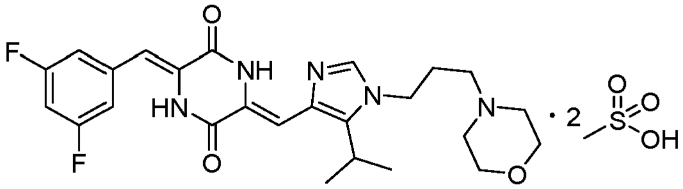

,

or

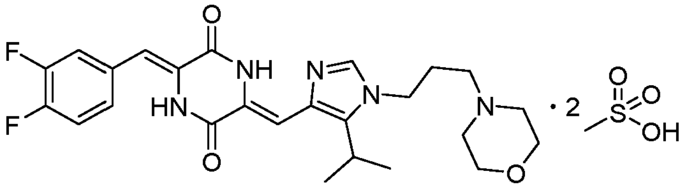

.

7. The compound of formula (I) as claimed in claim 1, the stereoisomer thereof, the tautomer thereof, the pharmaceutically acceptable salt thereof, or the solvate of any one of the foregoing, wherein the compound of formula (I) is any one of the following compounds:

a compound with a retention time of 6.29 min under the following conditions, which is one stereoisomer of

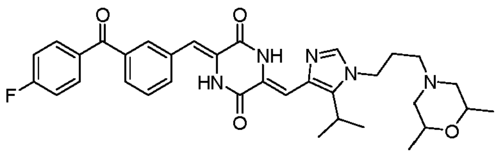

:

chromatographic column: Acclaim 120, C18, 5 μm, 4.6 * 150 mm, mobile phase: mobile phase A: MeOH, mobile phase B: 0.1% formic acid aqueous solution; flow rate: 1 mL/min, the mobile phase is washed according to the following gradient elution procedure:

| Time (min) | Mobile phase A (%, V/V) | Mobile phase B (%, V/V) |
|---|---|---|
| 0 | 40 | 60 |
| 0→6 | 40→100 | 60→0 |
| 6→10 | 100→40 | 0→60 |

;

a compound with a retention time of 6.48 min under the following conditions, which is one stereoisomer of

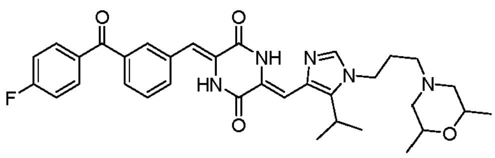

:

chromatographic column: Acclaim 120, C18, 5 μm, 4.6 * 150 mm, mobile phase: mobile phase A: MeOH, mobile phase B: 0.1% formic acid aqueous solution; flow rate: 1 mL/min, the mobile phase is washed according to the following gradient elution procedure:

| Time (min) | Mobile phase A (%, V/V) | Mobile phase B (%, V/V) |
|---|---|---|
| 0 | 40 | 60 |
| 0→6 | 40→100 | 60→0 |
| 6→10 | 100→40 | 0→60 |

8. A preparation method for a compound of formula (I), which comprises the following steps: carrying out a condensation reaction as shown below between a compound of formula (II) and a compound of formula (III) to obtain the compound of formula (I);

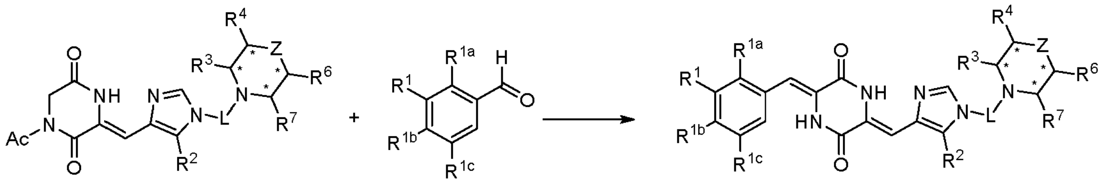

(II) (III) (I)

wherein *, L, Z, $R^1$, $R^{1a}$, $R^{1b}$, $R^{1c}$, $R^2$, $R^3$, $R^4$, $R^6$, and $R^7$ are as defined in at least one of claims 1 to 7.

9. A compound of formula (II):

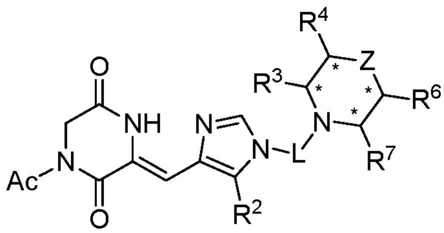

(II)

wherein *, $R^2$, $R^3$, $R^4$, $R^6$, $R^7$, L, and Z are as defined in at least one of claims 1 to 7.

10. The compound of formula (II) as claimed in claim 9, wherein the compound of formula (II) is any one of the following compounds:

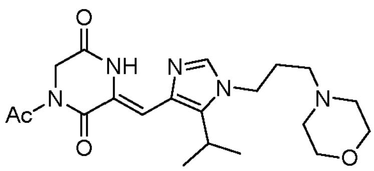

,

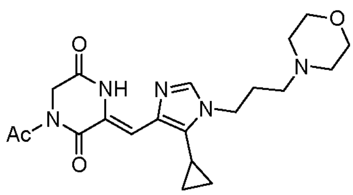

,

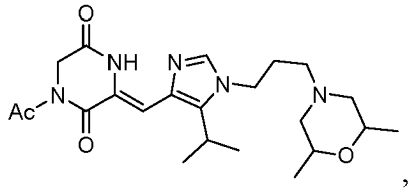,

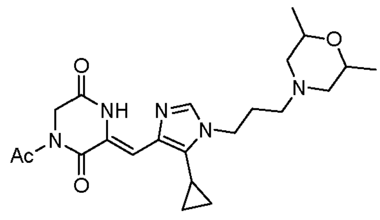,

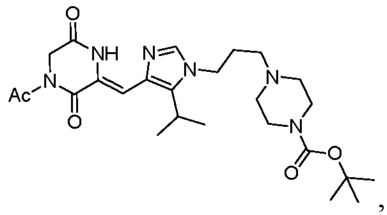,

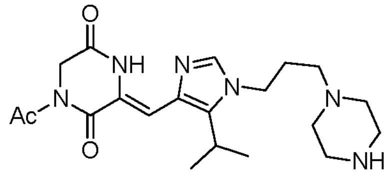,

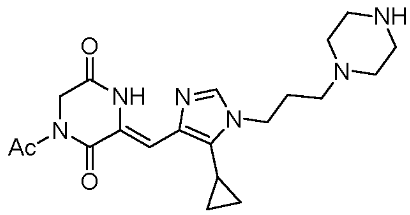,

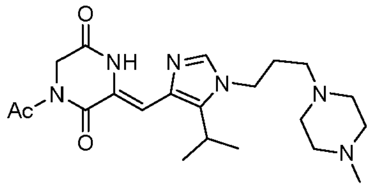,

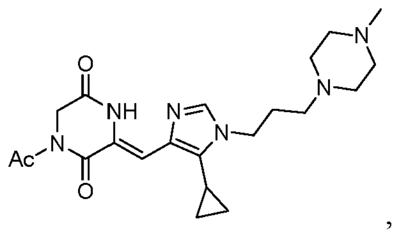,

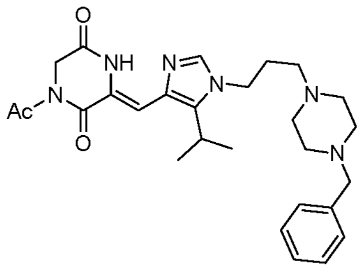,

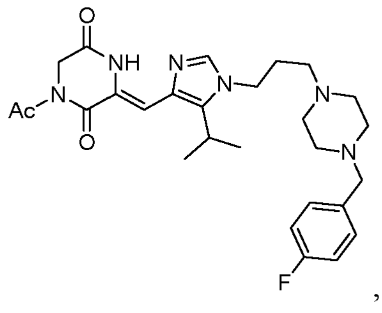,

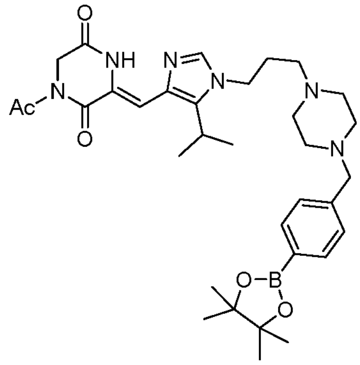,

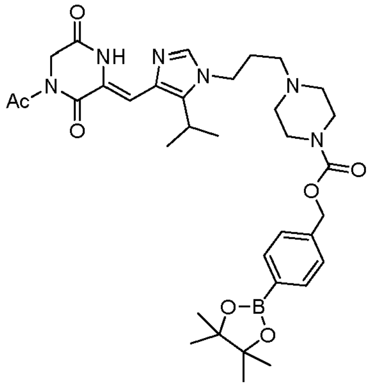,

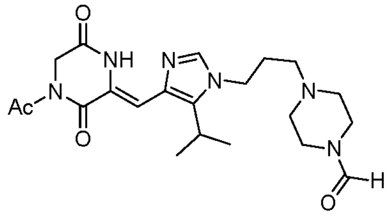,

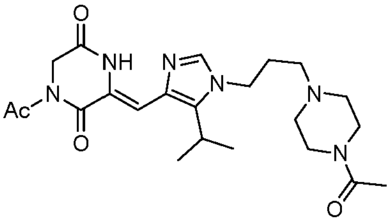,

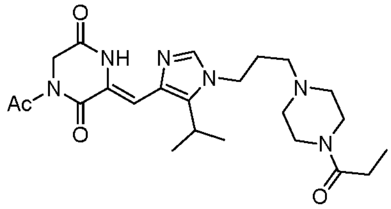,

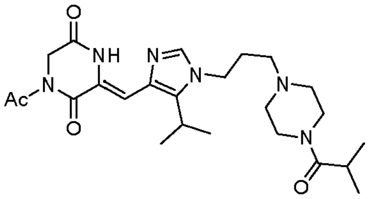,

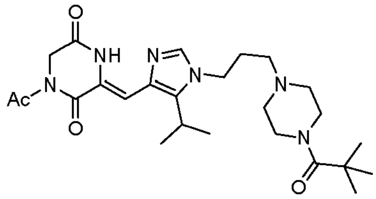,

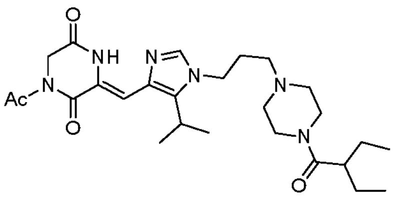,

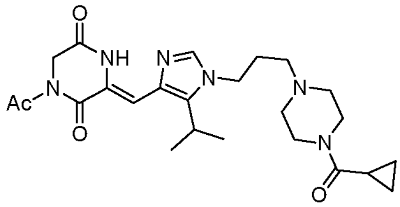,

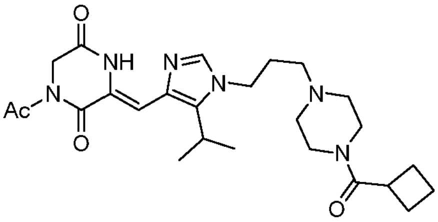,

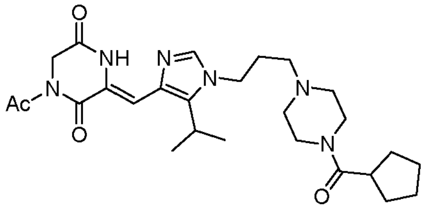,

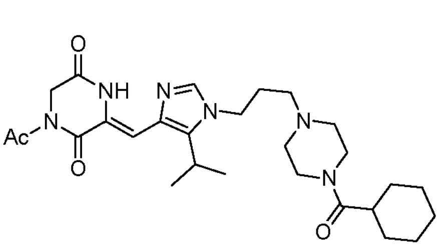,

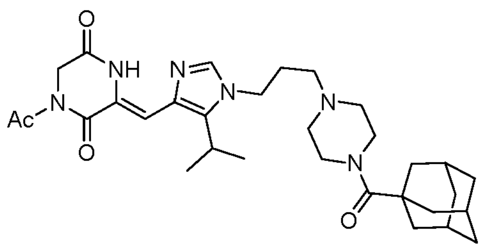,

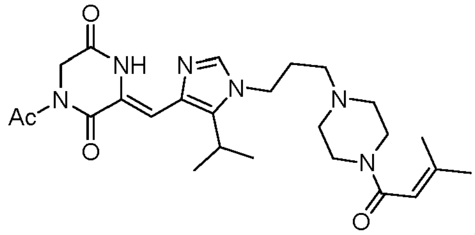,

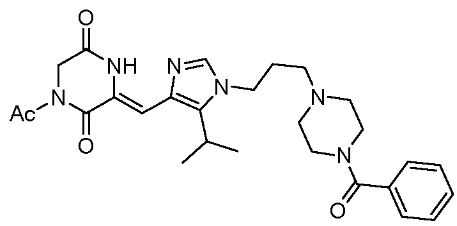,

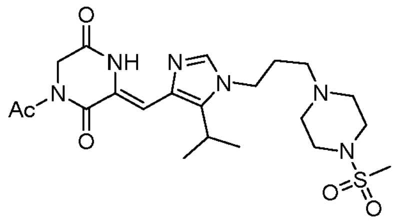,

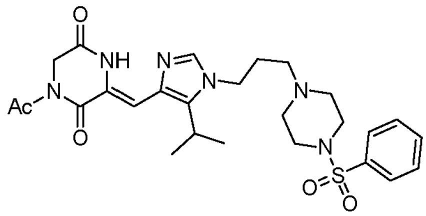,

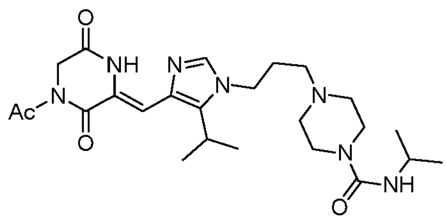,

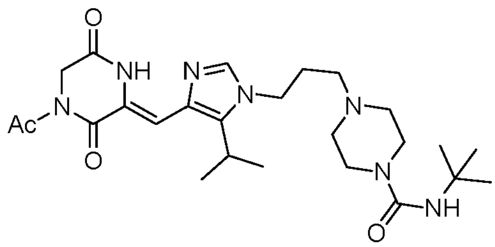,

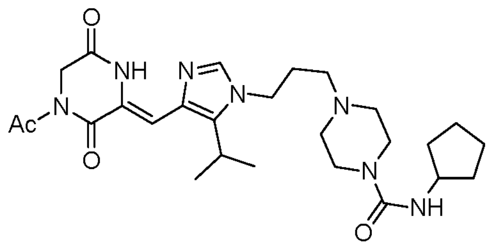,

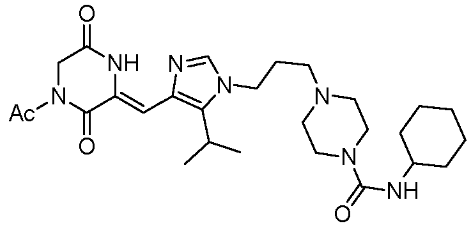,

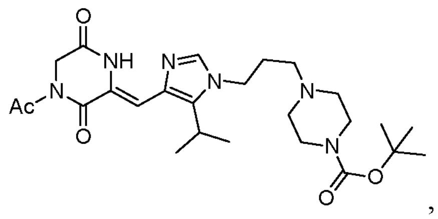,

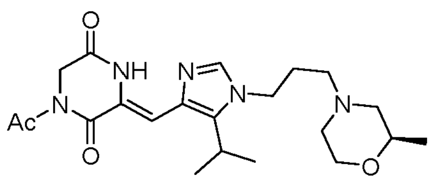,

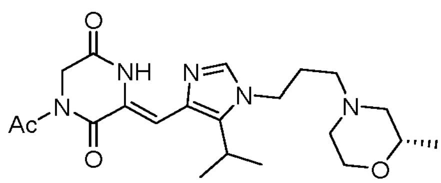,

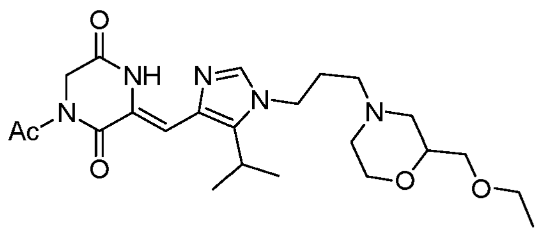,

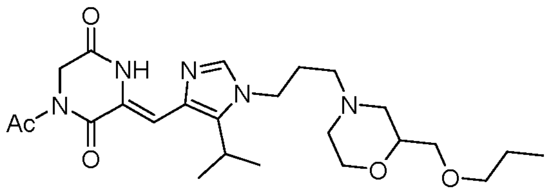

,

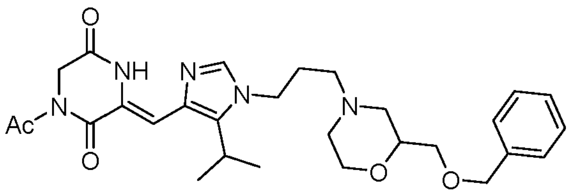

,

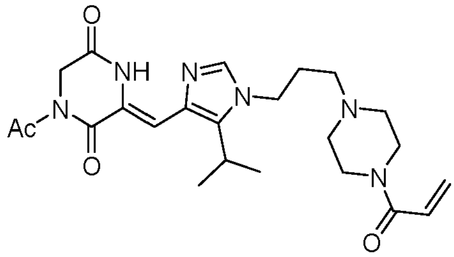

,

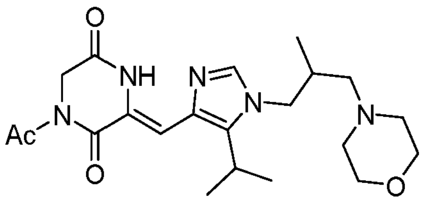

, or

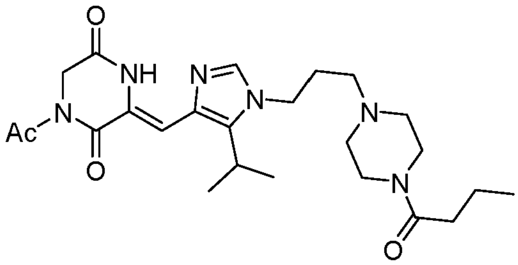

.

11. A pharmaceutical composition, comprising the compound of formula (I) as claimed in at least one of claims 1 to 7, the stereoisomer thereof, the tautomer thereof, the pharmaceutically acceptable salt thereof, or the solvate of any one of the foregoing, and a pharmaceutical excipient; preferably, the pharmaceutical excipient does not comprise a cosolvent.

12. The compound of formula (I) as claimed in at least one of claims 1 to 7, the stereoisomer thereof, the tautomer thereof, the pharmaceutically acceptable salt thereof, or the solvate of any one of the foregoing, or the pharmaceutical composition as claimed in claim 11 for use in preventing and/or treating cancer; the cancer is preferably one or more than one of lung cancer, pancreatic cancer, colon cancer, and liver cancer.

## Patentansprüche

1. Verbindung der Formel (I), ein Stereoisomer davon, ein Tautomer davon, ein pharmazeutisch verträgliches Salz davon oder ein Solvat von einem beliebigen des Vorhergehenden:

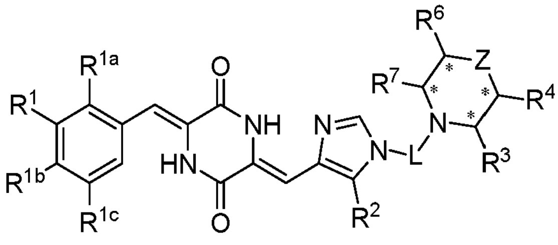

(I)

wobei ein Kohlenstoffatom mit "*" ein Kohlenstoffatom oder ein chirales Kohlenstoffatom ist, und wenn das Kohlenstoffatom mit "*" das chirale Kohlenstoffatom ist, es in einer S-Konfiguration und/oder einer R-Konfigura-

tion ist;

$R^1$ Wasserstoff, Deuterium, Halogen, $C_1$-$C_8$-Alkyl, $C_1$-$C_8$-Alkyl substituiert durch ein oder mehr als ein Halogen, $C_1$-$C_8$-Alkoxy, $C_1$-$C_8$-Alkoxy substituiert durch ein oder mehr als ein Halogen, Benzoyl, Benzoyl substituiert durch ein oder mehr als ein Halogen, Phenoxy oder Phenoxy substituiert durch ein oder mehr als ein Halogen ist;

$R^{1a}$, $R^{1b}$ und $R^{1c}$ unabhängig Wasserstoff, Deuterium, Halogen oder $C_1$-$C_8$-Alkyl sind;

oder $R^1$, $R^{1a}$ zusammen mit den Kohlenstoffatomen, an die sie gebunden sind, ein $C_6$-$C_{10}$-Aryl oder ein 3- bis 10-gliedriges Heteroaryl mit 1-5 Heteroatomen ausgewählt aus einem oder mehr als einem von N, O und S bilden;

oder $R^1$, $R^{1b}$ zusammen mit den Kohlenstoffatomen, an die sie gebunden sind, ein $C_6$-$C_{10}$-Aryl oder ein 3- bis 10-gliedriges Heteroaryl mit 1-5 Heteroatomen ausgewählt aus einem oder mehr als einem von N, O und S bilden;

oder $R^{1b}$, $R^{1c}$ zusammen mit den Kohlenstoffatomen, an die sie gebunden sind, ein $C_6$-$C_{10}$-Aryl oder ein 3- bis 10-gliedriges Heteroaryl mit 1-5 Heteroatomen ausgewählt aus einem oder mehr als einem von N, O und S bilden;

$R^2$ $C_1$-$C_8$-Alkyl oder $C_3$-$C_{10}$-Cycloalkyl ist;

L $C_1$-$C_8$-Heteroalkylen mit 1-4 Heteroatomen ausgewählt aus einem oder mehr als einem von N, O, S und Se oder $C_1$-$C_8$-Alkylen ist;

$R^3$, $R^4$, $R^6$ und $R^7$ unabhängig Wasserstoff, Deuterium, $C_1$-$C_8$-Alkyl, $C_1$-$C_8$-Alkyl substituiert durch ein oder mehr als ein $R^{3-1}$, $C_1$-$C_8$-Alkoxy, $C_3$-$C_{10}$-Cycloalkyl, $C_6$-$C_{10}$-Aryl oder 3- bis 10-gliedriges Heterocycloalkyl mit 1-5 Heteroatomen ausgewählt aus einem oder mehr als einem von N, O und S sind;

$R^{3-1}$ unabhängig O-$R^{3-1-1}$ ist, $R^{3-1-1}$ $C_1$-$C_8$-Alkyl oder $C_1$-$C_8$-Alkyl substituiert durch ein oder mehr als ein $R^{3-1-2}$ ist;

$R^{3-1-2}$ unabhängig $C_6$-$C_{10}$-Aryl ist;

Z O oder N($R^5$) ist;

$R^5$ Wasserstoff, Deuterium, $C_1$-$C_8$-Alkyl, $C_6$-$C_{10}$-Aryl substituiert durch ein oder mehr als ein $R^{5-1}$, Benzyl, Benzyl substituiert durch ein oder mehr als ein $R^{5-2}$, -C(=O)-$R^{5-3}$ oder -S(=O)$_2$-$R^{5-4}$ ist;

$R^{5-1}$ unabhängig Halogen ist;

$R^{5-2}$ unabhängig

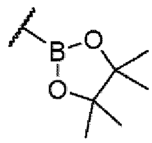

ist;

$R^{5-3}$ Wasserstoff, $C_1$-$C_8$-Alkoxy, Benzyloxy, Benzyloxy substituiert durch ein oder mehr als ein $R^{5-2}$, $C_1$-$C_8$-Alkyl, $C_3$-$C_{10}$-Cycloalkyl, $C_2$-$C_8$-Alkenyl, $C_6$-$C_{10}$-Aryl oder $NR^{5-3-1}R^{5-3-2}$ ist; $R^{5-3-1}$ und $R^{5-3-2}$ unabhängig Wasserstoff, $C_1$-$C_8$-Alkyl oder $C_3$-$C_{10}$-Cycloalkyl sind;

$R^{5-4}$ $C_1$-$C_8$-Alkyl oder $C_6$-$C_{10}$-Aryl ist.

**2.** Verbindung der Formel (I) nach Anspruch 1, das Stereoisomer davon, das Tautomer davon, das pharmazeutisch verträgliche Salz davon oder das Solvat von einem beliebigen des Vorhergehenden, wobei, wenn $R^1$ Halogen ist, das Halogen Fluor, Chlor, Brom oder Iod ist;

oder, wenn $R^1$ $C_1$-$C_8$-Alkyl ist, das $C_1$-$C_8$-Alkyl $C_1$-$C_4$-Alkyl ist;

oder, wenn $R^1$ $C_1$-$C_8$-Alkyl substituiert durch ein oder mehr als ein Halogen ist, das $C_1$-$C_8$-Alkyl substituiert durch ein oder mehr als ein Halogen $C_1$-$C_4$-Alkyl substituiert durch ein oder mehr als ein Halogen ist;

oder, wenn $R^1$ $C_1$-$C_8$-Alkoxy ist, das $C_1$-$C_8$-Alkoxy $C_1$-$C_4$-Alkoxy ist;

oder, wenn $R^1$ $C_1$-$C_8$-Alkoxy substituiert durch ein oder mehr als ein Halogen ist, das $C_1$-$C_8$-Alkoxy substituiert durch ein oder mehr als ein Halogen $C_1$-$C_4$-Alkoxy substituiert durch ein oder mehr als ein Halogen ist;

oder, wenn $R^1$ Benzoyl substituiert durch ein oder mehr als ein Halogen ist, das Halogen Fluor, Chlor, Brom oder Iod ist;

oder, wenn $R^1$ Phenoxy substituiert durch ein oder mehr als ein Halogen ist, das Halogen Fluor, Chlor, Brom oder Iod ist;

oder, wenn $R^{1a}$, $R^{1b}$ und $R^{1c}$ unabhängig Halogen sind, das Halogen Fluor, Chlor, Brom oder Iod ist;

oder, wenn $R^{1a}$, $R^{1b}$ und $R^{1c}$ unabhängig $C_1$-$C_8$-Alkyl sind, das $C_1$-$C_8$-Alkyl $C_1$-$C_4$-Alkyl ist;

oder, wenn $R^1$, $R^{1a}$ zusammen mit den Kohlenstoffatomen, an die sie gebunden sind, das $C_6$-$C_{10}$-Aryl bilden, das $C_6$-$C_{10}$-Aryl Phenyl ist;

oder, wenn $R^1$, $R^{1a}$ zusammen mit den Kohlenstoffatomen, an die sie gebunden sind, das 3- bis 10-gliedrige Heteroaryl mit 1-5 Heteroatomen ausgewählt aus einem oder mehr als einem von N, O und S bilden, das 3- bis 10-gliedrige Heteroaryl mit 1-5 Heteroatomen ausgewählt aus einem oder mehr als einem von N, O und S 4- bis 8-gliedriges Heteroaryl mit 1-2 Heteroatomen ausgewählt aus einem oder mehr als einem von N, O und S ist;
oder, wenn $R^2$ $C_1$-$C_8$-Alkyl ist, das $C_1$-$C_8$-Alkyl $C_1$-$C_4$-Alkyl ist;
oder, wenn $R^2$ $C_3$-$C_{10}$-Cycloalkyl ist, $C_3$-$C_{10}$-Cycloalkyl $C_3$-$C_6$-Cycloalkyl ist;
oder, wenn L $C_1$-$C_8$-Alkylen ist, das $C_1$-$C_8$-Alkylen $C_3$-$C_8$-Alkylen ist;
oder, wenn $R^3$, $R^4$, $R^6$ und $R^7$ unabhängig $C_1$-$C_8$-Alkyl sind, das $C_1$-$C_8$-Alkyl $C_1$-$C_4$-Alkyl ist;
oder, wenn $R^3$, $R^4$, $R^6$ und $R^7$ unabhängig $C_1$-$C_8$-Alkyl substituiert durch ein oder mehr als ein $R^{3-1}$ sind, das $C_1$-$C_8$-Alkyl $C_1$-$C_4$-Alkyl ist;
oder, wenn $R^{3-1-1}$ $C_1$-$C_8$-Alkyl ist, das $C_1$-$C_8$-Alkyl $C_1$-$C_4$-Alkyl ist;
oder, wenn $R^{3-1-1}$ $C_1$-$C_8$-Alkyl substituiert durch ein oder mehr als ein $R^{3-1-2}$ ist, das $C_1$-$C_8$-Alkyl $C_1$-$C_4$-Alkyl ist;
oder, wenn $R^{3-1-2}$ unabhängig $C_6$-$C_{10}$-Aryl ist, das $C_6$-$C_{10}$-Aryl Phenyl ist;
oder, wenn $R^5$ $C_1$-$C_8$-Alkyl ist, das $C_1$-$C_8$-Alkyl $C_1$-$C_4$-Alkyl ist;
oder, wenn $R^5$ $C_6$-$C_{10}$-Aryl substituiert durch ein oder mehr als ein $R^{5-1}$ ist, das $C_6$-$C_{10}$-Aryl Phenyl oder Naphthyl ist;
oder, wenn $R^{5-1}$ Halogen ist, das Halogen Fluor, Chlor, Brom oder Iod ist;
oder, wenn $R^{5-3}$ $C_1$-$C_8$-Alkoxy ist, das $C_1$-$C_8$-Alkoxy $C_1$-$C_4$-Alkoxy ist;
oder, wenn $R^{5-3}$ $C_1$-$C_8$-Alkyl ist, das $C_1$-$C_8$-Alkyl Methyl, Ethyl, n-Propyl, Isopropyl, tert-Butyl oder $-CH(C_2H_5)CH_2CH_3$ ist;
oder, wenn $R^{5-3}$ $C_3$-$C_{10}$-Cycloalkyl ist, das $C_3$-$C_{10}$-Cycloalkyl Cyclopropyl, Cyclobutyl, Cyclopentyl, Cyclohexyl oder Adamantyl ist;
oder, wenn $R^{5-3}$ $C_2$-$C_8$-Alkenyl ist, das $C_2$-$C_8$-Alkenyl $C_2$-$C_4$-Alkenyl ist;
oder, wenn $R^{5-3}$ $C_6$-$C_{10}$-Aryl ist, das $C_6$-$C_{10}$-Aryl Phenyl oder Naphthyl ist;
oder, wenn $R^{5-3-1}$ und $R^{5-3-2}$ unabhängig $C_1$-$C_8$-Alkyl sind, das $C_1$-$C_8$-Alkyl $C_1$-$C_4$-Alkyl ist;
oder, wenn $R^{5-3-1}$ und $R^{5-3-2}$ unabhängig $C_3$-$C_{10}$-Cycloalkyl sind, das $C_3$-$C_{10}$-Cycloalkyl $C_3$-$C_6$-Cycloalkyl ist;
oder, wenn $R^{5-4}$ $C_1$-$C_8$-Alkyl ist, das $C_1$-$C_8$-Alkyl $C_1$-$C_4$-Alkyl ist;
oder, wenn $R^{5-4}$ $C_6$-$C_{10}$-Aryl ist, das $C_6$-$C_{10}$-Aryl Phenyl oder Naphthyl ist.

3. Verbindung der Formel (I) nach Anspruch 1, das Stereoisomer davon, das Tautomer davon, das pharmazeutisch verträgliche Salz davon oder das Solvat von einem beliebigen des Vorhergehenden, wobei, wenn $R^1$ Halogen ist, das Halogen Fluor ist;

oder, wenn $R^1$ $C_1$-$C_8$-Alkyl ist, das $C_1$-$C_8$-Alkyl Methyl ist;
oder, wenn $R^1$ $C_1$-$C_8$-Alkyl substituiert durch ein oder mehr als ein Halogen ist, das $C_1$-$C_8$-Alkyl substituiert durch ein oder mehr als ein Halogen Trifluormethyl ist;
oder, wenn $R^1$ $C_1$-$C_8$-Alkoxy ist, das $C_1$-$C_8$-Alkoxy Methoxy ist;
oder, wenn $R^1$ $C_1$-$C_8$-Alkoxy substituiert durch ein oder mehr als ein Halogen ist, das $C_1$-$C_8$-Alkoxy substituiert durch ein oder mehr als ein Halogen Trifluormethoxy ist;
oder, wenn $R^1$ Benzoyl substituiert durch ein oder mehr als ein Halogen ist, das Halogen Fluor ist; bevorzugt das Benzoyl substituiert durch ein oder mehr als ein Halogen

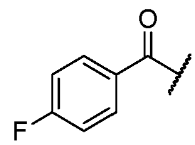

ist,
oder, wenn $R^1$ Phenoxy substituiert durch ein oder mehr als ein Halogen ist, das Halogen Fluor ist; bevorzugt das Phenoxy substituiert durch ein oder mehr als ein Halogen

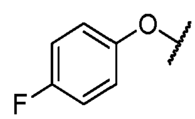

ist;
oder, wenn $R^{1a}$, $R^{1b}$ und $R^{1c}$ unabhängig Halogen sind, das Halogen Fluor ist;
oder, wenn $R^{1a}$, $R^{1b}$ und $R^{1c}$ unabhängig $C_1$-$C_8$-Alkyl sind, das $C_1$-$C_8$-Alkyl Methyl ist;
oder, wenn $R^1$, $R^{1a}$ zusammen mit den Kohlenstoffatomen, an die sie gebunden sind, das 3- bis 10-gliedrige

Heteroaryl mit 1-5 Heteroatomen ausgewählt aus einem oder mehr als einem von N, O und S bilden, das 3- bis 10-gliedrige Heteroaryl mit 1-5 Heteroatomen ausgewählt aus einem oder mehr als einem von N, O und S Pyridyl ist; bevorzugt das 3- bis 10-gliedrige Heteroaryl mit 1-5 Heteroatomen ausgewählt aus einem oder mehr als einem von N, O und S

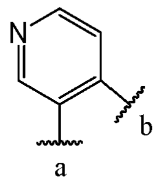

ist, mit einem a-Terminal gebunden an das Kohlenstoffatom, an das $R^1$ gebunden ist, und einem b-Terminal, das an das Kohlenstoffatom gebunden ist, an das $R^{1a}$ gebunden ist;
oder, wenn $R^2$ $C_1$-$C_8$-Alkyl ist, das $C_1$-$C_8$-Alkyl Methyl, Ethyl, Isopropyl oder tert-Butyl ist;
oder, wenn $R^2$ $C_3$-$C_{10}$-Cycloalkyl ist, das $C_3$-$C_{10}$-Cycloalkyl Cyclopropyl ist;
oder, wenn L $C_1$-$C_8$-Alkylen ist, das $C_1$-$C_8$-Alkylen

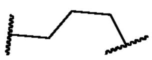, 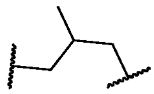,

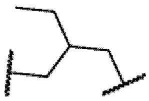, 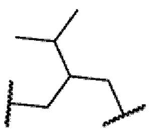, 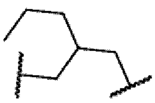 oder 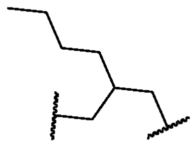

ist, wie

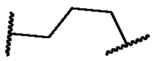;

oder, wenn $R^3$, $R^4$, $R^6$ und $R^7$ unabhängig $C_1$-$C_8$-Alkyl sind, das $C_1$-$C_8$-Alkyl Methyl oder Ethyl ist, wie Methyl;
oder, wenn $R^3$, $R^4$, $R^6$ und $R^7$ unabhängig $C_1$-$C_8$-Alkyl substituiert durch ein oder mehr als ein $R^{3-1}$ sind, das $C_1$-$C_8$-Alkyl Methyl ist;
oder, wenn $R^{3-1-1}$ $C_1$-$C_8$-Alkyl ist, das $C_1$-$C_8$-Alkyl Methyl, Ethyl, n-Propyl oder n-Butyl ist;
oder, wenn $R^{3-1-1}$ $C_1$-$C_8$-Alkyl substituiert durch ein oder mehr als ein $R^{3-1-2}$ ist, das $C_1$-$C_8$-Alkyl Methyl ist;
oder, wenn $R^5$ $C_1$-$C_8$ Alkyl ist, das $C_1$-$C_8$ Alkyl Methyl, Ethyl, n-Propyl, Isopropyl, n-Butyl oder 2-Methylpropyl ist, wie Methyl;
oder, wenn $R^5$ $C_6$-$C_{10}$-Aryl substituiert durch ein oder mehr als ein $R^{5-1}$ ist, das $C_6$-$C_{10}$-Aryl Phenyl ist;
oder, wenn $R^{5-1}$ Halogen ist, das Halogen Fluor ist;
oder, wenn $R^{5-3}$ $C_1$-$C_8$-Alkoxy ist, das $C_1$-$C_8$-Alkoxy tert-Butoxy ist;
oder, wenn $R^{5-3}$ $C_1$-$C_8$-Alkyl ist, das $C_1$-$C_8$-Alkyl $C_1$-$C_4$-Alkyl ist;
oder, wenn $R^{5-3}$ $C_3$-$C_{10}$-Cycloalkyl ist, $C_3$-$C_{10}$-Cycloalkyl $C_3$-$C_6$-Cycloalkyl ist;
oder, wenn $R^{5-3}$ $C_2$-$C_8$-Alkenyl ist, das $C_2$-$C_8$-Alkenyl

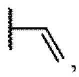, 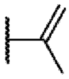, 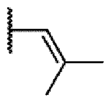

oder

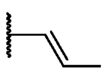

ist, wie

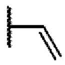 oder 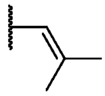 ;

oder, wenn $R^{5-3}$ $C_6$-$C_{10}$-Aryl ist, das $C_6$-$C_{10}$-Aryl Phenyl ist;
oder, wenn $R^{5-3-1}$ und $R^{5-3-2}$ unabhängig $C_1$-$C_8$-Alkyl sind, das $C_1$-$C_8$-Alkyl Isopropyl oder tert-Butyl ist;
oder, wenn $R^{5-3-1}$ und $R^{5-3-2}$ unabhängig $C_3$-$C_{10}$-Cycloalkyl sind, das $C_3$-$C_{10}$-Cycloalkyl Cyclopentyl oder Cyclohexyl ist;
oder, wenn $R^{5-4}$ $C_1$-$C_8$-Alkyl ist, das $C_1$-$C_8$-Alkyl Methyl ist;
oder, wenn $R^{5-4}$ $C_6$-$C_{10}$-Aryl ist, das $C_6$-$C_{10}$-Aryl Phenyl ist.

**4.** Verbindung der Formel (I) nach Anspruch 1, das Stereoisomer davon, das Tautomer davon, das pharmazeutisch verträgliche Salz davon oder das Solvat von einem beliebigen des Vorhergehenden, wobei $R^1$ Wasserstoff, Halogen, $C_1$-$C_8$-Alkyl, $C_1$-$C_8$-Alkoxy, $C_1$-$C_8$-Alkoxy substituiert durch ein oder mehr als ein Halogen, Benzoyl, Benzoyl substituiert durch ein oder mehr als ein Halogen, Phenoxy oder Phenoxy substituiert durch ein oder mehr als ein Halogen ist;

oder $R^{1b}$ Wasserstoff oder Halogen ist; wie Wasserstoff oder Fluor;
oder $R^{1c}$ Wasserstoff oder Halogen ist, wie Wasserstoff oder Fluor;
oder $R^2$ $C_1$-$C_8$-Alkyl ist;
oder L $C_1$-$C_8$-Alkylen ist;
oder $R^3$, $R^4$, $R^6$ und $R^7$ unabhängig Wasserstoff oder $C_1$-$C_8$-Alkyl sind;
oder $R^5$ Benzyl, -C(=O)-$R^{5-3}$ oder -S(=O)$_2$-$R^{5-4}$ ist;
oder $R^{5-3}$ Wasserstoff, $C_1$-$C_4$-Alkyl, $C_3$-$C_6$-Cycloalkyl, $C_2$-$C_8$-Alkenyl, Benzyloxy substituiert durch ein oder mehr als ein $R^{5-2}$, $C_6$-$C_{10}$-Aryl oder $NR^{5-3-1}R^{5-3-2}$ ist;
oder das pharmazeutisch verträgliche Salz der Verbindung der Formel (I) ein Salz ist, das aus der Verbindung der Formel (I) und einer pharmazeutisch verträglichen Säure zubereitet ist, die pharmazeutisch verträgliche Säure eine anorganische Säure oder eine organische Säure ist, die anorganische Säure bevorzugt Salzsäure ist und die organische Säure bevorzugt Methansulfonsäure ist.

**5.** Verbindung der Formel (I) nach Anspruch 1, das Stereoisomer davon, das Tautomer davon, das pharmazeutisch verträgliche Salz davon oder das Solvat von einem beliebigen des Vorhergehenden, wobei

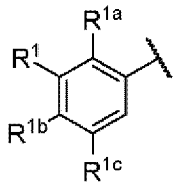 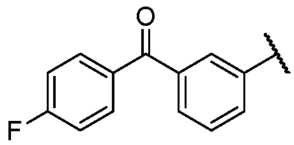 ,

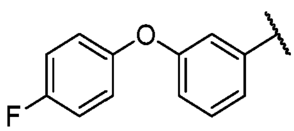 , 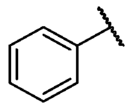 , 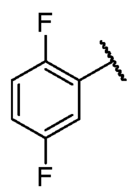 , 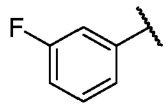 , 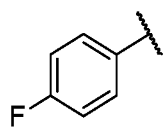 ,

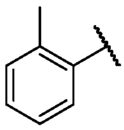 , 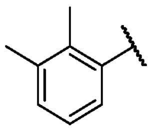 , 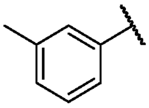 , 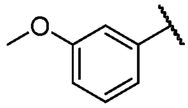 , 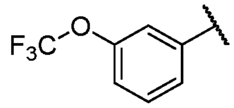 ,

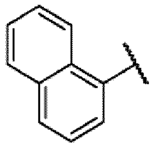, 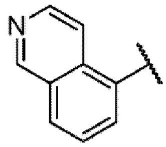, 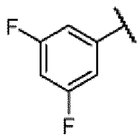 oder 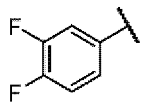

ist;

oder $R^2$ Isopropyl oder Cyclopropyl ist;

oder L

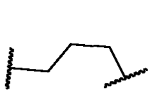 oder 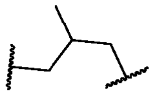

ist;

oder

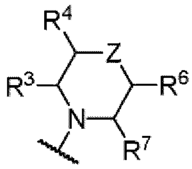 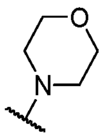, 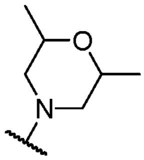, 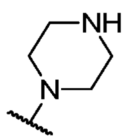, 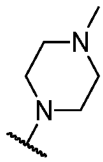,

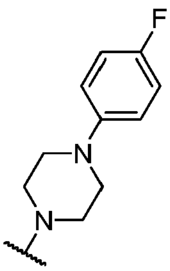, 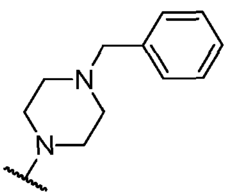, 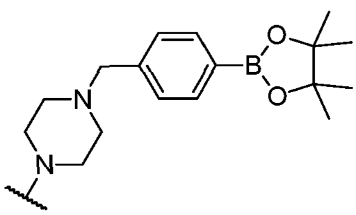, 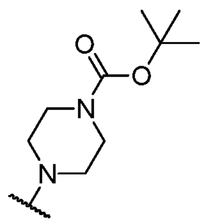,

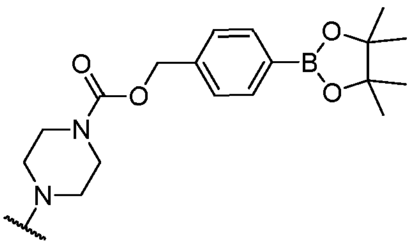, 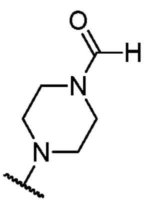, 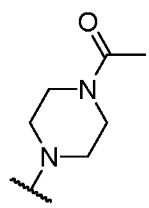, 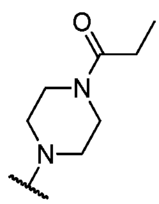,

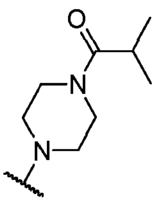, 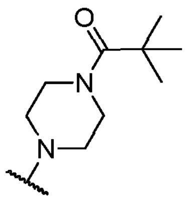, 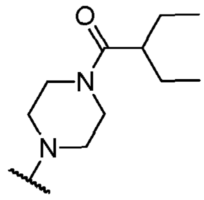, 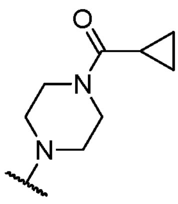, 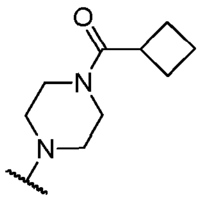,

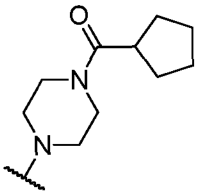 , 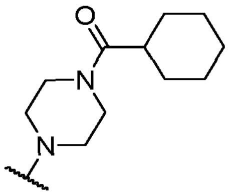 , 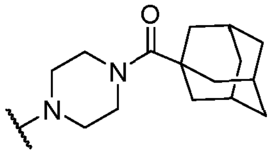 , 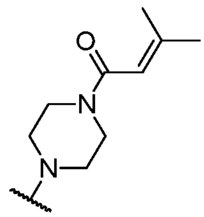 ,

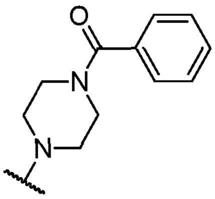 , 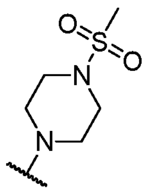 , 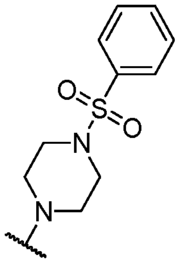 , 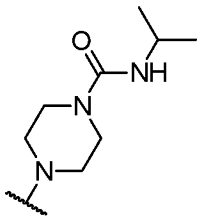 , 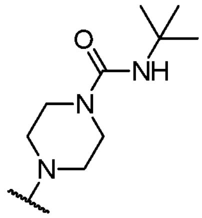 ,

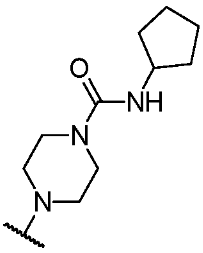 , 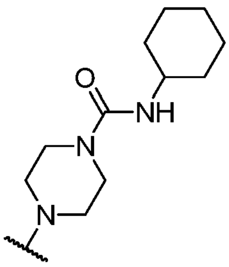 , 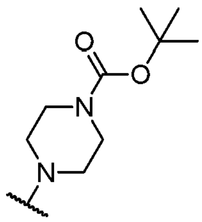 , 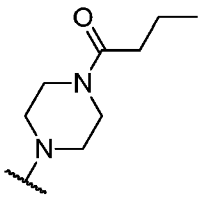 , 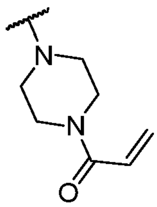 ,

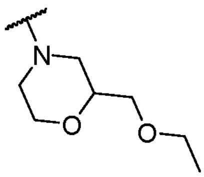 , 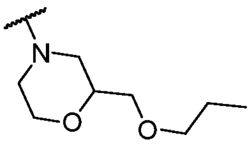 , 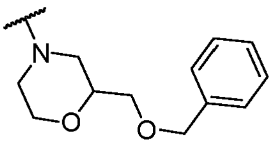 , 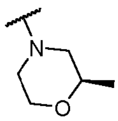

oder

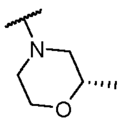

ist;
oder das pharmazeutisch verträgliche Salz der Verbindung der Formel (I) ein Salz zubereitet aus der Verbindung der Formel (I) und einer pharmazeutisch verträglichen Säure ist, die pharmazeutisch verträgliche Säure Salzsäure oder Methansulfonsäure ist;
oder das pharmazeutisch verträgliche Salz der Verbindung der Formel (I) ein Salz ist, das durch die Verbindung der Formel (I) und die pharmazeutisch verträgliche Säure in einem Molverhältnis von 1:2 gebildet ist.

**6.** Verbindung der Formel (I) nach Anspruch 1, das Stereoisomer davon, das Tautomer davon, das pharmazeutisch verträgliche Salz davon oder das Solvat von einem beliebigen des Vorhergehenden, wobei die Verbindung der Formel (I) eine beliebige der folgenden Verbindungen ist:

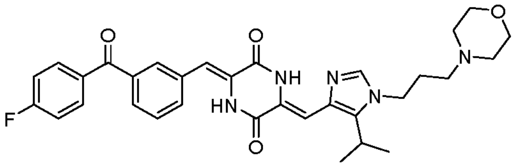,
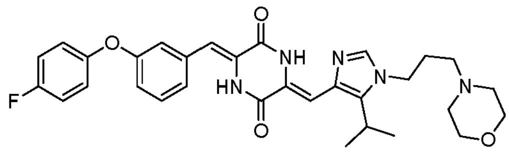,

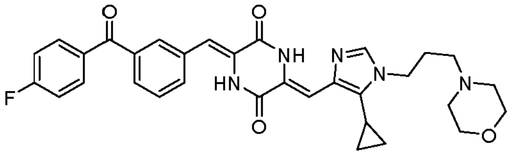,
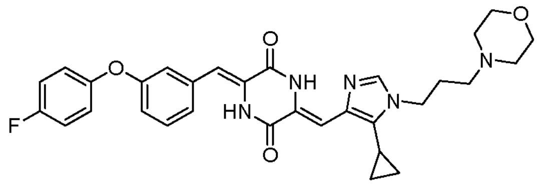,

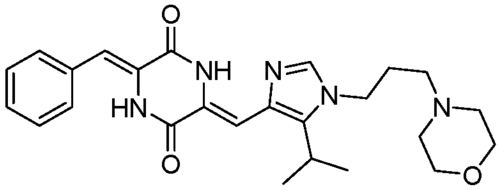,
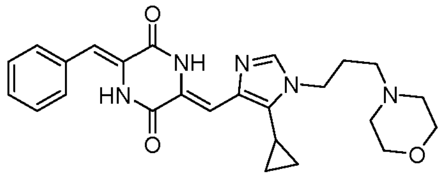,

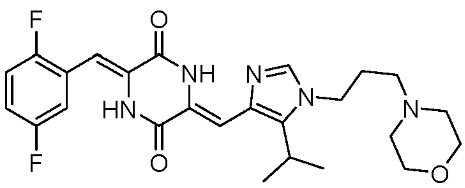,
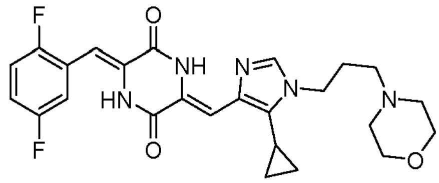,

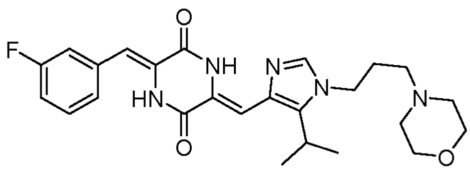,
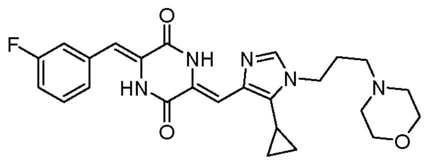,

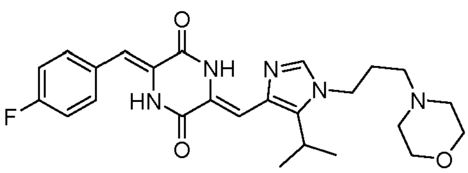,
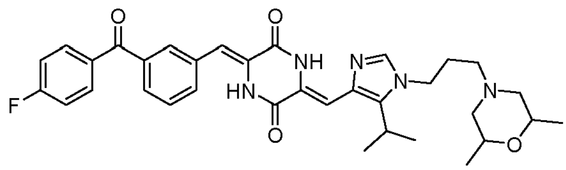,

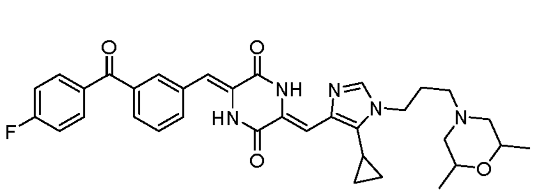,
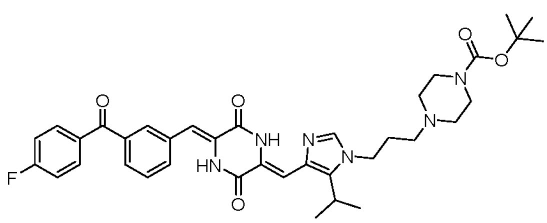,

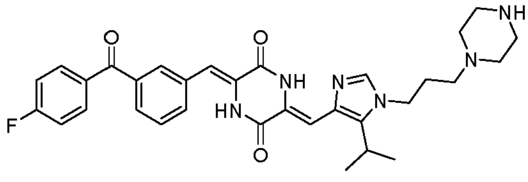,

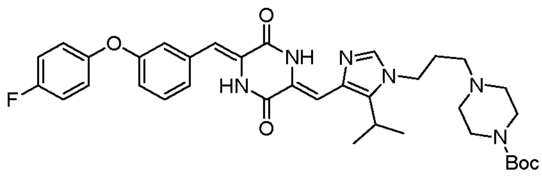,

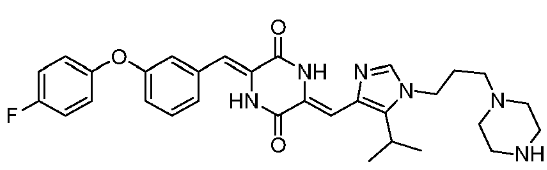,

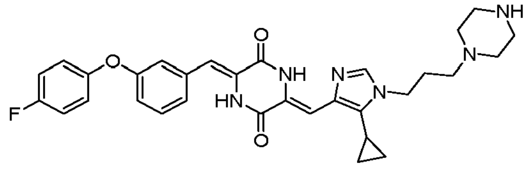,

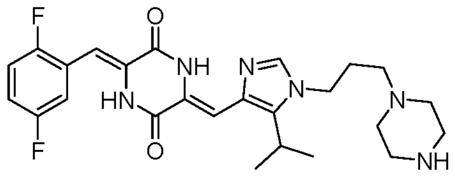,

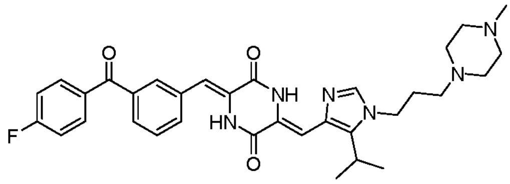,

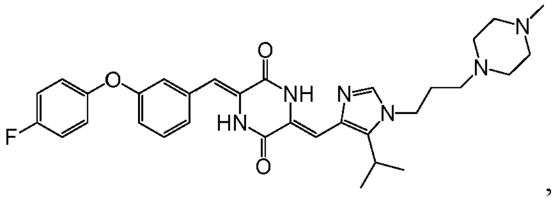,

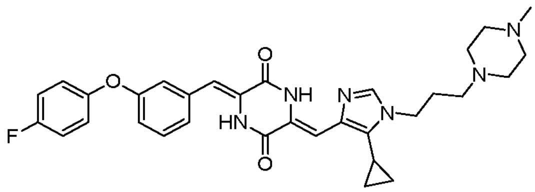,

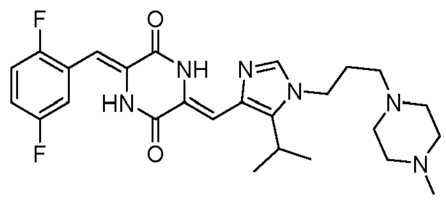,

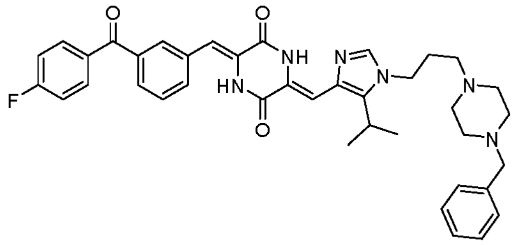,

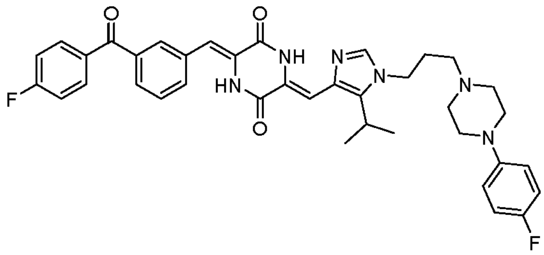,

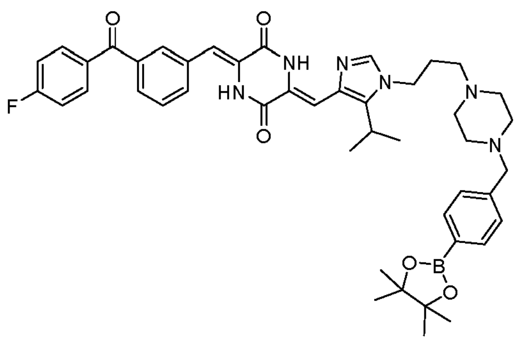,

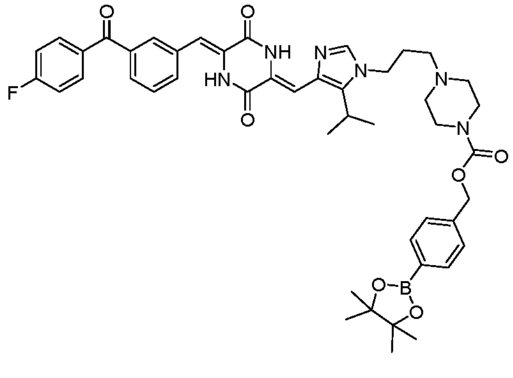,

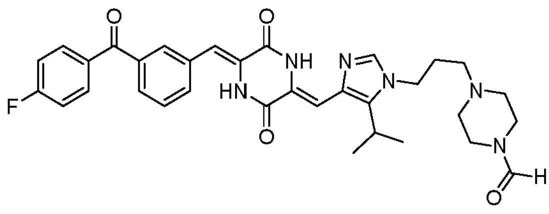,

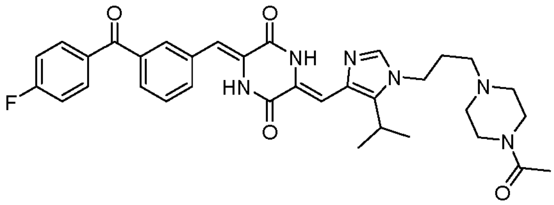,

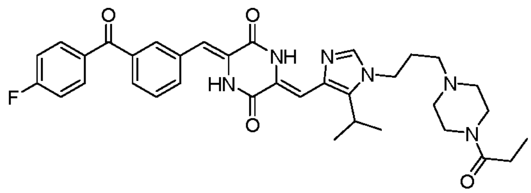,

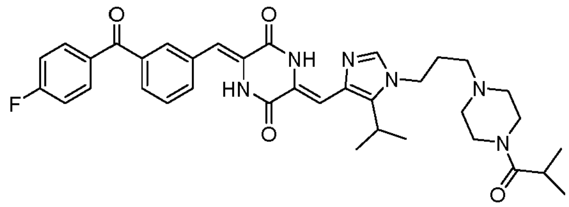,

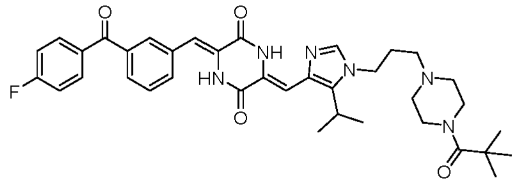,

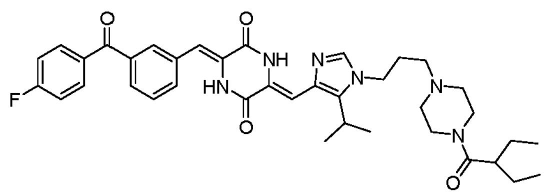,

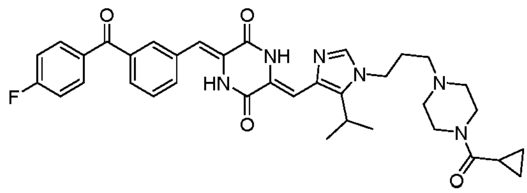,

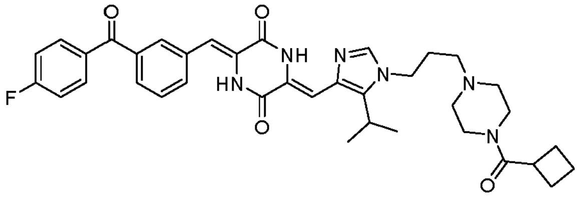,

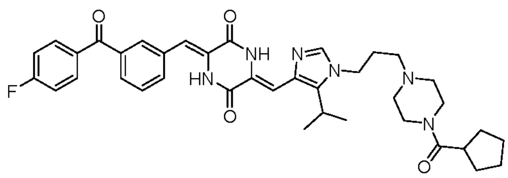,

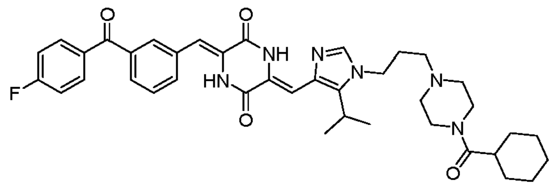,

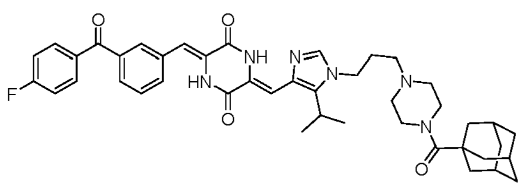,

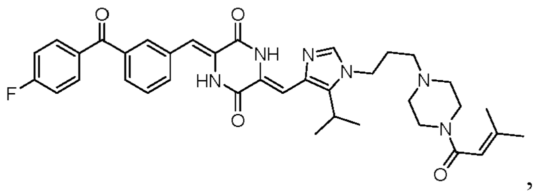,
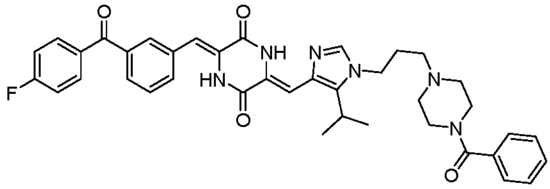,
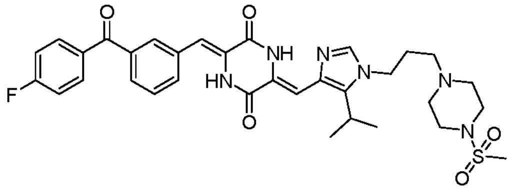,
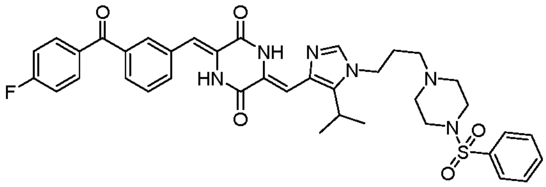,
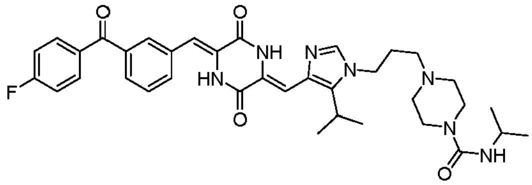,
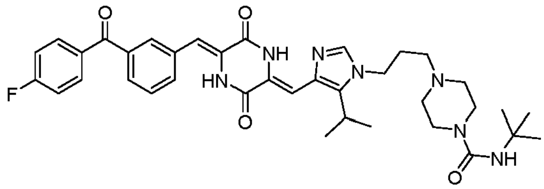,
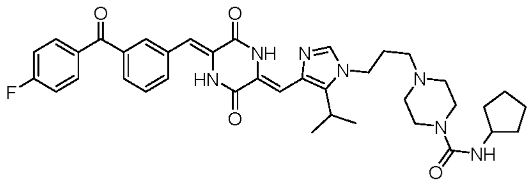,
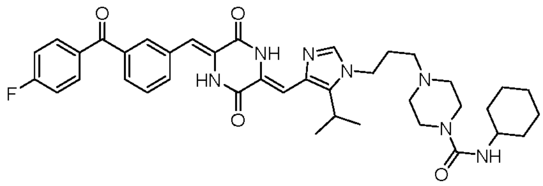,
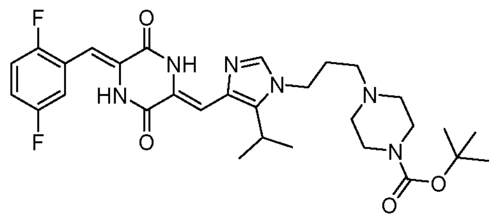,
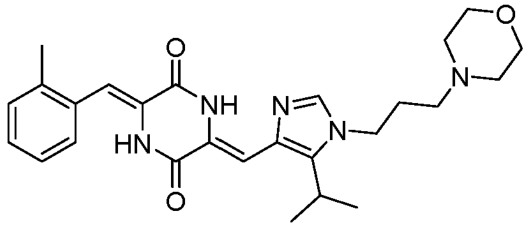,
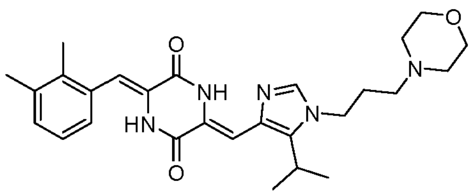,
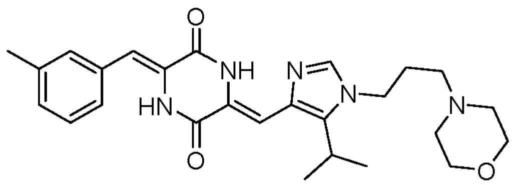,
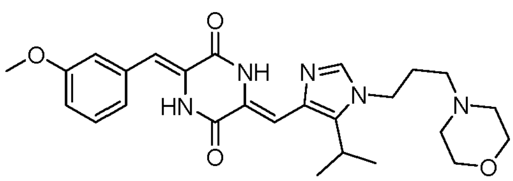,
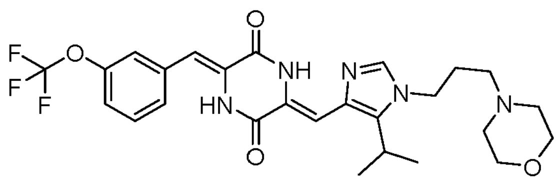,

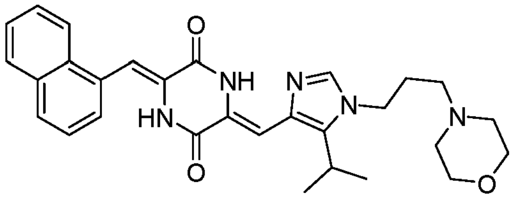,

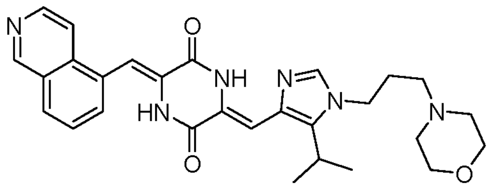,

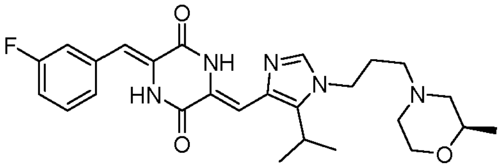,

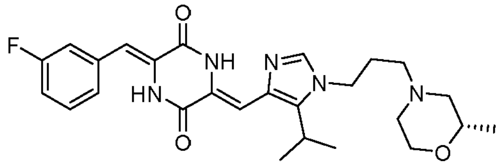,

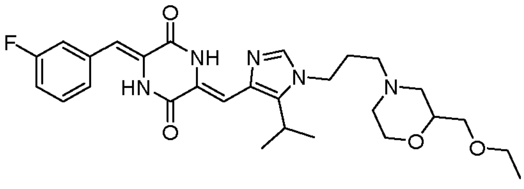,

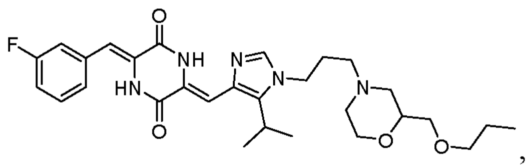,

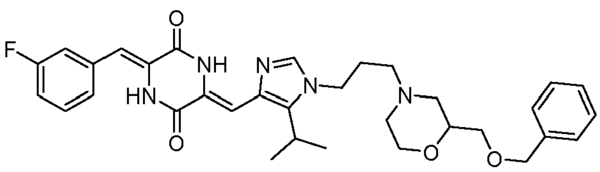,

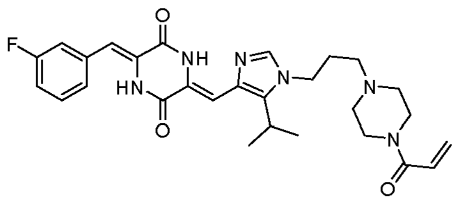,

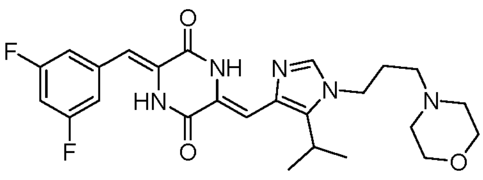,

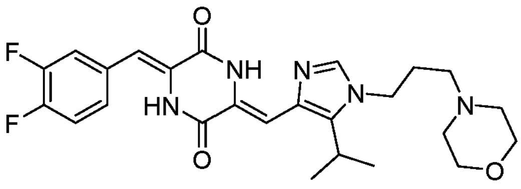,

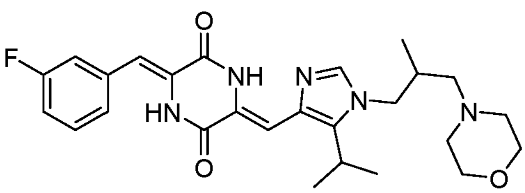,

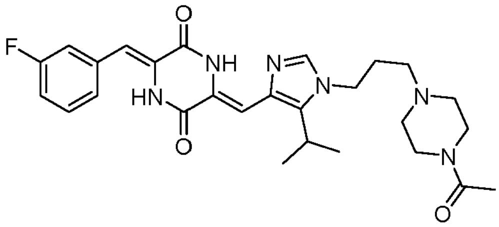,

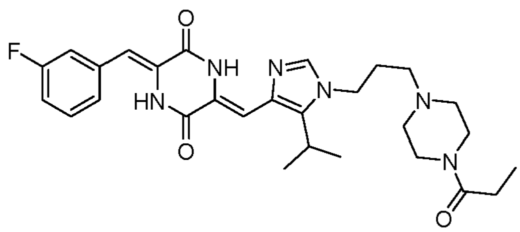,

oder

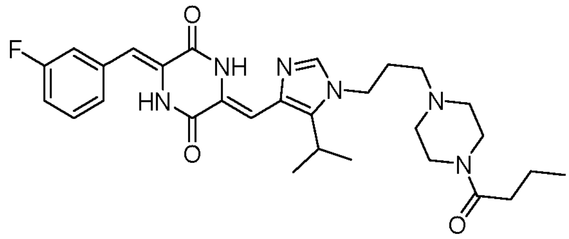

das pharmazeutisch verträgliche Salz der Verbindung der Formel (I) eine beliebige der folgenden Verbindungen ist:

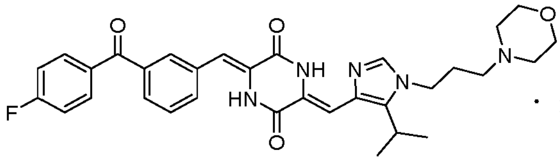 · 2HCl

,

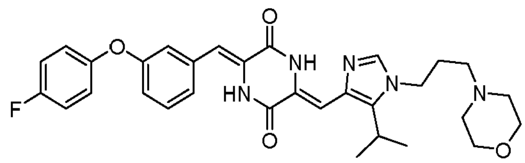 · 2HCl

,

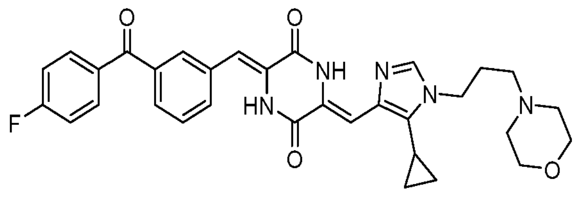 · 2HCl

,

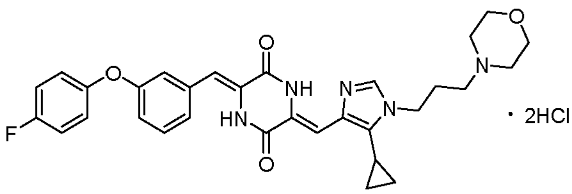 · 2HCl

,

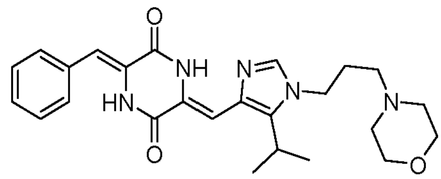 · 2HCl

,

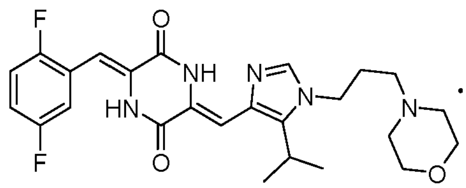 · 2HCl

,

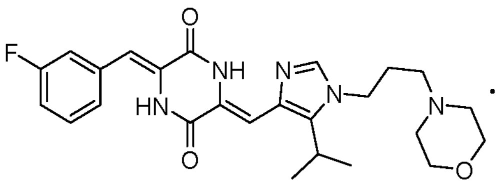 · 2HCl

,

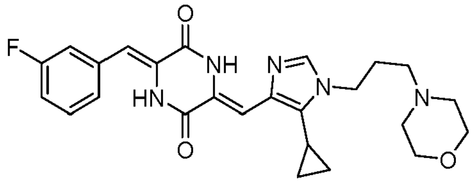 • 2HCl ,

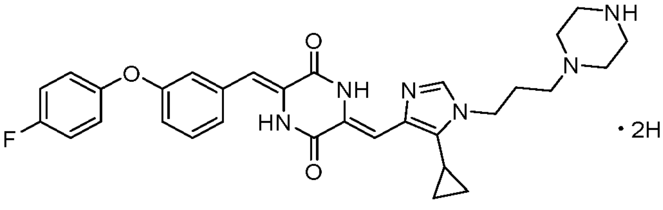 • 2HCl ,

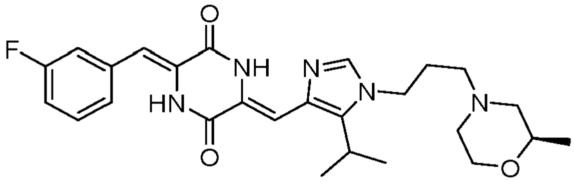 • 2HCl ,

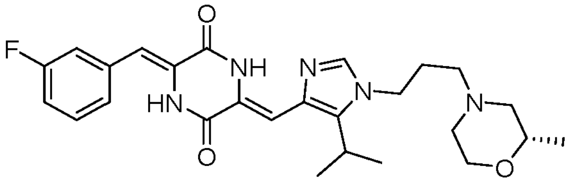 • 2HCl ,

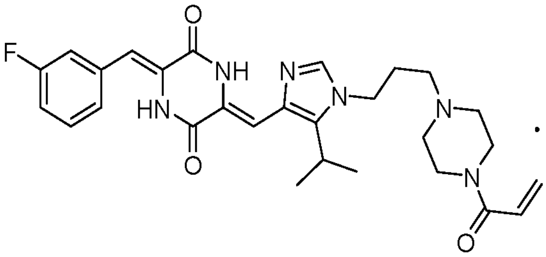 • 2HCl ,

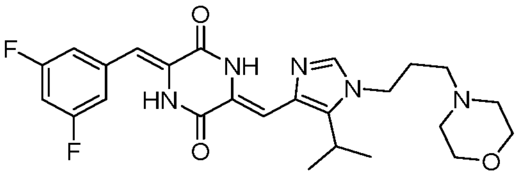 • 2HCl ,

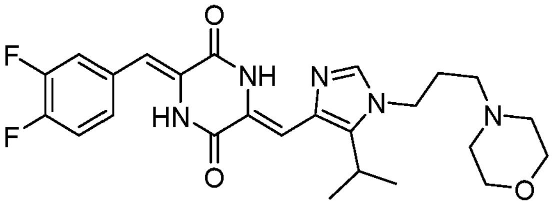 • 2HCl ,

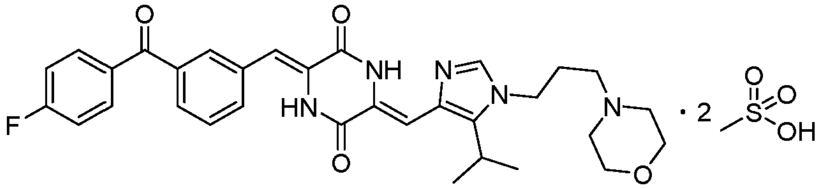

,

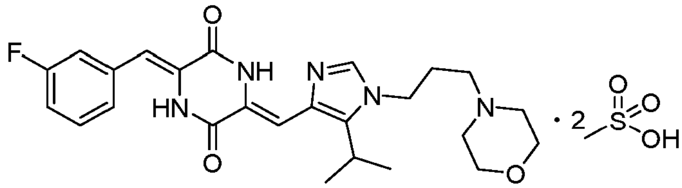

,

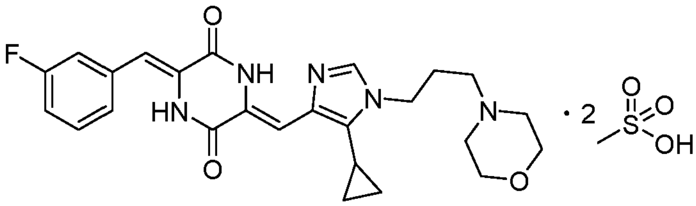

,

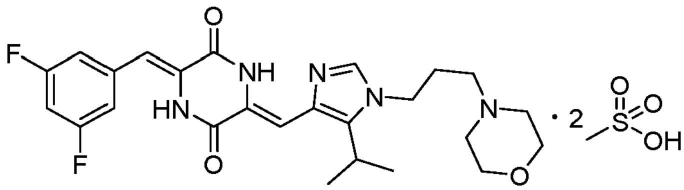

, oder

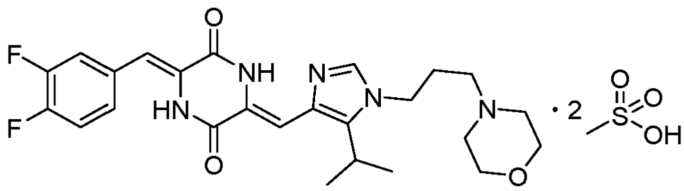

.

**7.** Verbindung der Formel (I) nach Anspruch 1, das Stereoisomer davon, das Tautomer davon, das pharmazeutisch verträgliche Salz davon oder das Solvat von einem beliebigen des Vorhergehenden, wobei die Verbindung der Formel (I) eine beliebige der folgenden Verbindungen ist:

eine Verbindung mit einer Retentionszeit von 6,29 min unter den folgenden Bedingungen,
die ein Stereoisomer von

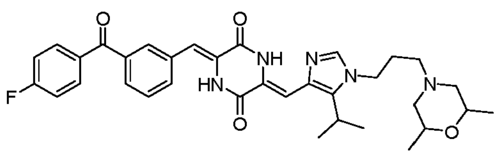

ist: chromatographische Säule: Acclaim 120, C18, 5 μm, 4,6 * 150 mm, mobile Phase: mobile Phase A: MeOH, mobile Phase B: 0,1 % wässrige Ameisensäurelösung; Flussrate: 1 ml/min, die mobile Phase wird gemäß dem folgenden Gradientenelutionsvorgang gewaschen:

| Zeit (min) | Mobile Phase A (%, V/V) | Mobile Phase B (%, V/V) |
|---|---|---|
| 0 | 40 | 60 |
| 0→6 | 40→100 | 60→0 |
| 6→10 | 100→40 | 0→60 |

eine Verbindung mit einer Retentionszeit von 6,48 min unter den folgenden Bedingungen, die ein Stereoisomer von

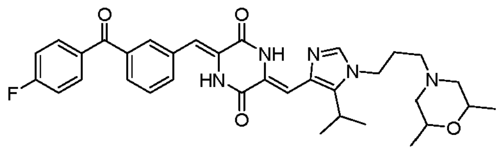

ist: chromatographische Säule: Acclaim 120, C18, 5 μm, 4,6 * 150 mm, mobile Phase: mobile Phase A: MeOH, mobile Phase B: 0,1 % wässrige Ameisensäurelösung; Flussrate: 1 ml/min, die mobile Phase wird gemäß dem folgenden Gradientenelutionsvorgang gewaschen:

| Zeit (min) | Mobile Phase A (%, V/V) | Mobile Phase B (%, V/V) |
|---|---|---|
| 0 | 40 | 60 |
| 0→6 | 40→100 | 60→0 |
| 6→10 | 100→40 | 0→60 |

**8.** Zubereitungsverfahren für eine Verbindung der Formel (I), das die folgenden Schritte umfasst: Durchführen einer Kondensationsreaktion wie unten gezeigt zwischen einer Verbindung der Formel (II) und einer Verbindung der Formel (III), um die Verbindung der Formel (I) zu erhalten;

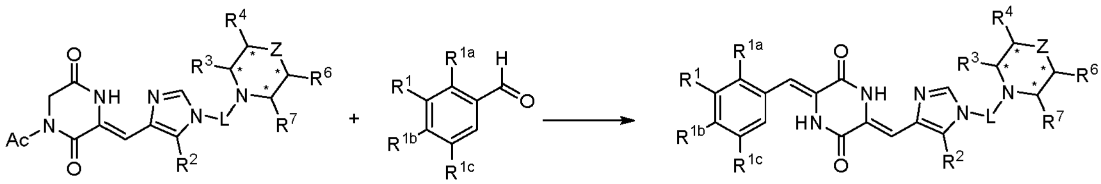

(II) (III) (I)

wobei *, L, Z, $R^1$, $R^{1a}$, $R^{1b}$, $R^{1c}$, $R^2$, $R^3$, $R^4$, $R^6$ und $R^7$ wie in zumindest einem der Ansprüche 1 bis 7 definiert sind.

**9.** Verbindung der Formel (II):

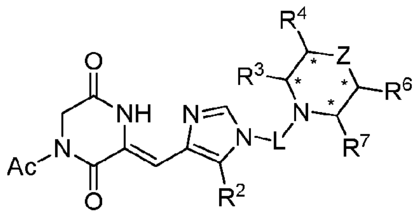

(II)

wobei *, $R^2$, $R^3$, $R^4$, $R^6$, $R^7$, L und Z wie in zumindest einem der Ansprüche 1 bis 7 definiert sind.

**10.** Verbindung der Formel (II) nach Anspruch 9, wobei die Verbindung der Formel (II) eine beliebige der folgenden Verbindungen ist:

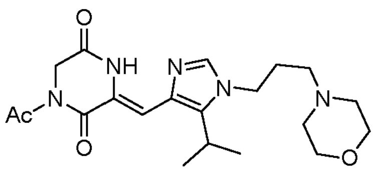,

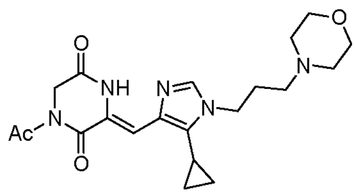,

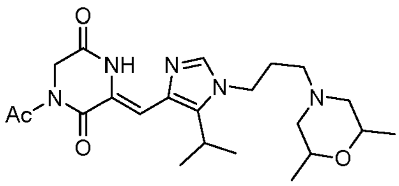,

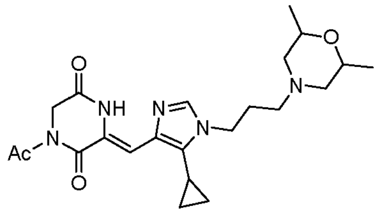,

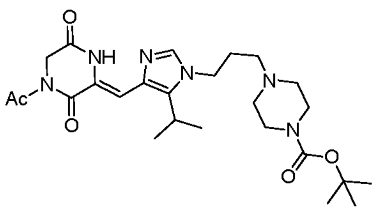,

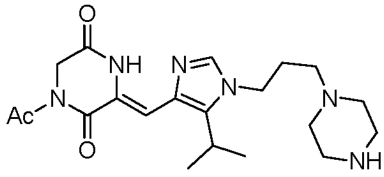,

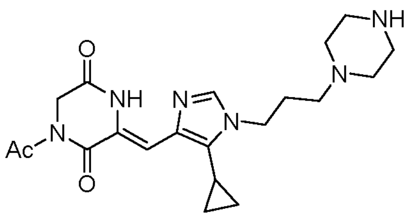,

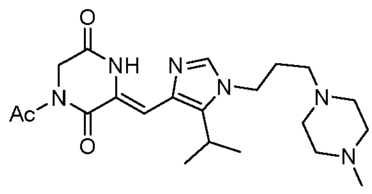,

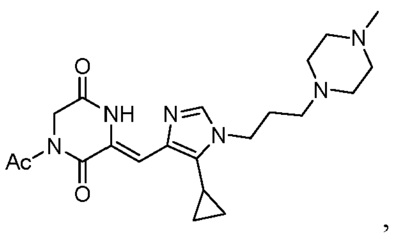,

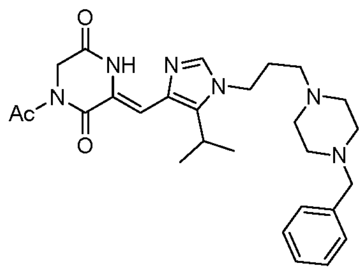,

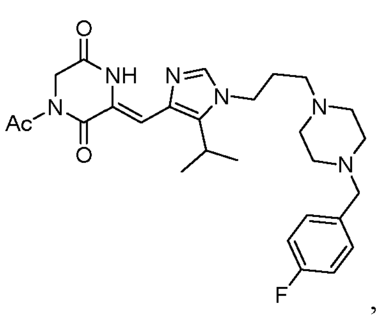,

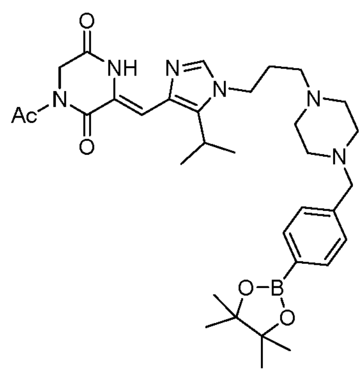,

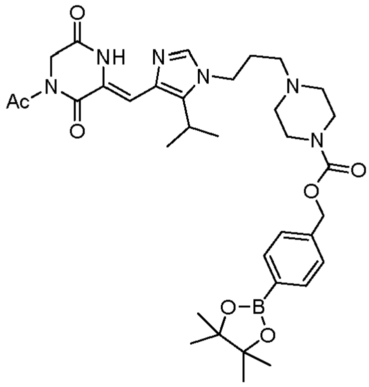
,

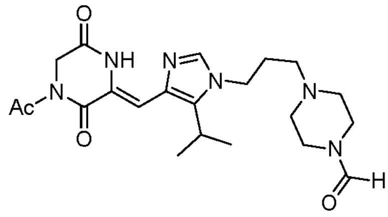
,

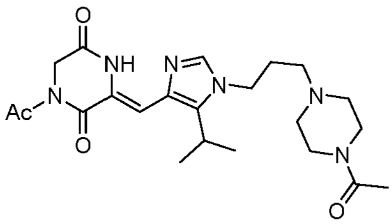
,

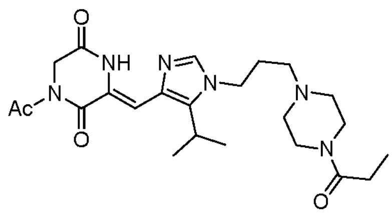
,

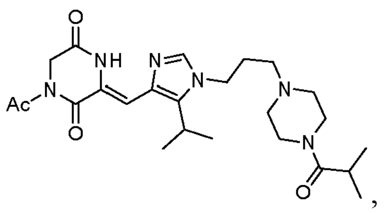
,

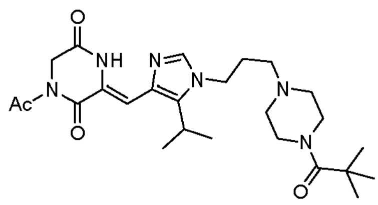
,

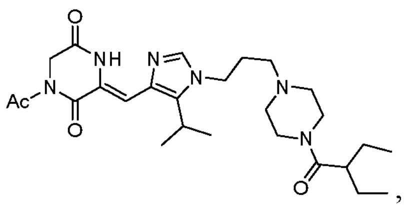
,

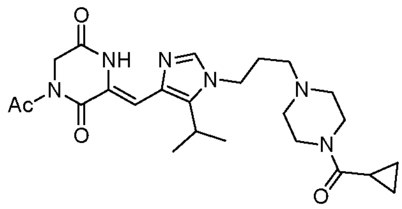
,

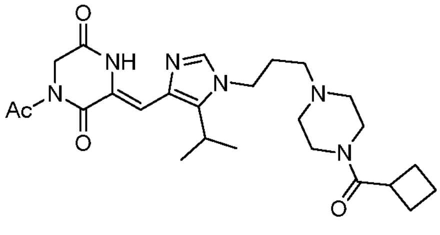
,

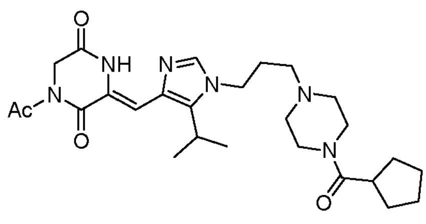
,

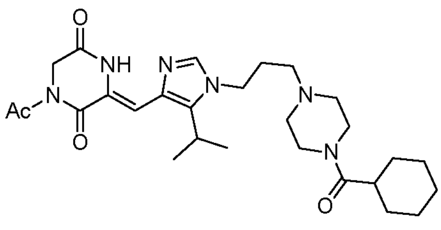
,

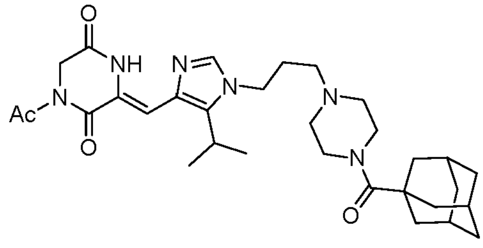
,

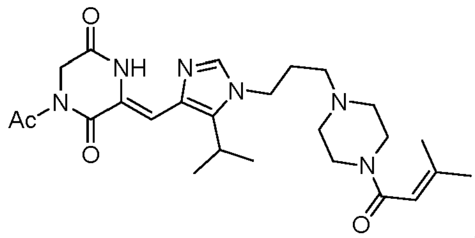,

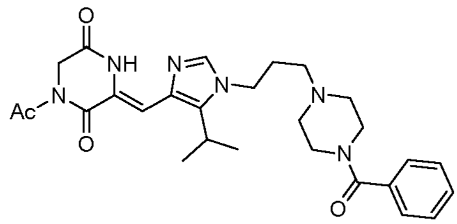,

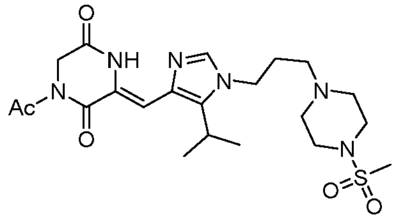,

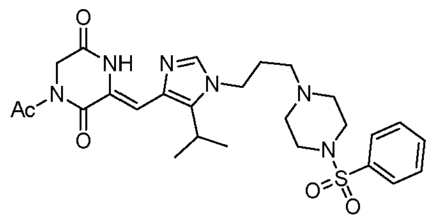,

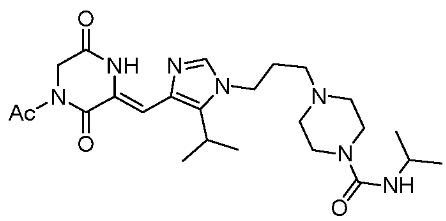,

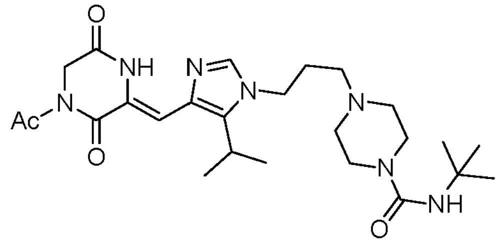,

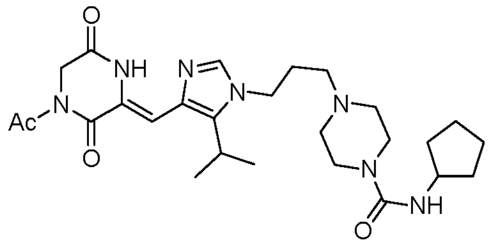,

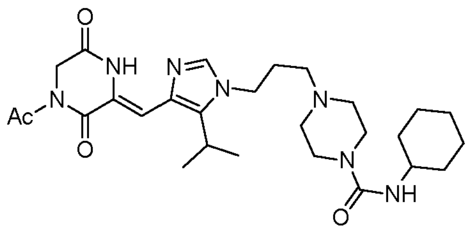,

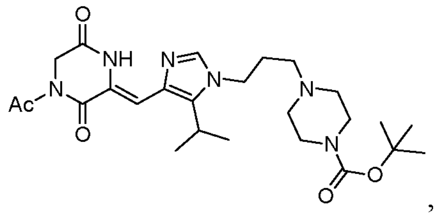,

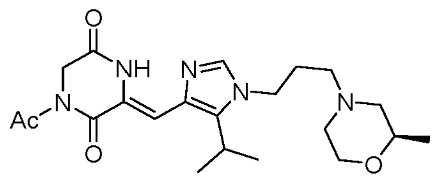,

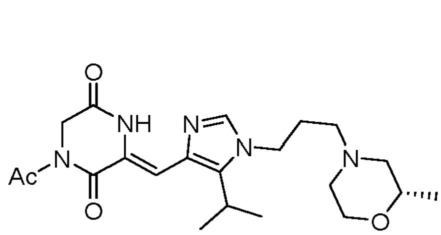,

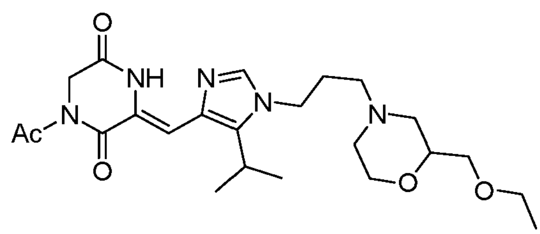,

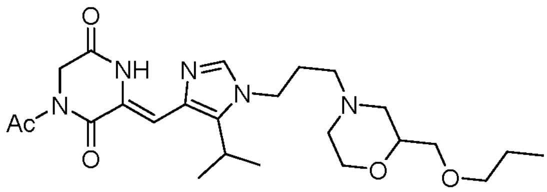

,

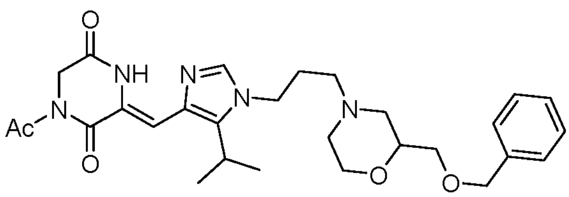

,

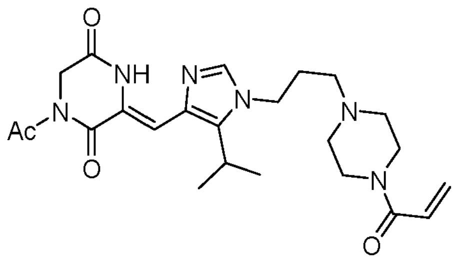

,

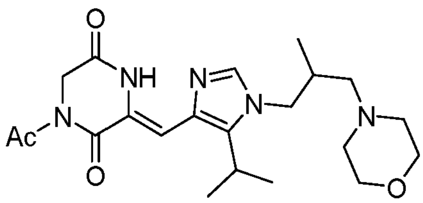

oder

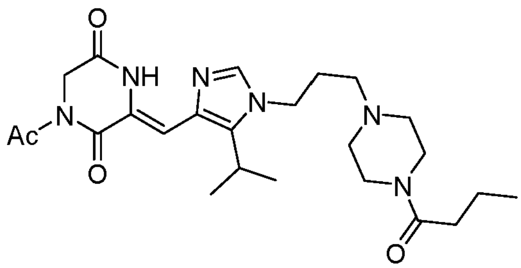

.

**11.** Pharmazeutische Zusammensetzung, umfassend die Verbindung der Formel (I) nach zumindest einem der Ansprüche 1 bis 7, das Stereoisomer davon, das Tautomer davon, das pharmazeutisch verträgliche Salz davon oder das Solvat von einem beliebigen des Vorhergehenden und einen pharmazeutischen Hilfsstoff; bevorzugt umfasst der pharmazeutische Hilfsstoff kein Co-Lösungsmittel.

**12.** Verbindung der Formel (I) nach zumindest einem der Ansprüche 1 bis 7, das Stereoisomer davon, das Tautomer davon, das pharmazeutisch verträgliche Salz davon oder das Solvat von einem beliebigen des Vorhergehenden oder die pharmazeutische Zusammensetzung nach Anspruch 11 zur Verwendung beim Vorbeugen und/oder Behandeln von Krebs; wobei der Krebs bevorzugt einer oder mehr als einer von Lungenkrebs, Bauchspeicheldrüsenkrebs, Darmkrebs und Leberkrebs ist.

## Revendications

**1.** Composé de formule (I), stéréoisomère de celui-ci, tautomère de celui-ci, sel acceptable pharmaceutiquement de celui-ci ou solvate de l'un quelconque de ce qui précède :

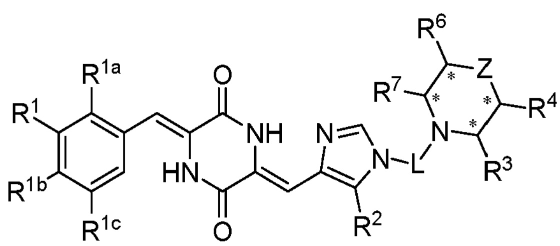

(I)

un atome de carbone avec « * » est un atome de carbone ou un atome de carbone chiral, et lorsque l'atome de

carbone avec « * » est l'atome de carbone chiral, il se trouve dans une configuration S et/ou une configuration R ;
$R^1$ est un hydrogène, un deutérium, un halogène, un alkyle en $C_1$-$C_8$, un alkyle en $C_1$-$C_8$ substitué par un ou plusieurs halogènes, un alcoxy en $C_1$-$C_8$, un alcoxy en $C_1$-$C_8$ substitué par un ou plusieurs halogènes, un benzoyle, un benzoyle substitué par un ou plusieurs halogènes, un phénoxy ou un phénoxy substitué par un ou plusieurs halogènes ;
$R^{1a}$, $R^{1b}$ et $R^{1c}$ sont indépendamment un hydrogène, un deutérium, un halogène ou un alkyle en $C_1$-$C_8$ ;
ou, $R^1$, $R^{1a}$ conjointement avec les atomes de carbone auxquels ils sont liés, forment un aryle en $C_6$-$C_{10}$ ou un hétéroaryle à 3 à 10 chaînons avec 1 à 5 hétéroatomes choisis parmi un ou plusieurs parmi N, O et S ;
ou, $R^1$, $R^{1b}$ conjointement avec les atomes de carbone auxquels ils sont liés, forment un aryle en $C_6$-$C_{10}$ ou un hétéroaryle à 3 à 10 chaînons avec 1 à 5 hétéroatomes choisis parmi un ou plusieurs parmi N, O et S ;
ou, $R^{1b}$, $R^{1c}$ conjointement avec les atomes de carbone auxquels ils sont liés forment un aryle en $C_6$-$C_{10}$ ou un hétéroaryle à 3 à 10 chaînons avec 1 à 5 hétéroatomes choisis parmi un ou plusieurs parmi N, O et S ;
$R^2$ est un alkyle en $C_1$-$C_8$ ou un cycloalkyle en $C_3$-$C_{10}$ ;
L est un hétéroalkylène en $C_1$-$C_8$ avec 1 à 4 hétéroatomes choisis parmi un ou plusieurs parmi N, O, S et Se ou un alkylène en $C_1$-$C_8$ ;
$R^3$, $R^4$, $R^6$ et $R^7$ sont indépendamment un hydrogène, un deutérium, un alkyle en $C_1$-$C_8$, un alkyle en $C_1$-$C_8$ substitué par un ou plusieurs $R^{3-1}$, un alcoxy en $C_1$-$C_8$, un cycloalkyle en $C_3$-$C_{10}$, un aryle en $C_6$-$C_{10}$, ou un hétérocycloalkyle à 3 à 10 chaînons avec 1 à 5 hétéroatomes choisis parmi un ou plusieurs parmi N, O et S ;
$R^{3-1}$ est indépendamment O-$R^{3-1-1}$, $R^{3-1-1}$ est un alkyle en $C_1$-$C_8$ ou un alkyle en $C_1$-$C_8$ substitué par un ou plusieurs $R^{3-1-2}$ ;
$R^{3-1-2}$ est indépendamment un aryle en $C_6$-$C_{10}$ ;
Z est O ou N($R^5$) ;
$R^5$ est un hydrogène, un deutérium, un alkyle en $C_1$-$C_8$, un aryle en $C_6$-$C_{10}$ substitué par un ou plusieurs $R^{5-1}$, un benzyle, un benzyle substitué par un ou plusieurs $R^{5-2}$, -C(=O)-$R^{5-3}$, ou - $S(=O)_2$-$R^{5-4}$ ;
$R^{5-1}$ est indépendamment un halogène ;
$R^{5-2}$ est indépendamment

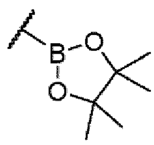

;

$R^{5-3}$ est un hydrogène, un alcoxy en $C_1$-$C_8$, un benzyloxy, un benzyloxy substitué par un ou plusieurs $R^{5-2}$, un alkyle en $C_1$-$C_8$, un cycloalkyle en $C_3$-$C_{10}$, un alcényle en $C_2$-$C_8$, un aryle en $C_6$-$C_{10}$ ou $NR^{5-3-1}R^{5-3-2}$ ; $R^{5-3-1}$ et $R^{5-3-2}$ sont indépendamment un hydrogène, un alkyle en $C_1$-$C_8$ ou un cycloalkyle en $C_3$-$C_{10}$ ;
$R^{5-4}$ est un alkyle en $C_1$-$C_8$ ou un aryle en $C_6$-$C_{10}$.

**2.** Composé de formule (I) selon la revendication 1, stéréoisomère de celui-ci, tautomère de celui-ci, sel acceptable pharmaceutiquement de celui-ci ou solvate de l'un quelconque de ce qui précède, dans lequel lorsque $R^1$ est un halogène, l'halogène est le fluor, le chlore, le brome ou l'iode ;

ou, lorsque $R^1$ est un alkyle en $C_1$-$C_8$, l'alkyle en $C_1$-$C_8$ est un alkyle en $C_1$-$C_4$ ;
ou, lorsque $R^1$ est un alkyle en $C_1$-$C_8$ substitué par un ou plusieurs halogènes, l'alkyle en $C_1$-$C_8$ substitué par un ou plusieurs halogènes est un alkyle en $C_1$-$C_4$ substitué par un ou plusieurs halogènes ;
ou, lorsque $R^1$ est un alcoxy en $C_1$-$C_8$, l'alcoxy en $C_1$-$C_8$ est un alcoxy en $C_1$-$C_4$ ;
ou, lorsque $R^1$ est un alcoxy en $C_1$-$C_8$ substitué par un ou plusieurs halogènes, l'alcoxy en $C_1$-$C_8$ substitué par un ou plusieurs halogènes est un alcoxy en $C_1$-$C_4$ substitué par un ou plusieurs halogènes ;
ou, lorsque $R^1$ est un benzoyle substitué par un ou plusieurs halogènes, l'halogène est le fluor, le chlore, le brome ou l'iode ;
ou, lorsque $R^1$ est un phénoxy substitué par un ou plusieurs halogènes, l'halogène est le fluor, le chlore, le brome ou l'iode ;
ou, lorsque $R^{1a}$, $R^{1b}$ et $R^{1c}$ sont indépendamment un halogène, l'halogène est le fluor, le chlore, le brome ou l'iode ;
ou, lorsque $R^{1a}$, $R^{1b}$ et $R^{1c}$ sont indépendamment un alkyle en $C_1$-$C_8$, l'alkyle en $C_1$-$C_8$ est un alkyle en $C_1$-$C_4$ ;
ou, lorsque $R^1$, $R^{1a}$ conjointement avec les atomes de carbone auxquels ils sont liés forment l'aryle en $C_6$-$C_{10}$, l'aryle en $C_6$-$C_{10}$ est un phényle ;
ou, lorsque $R^1$, $R^{1a}$ conjointement avec les atomes de carbone auxquels ils sont liés forment l'hétéroaryle à 3 à 10

chaînons avec 1 à 5 hétéroatomes choisis parmi un ou plusieurs parmi N, O et S, l'hétéroaryle à 3 à 10 chaînons avec 1 à 5 hétéroatomes choisis parmi un ou plusieurs parmi N, O et S est un hétéroaryle à 4 à 8 chaînons avec 1 à 2 hétéroatomes choisis parmi un ou plusieurs parmi N, O et S ;
ou, lorsque $R^2$ est un alkyle en $C_1$-$C_8$, l'alkyle en $C_1$-$C_8$ est un alkyle en $C_1$-$C_4$ ;
ou, lorsque $R^2$ est un cycloalkyle en $C_3$-$C_{10}$, le cycloalkyle en $C_3$-$C_{10}$ est un cycloalkyle en $C_3$-$C_6$ ;
ou, lorsque L est un alkylène en $C_1$-$C_8$, l'alkylène en $C_1$-$C_8$ est un alkylène en $C_3$-$C_8$ ;
ou, lorsque $R^3$, $R^4$, $R^6$ et $R^7$ sont indépendamment un alkyle en $C_1$-$C_8$, l'alkyle en $C_1$-$C_8$ est un alkyle en $C_1$-$C_4$ ;
ou, lorsque $R^3$, $R^4$, $R^6$ et $R^7$ sont indépendamment un alkyle en $C_1$-$C_8$ substitué par un ou plusieurs $R^{3-1}$, l'alkyle en $C_1$-$C_8$ est un alkyle en $C_1$-$C_4$ ;
ou, lorsque $R^{3-1-1}$ est un alkyle en $C_1$-$C_8$, l'alkyle en $C_1$-$C_8$ est un alkyle en $C_1$-$C_4$ ;
ou, lorsque $R^{3-1-1}$ est un alkyle en $C_1$-$C_8$ substitué par un ou plusieurs $R^{3-1-2}$, l'alkyle en $C_1$-$C_8$ est un alkyle en $C_1$-$C_4$ ;
ou, lorsque $R^{3-1-2}$ est indépendamment un aryle en $C_6$-$C_{10}$, l'aryle en $C_6$-$C_{10}$ est un phényle ;
ou, lorsque $R^5$ est un alkyle en $C_1$-$C_8$, l'alkyle en $C_1$-$C_8$ est un alkyle en $C_1$-$C_4$ ;
ou, lorsque $R^5$ est un aryle en $C_6$-$C_{10}$ substitué par un ou plusieurs $R^{5-1}$, l'aryle en $C_6$-$C_{10}$ est un phényle ou un naphtyle ;
ou, lorsque $R^{5-1}$ est un halogène, l'halogène est le fluor, le chlore, le brome ou l'iode ;
ou, lorsque $R^{5-3}$ est un alcoxy en $C_1$-$C_8$, l'alcoxy en $C_1$-$C_8$ est un alcoxy en $C_1$-$C_4$ ;
ou, lorsque $R^{5-3}$ est un alkyle en $C_1$-$C_8$, l'alkyle en $C_1$-$C_8$ est un méthyle, un éthyle, un n-propyle, un isopropyle, un tert-butyle ou $-CH(C_2H_5)CH_2CH_3$ ;
ou, lorsque $R^{5-3}$ est un cycloalkyle en $C_3$-$C_{10}$, le cycloalkyle en $C_3$-$C_{10}$ est un cyclopropyle, un cyclobutyle, un cyclopentyle, un cyclohexyle ou un adamantyle ;
ou, lorsque $R^{5-3}$ est un alcényle en $C_2$-$C_8$, l'alcényle en $C_2$-$C_8$ est un alcényle en $C_2$-$C_4$ ;
ou, lorsque $R^{5-3}$ est un aryle en $C_6$-$C_{10}$, l'aryle en $C_6$-$C_{10}$ est un phényle ou un naphtyle ;
ou, lorsque $R^{5-3-1}$ et $R^{5-3-2}$ sont indépendamment un alkyle en $C_1$-$C_8$, l'alkyle en $C_1$-$C_8$ est un alkyle en $C_1$-$C_4$ ;
ou, lorsque $R^{5-3-1}$ et $R^{5-3-2}$ sont indépendamment un cycloalkyle en $C_3$-$C_{10}$, le cycloalkyle en $C_3$-$C_{10}$ est un cycloalkyle en $C_3$-$C_6$ ;
ou, lorsque $R^{5-4}$ est un alkyle en $C_1$-$C_8$, l'alkyle en $C_1$-$C_8$ est un alkyle en $C_1$-$C_4$ ;
ou, lorsque $R^{5-4}$ est un aryle en $C_6$-$C_{10}$, l'aryle en $C_6$-$C_{10}$ est un phényle ou un naphtyle.

**3.** Composé de formule (I) selon la revendication 1, stéréoisomère de celui-ci, tautomère de celui-ci, sel acceptable pharmaceutiquement de celui-ci ou solvate de l'un quelconque de ce qui précède, dans lequel lorsque $R^1$ est un halogène, l'halogène est le fluor ;

ou, lorsque $R^1$ est un alkyle en $C_1$-$C_8$, l'alkyle en $C_1$-$C_8$ est un méthyle ;
ou, lorsque $R^1$ est un alkyle en $C_1$-$C_8$ substitué par un ou plusieurs halogènes, l'alkyle en $C_1$-$C_8$ substitué par un ou plusieurs halogènes est un trifluorométhyle ;
ou, lorsque $R^1$ est un alcoxy en $C_1$-$C_8$, l'alcoxy en $C_1$-$C_8$ est un méthoxy ;
ou, lorsque $R^1$ est un alcoxy en $C_1$-$C_8$ substitué par un ou plusieurs halogènes, l'alcoxy en $C_1$-$C_8$ substitué par un ou plusieurs halogènes est un trifluorométhoxy ;
ou, lorsque $R^1$ est un benzoyle substitué par un ou plusieurs halogènes, l'halogène est le fluor ; de préférence, le benzoyle substitué par un ou plusieurs halogènes est

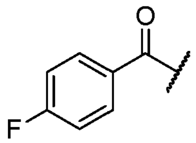
;

ou, lorsque $R^1$ est un phénoxy substitué par un ou plusieurs halogènes, l'halogène est le fluor ; de préférence, le phénoxy substitué par un ou plusieurs halogènes est

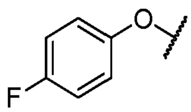
;

ou, lorsque $R^{1a}$, $R^{1b}$ et $R^{1c}$ sont indépendamment un halogène, l'halogène est le fluor ;

ou, lorsque $R^{1a}$, $R^{1b}$ et $R^{1c}$ sont indépendamment un alkyle en $C_1$-$C_8$, l'alkyle en $C_1$-$C_8$ est un méthyle ;
ou, lorsque $R^1$, $R^{1a}$ conjointement avec les atomes de carbone auxquels ils sont liés forment l'hétéroaryle à 3 à 10 chaînons avec 1 à 5 hétéroatomes choisis parmi un ou plusieurs parmi N, O et S, l'hétéroaryle à 3 à 10 chaînons avec 1 à 5 hétéroatomes choisis parmi un ou plusieurs parmi N, O et S est un pyridyle ; de préférence, l'hétéroaryle à 3 à 10 chaînons avec 1 à 5 hétéroatomes choisis parmi un ou plusieurs parmi N, O et S est

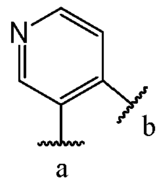 ,

avec une extrémité a liée à l'atome de carbone auquel $R^1$ est lié et une extrémité b liée à l'atome de carbone auquel $R^{1a}$ est lié ;
ou, lorsque $R^2$ est un alkyle en $C_1$-$C_8$, l'alkyle en $C_1$-$C_8$ est un méthyle, un éthyle, un isopropyle ou un tert-butyle ;
ou, lorsque $R^2$ est un cycloalkyle en $C_3$-$C_{10}$, le cycloalkyle en $C_3$-$C_{10}$ est un cyclopropyle ;
ou, lorsque L est un alkylène en $C_1$-$C_8$, l'alkylène en $C_1$-$C_8$ est

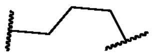 , 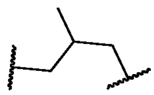 ,

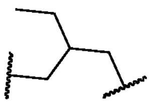 , 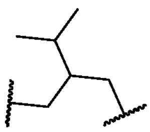 , 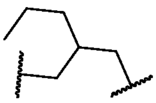 , ou 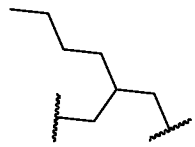 ,

par exemple

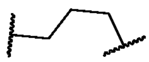 ;

ou, lorsque $R^3$, $R^4$, $R^6$ et $R^7$ sont indépendamment un alkyle en $C_1$-$C_8$, l'alkyle en $C_1$-$C_8$ est un méthyle ou un éthyle, par exemple un méthyle ;
ou, lorsque $R^3$, $R^4$, $R^6$ et $R^7$ sont indépendamment un alkyle en $C_1$-$C_8$ substitué par un ou plusieurs $R^{3-1}$, l'alkyle en $C_1$-$C_8$ est un méthyle ;
ou, lorsque $R^{3-1-1}$ est un alkyle en $C_1$-$C_8$, l'alkyle en $C_1$-$C_8$ est un méthyle, un éthyle, un n-propyle ou un n-butyle ;
ou, lorsque $R^{3-1-1}$ est un alkyle en $C_1$-$C_8$ substitué par un ou plusieurs $R^{3-1-2}$, l'alkyle en $C_1$-$C_8$ est un méthyle ;
ou, lorsque $R^5$ est alkyle en $C_1$-$C_8$, l'alkyle en $C_1$-$C_8$ est un méthyle, un éthyle, un n-propyle, un isopropyle, un n-butyle ou 2-méthylpropyle, par exemple un méthyle ;
ou, lorsque $R^5$ est un aryle en $C_6$-$C_{10}$ substitué par un ou plusieurs $R^{5-1}$, l'aryle en $C_6$-$C_{10}$ est un phényle ;
ou, lorsque $R^{5-1}$ est un halogène, l'halogène est le fluor ;
ou, lorsque $R^{5-3}$ est un alcoxy en $C_1$-$C_8$, l'alcoxy en $C_1$-$C_8$ est un tert-butoxy ;
ou, lorsque $R^{5-3}$ est un alkyle en $C_1$-$C_8$, l'alkyle en $C_1$-$C_8$ est un alkyle en $C_1$-$C_4$ ;
ou, lorsque $R^{5-3}$ est un cycloalkyle en $C_3$-$C_{10}$, le cycloalkyle en $C_3$-$C_{10}$ est un cycloalkyle en $C_3$-$C_6$ ;
ou, lorsque $R^{5-3}$ est un alcényle en $C_2$-$C_8$, l'alcényle en $C_2$-$C_8$ est

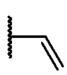 , 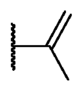 , 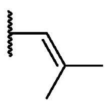 ,

ou

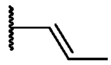,

par exemple

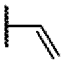 ou 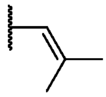 ;

ou, lorsque $R^{5-3}$ est un aryle en $C_6$-$C_{10}$, l'aryle en $C_6$-$C_{10}$ est un phényle ;
ou, lorsque $R^{5-3-1}$ et $R^{5-3-2}$ sont indépendamment un alkyle en $C_1$-$C_8$, l'alkyle en $C_1$-$C_8$ est un isopropyle ou un tert-butyle ;
ou, lorsque $R^{5-3-1}$ et $R^{5-3-2}$ sont indépendamment un cycloalkyle en $C_3$-$C_{10}$, le cycloalkyle en $C_3$-$C_{10}$ est un cyclopentyle ou un cyclohexyle ;
ou, lorsque $R^{5-4}$ est un alkyle en $C_1$-$C_8$, l'alkyle en $C_1$-$C_8$ est un méthyle ;
ou, lorsque $R^{5-4}$ est un aryle en $C_6$-$C_{10}$, l'aryle en $C_6$-$C_{10}$ est un phényle.

**4.** Composé de formule (I) selon la revendication 1, stéréoisomère de celui-ci, tautomère de celui-ci, sel acceptable pharmaceutiquement de celui-ci ou solvate de l'un quelconque de ce qui précède, dans lequel lorsque $R^1$ est un hydrogène, un halogène, un alkyle en $C_1$-$C_8$, un alcoxy en $C_1$-$C_8$, un alcoxy en $C_1$-$C_8$ substitué par un ou plusieurs halogènes, un benzoyle, un benzoyle substitué par un ou plusieurs halogènes, un phénoxy ou un phénoxy substitué par un ou plusieurs halogènes ;

ou, $R^{1b}$ est un hydrogène ou un halogène ; par exemple un hydrogène ou un fluor ;
ou, $R^{1c}$ est un hydrogène ou un halogène, par exemple un hydrogène ou un fluor ;
ou, $R^2$ est un alkyle en $C_1$-$C_8$ ;
ou, L est un alkylène en $C_1$-$C_8$ ;
ou, $R^3$, $R^4$, $R^6$ et $R^7$ sont indépendamment un hydrogène ou un alkyle en $C_1$-$C_8$ ;
ou, $R^5$ est un benzyle, -C(=O)-$R^{5-3}$ ou -S(=O)$_2$-$R^{5-4}$ ;
ou, $R^{5-3}$ est un hydrogène, un alkyle en $C_1$-$C_4$, un cycloalkyle en $C_3$-$C_6$, un alcényle en $C_2$-$C_8$, un benzyloxy substitué par un ou plusieurs groupes $R^{5-2}$, un aryle en $C_6$-$C_{10}$ ou $NR^{5-3-1}R^{5-3-2}$ ;
ou, le sel acceptable pharmaceutiquement du composé de formule (I) est un sel préparé à partir du composé de formule (I) et d'un acide acceptable pharmaceutiquement, l'acide acceptable pharmaceutiquement est un acide inorganique ou un acide organique, l'acide inorganique est de préférence l'acide chlorhydrique, et l'acide organique est de préférence l'acide méthanesulfonique.

**5.** Composé de formule (I) selon la revendication 1, stéréoisomère de celui-ci, tautomère de celui-ci, sel acceptable pharmaceutiquement de celui-ci ou solvate de l'un quelconque de ce qui précède, dans lequel

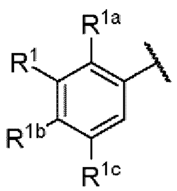 est 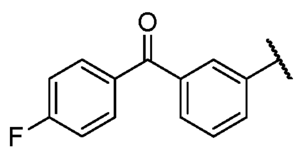,

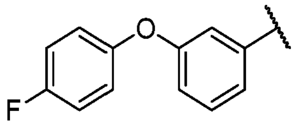, 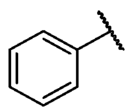, 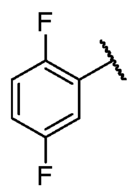, 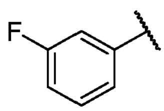, 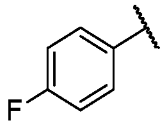,

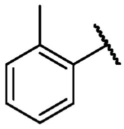 , 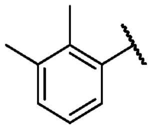 , 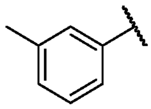 , 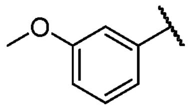 , 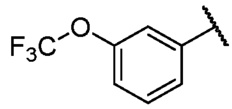 ,

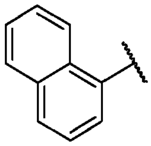 , 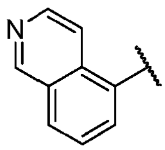 , 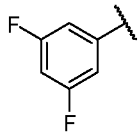 , ou 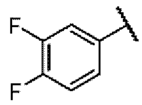 ;

ou, $R^2$ est un isopropyle ou un cyclopropyle ;
ou, L est

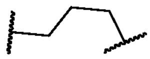 ou 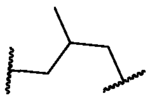 ;

ou,

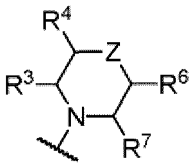 est 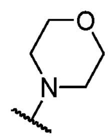 , 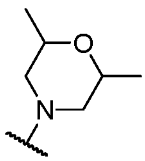 , 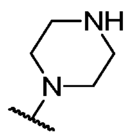 , 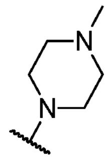 ,

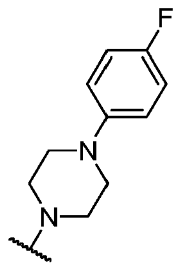 , 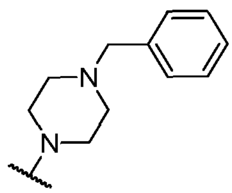 , 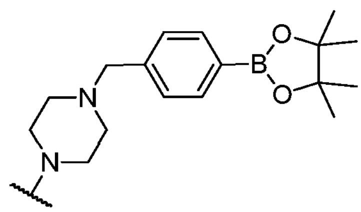 , 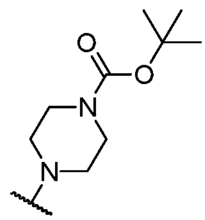 ,

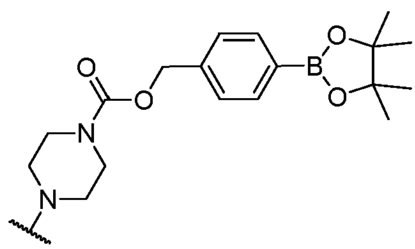 , 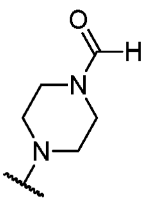 , 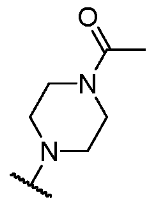 , 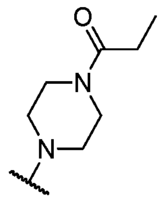 ,

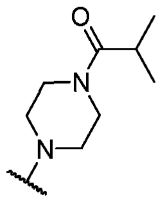 , 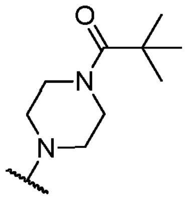 , 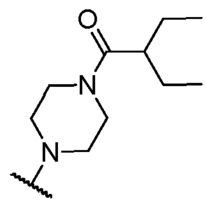 , 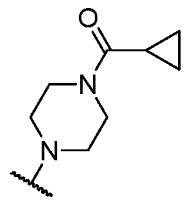 , 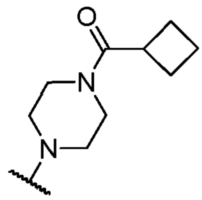 ,

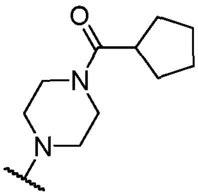 , 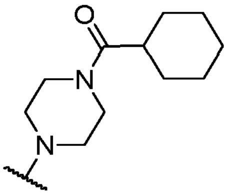 , 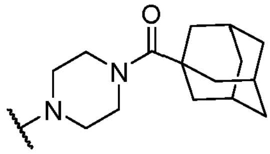 , 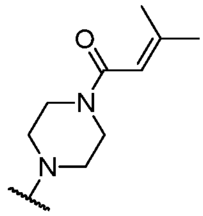 ,

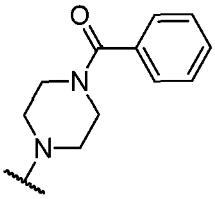 , 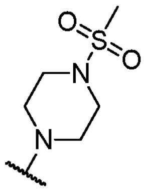 , 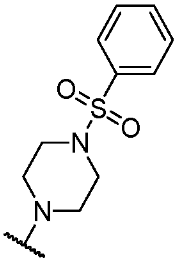 , 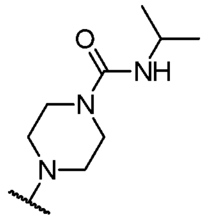 , 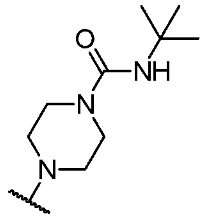 ,

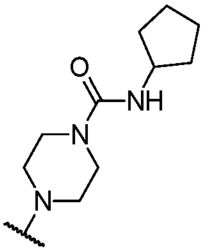 , 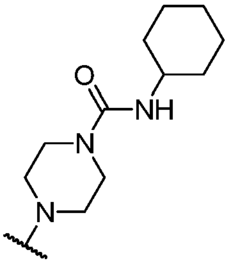 , 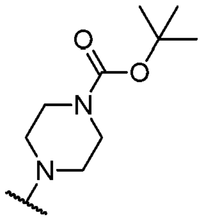 , 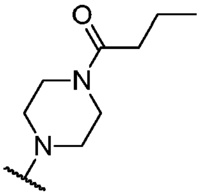 , 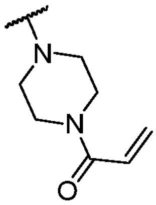 ,

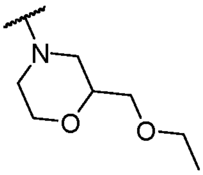 , 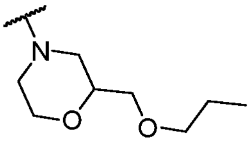 , 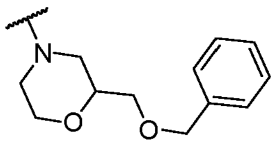 , 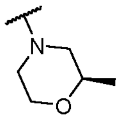 ,

ou

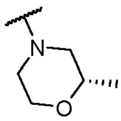 ;

ou, le sel acceptable pharmaceutiquement du composé de formule (I) est un sel préparé à partir du composé de formule (I) et d'un acide acceptable pharmaceutiquement, l'acide acceptable pharmaceutiquement est l'acide chlorhydrique ou l'acide méthanesulfonique ;

ou, le sel acceptable pharmaceutiquement du composé de formule (I) est un sel formé par le composé de formule (I) et l'acide acceptable pharmaceutiquement dans un rapport molaire de 1:2.

**6.** Composé de formule (I) selon la revendication 1, stéréoisomère de celui-ci, tautomère de celui-ci, sel acceptable pharmaceutiquement de celui-ci, ou solvate de l'un quelconque de ce qui précède, dans lequel le composé de formule (I) est l'un quelconque des composés suivants :

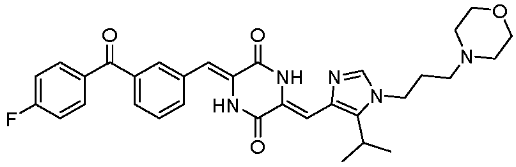,
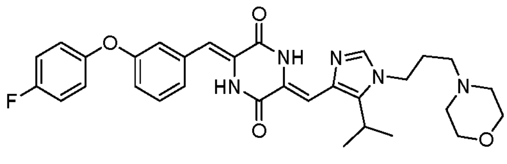,

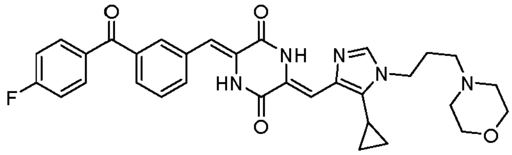,
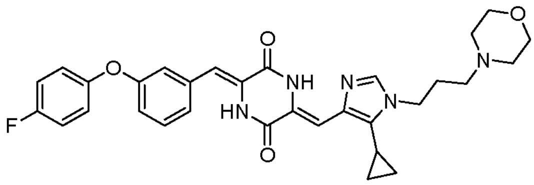,

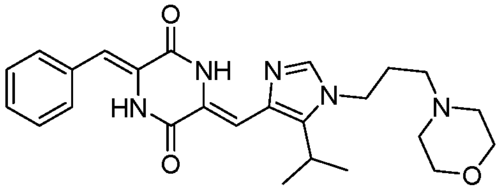,
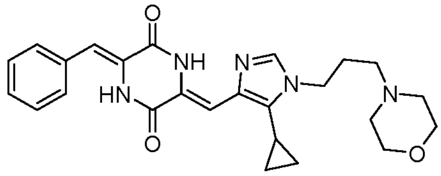,

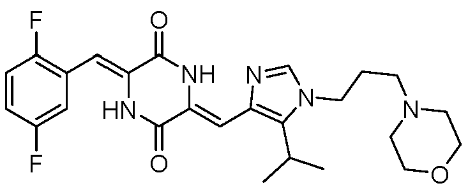,
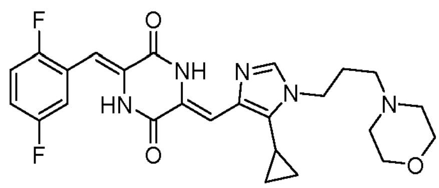,

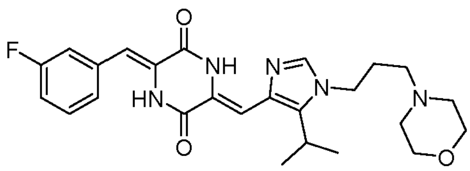,
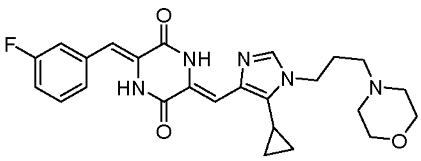,

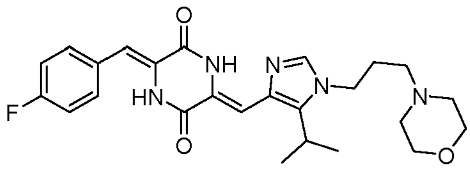,
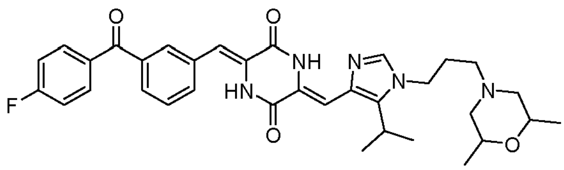,

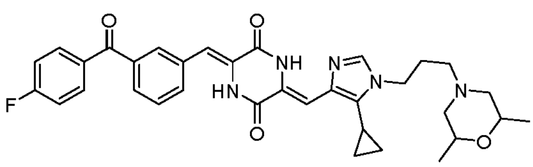,
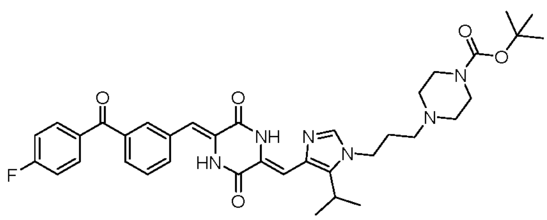,

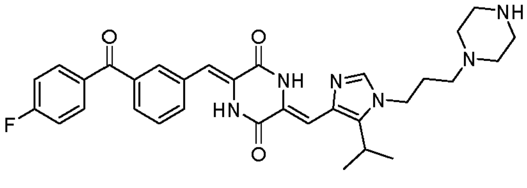,

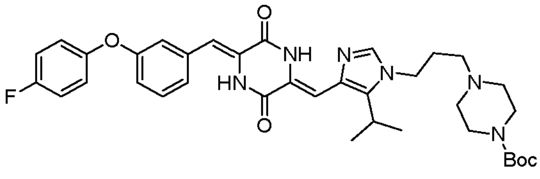,

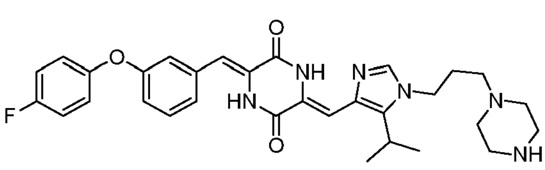,

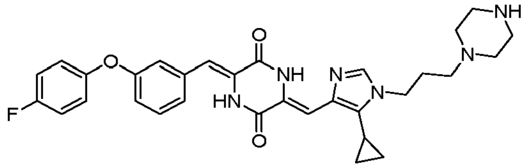,

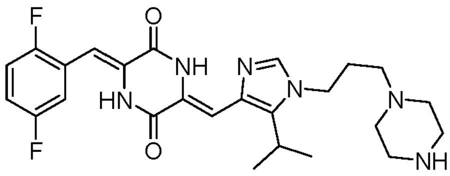,

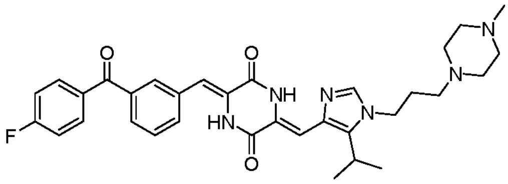,

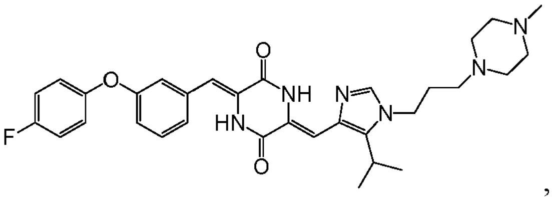,

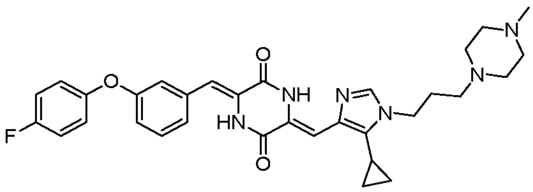,

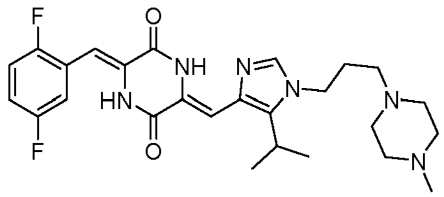,

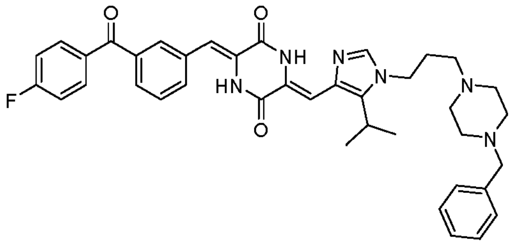,

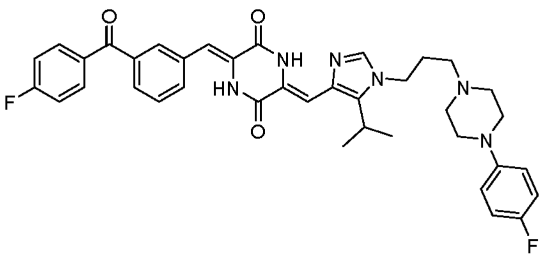,

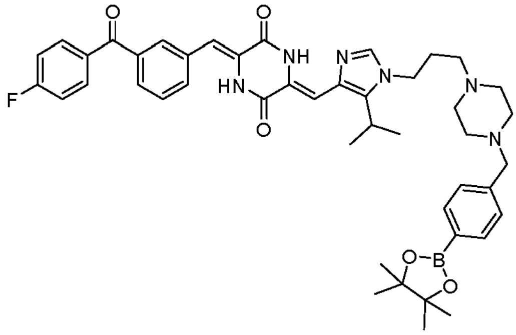,

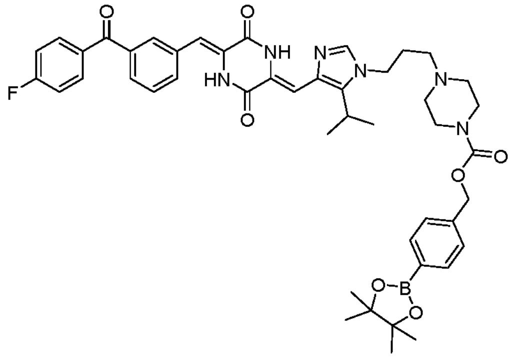,

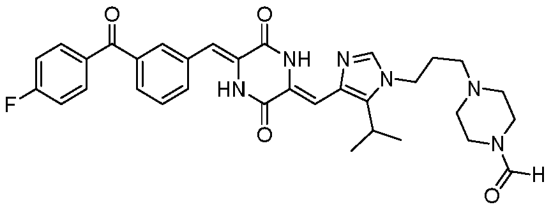,

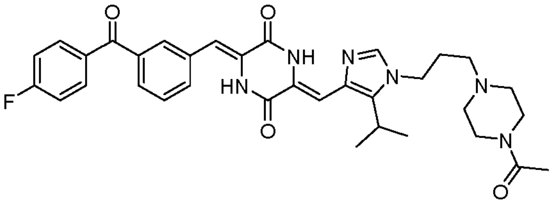,

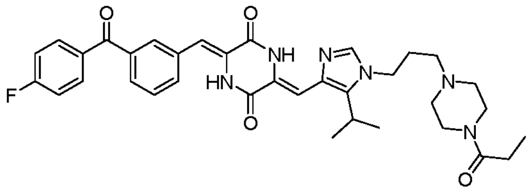,

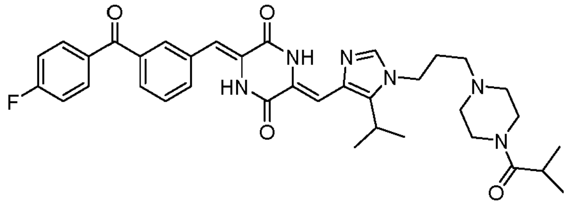,

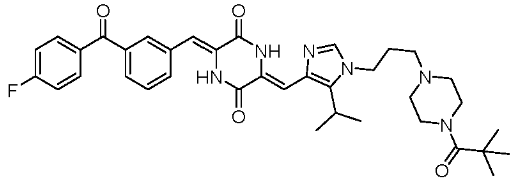,

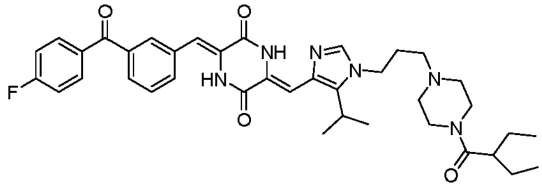,

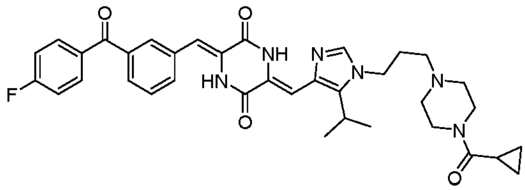,

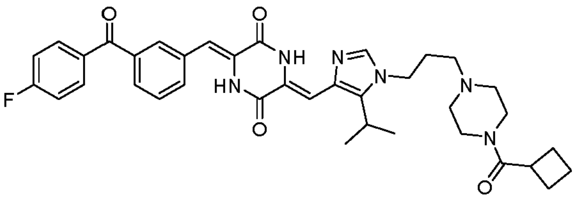,

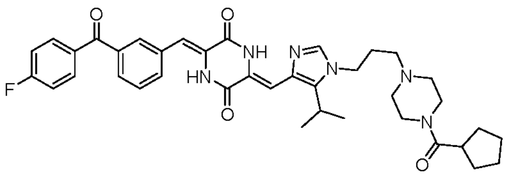,

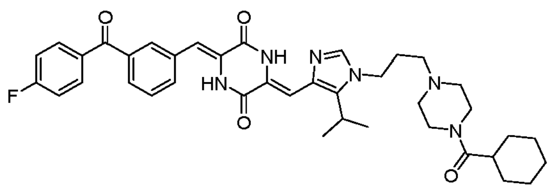,

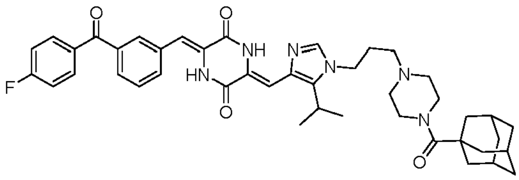,

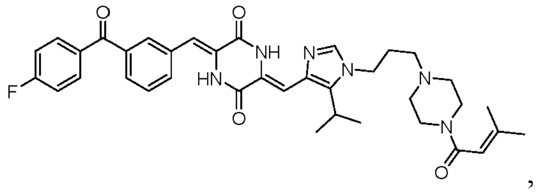,

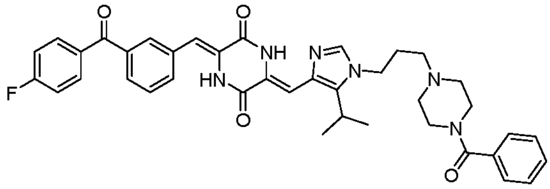,

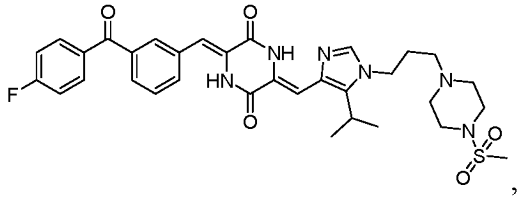,

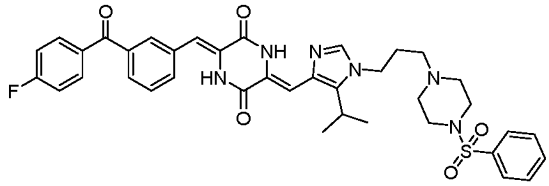,

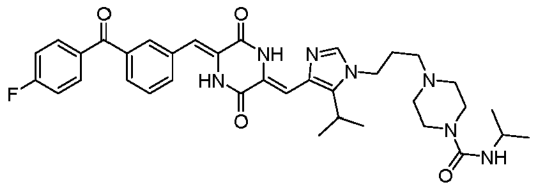,

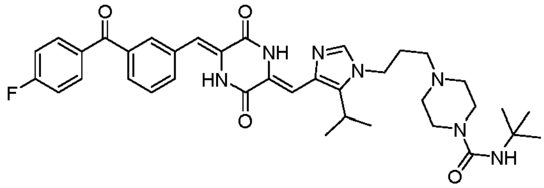,

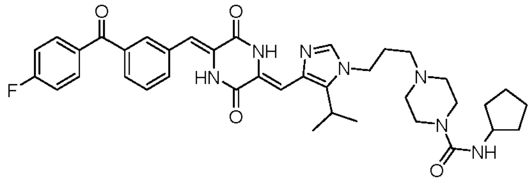,

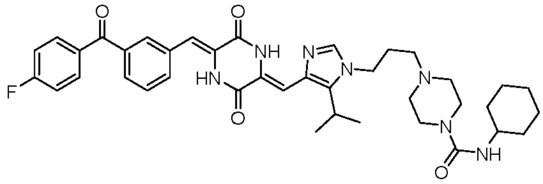,

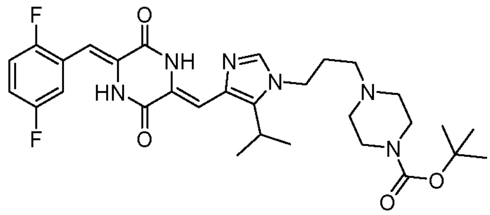,

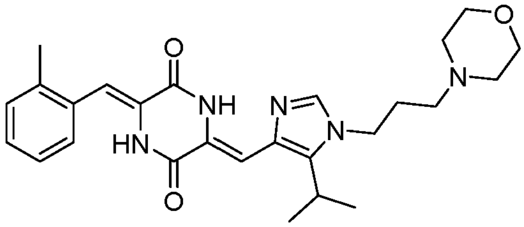,

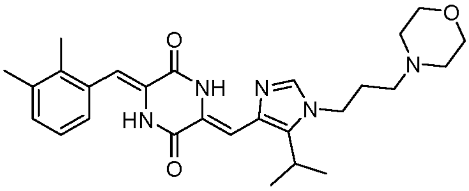,

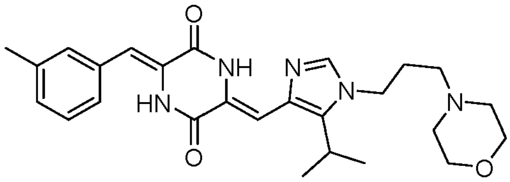,

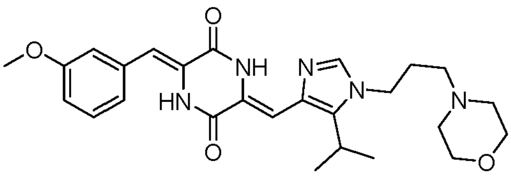,

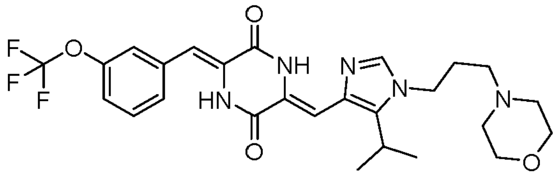,

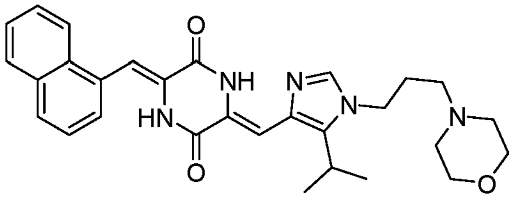,

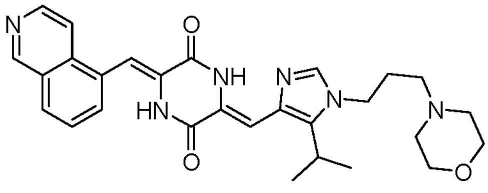,

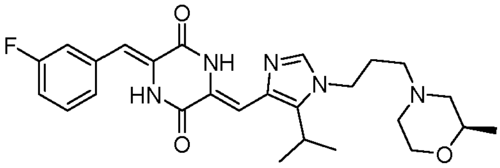,

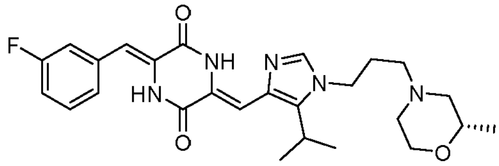,

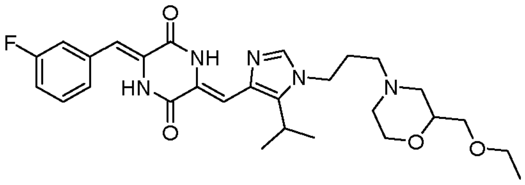,

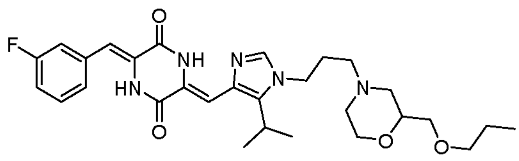,

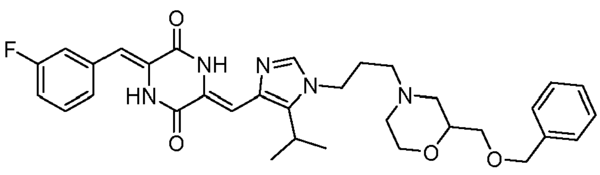,

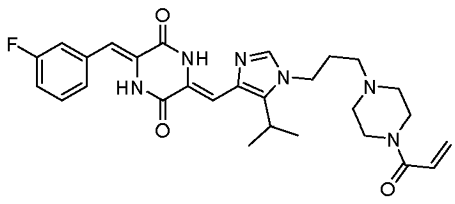,

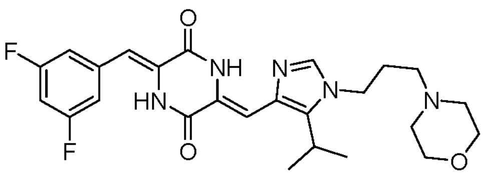,

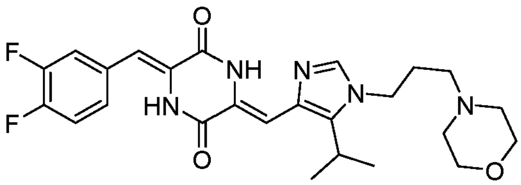,

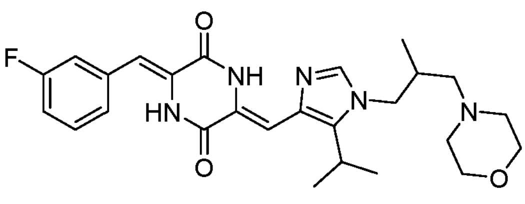,

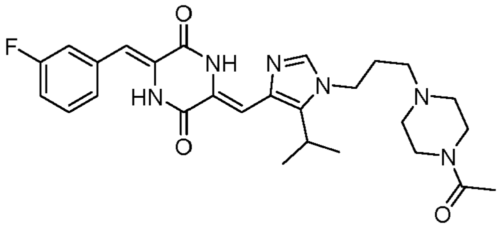,

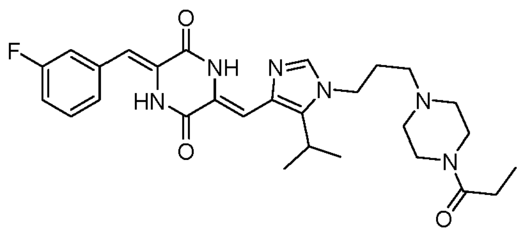,

ou

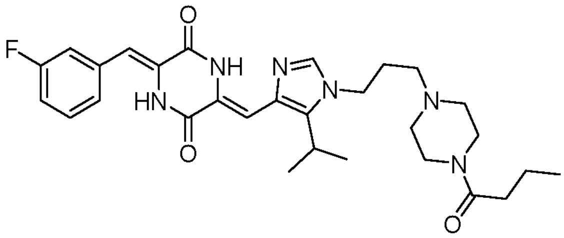

;

le sel acceptable pharmaceutiquement du composé de formule (I) est l'un quelconque des composés suivants :

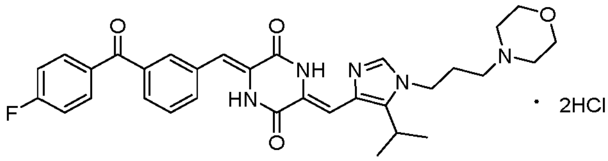

,

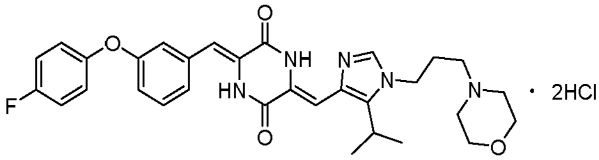

,

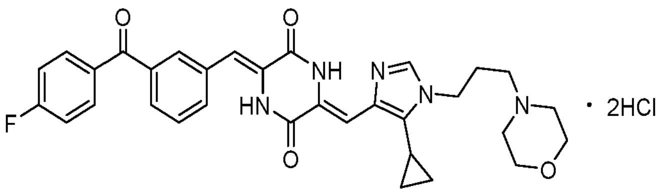

,

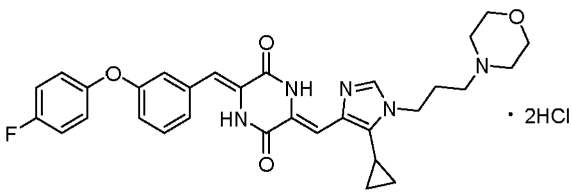

,

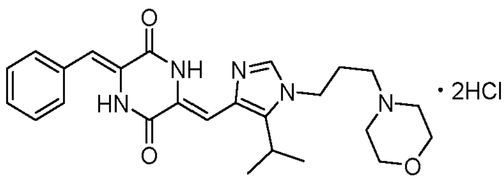

,

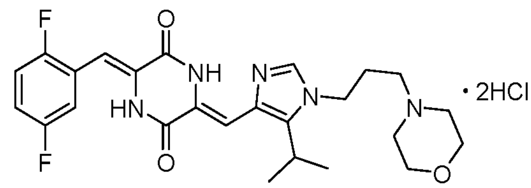

,

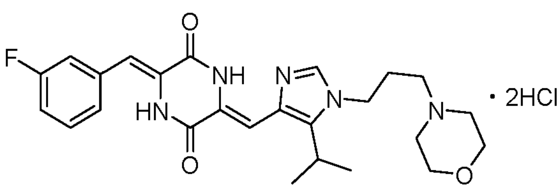

,

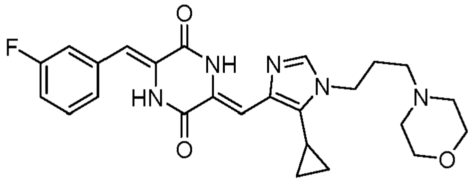

• 2HCl

,

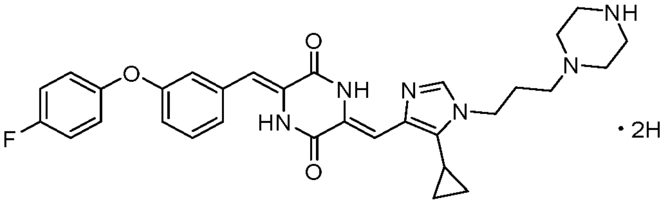

• 2HCl

,

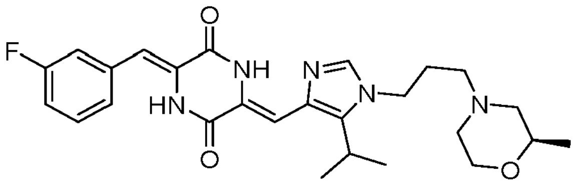

• 2HCl

,

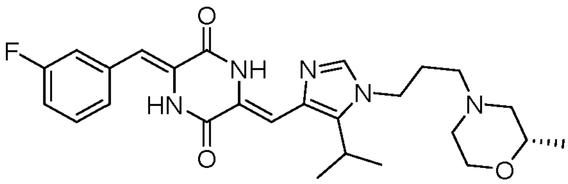

• 2HCl

,

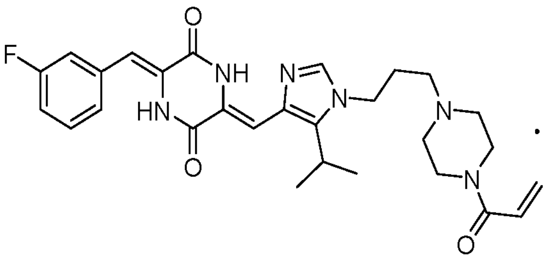

• 2HCl

,

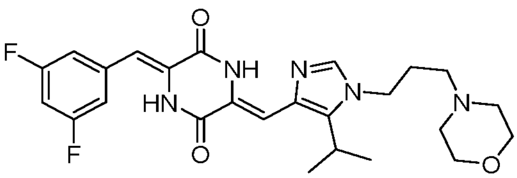

• 2HCl

,

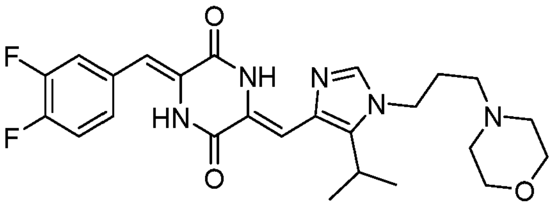

• 2HCl

,

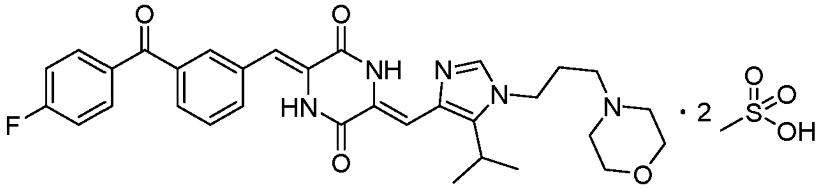

,

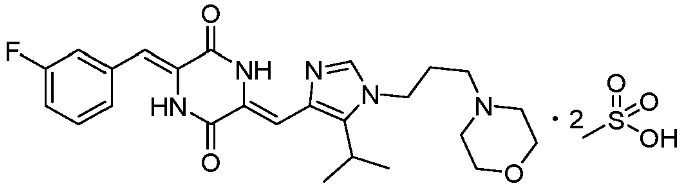

,

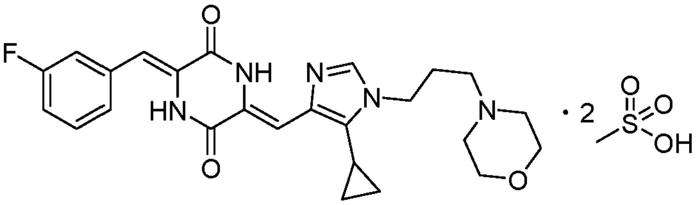

,

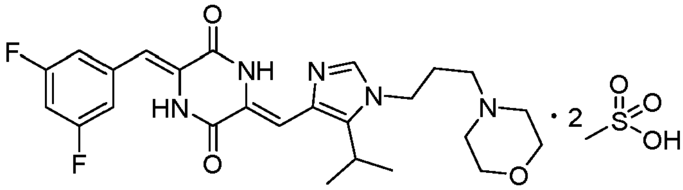

,

ou

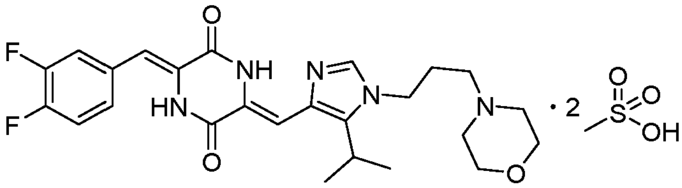

.

7. Composé de formule (I) selon la revendication 1, stéréoisomère de celui-ci, tautomère de celui-ci, sel acceptable pharmaceutiquement de celui-ci ou solvate de l'un quelconque de ce qui précède, dans lequel le composé de formule (I) est l'un quelconque des composés suivants :

un composé dont le temps de rétention est de 6,29 min dans les conditions suivantes, qui est un stéréoisomère de

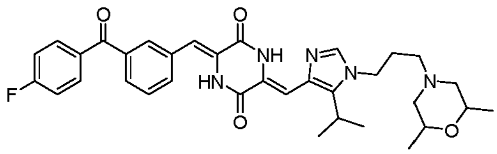

:

colonne chromatographique : Acclaim 120, C18, 5 μm, 4,6 * 150 mm, phase mobile : phase mobile A : MeOH, phase mobile B : solution aqueuse d'acide formique à 0,1 % ; débit : 1 ml/min, la phase mobile est lavée selon la procédure d'élution en gradient suivante :

| Temps (min) | Phase mobile A (%, V/V) | Phase mobile B (%, V/V) |
|---|---|---|
| 0 | 40 | 60 |
| 0→6 | 40→100 | 60→0 |
| 6→10 | 100→40 | 0→60 |

;

un composé dont le temps de rétention est de 6,48 min dans les conditions suivantes, qui est un stéréoisomère de

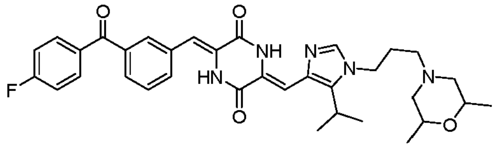

:

colonne chromatographique : Acclaim 120, C18, 5 μm, 4,6 * 150 mm, phase mobile : phase mobile A : MeOH, phase mobile B : solution aqueuse d'acide formique à 0,1 % ; débit : 1 ml/min, la phase mobile est lavée selon la procédure d'élution en gradient suivante :

| Temps (min) | Phase mobile A (%, V/V) | Phase mobile B (%, V/V) |
|---|---|---|
| 0 | 40 | 60 |
| 0→6 | 40→100 | 60→0 |
| 6→10 | 100→40 | 0→60 |

**8.** Procédé de préparation d'un composé de formule (I), qui comprend les étapes suivantes : la réalisation d'une réaction de condensation comme indiqué ci-dessous entre un composé de formule (II) et un composé de formule (III) pour obtenir le composé de formule (I) ;

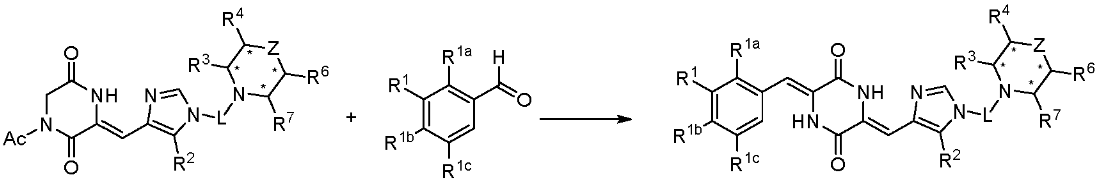

(II) (III) (I)

où *, L, Z, $R^1$, $R^{1a}$, $R^{1b}$, $R^{1c}$, $R^2$, $R^3$, $R^4$, $R^6$ et $R^7$ sont tels que définis dans au moins l'une des revendications 1 à 7.

**9.** Composé de formule (II)

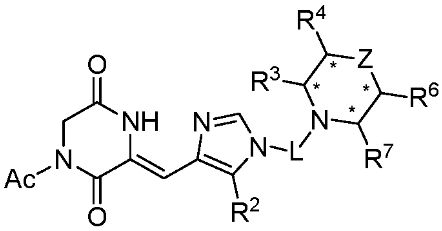

(II)

où *, $R^2$, $R^3$, $R^4$, $R^6$, $R^7$, L et Z sont tels que définis dans au moins l'une des revendications 1 à 7.

**10.** Composé de formule (II) selon la revendication 9, ledit composé de formule (II) étant l'un quelconque des composés

suivants :

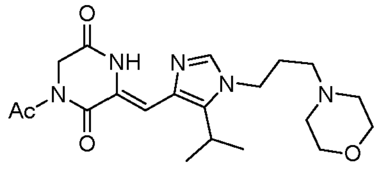 ,

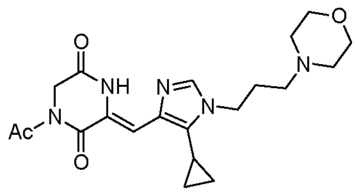 ,

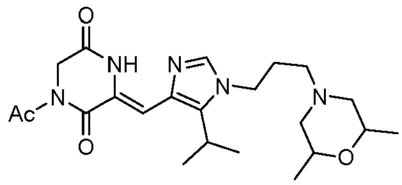 ,

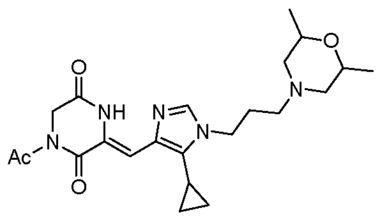 ,

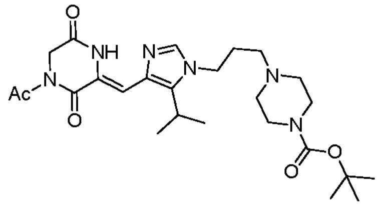 ,

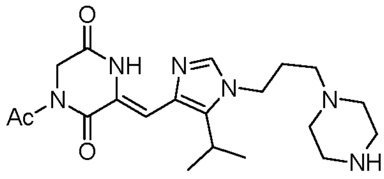 ,

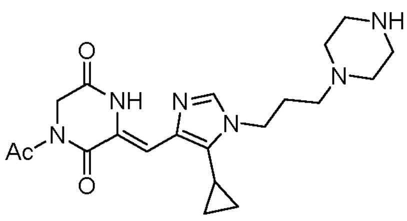 ,

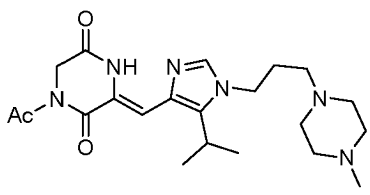 ,

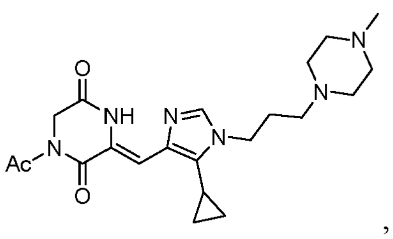 ,

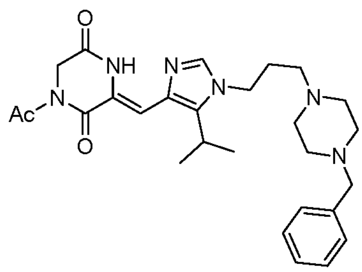 ,

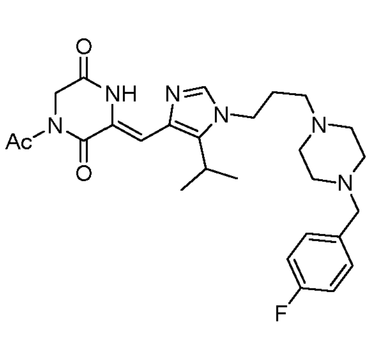 ,

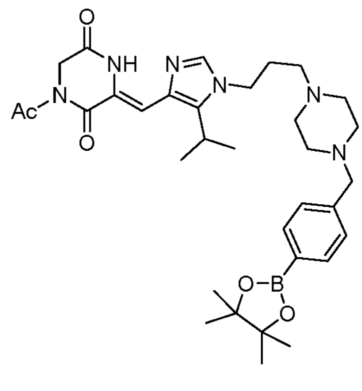 ,

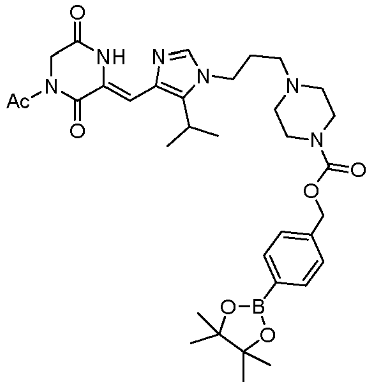
,

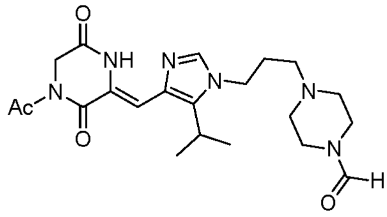
,

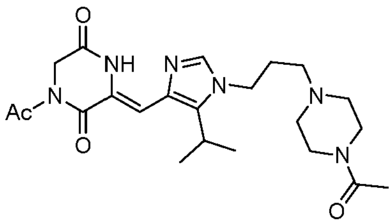
,

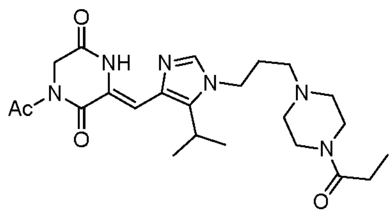
,

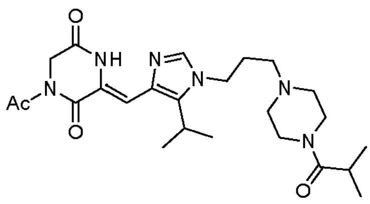
,

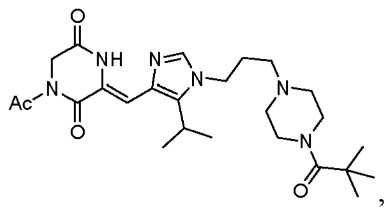
,

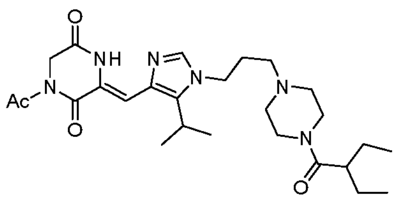
,

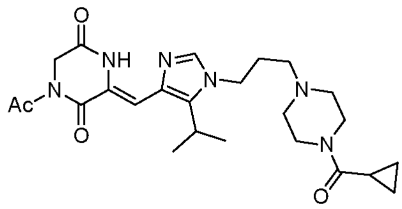
,

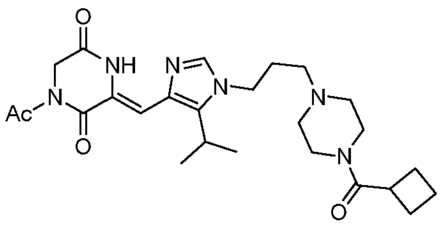
,

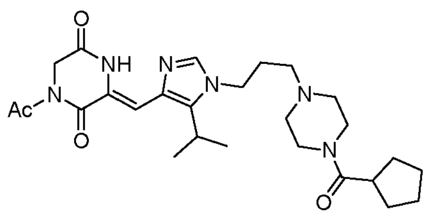
,

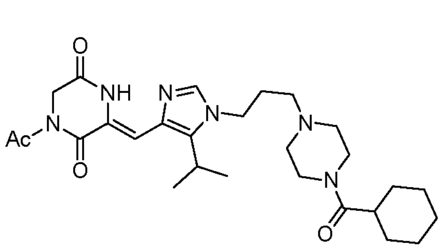
,

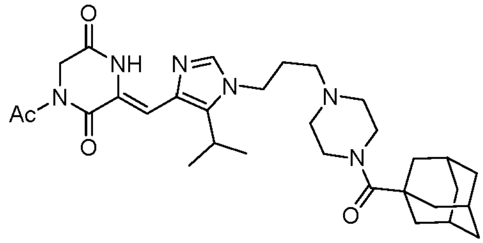
,

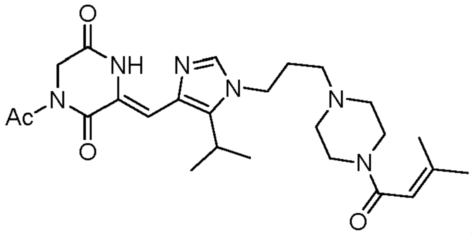
,

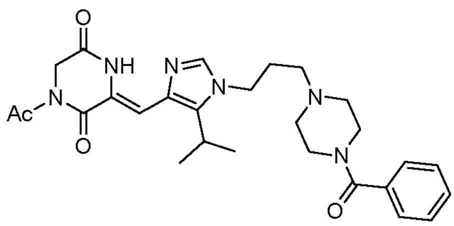
,

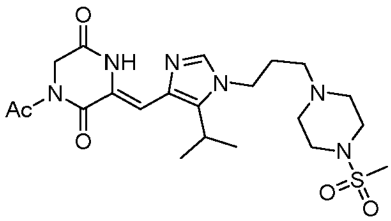
,

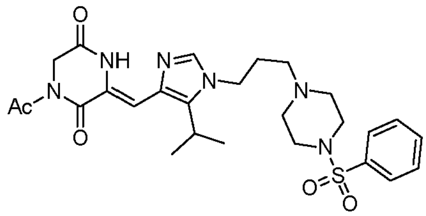
,

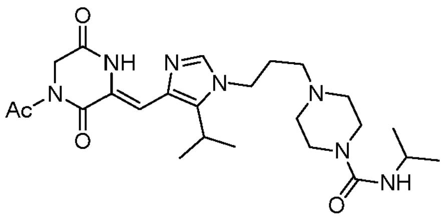
,

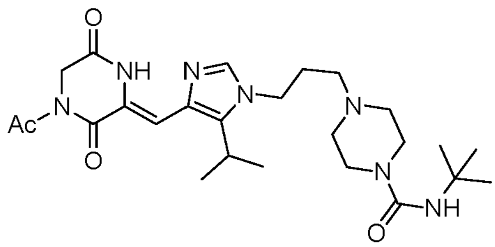
,

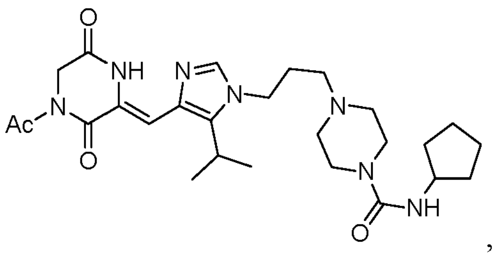
,

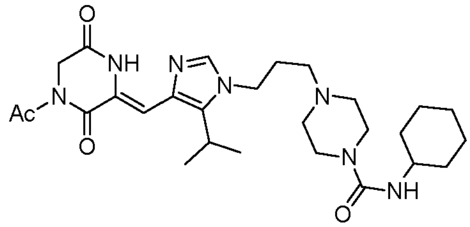
,

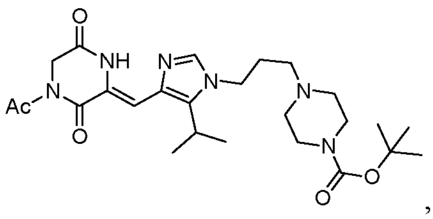
,

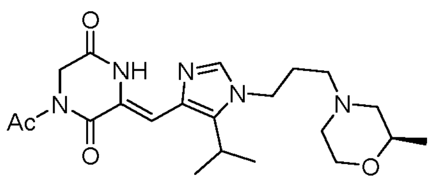
,

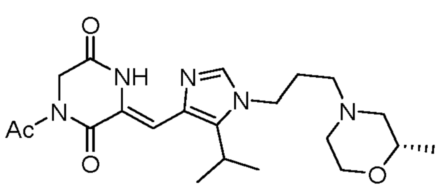
,

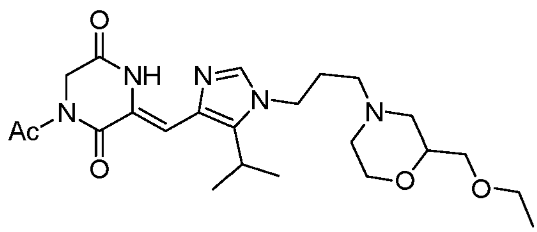
,

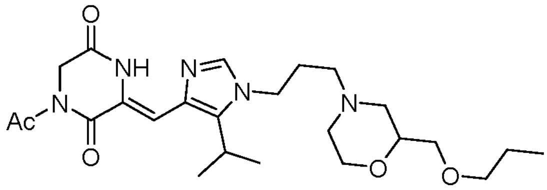

,

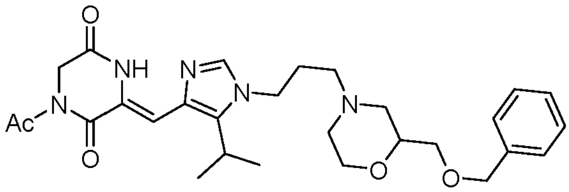

,

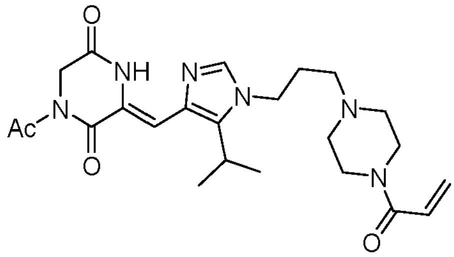

,

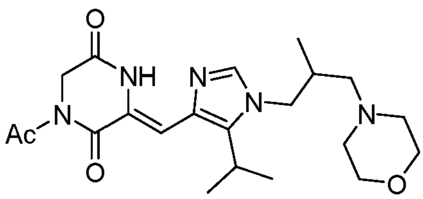

, ou

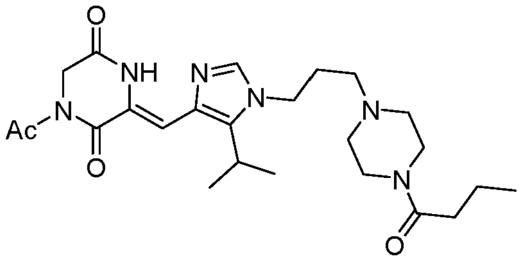

.

**11.** Composition pharmaceutique, comprenant le composé de formule (I) selon au moins l'une des revendications 1 à 7, stéréoisomère de celui-ci, tautomère de celui-ci, sel acceptable pharmaceutiquement de celui-ci ou solvate de l'un quelconque de ce qui précède, et un excipient pharmaceutique ; de préférence, l'excipient pharmaceutique ne comprend pas de co-solvant.

**12.** Composé de formule (I) selon au moins l'une des revendications 1 à 7, stéréoisomère de celui-ci, tautomère de celui-ci, sel acceptable pharmaceutiquement de celui-ci ou solvate de l'un quelconque de ce qui précède, ou composition pharmaceutique selon la revendication 11 destiné(e) à être utilisé(e) dans la prévention et/ou le traitement du cancer ; le cancer est de préférence un ou plusieurs parmi le cancer du poumon, le cancer du pancréas, le cancer du côlon et le cancer du foie.

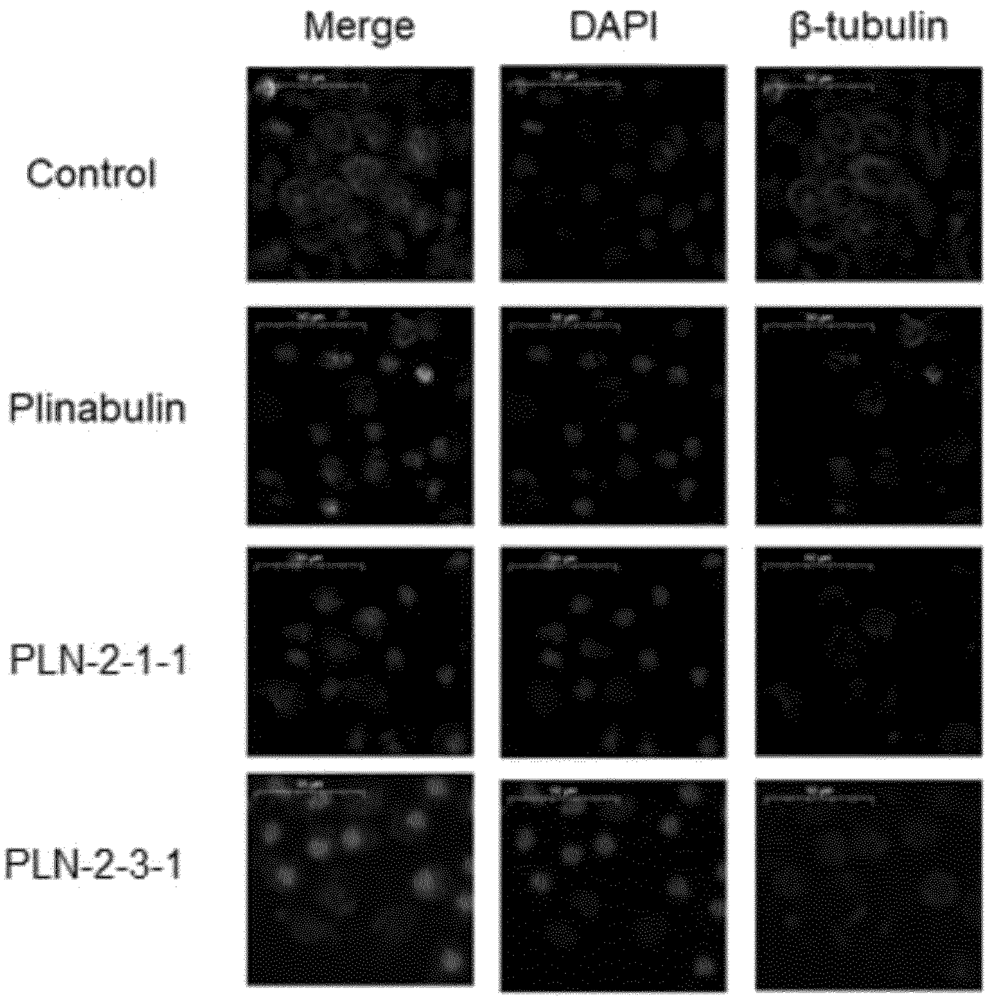


FIG. 1

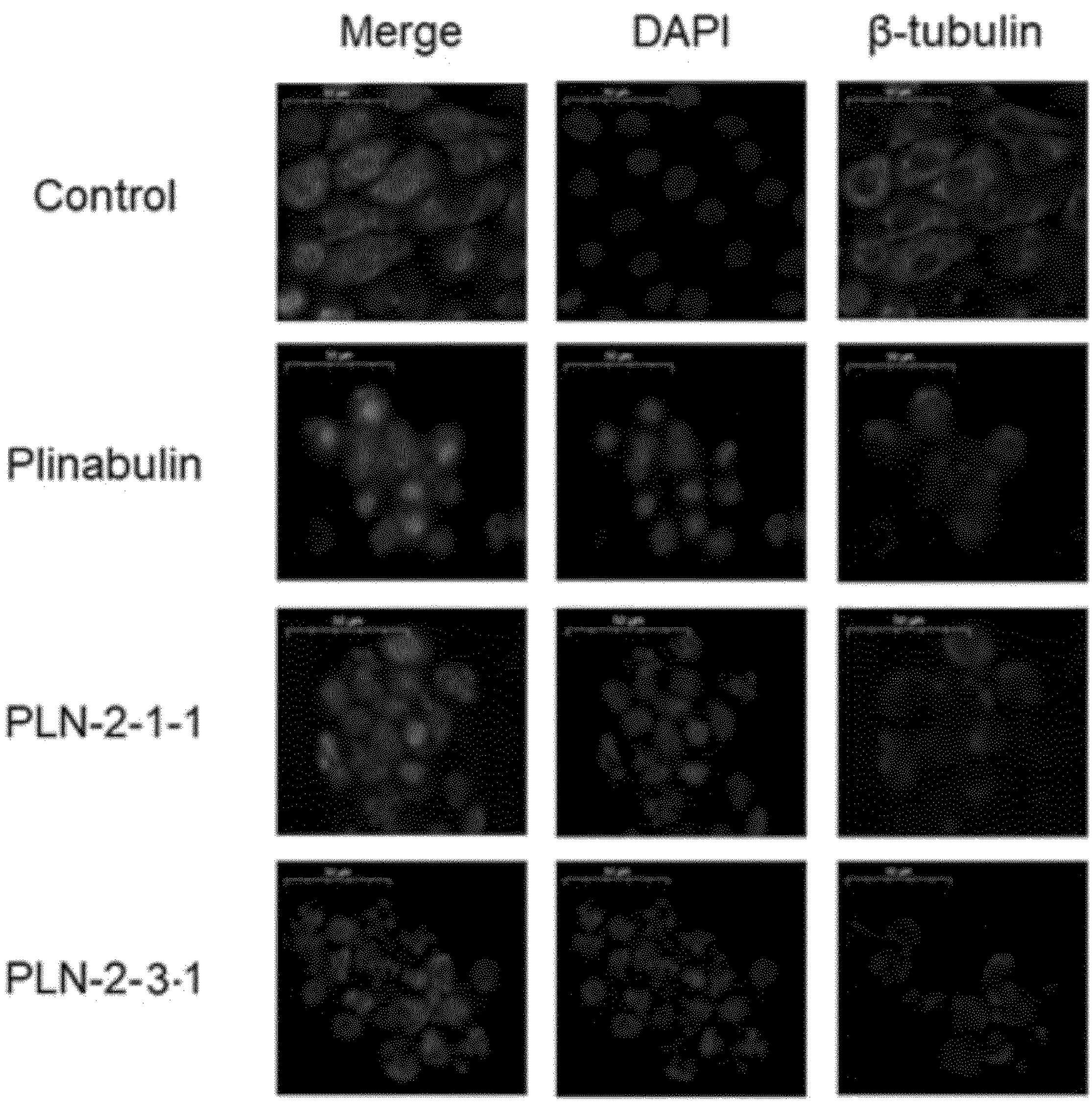


FIG. 2

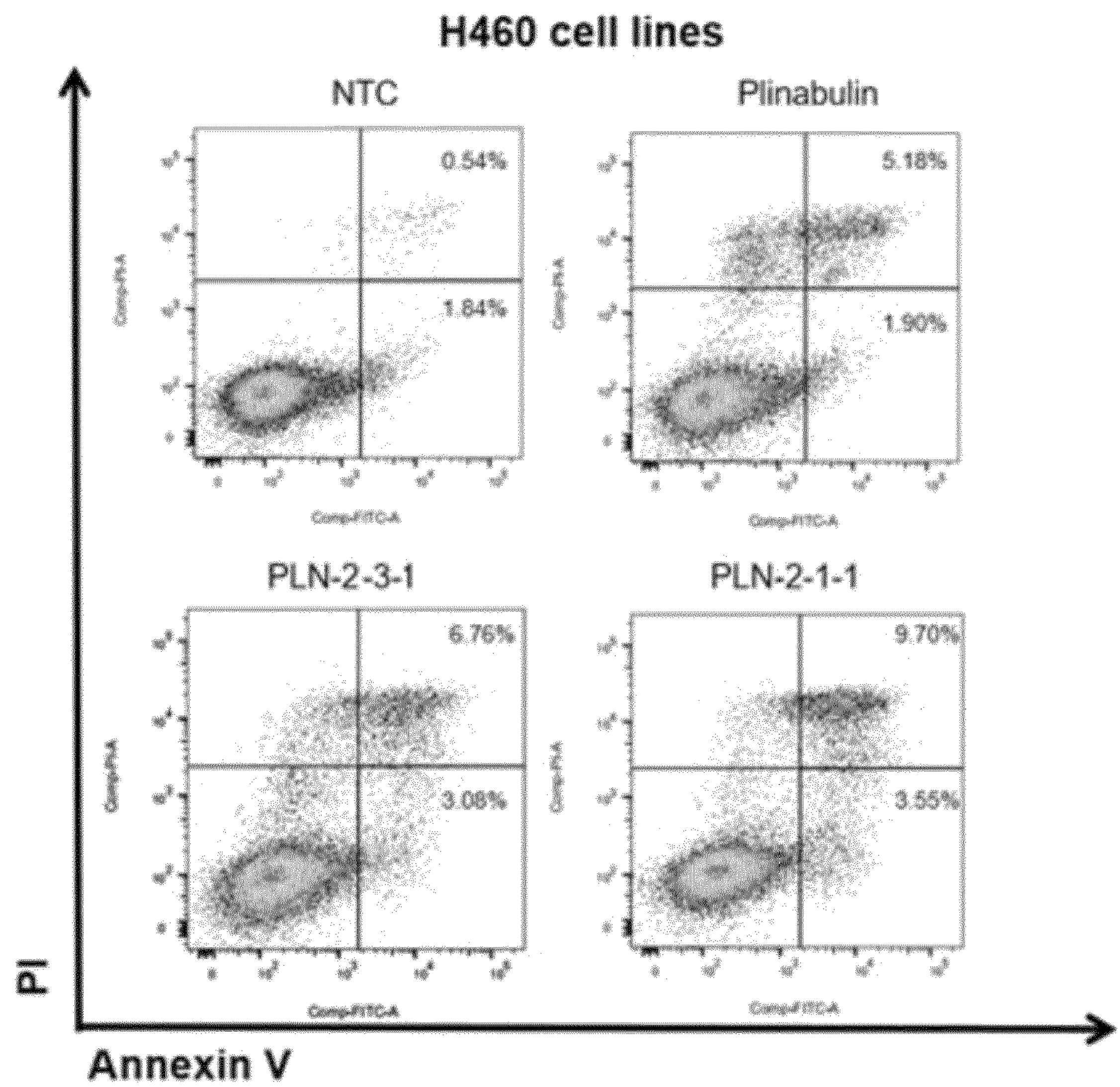
H460 cell lines
NTC
Plinabulin
PLN-2-3-1
PLN-2-1-1
0.54%
1.84%
5.18%
1.90%
6.76%
3.08%
9.70%
3.55%
PI
Annexin V

FIG. 3

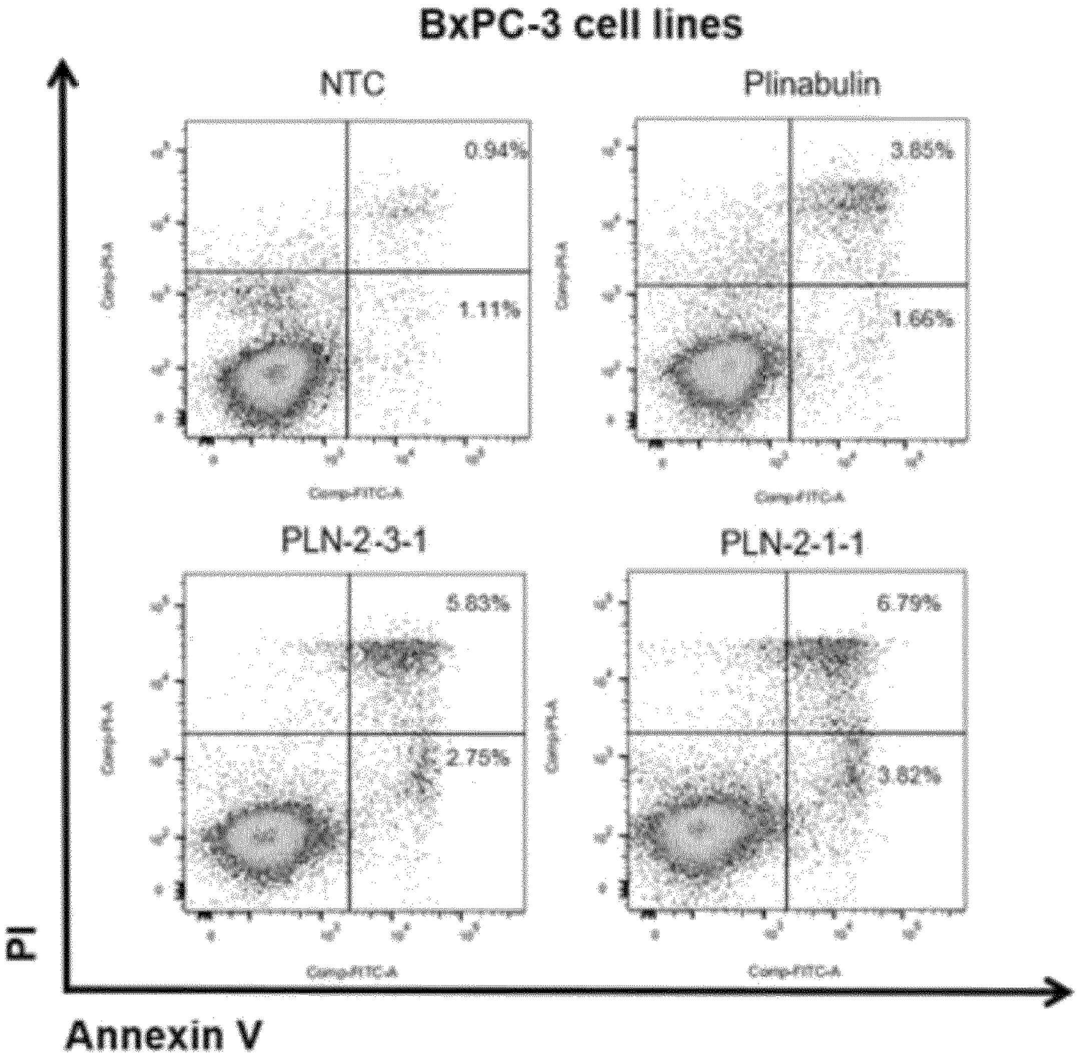
BxPC-3 cell lines
NTC
Plinabulin
PLN-2-3-1
PLN-2-1-1
0.94%
1.11%
3.85%
1.66%
5.83%
2.75%
6.79%
3.82%
Comp-PI-A
Comp-FITC-A
PI
Annexin V

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2020116248892
- WO 2012035436 A


### Non-patent literature cited in the description


- *Cell Reports*, 2019, vol. 28 (13), 3367-3386
- *CHEMICAL ABSTRACTS*, 714272-27-2
- **ASH** ; **ASH**. Handbook of Pharmaceutical Additives. Synapse Information Resources, Inc., 2002